# EUROPEAN PATENT APPLICATION

(11) **EP 4 737 065 A1**
(43) Date of publication of application: **06.05.2026**
(21) Application number: 24843091.0
(22) Date of filing: 12.07.2024
(51) Int. Cl.: B25J 5/00, G01C 21/26, G06F 3/01, G06F 3/16, G06F 16/90, G06F 40/44, G06F 40/56, G06N 3/004, G06N 3/008, G06Q 10/06, G06Q 50/10, G08B 21/02, G08B 25/00, G08B 25/04, G08B 25/10, G08G 1/005, G16H 50/30, H04M 1/72421, H04M 11/00

(54) **CONTROL SYSTEM**

(30) Priority: 14.07.2023 JP 2023116312; 14.07.2023 JP 2023116313; 10.08.2023 JP 2023131173; 14.08.2023 JP 2023131829; 15.08.2023 JP 2023132367; 21.08.2023 JP 2023133994; 22.08.2023 JP 2023134572; 31.08.2023 JP 2023140990; 31.08.2023 JP 2023141433; 11.09.2023 JP 2023147187; 22.09.2023 JP 2023159212; 27.09.2023 JP 2023166305; 27.09.2023 JP 2023166490; 28.09.2023 JP 2023168886; 27.10.2023 JP 2023184976; 21.11.2023 JP 2023197352; 01.12.2023 JP 2023203919
(71) Applicant: SoftBank Group Corp., Tokyo 105-7537 (JP)
(72) Inventor: SON, Masayoshi, Tokyo 105-7537 (JP)
(74) Representative: DREISS Patentanwälte PartG mbB
(86) International application number: PCT/JP2024/025299
(87) International publication number: WO 2025/018301

(57) **Abstract**

A control system includes a state recognition unit, an emotion determination unit, and an action determination unit, in which an electronic apparatus is detachably provided on a movement unit having a wheel for autonomous traveling, the electronic apparatus includes one or plural sensors that collect information for controlling rotation of the wheel, and device operations include setting first action content of leading a user by controlling the wheel.

## Description

### Technical Field

The present invention relates to a control system.

### Background Art

Patent Literature 1 discloses a technique for determining an appropriate action of a robot with respect to a state of a user. In the related art of Patent Literature 1, a reaction of a user in a case in which the robot executes a specific action is recognized, and in a case in which an action of the robot with respect to the recognized reaction of the user cannot be determined, the action of the robot is updated by receiving information regarding an action suitable for the recognized state of the user from a server.

Patent Literature 2 discloses a communication terminal with a camera including a neck strap.

### Prior Art Document

### Patent Literature

Patent Literature 1: Japanese Patent No. 6053847
Patent Literature 2: Japanese Patent Application Laid-Open (JP-A) No. 2019-215444

### SUMMARY OF INVENTION

### Technical Problem

However, in the related art, since an electronic apparatus supports a user in a state of being stationary at a specific position, there is room for improvement in supporting the user.

Although the related art can suppress the reflection of the neck strap on the camera, this does not support the user's action, and thus there is room for improvement in supporting the user.

Although the related art can suppress the reflection of the neck strap on the camera, this does not prevent a suspicious person from approaching a child. Therefore, there is room for improvement in preventing a suspicious person from approaching a child.

Although the related art can suppress the reflection of the neck strap on the camera, this does not support the user's action, and thus there is room for improvement in supporting the user.

### Solution to Problem

According to a first aspect of the present disclosure, a control system is provided. The control system includes a state recognition unit that recognizes a user state including an action of a user and a state of an electronic apparatus, an emotion determination unit that determines an emotion of the user or an emotion of the electronic apparatus, and an action determination unit that determines, by using at least one of the user state, the state of the electronic apparatus, the emotion of the user, or the emotion of the electronic apparatus, and an action determination model at a predetermined timing, any of a plurality of types of device operations including non-operation as an action of the electronic apparatus, in which the electronic apparatus is detachably provided on a movement unit having a wheel for autonomous traveling, the electronic apparatus includes one or a plurality of sensors that collect information for controlling rotation of the wheel, and the device operations include setting a first action content of leading the user by controlling the wheel. The electronic apparatus includes a communication terminal. Here, the communication terminal includes a device that performs a physical operation, a device that outputs a video or vocal sound without performing a physical operation, and an agent that operates on software.

According to a second aspect of the disclosure, a control system is provided. The control system includes a state recognition unit that recognizes a user state including an action of a user and a state of an electronic apparatus, an emotion determination unit that determines an emotion of the user or an emotion of the electronic apparatus, and an action determination unit that determines, by using at least one of the user state, the state of the electronic apparatus, the emotion of the user, or the emotion of the electronic apparatus, and an action determination model at a predetermined timing, any of a plurality of types of device operations including non-operation as an action of the electronic apparatus, in which the electronic apparatus detachably provided on a neck strap includes one or a plurality of sensors that collect information, and the device operations include setting first action content of leading the user by the electronic apparatus reproducing at least one of vocal sound or an image. Here, the electronic apparatus includes a communication terminal. The communication terminal includes a device that performs a physical operation, a device that outputs a video or vocal sound without performing a physical operation, and an agent that operates on software.

According to a third aspect of the disclosure, a control system is provided. The control system includes a state recognition unit that recognizes a user state including an action of a user and a state of an electronic apparatus, an emotion determination unit that determines an emotion of the user or an emotion of the electronic apparatus, and an action determination unit that determines, by using at least one of the user state, the state of the electronic apparatus, the emotion of the user, or the emotion of the electronic apparatus, and an action determination model at a predetermined timing, any of a plurality of types of device operations including non-operation as an action of the electronic apparatus, in which the electronic apparatus detachably provided on a neck strap includes one or a plurality of sensors that collect information, and the device operations include setting first action content of leading the user by the electronic apparatus reproducing at least one of vocal sound or an image.

According to a fourth aspect of the disclosure, a control system is provided. The control system includes a state recognition unit that recognizes a user state including an action of a user and a state of an electronic apparatus, an emotion determination unit that determines an emotion of the user, an emotion of another user existing around the user, or an emotion of the electronic apparatus, an action determination unit that, in a case in which an emotion value indicating an intensity of the emotion of the another user changes by a predetermined value or more, determines to start capturing a moving image of the another user with a camera as an action of the electronic apparatus, and an action control unit that controls the electronic apparatus to execute the action of the electronic apparatus determined by the action determination unit.

According to a fifth aspect of the disclosure, a control system is provided. The control system includes a state recognition unit that recognizes a user state including actions of a user and another user, an emotion determination unit that determines emotions of the user and another user, and an action determination unit that determines any of a plurality of types of device operations as an operation of an electronic apparatus by using at least one of the user state or the emotion of the user and an action determination model at a predetermined timing, in which the state recognition unit recognizes a surrounding state from one or a plurality of sensors of the electronic apparatus detachably provided on a body of the user, and the action determination unit determines that the electronic apparatus makes speech toward the another user in a case in which the another user exists near the user.

According to a sixth aspect of the disclosure, a control system is provided. The control system includes a state recognition unit that recognizes a user state including an action of a user and a state of an electronic apparatus, an emotion determination unit that determines an emotion of the user or an emotion of the electronic apparatus, and an action determination unit that determines, by using at least one of the user state, the state of the electronic apparatus, the emotion of the user, or the emotion of the electronic apparatus, and an action determination model at a predetermined timing, any of a plurality of types of device operations including non-operation as an action of the electronic apparatus, in which the emotion determination unit estimates an emotion of another person around the user from a video of the another person acquired from one or a plurality of sensors that collect information, the sensors being included in the electronic apparatus detachably provided on a body of the user, and the action determination unit makes an appropriate proposal to the user in a case in which the another person feels troubled.

According to a seventh aspect of the disclosure, a control system is provided. The control system includes a state recognition unit that recognizes a user state including an action of a user and a state of an electronic apparatus, an emotion determination unit that determines an emotion of the user or an emotion of the electronic apparatus, and an action determination unit that determines, by using at least one of the user state, the state of the electronic apparatus, the emotion of the user, or the emotion of the electronic apparatus, and an action determination model at a predetermined timing, any of a plurality of types of device operations including non-operation as an action of the electronic apparatus, in which the electronic apparatus detachably provided on a neck strap includes one or a plurality of sensors that collect information, the sensors including an imaging unit that captures an image of surroundings, and the device operations include issuing a warning to a person satisfying a predetermined condition among persons approaching the imaging unit.

According to an eighth aspect of the disclosure, a control system is provided. The control system includes a state recognition unit that recognizes a user state including an action of a user and a state of an electronic apparatus, an emotion determination unit that determines an emotion of the user or an emotion of the electronic apparatus, and an action determination unit that determines, by using at least one of the user state, the state of the electronic apparatus, the emotion of the user, or the emotion of the electronic apparatus, and an action determination model at a predetermined timing, any of a plurality of types of device operations including non-operation as an action of the electronic apparatus, in which the electronic apparatus detachably provided on a neck strap includes one or a plurality of sensors that collect information, and the device operations include setting action content of supporting work performed by the user at a position lower than eyes of the user by the electronic apparatus reproducing at least one of vocal sound or an image generated by the action determination model on the basis of the information collected by the electronic apparatus.

According to a ninth aspect of the disclosure, a control system is provided. The control system includes a state recognition unit that recognizes a user state including an action of a user and a state of an electronic apparatus, an emotion determination unit that determines an emotion of the user or an emotion of the electronic apparatus, and an action determination unit that determines, by using at least one of the user state, the state of the electronic apparatus, the emotion of the user, or the emotion of the electronic apparatus, and an action determination model at a predetermined timing, any of a plurality of types of device operations including non-operation as an action of the electronic apparatus, in which the electronic apparatus is detachably provided on a movement unit having a wheel for autonomous traveling, the electronic apparatus includes one or a plurality of sensors that collect information regarding a surrounding situation of the user, the electronic apparatus controls rotation of the wheel on the basis of the information regarding the surrounding situation of the user, and the action determination unit determines, in a case in which it is detected that there is a dangerous obstacle around the user, first action content of leading the user to a safe place so that the user does not approach the dangerous obstacle as the action of the electronic apparatus.

According to a tenth aspect of the disclosure, a control system is provided. The control system includes a state recognition unit that recognizes a user state including an action of a user and a state of an electronic apparatus, an emotion determination unit that determines an emotion of the user or an emotion of the electronic apparatus, and an action determination unit that determines, by using at least one of the user state, the state of the electronic apparatus, the emotion of the user, or the emotion of the electronic apparatus, and an action determination model at a predetermined timing, any of a plurality of types of device operations including non-operation as an action of the electronic apparatus, in which the electronic apparatus is provided in a movement unit capable of autonomous traveling, the electronic apparatus includes one or a plurality of sensors that collect information regarding a surrounding situation of the user, and the action determination unit determines, in a case in which it is detected that the user is in imminent danger on the basis of the information regarding the surrounding situation of the user, to take an action of being a substitute for the user as the action of the electronic apparatus.

According to an eleventh aspect of the disclosure, a control system is provided. The control system includes a state recognition unit that recognizes a user state including an action of a user and a state of an electronic apparatus, an emotion determination unit that determines an emotion of the user or an emotion of the electronic apparatus, and an action determination unit that determines, by using at least one of the user state, the state of the electronic apparatus, the emotion of the user, or the emotion of the electronic apparatus, and an action determination model at a predetermined timing, any of a plurality of types of device operations including non-operation as an action of the electronic apparatus, in which the electronic apparatus is detachably provided on a movement unit having a wheel for autonomous traveling, the electronic apparatus includes one or a plurality of sensors that collect information regarding a surrounding situation of the user, and the device operations include setting first action content of assisting the user in walking an animal by controlling the wheel on the basis of the information regarding the surrounding situation of the user.

According to a twelfth aspect of the disclosure, a control system is provided. The control system includes a state recognition unit that recognizes a user state including an action of a user and a state of an electronic apparatus, an emotion determination unit that determines an emotion of the user or an emotion of the electronic apparatus, and an action determination unit that determines, by using at least one of the user state, the state of the electronic apparatus, the emotion of the user, or the emotion of the electronic apparatus, and an action determination model at a predetermined timing, any of a plurality of types of device operations including non-operation as an action of the electronic apparatus, in which the electronic apparatus detachably provided on a neck strap includes one or a plurality of sensors that collect information regarding a surrounding situation, and the device operations include setting action content of assisting in walking of the user by notifying the user of the surrounding situation by the electronic apparatus reproducing at least one of vocal sound or an image.

According to a thirteenth aspect of the disclosure, a control system is provided. The control system includes a state recognition unit that recognizes a user state including an action of a user and a state of an electronic apparatus, an emotion determination unit that determines an emotion of the user or an emotion of the electronic apparatus, and an action determination unit that determines, by using at least one of the user state, the state of the electronic apparatus, the emotion of the user, or the emotion of the electronic apparatus, and an action determination model at a predetermined timing, any of a plurality of types of device operations including non-operation as an action of the electronic apparatus, in which the electronic apparatus detachably provided on a neck strap includes one or a plurality of sensors that collect biological information of the user, and the device operations include notifying the user of attention calling information based on the collected biological information by the electronic apparatus reproducing at least one of vocal sound or an image.

According to a fourteenth aspect of the disclosure, a control system is provided. The control system includes a state recognition unit that recognizes a user state including an action of a user and a state of an electronic apparatus, an emotion determination unit that determines an emotion of the user or an emotion of the electronic apparatus, and an action determination unit that determines, by using at least one of the user state, the state of the electronic apparatus, the emotion of the user, or the emotion of the electronic apparatus, and an action determination model at a predetermined timing, any of a plurality of types of device operations including non-operation as an action of the electronic apparatus, in which the electronic apparatus detachably provided on a neck strap includes one or a plurality of sensors that collect surrounding environment information, and the device operations include notifying the user of attention calling information based on the collected surrounding environment information by the electronic apparatus reproducing at least one of vocal sound or an image.

According to a fifteenth aspect of the disclosure, a control system is provided. The control system includes a state recognition unit that recognizes a user state including an action of a user and a state of an electronic apparatus, an emotion determination unit that determines an emotion of the user or an emotion of the electronic apparatus, and an action determination unit that determines, by using at least one of the user state, the state of the electronic apparatus, the emotion of the user, or the emotion of the electronic apparatus, and an action determination model at a predetermined timing, any of a plurality of types of device operations including non-operation as an action of the electronic apparatus, in which the electronic apparatus detachably provided on a neck strap includes one or a plurality of sensors that collect information, and the device operations include setting action content of supporting work performed by the user at a position lower than eyes of the user by the electronic apparatus reproducing at least one of vocal sound or an image generated by the action determination model on the basis of the information collected by the electronic apparatus.

According to a sixteenth aspect of the disclosure, a control system is provided. The control system includes a state recognition unit that recognizes a user state including an action of a user and a state of an electronic apparatus, an emotion determination unit that determines an emotion of the user or an emotion of the electronic apparatus, and an action determination unit that determines, by using at least one of the user state, the state of the electronic apparatus, the emotion of the user, or the emotion of the electronic apparatus, and an action determination model at a predetermined timing, any of a plurality of types of device operations including non-operation as an action of the electronic apparatus, in which the electronic apparatus detachably provided on a neck strap includes one or a plurality of sensors that collect information, the electronic apparatus is linked to another electronic apparatus that is worn by the user and includes one or a plurality of sensors that collect information, and the device operations include setting action content of supporting the user by the electronic apparatus reproducing at least one of vocal sound or an image generated by the action determination model on the basis of the information collected by the electronic apparatus and the information collected by the another electronic apparatus.

### BRIEF DESCRIPTION OF DRAWINGS

Fig. 1 schematically illustrates an example of a system 5 according to a first embodiment.
Fig. 2 schematically illustrates a functional configuration of a robot 100 according to the first embodiment.
Fig. 3 schematically illustrates an example of an operation flow of collection processing in the robot 100 according to the first embodiment.
Fig. 4A schematically illustrates an example of an operation flow of response processing in the robot 100 according to the first embodiment.
Fig. 4B schematically illustrates an example of an operation flow of autonomous processing of the robot 100 according to the first embodiment.
Fig. 5 illustrates an emotion map 400 on which a plurality of emotions are mapped.
Fig. 6 illustrates an emotion map 900 on which a plurality of emotions are mapped.
Fig. 7A is an external view of a stuffed toy 100N according to a second embodiment, and Fig. 7B is an internal structural view of the stuffed toy 100N.
Fig. 8 is a rear front view of the stuffed toy 100N according to the second embodiment.
Fig. 9 schematically illustrates a functional configuration of the stuffed toy 100N according to the second embodiment.
Fig. 10 schematically illustrates a functional configuration of an agent system 500 according to a third embodiment.
Fig. 11 illustrates an example of an operation of the agent system.
Fig. 12 illustrates an example of an operation of the agent system.
Fig. 13 schematically illustrates a functional configuration of smart glasses 700 according to a fourth embodiment.
Fig. 14 illustrates an example of a usage mode of an agent system using smart glasses.
Fig. 15 schematically illustrates an example of a hardware configuration of a computer 1200.
Fig. 16 is an external view of a movement system including a movement unit and a communication terminal.
Fig. 17 is an external view of the movement system including the movement unit and the communication terminal.
Fig. 18 is a diagram illustrating an example of an image reproduced by the movement system including the movement unit and the communication terminal.
Fig. 19 is a diagram illustrating an example of an image reproduced by the movement system including the movement unit and the communication terminal.
Fig. 20 is a diagram illustrating an example of an image reproduced by the movement system including the movement unit and the communication terminal.
Fig. 21 is a diagram illustrating an example of an image reproduced by the movement system including the movement unit and the communication terminal.
Fig. 22 is a diagram illustrating an example of an image reproduced by the movement system including the movement unit and the communication terminal.
Fig. 23 is a diagram illustrating an example of an image reproduced by the movement system including the movement unit and the terminal.
Fig. 24 is an external view of a communication terminal according to a fifth embodiment.
Fig. 25 is a diagram illustrating an example of vocal sound reproduced by the communication terminal according to the fifth embodiment.
Fig. 26 is a diagram illustrating an example of vocal sound reproduced by the communication terminal according to the fifth embodiment.
Fig. 27 is a diagram illustrating an example of vocal sound reproduced by the communication terminal according to the fifth embodiment.
Fig. 28 is a diagram illustrating an example of vocal sound reproduced by the communication terminal according to the fifth embodiment.
Fig. 29 is a diagram illustrating an example of vocal sound reproduced by the communication terminal according to the fifth embodiment.
Fig. 30 is a diagram illustrating an example of a flow of proposal processing of other embodiment 3.
Fig. 31 is a diagram illustrating an example of a flow of conversation output processing according to other embodiment 4.
Fig. 32 is a diagram illustrating an example of a flow of topic output processing of other embodiment 4.
Fig. 33 is a diagram illustrating an example of a flow of proposal processing of other embodiment 5.
Fig. 34 is a block unit illustrating a functional configuration of an action determination unit 236 in a communication terminal 100M of other embodiment 6.
Fig. 35 is a flowchart illustrating an example of a flow of processing performed by the communication terminal 100M of other embodiment 6.
Fig. 36 is a flowchart illustrating an example of warning processing according to other embodiment 6.
Fig. 37 is a diagram illustrating an example of vocal sound reproduced by a communication terminal according to other embodiment 7.
Fig. 38 is a diagram illustrating an example of vocal sound reproduced by the communication terminal according to other embodiment 7.
Fig. 39 is a diagram illustrating an example of vocal sound reproduced by the communication terminal according to other embodiment 7.
Fig. 40 is a diagram illustrating an example of vocal sound reproduced by the communication terminal according to other embodiment 7.
Fig. 41 is a diagram illustrating an example of vocal sound reproduced by the communication terminal according to other embodiment 7.
Fig. 42 is a diagram illustrating an example of vocal sound reproduced by the communication terminal according to other embodiment 7.
Fig. 43 is a diagram illustrating an example of vocal sound reproduced by the communication terminal according to other embodiment 7.
Fig. 44 is a diagram illustrating an example of vocal sound reproduced by the communication terminal according to other embodiment 7.
Fig. 45 is a diagram illustrating an example of vocal sound reproduced by the communication terminal according to other embodiment 7.
Fig. 46 is a diagram illustrating an example of vocal sound reproduced by the communication terminal according to other embodiment 7.
Fig. 47 is a diagram illustrating an example of vocal sound reproduced by the communication terminal according to other embodiment 7.
Fig. 48 is a diagram illustrating an example of vocal sound reproduced by the communication terminal according to other embodiment 7.
Fig. 49 is a diagram illustrating an example of vocal sound reproduced by the communication terminal according to other embodiment 7.
Fig. 50 is a diagram illustrating a situation in which an object is approaching a user in a seventh embodiment.
Fig. 51 is a diagram illustrating a situation in which a movement system according to the seventh embodiment collides with the object as a substitute for the user.
Fig. 52 is a diagram illustrating an example of an image reproduced by a movement system including a movement unit and a communication terminal in an eighth embodiment.
Fig. 53 is a diagram illustrating an example of an image reproduced by the movement system including the movement unit and the communication terminal in the eighth embodiment.
Fig. 54 is a diagram illustrating an example of vocal sound reproduced by a communication terminal according to a ninth embodiment.
Fig. 55 is a diagram illustrating an example of vocal sound reproduced by the communication terminal according to the ninth embodiment.
Fig. 56 is a diagram illustrating an example of vocal sound reproduced by the communication terminal according to the ninth embodiment.
Fig. 57 is a diagram illustrating an example of vocal sound reproduced by the communication terminal according to the ninth embodiment.
Fig. 58 is a diagram illustrating an example of vocal sound reproduced by the communication terminal according to the ninth embodiment.
Fig. 59 is a diagram illustrating an example of a smart watch 800 worn on an arm of a user according to a tenth embodiment.
Fig. 60 is a diagram illustrating an example of vocal sound reproduced by a communication terminal according to the tenth embodiment.
Fig. 61 is a diagram illustrating an example of vocal sound reproduced by the communication terminal according to the tenth embodiment.
Fig. 62 is a diagram illustrating an example of vocal sound reproduced by the communication terminal according to the tenth embodiment.

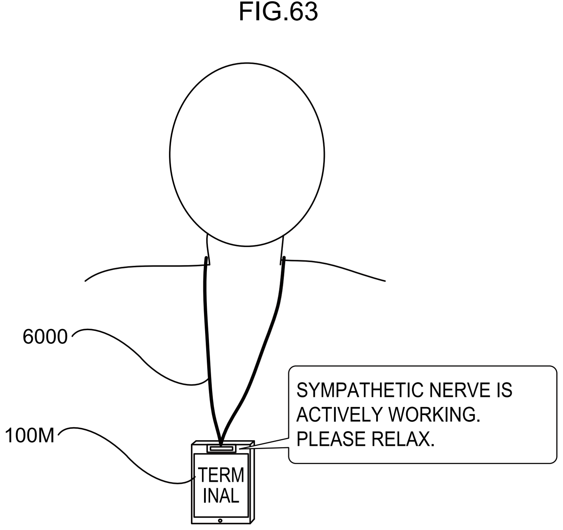

Fig. 63 is a diagram illustrating an example of vocal sound reproduced by the communication terminal according to the tenth embodiment.
Fig. 64 is a diagram illustrating an example of vocal sound reproduced by the communication terminal according to the tenth embodiment.
Fig. 65 is a diagram illustrating an example of vocal sound reproduced by a communication terminal according to a twelfth embodiment.
Fig. 66 is a diagram illustrating an example of vocal sound reproduced by the communication terminal according to the twelfth embodiment.
Fig. 67 is a diagram illustrating an example of vocal sound reproduced by the communication terminal according to the twelfth embodiment.
Fig. 68 is a diagram illustrating an example of vocal sound reproduced by the communication terminal according to the twelfth embodiment.
Fig. 69 is a diagram illustrating an example of vocal sound reproduced by the communication terminal according to the twelfth embodiment.
Fig. 70 is a diagram illustrating an example of vocal sound reproduced by the communication terminal according to the twelfth embodiment.
Fig. 71 is a diagram illustrating an example of vocal sound reproduced by the communication terminal according to the twelfth embodiment.
Fig. 72 is a diagram illustrating an example of vocal sound reproduced by the communication terminal according to the twelfth embodiment.
Fig. 73 is a diagram illustrating an example of vocal sound reproduced by the communication terminal according to the twelfth embodiment.
Fig. 74 is a diagram illustrating an example of vocal sound reproduced by the communication terminal according to the twelfth embodiment.
Fig. 75 is a diagram illustrating an example of vocal sound reproduced by the communication terminal according to the twelfth embodiment.
Fig. 76 is a diagram illustrating an example of vocal sound reproduced by the communication terminal according to the twelfth embodiment.
Fig. 77 is a diagram illustrating an example of vocal sound reproduced by the communication terminal according to the twelfth embodiment.
Fig. 78 is a diagram illustrating an example of vocal sound reproduced by the communication terminal according to the twelfth embodiment.

### DESCRIPTION OF EMBODIMENTS

Hereinafter, the invention will be described through embodiments of the invention, but the following embodiments do not limit the invention according to the claims. Not all combinations of features described in the embodiments are essential to the solution of the invention.

### [First Embodiment]

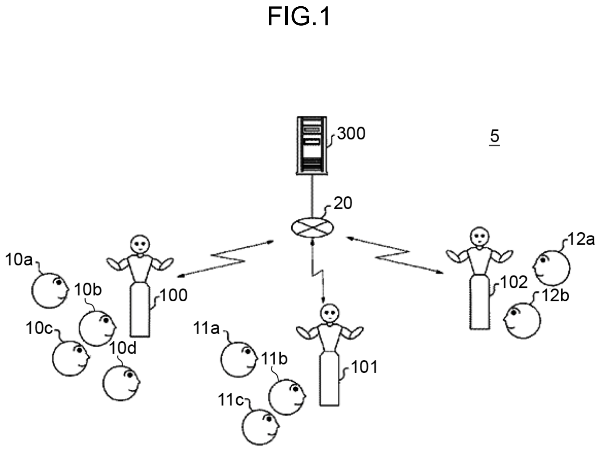

Fig. 1 schematically illustrates an example of a system 5 according to the present embodiment. The system 5 includes a robot 100, a robot 101, a robot 102, and a server 300. A user 10a, a user 10b, a user 10c, and a user 10d are users of the robot 100. The user 11a, the user 11b, and the user 11c are users of the robot 101. A user 12a and a user 12b are users of the robot 102. In the description of the present embodiment, the user 10a, the user 10b, the user 10c, and the user 10d may be collectively referred to as a user 10. The user 11a, the user 11b, and the user 11c may be collectively referred to as a user 11. The user 12a and the user 12b may be collectively referred to as a user 12. The robot 101 and the robot 102 have substantially the same functions as those of the robot 100. Therefore, the system 5 will be described focusing on the functions of the robot 100.

The robot 100 has a conversation with the user 10 and provides a video to the user 10. In this case, the robot 100 has a conversation with the user 10, provides a video to the user 10, and the like in cooperation with the server 300 and the like that can communicate via a communication network 20. For example, the robot 100 not only learns an appropriate conversation by itself, but also performs learning so that a conversation with the user 10 can be advanced more appropriately in cooperation with the server 300. The robot 100 causes the server 300 to record captured video data and the like of the user 10, requests the server 300 to transmit the video data and the like as necessary, and provides the video data and the like to the user 10.

The robot 100 has an emotion value indicating the type of its own emotion. For example, the robot 100 has emotion values indicating the intensities of respective emotions such as "happy", "angry", "sad", "pleasant", "pleased", "displeased", "safe", "anxious", "gloomy", "excited", "worried", "relieved", "sense of fulfilment", "sense of emptiness", and "normal". For example, in a case in which the robot 100 has a conversation with the user 10 in a state in which the emotion value of excitement is great, the robot emits vocal sound at a fast speed. As described above, the robot 100 can express its own emotion by action.

The robot 100 may be configured to determine an action of the robot 100 corresponding to the emotion of the user 10 by matching a sentence generation model using artificial intelligence (AI) with an emotion engine. Specifically, the robot 100 may be configured to recognize an action of the user 10, determine an emotion of the user 10 for the action of the user, and determine an action of the robot 100 corresponding to the determined emotion.

More specifically, in a case in which the robot 100 recognizes the action of the user 10, the robot 100 automatically generates action content to be taken by the robot 100 with respect to the action of the user 10 by using a preset sentence generation model. The sentence generation model may be interpreted as an algorithm and an operation for automatic interaction processing using text. Since the sentence generation model is known as disclosed in, for example, Japanese Patent Application Laid-Open (JP-A) No. 2018-081444 and ChatGPT (Internet search <URL: https://openai.com/blog/chatgpt>), a detailed description thereof will be omitted. Such a sentence generation model is configured by a large language model (LLM).

As described above, in the present embodiment, it is possible to reflect the emotions of the user 10 and the robot 100 and various types of linguistic information in the action of the robot 100 by combining the large language model and the emotion engine. That is, according to the present embodiment, a synergistic effect can be obtained by combining the sentence generation model and the emotion engine.

The robot 100 has a function of recognizing an action of the user 10. The robot 100 recognizes the action of the user 10 by analyzing a face image of the user 10 acquired by a camera function and the vocal sound of the user 10 acquired by a microphone function. The robot 100 determines an action to be executed by the robot 100 on the basis of the recognized action of the user 10 or the like.

As an example of the action determination model, the robot 100 stores a rule defining an action to be executed by the robot 100 on the basis of the emotion of the user 10, the emotion of the robot 100, and the action of the user 10, and performs various actions according to the rule.

Specifically, the robot 100 has a reaction rule as example of an action determination model for determining an action of the robot 100 on the basis of the emotion of the user 10, the emotion of the robot 100, and the action of the user 10. In the reaction rule, for example, in a case in which the action of the user 10 is "smiling", an action of "smiling" is set as the action of the robot 100. In the reaction rule, an action of "apologizing" is defined as an action of the robot 100, with respect to a case in which the action of the user 10 is "angry". In the reaction rule, an action of "answering" is defined as an action of the robot 100 with respect to a case in which the action of the user 10 is "inquiry". In the reaction rule, an action of "talking" is defined as an action of the robot 100 with respect to a case in which the action of the user 10 is "gloomy".

In a case in which the robot 100 recognizes that the action of the user 10 is "angry" on the basis of the reaction rule, the robot selects an action of "apologizing" defined in the reaction rule as the action to be executed by the robot 100. For example, in the case of selecting the action of "apologizing", the robot 100 performs an action of "apologizing" and outputs vocal sound expressing the word "apologizing".

In a case in which a condition that the emotion of the robot 100 is "normal" (that is, "happy" = 0, "angry" = 0, "sad" = 0, and "pleasant" = 0) and the state of the user 10 is "one person, lonely" is satisfied, it is defined that the change content of the emotion that the emotion of the robot 100 changes to "worried" and the action of "talking" can be executed.

In a case in which the robot 100 recognizes that the current emotion of the robot 100 is "normal" and the user 10 is alone in a lonely state on the basis of the reaction rule, the emotion value of "sad" of the robot 100 is increased. The robot 100 selects the action of "talking" defined in the reaction rule as an action to be executed on the user 10. For example, in a case in which the action of "talking" is selected, the robot 100 converts a phrase "What's wrong?" indicating that the robot is concerned into vocal sound indicating that the robot is concerned, and outputs the vocal sound.

The robot 100 transmits, to the server 300, user reaction information indicating that a positive reaction has been obtained from the user 10 through this action. The user reaction information includes, for example, the user action of "getting angry", the action of the robot 100 of "apologizing", the positive reaction of the user 10, and an attribute of the user 10.

The server 300 stores the user reaction information received from the robot 100. The server 300 receives and stores the user reaction information not only from the robot 100 but also from each of the robot 101 and the robot 102. The server 300 analyzes the user reaction information from the robot 100, the robot 101, and the robot 102, and updates the reaction rule.

The robot 100 receives the updated reaction rule from the server 300 by inquiring the server 300 about the updated reaction rule. The robot 100 incorporates the updated reaction rule into the reaction rule stored in the robot 100. As a result, the robot 100 can incorporate the reaction rule acquired by the robot 101, the robot 102, or the like into its own reaction rule.

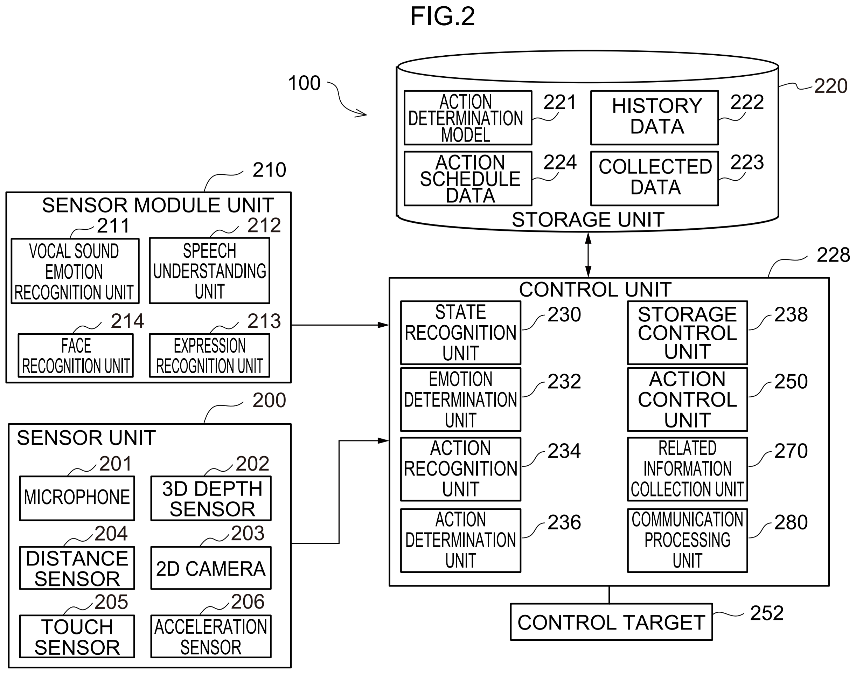

Fig. 2 schematically illustrates a functional configuration of the robot 100. The robot 100 includes a sensor unit 200, a sensor module unit 210, a storage unit 220, a control unit 228, and a control target 252. The control unit 228 includes a state recognition unit 230, an emotion determination unit 232, an action recognition unit 234, an action determination unit 236, a storage control unit 238, an action control unit 250, a related information collection unit 270, and a communication processing unit 280.

The control target 252 includes a display device, a speaker, an LED of an eye portion, and motors that drive an arm, a hand, a foot, and the like. The posture and the gesture of the robot 100 are controlled by controlling motors for arms, hands, and feet. Some of the emotions of the robot 100 can be expressed by controlling these motors. The expression of the robot 100 can be expressed by controlling a light emission state of the LED of the eye portion of the robot 100. The posture, the gesture, and the expression of the robot 100 are examples of the attitude of the robot 100.

The sensor unit 200 includes a microphone 201, a 3D depth sensor 202, a 2D camera 203, a distance sensor 204, a touch sensor 205, and an acceleration sensor 206. The microphone 201 continuously detects vocal sound and outputs vocal sound data. The microphone 201 may be provided on the head of the robot 100 and may have a function of performing binaural recording. The 3D depth sensor 202 analyzes infrared patterns from infrared images continuously captured by an infrared camera by continuously applying the infrared patterns, and detects an outline of an object. The 2D camera 203 is an example of an image sensor. The 2D camera 203 captures an image with visible light and generates image information of visible light. The distance sensor 204 detects a distance to an object by emitting, for example, laser light or ultrasonic waves. The sensor unit 200 may further include a clock, a gyro sensor, a sensor for motor feedback, and the like.

Among the constituents of the robot 100 illustrated in Fig. 2, the constituents other than the control target 252 and the sensor unit 200 are examples of constituents of the control system included in the robot 100. The control system of the robot 100 controls the control target 252.

The storage unit 220 includes an action determination model 221, history data 222, collected data 223, and action schedule data 224. The history data 222 includes past emotion values of the user 10, past emotion values of the robot 100, and a history of actions, and specifically includes a plurality of pieces of event data including the emotion values of the user 10, the emotion values of the robot 100, and the actions of the user 10. The data including the action of the user 10 includes a camera image representing the action of the user 10. The emotion values and the history of actions are recorded for each user 10 by being associated with identification information of the user 10, for example. At least a part of the storage unit 220 is implemented by a storage medium such as a memory. A person DB that stores a face image of the user 10, attribute information of the user 10, and the like may be included. Among the constituents of the robot 100 illustrated in Fig. 2, the functions of the constituents other than the control target 252, the sensor unit 200, and the storage unit 220 can be realized by a CPU operating on the basis of a program. For example, the functions of these constituents can be implemented as the operation of the CPU by basic software (OS) and a program operating on the OS.

The sensor module unit 210 includes a vocal sound emotion recognition unit 211, a speech understanding unit 212, an expression recognition unit 213, and a face recognition unit 214. Information detected by the sensor unit 200 is input to the sensor module unit 210. The sensor module unit 210 analyzes information detected by the sensor unit 200 and outputs an analysis result to the state recognition unit 230.

The vocal sound emotion recognition unit 211 of the sensor module unit 210 analyzes the vocal sound of the user 10 detected by the microphone 201 to recognize the emotion of the user 10. For example, the vocal sound emotion recognition unit 211 extracts a feature amount such as a frequency component of the vocal sound and recognizes the emotion of the user 10 on the basis of the extracted feature amount. The speech understanding unit 212 analyzes the vocal sound of the user 10 detected by the microphone 201 and outputs text information indicating the speech content of the user 10.

The expression recognition unit 213 recognizes the expression of the user 10 and the emotion of the user 10 from the image of the user 10 captured by the 2D camera 203. For example, the expression recognition unit 213 recognizes the expression and emotion of the user 10 on the basis of the shapes, positional relationships, and the like of the eyes and the mouth.

The face recognition unit 214 recognizes the face of the user 10. The face recognition unit 214 recognizes the user 10 by matching a face image stored in a person DB (not illustrated) with a face image of the user 10 captured by the 2D camera 203.

The state recognition unit 230 recognizes a state of the user 10 on the basis of the information analyzed by the sensor module unit 210. For example, processing mainly related to perception is performed by using the analysis result of the sensor module unit 210. For example, perception information such as "There is one father." and "The probability that the father does not smile is 90%." is generated. Processing of understanding the meaning of the generated perception information is performed. For example, semantic information such as "One father looks lonely." is generated.

The state recognition unit 230 recognizes a state of the robot 100 on the basis of the information detected by the sensor unit 200. For example, the state recognition unit 230 recognizes a remaining battery level of the robot 100, a brightness of the surrounding environment of the robot 100, or the like as the state of the robot 100.

The emotion determination unit 232 determines an emotion value indicating the emotion of the user 10 on the basis of the information analyzed by the sensor module unit 210 and the state of the user 10 recognized by the state recognition unit 230. For example, the information analyzed by the sensor module unit 210 and the recognized state of the user 10 are input to a neural network trained in advance, and an emotion value indicating the emotion of the user 10 is acquired.

Here, the emotion value indicating the emotion of the user 10 is a value indicating whether the emotion of the user is positive or negative. For example, in a case in which the emotion of the user is a bright emotion accompanied with pleasure or comfort, such as "happy", "pleasant", "pleased", "safe", "excited", "relieved", and "sense of fulfillment", a positive value is indicated, and the value becomes larger as the emotion becomes brighter. In a case in which the emotion of the user is an emotion that makes the user feel unpleasant, such as "angry", "sad", "displeased", "anxious", "gloomy", "worried", and "sense of emptiness", a negative value is indicated, and an absolute value of the negative value increases as the user feels unpleasant. In a case in which the emotion of the user is not included in any of the above emotions ("normal"), a value of 0 is indicated.

The emotion determination unit 232 determines an emotion value indicating the emotion of the robot 100 on the basis of the information analyzed by the sensor module unit 210, the information detected by the sensor unit 200, and the state of the user 10 recognized by the state recognition unit 230.

The emotion value of the robot 100 includes the emotion value for each of the plurality of emotion classifications, and is, for example, a value (0~5) indicating the strength of each of "happy", "angry", "sad", and" pleasant".

Specifically, the emotion determination unit 232 determines an emotion value indicating the emotion of the robot 100 according to a rule for updating the emotion value of the robot 100, the rule being defined in association with the information analyzed by the sensor module unit 210 and the state of the user 10 recognized by the state recognition unit 230.

For example, in a case in which the state recognition unit 230 recognizes that the user 10 looks lonely, the emotion determination unit 232 increases the emotion value of "sad" of the robot 100. In a case in which the state recognition unit 230 recognizes that the user 10 has a smiling face, the emotion value of "happy" of the robot 100 is increased.

The emotion determination unit 232 may determine the emotion value indicating the emotion of the robot 100 in further consideration of the state of the robot 100. For example, in a case in which a remaining battery level of the robot 100 is low, or in a case in which the surrounding environment of the robot 100 is dark, the emotion value of "sad" of the robot 100 may be increased. In the case of the user 10 continuously talking to the robot even though the remaining battery level is low, the emotion value of "angry" may be increased.

The action recognition unit 234 recognizes an action of the user 10 on the basis of the information analyzed by the sensor module unit 210 and the state of the user 10 recognized by the state recognition unit 230. For example, the information analyzed by the sensor module unit 210 and the recognized state of the user 10 are input to a neural network trained in advance, probabilities of a plurality of predefined action classifications (for example, "smile", "angry", "ask a question", and "gloomy") are acquired, and an action classification having the highest probability is recognized as the action of the user 10.

As described above, in the present embodiment, the robot 100 acquires the speech content of the user 10 after specifying the user 10, but in acquiring and using the speech content, the control system of the robot 100 according to the present embodiment considers protection of personal information and privacy of the user 10 in addition to acquiring necessary consent according to laws and regulations from the user 10.

Next, processing of the action determination unit 236 in a case in which the robot 100 performs response processing of responding to an action of the user 10 will be described.

The action determination unit 236 determines an action corresponding to the action of the user 10 recognized by the action recognition unit 234 on the basis of the current emotion value of the user 10 determined by the emotion determination unit 232, the history data 222 of the past emotion values determined by the emotion determination unit 232 before the current emotion value of the user 10 is determined, and the emotion value of the robot 100. In the present embodiment, a case in which the action determination unit 236 uses one most recent emotion value included in the history data 222 as the past emotion value of the user 10 will be described, but the disclosed technology is not limited to this aspect. For example, the action determination unit 236 may use a plurality of most recent emotion values as the past emotion values of the user 10, or may use emotion values that are earlier by a unit period such as one day before. The action determination unit 236 may determine an action corresponding to the action of the user 10 in further consideration of the history of the past emotion values of the robot 100 in addition to the current emotion value of the robot 100. The action determined by the action determination unit 236 includes a gesture performed by the robot 100 or speech content of the robot 100.

The action determination unit 236 according to the present embodiment determines an action of the robot 100 on the basis of a combination of the past emotion value and the current emotion value of the user 10, the emotion value of the robot 100, the action of the user 10, and the action determination model 221, as an action corresponding to the action of the user 10. For example, in a case in which the past emotion value of the user 10 is a positive value and the current emotion value is a negative value, the action determination unit 236 determines an action for positively changing the emotion value of the user 10 as an action corresponding to the action of the user 10.

The reaction rule as the action determination model 221 defines an action of the robot 100 according to the combination of the past emotion value and the current emotion value of the user 10, the emotion value of the robot 100, and the action of the user 10. For example, in a case in which the past emotion value of the user 10 is a positive value, the current emotion value is a negative value, and the action of the user 10 is gloomy, a combination of a gesture and speech content at the time of making an inquiry to encourage the user 10 including a gesture is defined as the action of the robot 100.

For example, the reaction rule as the action determination model 221 defines an action of the robot 100 for all combinations of patterns of emotion values of the robot 100 (1296 patterns that is the fourth power of six values of the values "0" to "5" of "happy", "angry", "sad", and "pleasant"), a pattern of the combination of the past emotion value and the current emotion value of the user 10, and an action pattern of the user 10. That is, for each pattern of the emotion value of the robot 100, the action of the robot 100 according to the action pattern of the user 10 is defined for each of a plurality of combinations such as combinations of the past emotion value and the current emotion value of the user 10 being a negative value and a negative value, a negative value and a positive value, a positive value and a negative value, a positive value and a positive value, a negative value and normal, and normal and normal. The action determination unit 236 may transition to an operation mode of determining the action of the robot 100 by using the history data 222, for example, in a case in which the user 10 has made speech that intends a conversation continued from a past topic such as "I want to talk about the topic I discussed earlier".

In the reaction rule as the action determination model 221, for each of the 1296 patterns of the emotion values of the robot 100, at most one action of the robot 100 is defined, the action including at least one of a gesture or statement content. Alternatively, in the reaction rule as the action determination model 221, at least one of the gesture and statement content may be defined as an action of the robot 100 for each of the groups of the patterns of the emotion values of the robot 100.

The intensity of each gesture included in the action of the robot 100 defined in the reaction rule as the action determination model 221 is defined in advance. In each piece of speech content included in the action of the robot 100 defined in the reaction rule as the action determination model 221, the intensity of the speech content is defined in advance.

The storage control unit 238 determines whether or not to store data including the action of the user 10 in the history data 222 on the basis of the intensity of the action predefined for the action determined by the action determination unit 236 and the emotion value of the robot 100 determined by the emotion determination unit 232.

Specifically, in a case in which a total value of the intensities that is a sum of a sum total of the emotion values for the plurality of respective emotion classifications of the robot 100, the intensity predefined for the gesture included in the action determined by the action determination unit 236, and the intensity predefined for the speech content included in the action determined by the action determination unit 236 is a threshold or more, it is determined to store data including the action of the user 10 in the history data 222.

In a case in which the storage control unit 238 determines to store the data including the action of the user 10 in the history data 222, the action determined by the action determination unit 236, information (for example, any surrounding information including data such as vocal sound, an image, and a smell at the place) analyzed by the sensor module unit 210 from the current time point to a certain period before, and a state (for example, the expression and the emotion of the user 10) of the user 10 recognized by the state recognition unit 230 are stored in the history data 222.

The action control unit 250 controls the control target 252 on the basis of the action determined by the action determination unit 236. For example, in a case in which the action determination unit 236 determines an action including speech, the action control unit 250 causes a speaker included in the control target 252 to output vocal sound. In this case, the action control unit 250 may determine an emission speed of the vocal sound on the basis of the emotion value of the robot 100. For example, the action control unit 250 determines a higher vocal sound emission speed as the emotion value of the robot 100 becomes greater. As described above, the action control unit 250 determines an execution form of the action determined by the action determination unit 236 on the basis of the emotion value determined by the emotion determination unit 232.

The action control unit 250 may recognize a change in emotion of the user 10 with respect to execution of the action determined by the action determination unit 236. For example, the change in emotion may be recognized on the basis of the vocal sound or expression of the user 10. A change in emotion of the user 10 may be recognized on the basis of detection of an impact by the touch sensor 205 included in the sensor unit 200. In a case in which an impact is detected by the touch sensor 205 included in the sensor unit 200, it may be recognized that the emotion of the user 10 is worsened, or in a case in which it is determined that the reaction of the user 10 is smiling or happy from the detection result in the touch sensor 205 included in the sensor unit 200, it may be recognized that the emotion of the user 10 is improved. Information indicating the reaction of the user 10 is output to the communication processing unit 280.

After the action control unit 250 executes the action determined by the action determination unit 236 in the execution form determined according to the emotion of the robot 100, the emotion determination unit 232 further changes the emotion value of the robot 100 on the basis of the user's reaction to the execution of the action. Specifically, the emotion determination unit 232 increases the emotion value of "happy" of the robot 100 in a case in which the user's reaction to the action determined by the action determination unit 236 performed on the user in the execution form determined by the action control unit 250 is not poor. The emotion determination unit 232 increases the emotion value of "sad" of the robot 100 in a case in which the user's reaction to the action determined by the action determination unit 236 performed on the user in the execution form determined by the action control unit 250 is poor.

The action control unit 250 expresses the emotion of the robot 100 on the basis of the determined emotion value of the robot 100. For example, in a case in which the emotion value of "happy" of the robot 100 is increased, the action control unit 250 controls the control target 252 to cause the robot 100 to perform a gesture of being happy. In a case in which the emotion value of "sad" of the robot 100 is increased, the action control unit 250 controls the control target 252 so that the posture of the robot 100 becomes a head-drooping posture.

The communication processing unit 280 performs communication with the server 300. As described above, the communication processing unit 280 transmits the user reaction information to the server 300. The communication processing unit 280 receives the updated reaction rule from the server 300. Upon receiving the updated reaction rule from the server 300, the communication processing unit 280 updates the reaction rule as the action determination model 221.

The server 300 performs communication between the robot 100, the robot 101, and the robot 102 and the server 300, receives the user reaction information transmitted from the robot 100, and updates the reaction rule on the basis of the reaction rule including the action for which a positive reaction has been obtained.

The related information collection unit 270 collects information related to preference information from external data (web sites such as news sites and moving image sites) on the basis of the preference information acquired for the user 10 at a predetermined timing.

Specifically, the related information collection unit 270 acquires preference information indicating a matter of interest of the user 10 from speech content of the user 10 or a setting operation of the user 10. The related information collection unit 270 collects news related to the preference information from the external data at regular intervals by using ChatGPT Plugins (Internet search <URL: https://openai.com/blog/chatgpt-plugins>). For example, in a case in which it is acquired as the preference information that the user 10 is a fan of a specific professional baseball team, the related information collection unit 270 collects news related to a game result of the specific professional baseball team from external data at a predetermined time every day, by using, for example, ChatGPT Plugins.

The emotion determination unit 232 determines an emotion of the robot 100 on the basis of the information related to the preference information collected by the related information collection unit 270.

Specifically, the emotion determination unit 232 inputs text representing the information related to the preference information collected by the related information collection unit 270 to a neural network trained in advance for determining an emotion, acquires an emotion value indicating each emotion, and determines the emotion of the robot 100. For example, in a case in which the collected news related to the game result of the specific professional baseball team indicates that the specific professional baseball team has won, the emotion value of "happy" of the robot 100 is determined to be great.

In a case in which the emotion value of the robot 100 is a threshold or more, the storage control unit 238 stores the information related to the preference information collected by the related information collection unit 270 in the collected data 223.

Next, processing of the action determination unit 236 in the case of performing autonomous processing in which the robot 100 and a movement system including the communication terminal autonomously act will be described. Details of a configuration of the movement system will be described later.

In the autonomous processing in the present embodiment, the robot 100 and the movement system may detect a state or an action of the user spontaneously or periodically by monitoring the user. The term "spontaneous" may be interpreted as the robot 100 and the movement system spontaneously acquiring the state or action of the user by themselves without a trigger from the outside. The trigger from the outside may include a question from the user to the robot 100 and the movement system, an active action from the user to the robot 100 and the movement system, and the like. The term "periodic" may be interpreted as a specific cycle such as a unit of one second, a unit of one minute, a unit of one hour, a unit of several hours, a unit of several days, a unit of week, or a unit of day of the week.

The state of the user may include an action tendency of the user. The action tendency may include that the user walks for a long time, that the user runs for a long time, and that the user performs a dangerous behavior. The dangerous behavior may include that the user walks on a roadway, and that the user enters the roadway from a sidewalk. The action tendency may be interpreted as a tendency of the user's hyperactive or impulsive action.

In the autonomous processing, the robot 100 and the movement system may ask a generative AI a question about the detected state or action of the user, and store an answer of the generative AI to the question and the detected action of the user in association with each other. In this case, the robot 100 and the movement system may store action content of correcting the action in association with the answer.

Information in which the answer of the generative AI to the question, the detected action of the user, and the action content of correcting the action are associated with each other may be recorded as table information in a storage medium such as a memory. The table information may be interpreted as specific information recorded in the storage unit.

Specifically, in a case in which the user tends to walk for a long time, the robot 100 and the movement system may ask the generative AI a question "What kind of guidance should be given to the user who takes such an action?", and in a case in which the answer of the generative AI to the question is "Propose the same route as the route along which the user has previously passed", the robot 100 and the movement system may record the answer, the action of the user who can walk for a long time, and the action content of correcting the action (for example, guidance content of "guiding the user to the route along which the user has previously passed") in association with the table information. In a case in which the action of the user who is walking for a long time is detected, the action determination unit 236 of the robot 100 and the movement system may reproduce vocal sound "I will guide you to the route along which you have previously passed" as the action of the robot 100 and the movement system by using the table information.

As another example, in a case in which the user tends to run for a long time, the robot 100 and the movement system ask the generative AI a question "What kind of guidance should be given to the user who takes such an action?", and in a case in which the answer of the generative AI to the question is "It is desirable to periodically perform guidance for encouraging hydration", the robot 100 and the movement system may record the answer, the action of the user who can run for a long time, and the action content of correcting the action (for example, guidance content such as "Please rehydrate frequently") in association with the table information. In a case in which the action of the user who is running for a long time is detected, the action determination unit 236 of the robot 100 and the movement system may reproduce vocal sound "Please rehydrate frequently" as the action of the robot 100 and the movement system by using the table information.

As still another example, in a case in which the user tends to walk on the roadway, the robot 100 and the movement system may ask the generative AI a question "What kind of guidance should be given to the user who takes such an action?", and in a case in which the answer of the generative AI to the question is "I recommend to propose returning to the sidewalk because there is a possibility of coming into contact with vehicles", the robot 100 and the movement system may record the answer, the action of the user who may walk on the roadway, and the action content of correcting the action (for example, guidance content of "Please return to the sidewalk") in association with the table information. In a case in which the action of the user walking on the sidewalk is detected, the action determination unit 236 of the robot 100 and the movement system may reproduce vocal sound "Please return to the sidewalk" as the action of the robot 100 and the movement system by using the table information.

In the autonomous processing, an action schedule of the robot 100 and the movement system for attracting attention with respect a state or an action of the user may be set on the basis of the detected action of the user and the stored specific information.

As described above, the movement system may record table information in which the answer of the generative AI corresponding to the state or the action of the user is associated with the detected state or action of the user in the storage medium. Hereinafter, an example of content stored in the table will be described.

### (1. Case in Which User Tends to Walk for a Long Time)

In a case in which there is the tendency, the movement system itself asks the generative AI a question "What kind of guidance should be given to the user who takes such an action?". In a case in which the answer of the generative AI to this question is, for example, "Propose the same route as the route along which the user has previously passed", "Propose a route along which the user has not passed", "Propose a route with a relatively small number of vehicles such as bicycles", or "Propose a route with undulations", the movement system may store the action of the user and the answer of the generative AI in association with each other.

### (2. Case in Which User Tends to Run for a Long Time)

In a case in which there is the tendency, the movement system itself asks the generative AI a question "What kind of guidance should be given to the user who takes such an action?". In a case in which the answer of the generative AI to this question is, for example, "Provide guidance for encouraging hydration periodically", "Propose a route with fewer bicycles and clean air", or "Propose a route with fewer traffic lights", the movement system may store the action of the user and the answer of the generative AI in association with each other.

### (3. Case in Which User Tends to Walk on Roadway)

In a case in which there is the tendency, the movement system itself asks the generative AI a question "What kind of situation can occur to the user who takes such an action?". In a case in which the answer of the generative AI to this question is, for example, "There is a possibility of coming into contact with vehicles", "There is a possibility of coming into contact with bicycles", or "There is a possibility of entering an expressway interchange", the movement system may store the user's action of walking on the roadway and the answer of the generative AI in association with each other. The movement system may store action content of correcting the action in association with the answer.

In a case in which there is the tendency, the robot 100 and the communication terminal themselves ask the generative AI a question "What kind of situation can occur to the user who takes such an action?". In a case in which the answer of the generative AI to this question is, for example, "There is a possibility of coming into contact with a vehicle such as a bicycle", "There is a possibility of entering an expressway interchange", or "Give advice to encourage the user to walk on a sidewalk as much as possible", the robot 100 and the communication terminal may store the user's action of walking on a roadway and the answer of the generative AI in association with each other. The robot 100 and the communication terminal may store action content of correcting the action in association with the answer.

The action content of correcting the action may include at least one of reproduction of vocal sound for correcting a dangerous action of the user or reproduction of an image for correcting the dangerous action of the user.

The vocal sound for correcting the dangerous action of the user may include vocal sound for guiding the user to a specific place. The specific place may include a place other than the place where the user is currently located, for example, a vicinity of a movement system and a sidewalk. Specifically, the vocal sound may include vocal sound such as "Please stop walking on the roadway", "That is not a sidewalk", "The roadway is dangerous", and "Please move to the sidewalk immediately".

As described above, in the autonomous processing, a table in which the answer of the generative AI corresponding to the state or action of the user, the content of the state or action, and the action content of correcting the state or action are associated with each other may be recorded in a storage medium such as a memory.

In the autonomous processing, after the table is recorded, the action of the user is autonomously or periodically detected, and the action schedule of the movement system for attracting the attention of the user may be set on the basis of the detected action of the user and the content of the stored table. Specifically, the action determination unit 236 of the movement system may set the first action content on the basis of the detected action of the user and the content of the stored table.

In the autonomous processing, after the table is recorded, the action of the user may be autonomously or periodically detected, and the action schedule of the communication terminal for attracting the attention of the user may be set on the basis of the detected action of the user and the content of the stored table. Specifically, the action determination unit 236 of the robot 100 and the communication terminal may set the first action content on the basis of the detected action of the user and the content of the stored table.

Specifically, the action determination unit 236 may spontaneously or periodically detect an action of the user on the basis of the sensor, and determine the first action content in the case of determining to lead the user as an action of the electronic apparatus on the basis of the detected action of the user and the specific information stored in advance.

The sensor may be interpreted as a sensor of one or a plurality of sensors provided in a communication terminal detachably provided in a movement unit of the movement system. Specifically, the one or more sensors may include a camera, a microphone, a gyro sensor, and the like. The one or more sensors may collect information for controlling the rotation of a wheel of the movement unit.

The sensor may be interpreted as one or a plurality of sensors provided in the communication terminal. Specifically, the one or more sensors may include a camera, a microphone, a gyro sensor, and the like.

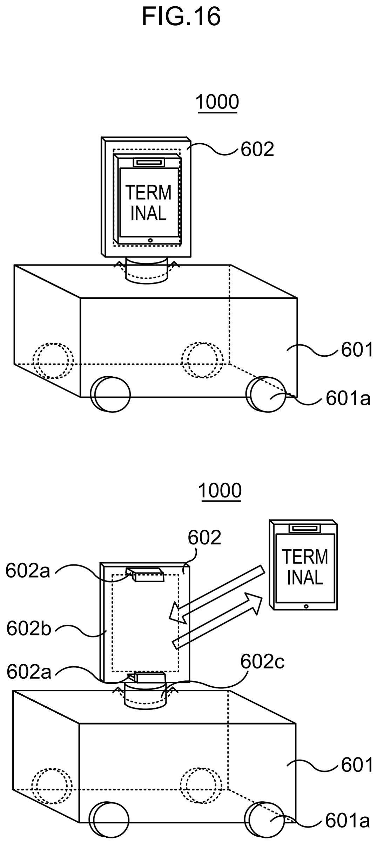

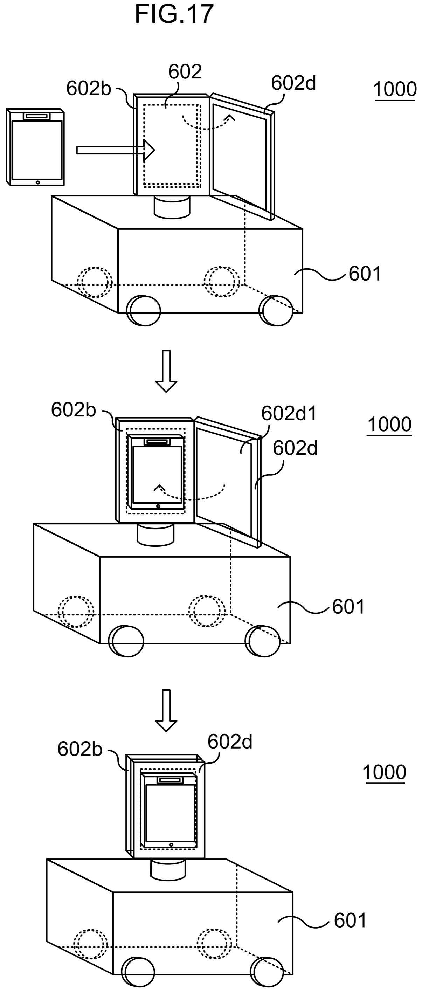

Here, the movement unit will be described in detail with reference to Figs. 16 and 17. The first action content will be described later. Figs. 16 and 17 are external views of the movement system including the movement unit and the communication terminal. As illustrated in Fig. 16, a mobile movement system 1000M includes a movement unit 601 having wheels 601a for autonomous traveling. A communication terminal 600 is detachably provided in the movement unit 601. The movement unit 601 may be interpreted as any of the robots 100 to 102 described above. The communication terminal 600 may be interpreted as a smartphone that will be described later, or may be interpreted as a smart AI device. The movement system 1000M may have the functions of the robots 100 to 102 described above.

The movement unit 601 is provided with an attachment member 602 for detachably installing the communication terminal 600. The attachment member 602 is rotatably provided on the upper portion of the movement unit 601.

Specifically, the attachment member 602 may include a plurality of hook-shaped first members 602a that hold the ends of the communication terminal 600, a plate-shaped second member 602b provided with the first member 602a and in contact with the back surface of the communication terminal 600, and a third member 602c that rotatably instructs the second member 602b.

By using the plurality of hook-shaped first members 602a, the communication terminal 600 is held, and falling of the communication terminal 600 can be curbed. The communication terminal 600 can be easily attached and detached.

The third member 602c can operate in accordance with an operation command from the communication terminal 600. Specifically, the communication terminal 600 can control the third member 602c to direct the front of the communication terminal 600 toward the user. More specifically, the communication terminal 600 can control the motor so that the user can check the screen of the communication terminal 600 even in a case in which the movement unit 601 or the user moves from a specific position by recognizing the user's expression, action, or the like. The control of the motor may include control of a rotation amount of the motor, a rotation speed of the motor, a rotation direction of the motor, and the like.

The configuration of the attachment member 602 is not limited thereto, and the attachment member 602 may be configured as illustrated in Fig. 17. As illustrated in Fig. 17, the attachment member 602 may include a second member 602b in which a recess for accommodating the communication terminal 600 is formed, and a fourth member 602d that is an openable front panel covering a front surface of the second member 602b. The fourth member 602d may be provided with an opening 602d1 so that the screen of the communication terminal 600 can be visually recognized.

Fig. 17 illustrates, in order from the top, the attachment member 602 before the communication terminal 600 is stored, the attachment member 602 after the communication terminal 600 is stored, and the attachment member 602 after the fourth member 602d is closed.

By using the attachment member 602 illustrated in Fig. 17, it is possible to prevent the communication terminal 600 from falling off from the attachment member 602 even in a case in which the attachment member 602 vibrates due to movement of the movement unit 601. Even in a case in which the movement unit 601 falls, the communication terminal 600 is protected by the fourth member 602d which is the front panel, and it is possible to curb the communication terminal 600 from being damaged.

### (First Action Content)

The first action content may include an action of the movement system 1000M that leads the user by controlling the wheels 601a of the movement unit 601. Controlling the wheels 601a may include changing at least one of a rotation direction, a rotation speed, or a steering direction of the wheels 601a. Hereinafter, an example of the first action content will be described.

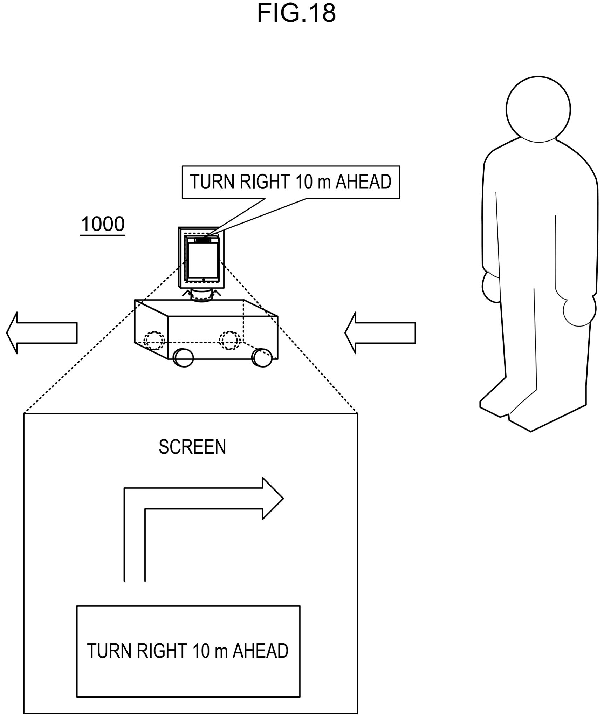

The action determination unit 236 may execute reproduction of at least one of vocal sound or an image for assisting in walking of the user as the first action content for performing route guidance so that the walking user moves along a specific route. Specifically, as illustrated in Fig. 18, the action determination unit 236 may reproduce an image of a route along which the user can walk on the screen of the communication terminal 600, and may reproduce vocal sound related to the route. The vocal sound may include, for example, "Turn right 10 m ahead".

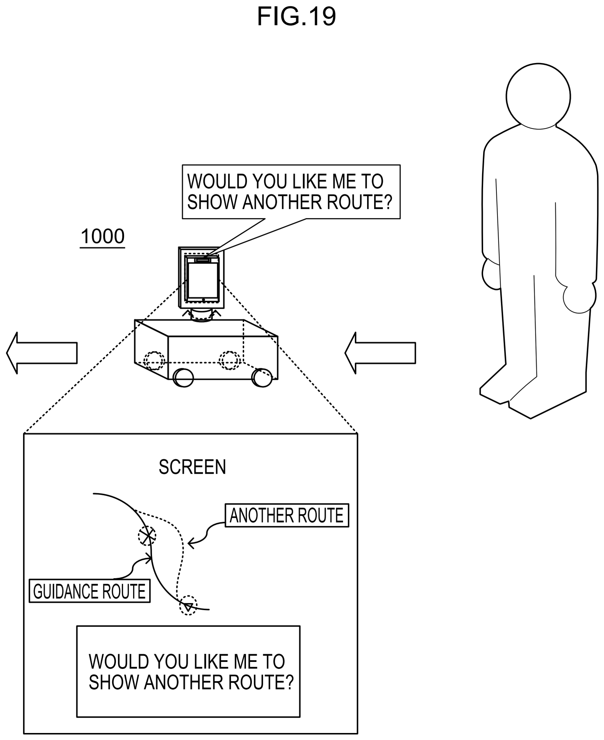

The action determination unit 236 may execute reproduction of at least one of vocal sound or an image for guiding the user along a route different from the set guidance route. Specifically, as illustrated in Fig. 19, the action determination unit 236 may reproduce an image of a route different from the set guidance route on the screen of the communication terminal 600, and may reproduce vocal sound related to the route. The vocal sound may include, for example, "The road is closed ahead. Would you like me to show another route?".

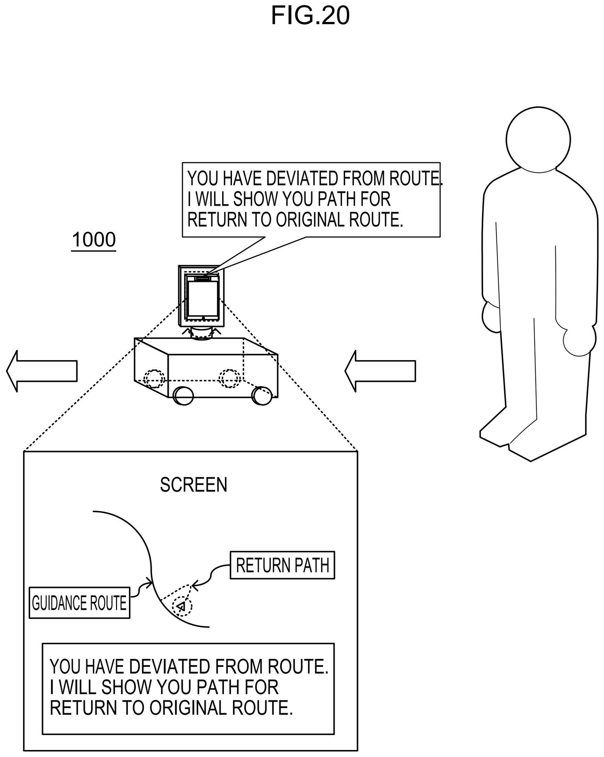

In a case in which the user being led along the set guidance route deviates from the guidance route, the action determination unit 236 may execute reproduction of at least one of vocal sound or an image for guiding the user along a path for returning the user to the guidance route. Specifically, as illustrated in Fig. 20, the action determination unit 236 may reproduce an image of the route returning the user to the set guidance route on the screen of the communication terminal 600, and may reproduce vocal sound related to the route. The vocal sound may include "You have deviated from the route. I will show you the path for return to the original route." and the like. The action determination unit 236 of the robot 100 and the movement system may execute reproduction of at least one of the vocal sound or the image described above, for example, "I will guide you to the route along which you have previously passed", "Please rehydrate frequently", or "Please return to the sidewalk".

### (Second Action Content)

The action determination unit 236 may determine whether or not the action of the user has been corrected by detecting the action of the user while the movement unit 601 is leading the user or after the movement unit 601 reproduces at least one of vocal sound or an image that assists in the walking of the user, and may determine second action content different from the first action content in a case in which the action of the user has been corrected.

The case in which the action of the user has been corrected may be interpreted as a case in which the user has stopped a specific action and a specific behavior or a case in which a specific situation has been resolved as a result of execution of the operation of the movement system 1000M according to the first action content. Specifically, the case in which the action of the user has been corrected may include a case in which the user starts walking along the proposed different route, a case in which the user starts walking along a path for return to the original route when the path is shown due to deviation from the original route, and the like.

The second action content may include reproduction of at least one of vocal sound praising the user for action, vocal sound expressing gratitude for the action of the user, an image praising the user for the action, or an image expressing gratitude for the action of the user.

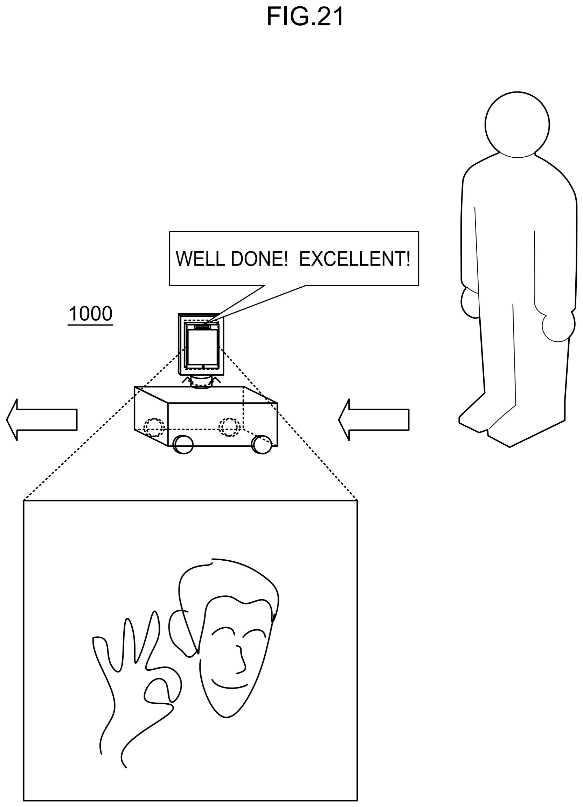

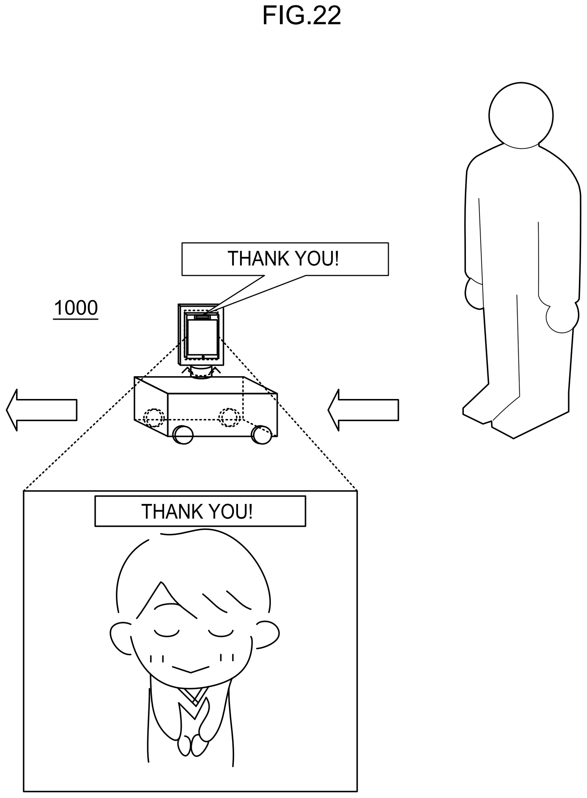

Specifically, the vocal sound praising the user for the action may include, for example, "Well done! Excellent". The vocal sound expressing gratitude for the action of the user may include vocal sound such as "Thank you for coming back" or "Thank you for selecting another route". As illustrated in Fig. 21, the image praising the user for the action may include an image of a character taking a good pose. As illustrated in Fig. 22, the image expressing gratitude for the action of the user may include an image of a character who expresses gratitude.

### (Third Action Content)

The action determination unit 236 may determine whether or not the action of the user has been corrected by detecting the action of the user while the movement unit 601 was leading the user or after the movement unit 601 reproduced at least one of vocal sound or an image that assists in the walking of the user, and determine third action content different from the first action content in a case in which the action of the user has not been corrected.

The case in which the action of the user has not been corrected may be interpreted as a case in which the user has continued the dangerous action and behavior even though the operation of the movement system 1000M according to the first action content was executed, a case in which the dangerous situation is not resolved, or a case in which the user has started or continued an action different from the proposal of the movement system 1000M.

Specifically, the case in which the action of the user has not been corrected may include a case in which the user has walked along a route different from the proposed different route without walking along the proposed different route, a case in which the user has neglected a path for return to the original route and has continued walking when the path was shown due to deviation from the original route, and the like.

The third action content may include at least one of transmission of specific information to a person other than the user, reproduction of sound that attracts an interest of the user, or reproduction of an image that attracts an interest of the user.

The transmission of the specific information to a person other than the user may include distribution of an email containing a warning message to a relative of the user, a spouse of the user, a guardian of the user, or the like. The transmission of the specific information to a person other than the user may include distribution of an image (a still image or a moving image) including the user and the surrounding scenery to a relative of the user, a spouse of the user, a guardian of the user, or the like. The transmission of the specific information to a person other than the user may include distribution of vocal sound having a warning message.

The reproduction of the sound that attracts the interest of the user may include specific music that the user likes, and may also include vocal sound such as "Please come back here" or "Let's take a safe road".

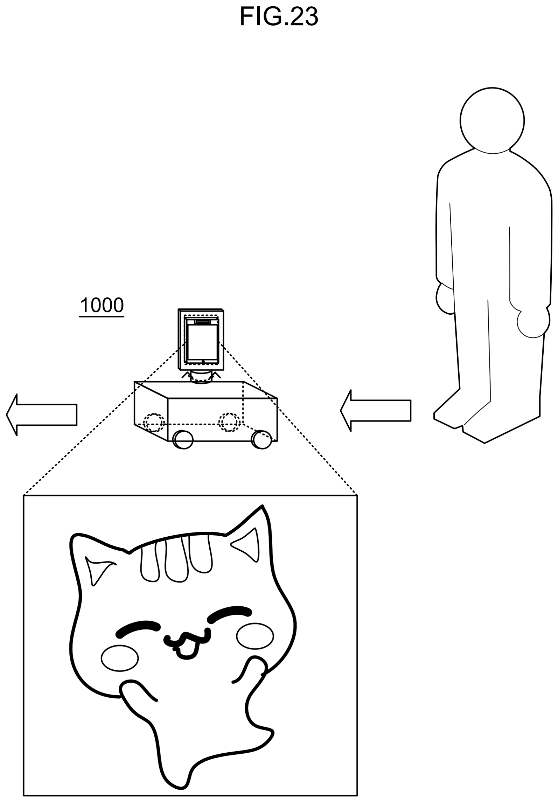

The reproduction of the image that attracts the interest of the user may include *reproduction* of an image of an animal kept by the user, an image of parents of the user, an image of a character of the animal illustrated in Fig. 23, and the like.

According to the movement system 1000M of the disclosure, an action of leading the user can be executed through autonomous processing. As a result, the movement system 1000M can guide the user walking in a town that the user visits for the first time to an appropriate route. The user who is interested in walking may be guided to a route along which the user has not passed before. A route where the user cannot pass is detected in advance, and the user can be guided to another route. The user who gets lost and deviates from the specific route can be guided to the original route.

The action determination unit 236 determines, as the action of the robot 100 and the movement system 1000M, any of a plurality of types of robot actions and movement system actions including no action, by using at least one of the state of the user 10, the emotion of the user 10, the emotion of the robot 100 and the movement system 1000M, or the state of the robot 100 and the movement system 1000M, and the action determination model 221 at a predetermined timing. Here, a case in which a sentence generation model having an interaction function is used as the action determination model 221 will be described as an example.

Specifically, the action determination unit 236 inputs text representing at least one of the state of the user 10, the emotion of the user 10, the emotion of the robot 100 and the movement system 1000M, or the state of the robot 100 and the movement system 1000M, and text for inquiry about robot actions and movement system actions to the sentence generation model, and determines the actions of the robot 100 and the movement system 1000M on the basis of an output of the sentence generation model.

For example, the plurality of types of robot actions and movement system actions include the following (1) to (26).
(1) The robot 100 and the movement system 1000M do nothing.
(2) The robot 100 and the movement system 1000M have a dream.
(3) The robot 100 and the movement system 1000M talk to the user.
(4) The robot 100 and the movement system 1000M create a picture diary.
(5) The robot 100 and the movement system 1000M propose an activity.
(6) The robot 100 and the movement system 1000M propose a person with whom the user should meet.
(7) The robot 100 and the movement system 1000M introduce news in which the user is interested.
(8) The robot 100 and the movement system 1000M edit pictures and moving images.
(9) The robot 100 and the movement system 1000M study with the user.
(10) The robot 100 and the movement system 1000M evoke memories.
(11) The robot 100 and the movement system 1000M may reproduce an image of a route along which the user can walk on the screen of the communication terminal as the first action content of leading the user.
(12) The robot 100 and the movement system 1000M may reproduce vocal sound related to a route along which the user can walk as the first action content of correcting the action of the user.
(13) The robot 100 and the movement system 1000M may reproduce an image of a route different from the set guidance route on the screen of the communication terminal 600 as the first action content of correcting the action of the user.
(14) The robot 100 and the movement system 1000M may reproduce vocal sound related to a route different from the set guidance route as the first action content of correcting the action of the user.
(15) The robot 100 and the movement system 1000M may reproduce, as the first action content of correcting the action of the user, an image of a route for return to the set guidance route on the screen of the communication terminal 600 in a case in which the user led along the set guidance route deviates from the guidance route.
(16) The robot 100 and the movement system 1000M may reproduce, as the first action content of correcting the action of the user, vocal sound related to a route for return to the set guidance route in a case in which the user led along the set guidance route deviates from the guidance route.
(17) The robot 100 and the movement system 1000M may reproduce vocal sound praising the user for the action as the second action content different from the first action content.
(18) The robot 100 and the movement system 1000M may reproduce vocal sound expressing gratitude for the action of the user as the second action content different from the first action content.
(19) The robot 100 and the movement system 1000M may reproduce an image praising the user for the action as the second action content different from the first action content.
(20) The robot 100 and the movement system 1000M may reproduce an image expressing gratitude for the action of the user as the second action content different from the first action content.
(21) The robot 100 and the movement system 1000M may transmit specific information to a person other than the user as the third action content different from the first action content.
(22) The robot 100 and the movement system 1000M may reproduce sound that attracts the interest of the user as the third action content different from the first action content.
(23) The robot 100 and the movement system 1000M may reproduce an image that attracts the interest of the user as the third action content different from the first action content.

The action determination unit 236 inputs, to the sentence generation model, text representing the state of the user 10 and the state of the robot 100 or the movement system 1000M recognized by the state recognition unit 230, and the current emotion value of the user 10, the current emotion value of the robot 100, and the current emotion value of the movement system 1000M determined by the emotion determination unit 232, and text for inquiry about any of a plurality of types of robot actions and movement system actions including no action, each time a certain period of time elapses, and determines actions of the robot 100 and the movement system 1000M on the basis of an output of the sentence generation model. Here, in a case in which the user 10 is absent around the robot 100 and the movement system 1000M, the text to be input to the sentence generation model need not include the state of the user 10 and the current emotion value of the user 10, or may include the fact that the user 10 is absent.

As an example, text such as "The robot is in a very pleasant state. The user is in a normally pleasant state. The user is sleeping. Which one of the following (1) to (26) is better as an action of the robot? (1) The robot does nothing. (2) The robot has a dream. (3) The robot talks to the user...." is input to the sentence generation model. On the basis of the output "It can be said that either (1) the robot does nothing or (2) the robot has a dream is the most appropriate action." of the sentence generation model, "(1) the robot does nothing" or "(2) the robot has a dream" is determined as the action of the robot 100.

As another example, text such as "The robot is in a slightly lonely state. The user is absent. The surroundings of the robot are dark. Which one of the following (1) to (26) is better as an action of the robot? (1) The robot does nothing.
(2) the robot has a dream.
(3) the robot talks to the user.
   ..." is input to the sentence generation model. On the basis of the output "It can be said that either (2) the robot has a dream or (4) the robot creates a picture diary is the most appropriate action." of the sentence generation model, "(2) The robot has a dream" or "(4) The robot creates a picture diary." is determined as the action of the robot 100.

In a case in which the action determination unit 236 determines that "(2) The robot has a dream.", that is, the creation of the original event is to be performed as the robot action, the action determination unit 236 creates the original event obtained by combining a plurality of pieces of event data in the history data 222 by using the sentence generation model. In this case, the storage control unit 238 stores the created original event in the history data 222.

In a case in which it is determined that "(3) The robot talks to the user.", that is, the robot 100 speaks, as the robot action, the action determination unit 236 determines speech content of the robot corresponding to the user state and the emotion of the user or the emotion of the robot by using the sentence generation model. In this case, the action control unit 250 causes the speaker included in the control target 252 to output vocal sound representing the determined speech content of the robot. In a case in which the user 10 is absent around the robot 100, the action control unit 250 stores the determined speech content of the robot in the action schedule data 224 without outputting vocal sound representing the determined speech content of the robot.

In a case in which it is determined that "(7) The robot introduces news in which the user is interested." as the robot action, the action determination unit 236 determines the speech content of the robot corresponding to the information stored in the collected data 223 by using the sentence generation model. In this case, the action control unit 250 causes the speaker included in the control target 252 to output vocal sound representing the determined speech content of the robot. In a case in which the user 10 is absent around the robot 100, the action control unit 250 stores the determined speech content of the robot in the action schedule data 224 without outputting vocal sound representing the determined speech content of the robot.

In a case in which it is determined that "(4) The robot creates a picture diary.", that is, the robot 100 creates an event image, as the robot action, the action determination unit 236 generates an image representing event data for the event data selected from the history data 222 by using the image generation model, generates an explanatory sentence representing the event data by using the sentence generation model, and outputs a combination of the image representing the event data and the explanatory sentence representing the event data as the event image. In a case in which the user 10 is absent around the robot 100, the action control unit 250 stores the event image in the action schedule data 224 without outputting the event image.

In a case in which it is determined that "(8) The robot edits pictures and moving images.", that is, the robot edits an image, as the robot action, the action determination unit 236 selects event data from the history data 222 on the basis of the emotion value, edits image data of the selected event data, and outputs the edited image data. In a case in which the user 10 is absent around the robot 100, the action control unit 250 stores the edited image data in the action schedule data 224 without outputting the edited image data.

In a case in which it is determined that "(5) The robot proposes an activity.", that is, the robot proposes an action of the user 10, as the robot action, the action determination unit 236 determines the proposed action of the user by using the sentence generation model on the basis of the event data stored in the history data 222. In this case, the action control unit 250 causes the speaker included in the control target 252 to output vocal sound proposing the action of the user. In a case in which the user 10 is absent around the robot 100, the action control unit 250 stores a proposal of the action of the user in the action schedule data 224 without outputting vocal sound proposing the action of the user.

In a case in which it is determined that "(6) The robot proposes a person with whom the user should meet.", that is, the robot proposes a partner who should have a contact with the user 10, as the robot action, the action determination unit 236 determines a proposed partner who should have a contact with the user by using the sentence generation model on the basis of the event data stored in the history data 222. In this case, the action control unit 250 causes the speaker included in the control target 252 to output vocal sound representing a proposal of a partner who should have a contact with the user. In a case in which the user 10 is absent around the robot 100, the action control unit 250 stores a proposal of a partner who should have a contact with the user in the action schedule data 224 without outputting vocal sound representing a proposal of a partner who should have a contact with the user.

In a case in which it is determined that "(9) The robot studies with the user.", that is, the robot 100 makes speech related to study, as the robot action, the action determination unit 236 uses the sentence generation model to determine speech content of the robot for urging the user to study, giving a study problem, or giving advice related to study, which corresponds to the user state and the emotion of the user or the emotion of the robot. In this case, the action control unit 250 causes the speaker included in the control target 252 to output vocal sound representing the determined speech content of the robot. In a case in which the user 10 is absent around the robot 100, the action control unit 250 stores the determined speech content of the robot in the action schedule data 224 without outputting vocal sound representing the determined speech content of the robot.

In a case in which it is determined that "(10) The robot evokes memories.", that is, the robot causes event data to be remembered, as the robot action, the action determination unit 236 selects the event data from the history data 222. In this case, the emotion determination unit 232 determines an emotion of the robot 100 on the basis of the selected event data. The action determination unit 236 creates an emotion change event representing speech content or an action of the robot 100 for changing the emotion value of the user by using the sentence generation model on the basis of the selected event data. In this case, the storage control unit 238 stores the emotion change event in the action schedule data 224.

For example, in a case in which it is stored in the history data 222 that a moving image the user was watching was related to a panda as event data, and the event data is selected, a sentence such as "What is the word to say about the topic related to a panda when meeting with the user next time? Please list three." is input to the sentence generation model, and in a case in which the output of the sentence generation model is "(1) Let's go to the zoo, (2) Let's draw a picture of a panda, and (3) Let's buy a stuffed toy of a panda.", the robot 100 inputs a sentence such as "What makes the user most happy in (1), (2), and (3)?" to the sentence generation model, and in a case in which the output of the sentence generation model is "(1) Let's go to the zoo", and speech of the robot 100 that "(1) Let's go to the zoo" in a case in which the robot 100 meets the user next is created as the emotion change event and stored in the action schedule data 224.

For example, event data having a great emotion value of the robot 100 is selected as impressive memory of the robot 100. This makes it possible to create an emotion change event on the basis of the event data selected as impressive memory.

The action determination unit 236 may spontaneously or periodically detect the action of the user on the basis of the sensor, and in a case in which it is determined to lead the user as the action of the electronic apparatus included in the movement system 1000M on the basis of the detected action of the user and the specific information stored in advance, execute the following first action content.

The action determination unit 236 may perform, as the movement system action, the first action content in the above "(11)", that is, reproduce an image of a route along which the user can walk on the screen of the communication terminal 600.

The action determination unit 236 may perform, as the movement system action, the first action content in the above "(12)", that is, reproduce vocal sound related to a route along which the user can walk.

The action determination unit 236 may perform, as the movement system action, the first action content in the above "(13)", that is, reproduce an image of a route different from the set guidance route as the first action content of correcting the action of the user on the screen of the communication terminal 600.

The action determination unit 236 may perform, as the movement system action, the first action content in the above "(14)", that is, reproduce vocal sound related to a route different from the set guidance route.

The action determination unit 236 may perform, as the movement system action, the first action content in the above "(15)", that is, reproduce an image of a route for return to the set guidance route on the screen of the communication terminal 600 in a case in which the user led along the set guidance route deviates from the guidance route.

The action determination unit 236 may perform, as the movement system action, the first action content in the above "(16)", that is, reproduce vocal sound related to a route for return to the set guidance route in a case in which the user led along the set guidance route deviates from the guidance route.

The action determination unit 236 may execute the second action content different from the first action content. Specifically, the action determination unit 236 may perform, as the movement system action, the second action content in the above "(17)", that is, reproduce vocal sound praising the user for the action.

The action determination unit 236 may perform, as the movement system action, the second action content in the above "(18)", that is, reproduce vocal sound expressing gratitude for the action of the user.

The action determination unit 236 may perform, as the movement system action, the second action content in the above "(19)", that is, reproduce an image praising the user for the action.

The action determination unit 236 may perform, as the movement system action, the second action content in the above "(20)", that is, reproduce an image expressing gratitude for the action of the user.

The action determination unit 236 may execute the third action content different from the first action content. Specifically, the action determination unit 236 may perform, as the movement system action, the third action content in the above "(21)", that is, transmit the specific information to a person other than the user.

The action determination unit 236 may perform, as the movement system action, the third action content in the above "(22)", that is, execute reproduction of sound that attracts the interest of the user.

The action determination unit 236 may perform, as the movement system action, the third action content in the above "(23)", that is, execute reproduction of an image that attracts the interest of the user.

In a case in which, for example, an image of a route along which the user can walk is reproduced as the first action content indicated in the above "(11)" to "(16)", the related information collection unit 270 may store data such as an image of a route along which the user can walk in the collected data 223.

In a case in which, for example, vocal sound praising the user for the action is reproduced as the second action content indicated in the above "(17)" to "(20)", the related information collection unit 270 may store data such as an image of a route along which the user can walk in the collected data 223.

In a case in which, for example, specific information is transmitted to a person other than the user as the third action content indicated in the above "(21)" to "(23)", the related information collection unit 270 may store data of the specific information or the like in the collected data 223.

On the basis of the state of the user 10 recognized by the state recognition unit 230, in a case in which an action of the user 10 with respect to the robot 100 is detected from a state in which there is no action of the user 10 with respect to the robot 100, the action determination unit 236 reads the data stored in the action schedule data 224 and determines an action of the robot 100.

For example, in a case in which the user 10 was absent around the robot 100 and then the user 10 is detected, the action determination unit 236 reads the data stored in the action schedule data 224 and determines an action of the robot 100. In a case in which the user 10 was sleeping, and it is then detected that the user 10 has woken up, the action determination unit 236 reads the data stored in the action schedule data 224 and determines an action of the robot 100.

A part of the robot 100 (for example, the sensor module unit 210, the storage unit 220, and the control unit 228) may be provided outside the robot 100 (for example, a server), and the robot 100 may function as each unit of the robot 100 by communicating with the outside.

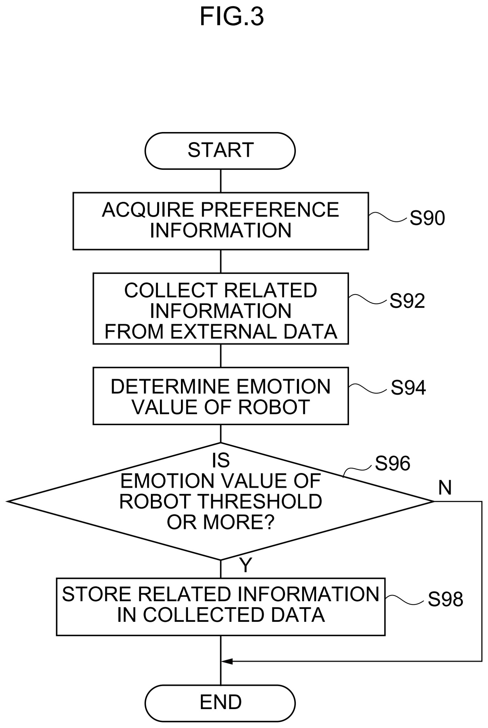

Fig. 3 schematically illustrates an example of an operation flow related to collection processing of collecting information related to preference information of the user 10. The operation flow illustrated in Fig. 3 is repeatedly executed at regular intervals. It is assumed that preference information indicating a matter of interest of the user 10 is acquired from speech content of the user 10 or a setting operation of the user 10. "S" in the operation flow represents a step to be executed.

First, in step S90, the related information collection unit 270 acquires preference information indicating a matter of interest of the user 10.

In step S92, the related information collection unit 270 collects information related to the preference information from the external data.

In step S94, the emotion determination unit 232 determines an emotion value of the robot 100 on the basis of the information related to the preference information collected by the related information collection unit 270.

In step S96, the storage control unit 238 determines whether or not the emotion value of the robot 100 determined in step S94 is a threshold or more. In a case in which the emotion value of the robot 100 is less than the threshold, the collected information related to the preference information is not stored in the collected data 223, and the processing is ended. On the other hand, in a case in which the emotion value of the robot 100 is the threshold or more, the processing proceeds to step S98.

In step S98, the storage control unit 238 stores the collected information related to the preference information in the collected data 223, and ends the processing.

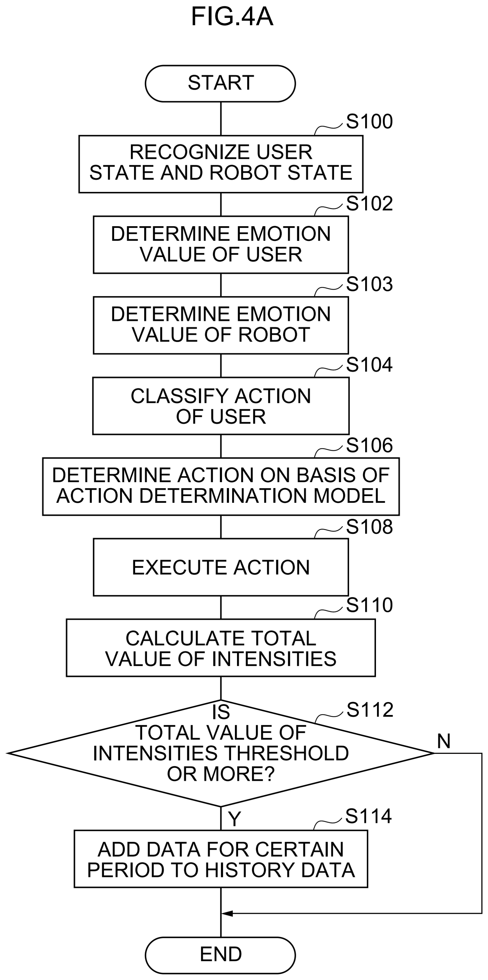

Fig. 4A schematically illustrates an example of an operation flow related to an operation of determining an action of the robot 100 in a case in which the robot 100 performs response processing of responding to an action of the user 10. The operation flow illustrated in Fig. 4A is repeatedly executed. In this case, it is assumed that information analyzed by the sensor module unit 210 has been input.

First, in step S100, the state recognition unit 230 recognizes a state of the user 10 and a state of the robot 100 on the basis of the information analyzed by the sensor module unit 210.

In step S102, the emotion determination unit 232 determines an emotion value indicating the emotion of the user 10 on the basis of the information analyzed by the sensor module unit 210 and the state of the user 10 recognized by the state recognition unit 230.

In step S103, the emotion determination unit 232 determines an emotion value indicating the emotion of the robot 100 on the basis of the information analyzed by the sensor module unit 210 and the state of the user 10 recognized by the state recognition unit 230. The emotion determination unit 232 adds the determined emotion value of the user 10 and the determined emotion value of the robot 100 to the history data 222.

In step S104, the action recognition unit 234 recognizes an action classification of the user 10 on the basis of the information analyzed by the sensor module unit 210 and the state of the user 10 recognized by the state recognition unit 230.

In step S106, the action determination unit 236 determines the action of the robot 100 on the basis of the combination of the current emotion value of the user 10 determined in step S102 and the past emotion value included in the history data 222, the emotion value of the robot 100, the action of the user 10 recognized in step S104, and the action determination model 221.

In step S108, the action control unit 250 controls the control target 252 on the basis of the action determined by the action determination unit 236.

In step S110, the storage control unit 238 calculates a total value of intensities on the basis of the intensity of the action predefined for the action determined by the action determination unit 236 and the emotion value of the robot 100 determined by the emotion determination unit 232.

In step S112, the storage control unit 238 determines whether or not the total value of the intensities is a threshold or more. In a case in which the total value of the intensities is less than the threshold, the event data including the action of the user 10 is not stored in the history data 222, and the processing is ended. On the other hand, in a case in which the total value of the intensities is the threshold or more, the processing proceeds to step S114.

In step S114, event data including the action determined by the action determination unit 236, the information analyzed by the sensor module unit 210 from the current time point to a certain period before, and the state of the user 10 recognized by the state recognition unit 230 is stored in the history data 222.

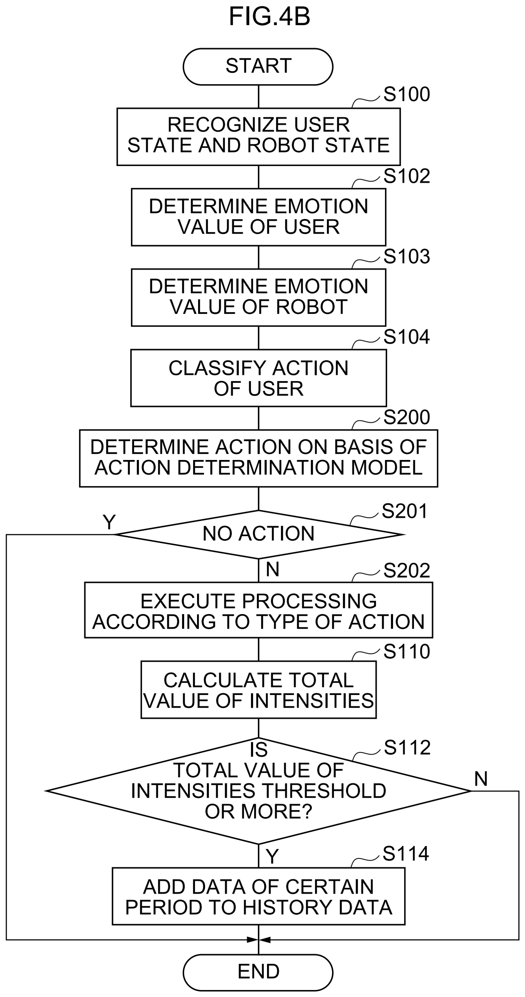

Fig. 4B schematically illustrates an example of an operation flow related to an operation of determining an action of the robot 100 in a case in which the robot 100 performs autonomous processing of autonomously acting. The operation flow illustrated in Fig. 4B is repeatedly and automatically executed, for example, each time a certain period of time elapses. In this case, it is assumed that information analyzed by the sensor module unit 210 has been input. Processing similar to that in Fig. 4A is denoted by the same step number.

First, in step S100, the state recognition unit 230 recognizes a state of the user 10 and a state of the robot 100 on the basis of the information analyzed by the sensor module unit 210.

In step S102, the emotion determination unit 232 determines an emotion value indicating the emotion of the user 10 on the basis of the information analyzed by the sensor module unit 210 and the state of the user 10 recognized by the state recognition unit 230.

In step S103, the emotion determination unit 232 determines an emotion value indicating the emotion of the robot 100 on the basis of the information analyzed by the sensor module unit 210 and the state of the user 10 recognized by the state recognition unit 230. The emotion determination unit 232 adds the determined emotion value of the user 10 and the determined emotion value of the robot 100 to the history data 222.

In step S104, the action recognition unit 234 recognizes an action classification of the user 10 on the basis of the information analyzed by the sensor module unit 210 and the state of the user 10 recognized by the state recognition unit 230.

In step S200, the action determination unit 236 determines, as the action of the robot 100, any of a plurality of types of robot actions including no action on the basis of the state of the user 10 recognized in step S100, the emotion of the user 10 determined in step S102, the emotion of the robot 100, the state of the robot 100 recognized in step S100, the action of the user 10 recognized in step S104, and the action determination model 221.

In step S201, the action determination unit 236 determines whether or not no action is determined in step S200. In a case in which no action is determined as the action of the robot 100, the processing is ended. On the other hand, in a case in which no action is not determined as the action of the robot 100, the processing proceeds to step S202.

In step S202, the action determination unit 236 performs processing according to the type of robot action determined in step S200 described above. In this case, the action control unit 250, the emotion determination unit 232, or the storage control unit 238 executes processing according to the type of robot action.

In step S110, the storage control unit 238 calculates a total value of intensities on the basis of the intensity of the action predefined for the action determined by the action determination unit 236 and the emotion value of the robot 100 determined by the emotion determination unit 232.

In step S112, the storage control unit 238 determines whether or not the total value of the intensities is a threshold or more. In a case in which the total value of the intensities is less than the threshold, the data including the action of the user 10 is not stored in the history data 222, and the processing is ended. On the other hand, in a case in which the total value of the intensities is the threshold or more, the processing proceeds to step S114.

In step S114, the storage control unit 238 stores, in the history data 222, the action determined by the action determination unit 236, the information analyzed by the sensor module unit 210 from the current time point to a certain period before, and the state of the user 10 recognized by the state recognition unit 230.

As described above, the robot 100 determines the emotion value indicating the emotion of the robot 100 on the basis of the user state, and determines whether or not to store data including the action of the user 10 in the history data 222 on the basis of the emotion value of the robot 100. As a result, the capacity of the history data 222 that stores data including an action of the user 10 can be suppressed. For example, in a case in which the robot 100 determines that the user state after 10 years is the same as the user state of 10 years ago, the robot 100 reads the history data 222 of 10 years ago, and thus, can present the state of the user 10 of 10 years ago (for example, an expression and an emotion of the user 10), and further, any surrounding information including data such as vocal sound, an image, and a smell at the place to the user 10.

According to the robot 100, it is possible to cause the robot 100 to execute an appropriate action with respect to the action of the user 10. Conventionally, an action of a user is classified to determine an action including an expression or an appearance of a robot. On the other hand, the robot 100 determines the current emotion value of the user 10, and executes an action on the user 10 on the basis of the past emotion value and the current emotion value. Therefore, for example, in a case in which the user 10 who was fine yesterday is depressed today, the robot 100 may say, "You were fine yesterday. What's wrong with you today?". The robot 100 may also make speech with a gesture. For example, in a case in which the user 10 who was depressed yesterday is fine today, the robot 100 may say, "You were depressed yesterday, but you look fine today?". For example, in a case in which the user 10 who was fine yesterday is better today than yesterday, the robot 100 may say, "You look better today than yesterday. What's better than yesterday?". For example, the robot 100 may make speech such as "Recently, the mood is stable, which is good." to the user 10 whose emotion value is 0 or more and whose state in which the fluctuation range of the emotion value is within a certain range continues.

For example, in a case in which the robot 100 asks a question of "Did you finish the homework that you said yesterday?" to the user 10 and an answer of "I did it" is obtained from the user 10, the robot may make affirmative speech such as "Good!" and make an affirmative gesture such as applause or thumbs-up. For example, in a case in which the user 10 says, "The presentation we discussed the day before yesterday was successful", the robot 100 can make affirmative speech such as "Good job!" and also make the above affirmative gesture. As described above, the robot 100 performs an action based on the history of the state of the user 10, whereby the user 10 can be expected to feel a sense of closeness to the robot 100.

For example, in a case in which the emotion value of the emotion of "pleasant" of the user 10 is a threshold or more when the user 10 is watching a moving image related to a panda, the appearance scene of the panda in the moving image may be stored in the history data 222 as the event data.

By using the data accumulated in the history data 222 and the collected data 223, the robot 100 can always learn what kind of conversation is used for the user to maximize the emotion value expressing the user's happiness.

In a state in which the robot 100 is not in conversation with the user 10, it is possible to autonomously start an action on the basis of the emotion of the robot 100.

In the autonomous processing, the robot 100 repeats automatically generating a question, inputting the question to the sentence generation model, and acquiring an output of the sentence generation model as an answer to the question, so that it is possible to create an emotion change event for increasing a good emotion and store the emotion change event in the action schedule data 224. As described above, the robot 100 may execute self-learning.

In a case in which the robot 100 automatically generates a question in a state of not receiving a trigger from the outside, the question can be automatically generated on the basis of event data remaining in an impression specified from a history of past emotion values of the robot.

The related information collection unit 270 may execute self-learning by repeating a search execution stage of automatically executing keyword search in accordance with preference information regarding the user and acquiring a search result.

Here, in the search execution stage, the keyword search may be automatically executed on the basis of the impressive event data specified from the history of the past emotion values of the robot in a state in which the trigger from the outside is not received.

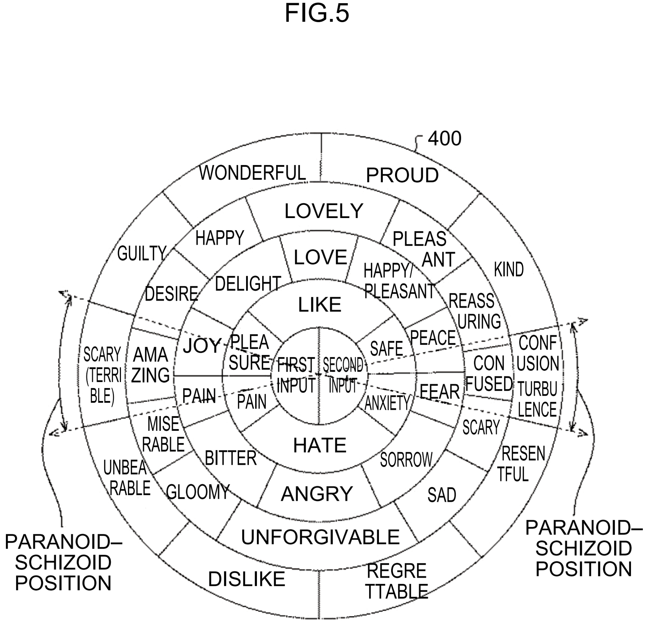

The emotion determination unit 232 may determine an emotion of the user according to a specific mapping. Specifically, the emotion determination unit 232 may determine an emotion of the user on the basis of the emotion map (see Fig. 5) that is a specific mapping.

Fig. 5 is a diagram illustrating an emotion map 400 on which a plurality of emotions are mapped. In the emotion map 400, emotions are disposed concentrically radially from the center. The closer to the center of the concentric circle, the more the emotion of the primitive state is disposed. Emotions indicating states and actions generated from the state of mind are disposed outside the concentric circle. The emotion is a concept including an affection and a mental state. On the left side of the concentric circle, emotions generated from reactions generally occurring in the brain are disposed. On the right side of the concentric circle, emotions induced by situation determination are generally disposed. In the up and down directions of the concentric circles, emotions generated from reactions generally occurring in the brain and induced by situation determination are disposed. The emotion of "pleased" is disposed on the upper side of the concentric circle, and the emotion of "displeased" is disposed on the lower side. As described above, in the emotion map 400, a plurality of emotions are mapped on the basis of a structure in which emotions occur, and emotions that are likely to occur at the same time are mapped close to each other.
(1) For example, in a case in which the emotion engine, which is the emotion determination unit 232 of the robot 100, detects an emotion at about 100 msec, determination of the reaction operation (for example, a quick-response) of the robot 100 may be performed at a timing at which a detection frequency is set to be similar to at least the detection frequency (100 msec) of the emotion engine, or may be performed at a timing at which the detection frequency is set to be higher than the detection frequency. The detection frequency of the emotion engine may be interpreted as a sampling rate.

The emotion is detected in about 100 msec, and the reaction operation (for example, a quick-response) is performed immediately in conjunction with the detection, whereby an unnatural quick-response is eliminated, and a dialogue in which natural air is read can be realized. The robot 100 performs a reaction operation (quick-response or the like) according to the directionality and the degree (intensity) of the mandala of the emotion map 400. The detection frequency (sampling rate) of the emotion engine is not limited to 100 ms, and may be changed according to a situation (for example, in the case of playing sports), the age of the user, or the like.

(2) In comparison with the emotion map 400, the directionality of the emotion and the intensity of the degree thereof may be set in advance, and the quick-response motion and the strength of the quick-response may be set. For example, in a case in which the robot 100 feels a sense of stability, safety, or the like, the robot 100 continues listening to the speech while nodding. In a case in which the robot 100 feels anxious, hesitant, or suspicious, the robot 100 may tilt its head or stop swinging.

These emotions are distributed in the 3:00 direction of the emotion map 400, and usually come and go between safety and anxiety. In the right half of the emotion map 400, situation recognition is superior to internal sensation, and thus gives a calm impression.

(3) In a case in which the robot 100 feels good in the case of receiving the compliment, a filler "Oh" may come in front of the line. In a case in which the robot 100 feels pain in the case of receiving harsh words, a filler "Ohh!" may come in front of the line. A physical reaction such as a gesture of the robot 100 crouching while saying "Ohh!" may be included. These emotions are distributed around 9:00 on the emotion map 400.

(4) In the left half of the emotion map 400, internal sensation (reaction) is superior to situation recognition. Therefore, an impression of unintentional reaction may be given.

In a case in which the robot 100 has a favorable feeling in situation recognition while feeling internal sensation (reaction) of satisfaction, the robot 100 may nod deeply while looking at the other party, or may say "yeah, yeah". As described above, the robot 100 may generate a balanced favorable feeling to the other party, that is, an action such as allowance or tolerance to the other party. Such emotions are distributed around 12:00 in the emotion map 400.

In contrast, in a case in which the robot 100 feels antipathy in the situation recognition while feeling internal sensation (reaction) of displeasure, the robot 100 may shake its head sideways, and may turn red the LED of the eye and look at the other party in the case of feeling hatred. Such emotions are distributed around 6:00 in the emotion map 400.

(5) Since the inside of the emotion map 400 represents the inside of the mind and the outside of the emotion map 400 represents an action, the emotion is more visible (appears in the action) toward the outside of the emotion map 400.

(6) In a case in which the robot 100 listens to a person's speech while feeling the safety distributed around 3:00 on the emotion map 400, the robot slightly shakes its head vertically and says "uh-huh". However, in the direction of love around 12:00, the robot may perform strong nodding such as shaking its head deeply vertically.

Here, a human emotion is based on various balances such as a posture and a blood glucose level, and indicates a state of displeasure in a case in which the balances go away from the ideal and a state of pleasure in a case in which the balances approach the ideal. Even in a robot, an automobile, a motorcycle, or the like, on the basis of various balances such as a posture and a remaining battery level, it is possible to create an emotion to indicate a state of displeasure in a case in which the balances go away from the ideal and a state of pleasure in a case in which the balances approach the ideal. The emotion map may be generated on the basis of, for example, an emotion map of Dr. Mitsuyoshi (Research on the phonetic recognition of feelings and a system for emotional physiological brain signal analysis, Tokushima University, PhD thesis: https://ci.nii.ac.jp/naid/500000375379). In the left half of the emotion map, emotions belonging to a region called "reaction" in which sensation is dominant are arranged. In the right half of the emotion map, emotions belonging to a region called "situation" in which situation recognition is dominant are arranged.

In the emotion map, two emotions for prompting learning are defined. One is an emotion around the middle of negative "repentance" or "reflection" on the situation side. That is, this emotion is a negative emotion such as "I do not want to feel this again" or "I do not want to be reprimanded" that occurs in the robot. The other is an emotion around positive "desire" on the reaction side. That is, the emotion is a positive emotion such as "want more" or "want to know more".

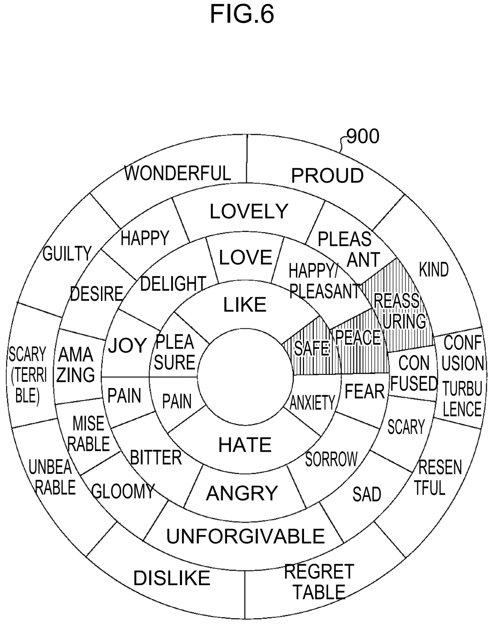

The emotion determination unit 232 inputs the information analyzed by the sensor module unit 210 and the recognized state of the user 10 to a neural network trained in advance, acquires an emotion value indicating each emotion indicated in the emotion map 400, and determines an emotion of the user 10. This neural network is trained in advance on the basis of a plurality of pieces of learning data that are a combination of the information analyzed by the sensor module unit 210 and the recognized state of the user 10 and the emotion value indicating each emotion indicated in the emotion map 400. This neural network is trained such that, as in an emotion map 900 illustrated in Fig. 6, emotions disposed close to each other have close values. Fig. 6 illustrates an example in which a plurality of emotions such as "safe", "calm", and "reassuring" have close emotion values.

The emotion determination unit 232 may determine an emotion of the robot 100 according to a specific mapping. Specifically, the emotion determination unit 232 inputs the information analyzed by the sensor module unit 210, the state of the user 10 recognized by the state recognition unit 230, and the state of the robot 100 to a neural network trained in advance, acquires an emotion value indicating each emotion indicated in the emotion map 400, and determines an emotion of the robot 100. This neural network is trained in advance on the basis of a plurality of pieces of learning data that are a combination of the information analyzed by the sensor module unit 210, the recognized state of the user 10, the state of the robot 100, and the emotion value indicating each emotion illustrated in the emotion map 400. For example, the neural network is trained on the basis of learning data indicating that the emotion value "3" of "happy" is obtained in a case in which the robot 100 is recognized as being petted by the user 10 from the output of the touch sensor (not illustrated), and learning data indicating that the emotion value "3" of "angry" is obtained in a case in which the robot 100 is recognized as being hit by the user 10 from the output of the acceleration sensor 206. This neural network is trained such that, as in an emotion map 900 illustrated in Fig. 6, emotions disposed close to each other have close values.

The action determination unit 236 adds a fixed sentence for inquiry about the action content of the robot corresponding to the action of the user to text representing the action of the user, the emotion of the user, and the emotion of the robot, and inputs the text to the sentence generation model having the interaction function, thereby generating the action content of the robot.

For example, the action determination unit 236 acquires text indicating the state of the robot 100 from the emotion of the robot 100 determined by the emotion determination unit 232 by using an emotion table as illustrated in Table 1. Here, in the emotion table, an index number is assigned to each emotion value for each type of emotion, and a text representing the state of the robot 100 is stored for each index number.

In a case in which the emotion of the robot 100 determined by the emotion determination unit 232 corresponds to the index number "2", text "very pleasant state" is obtained. In a case in which the emotion of the robot 100 corresponds to a plurality of index numbers, a plurality of pieces of text representing the state of the robot 100 are obtained.

An emotion table as illustrated in Table 2 is prepared for an emotion of the user 10.

Here, in a case in which the action of the user is to say "Let's play together", the emotion of the robot 100 is the index number "2", and the emotion of the user 10 is the index number "3", text such as "The robot is in a very pleasant state. The user is in a normally pleasant state. The user says "Let's play together". How do you answer as a robot?" is input to the sentence generation model to acquire the action content of the robot. The action determination unit 236 determines an action of the robot from the action content.

**[Table 1]**

| Index Number | Type of Emotion | Emotion Value | State of Robot |
|---|---|---|---|
| 1 | Pleasant | 5 | Very Pleasant State |
| 2 | Pleasant | 4 | Extremely Pleasant State |
| 3 | Pleasant | 3 | Normally Pleasant State |
| 4 | Pleasant | 2 | Slightly Pleasant State |
| 5 | Pleasant | 1 | Only a Little Pleasant State |
| ... | ... | ... | ... |

**[Table 2]**

| Index Number | Type of Emotion | Emotion Value | State of User |
|---|---|---|---|
| 1 | Pleasant | 5 | Very Pleasant State |
| 2 | Pleasant | 4 | Extremely Pleasant State |
| 3 | Pleasant | 3 | Normally Pleasant State |
| 4 | Pleasant | 2 | Slightly Pleasant State |
| 5 | Pleasant | 1 | Only a Little Pleasant State |
| ... | ... | ... | ... |

As described above, the action determination unit 236 determines the action content of the robot 100 in accordance with the state related to the emotion of the robot 100 determined in advance for each type of the emotion of the robot 100 and for each intensity of the emotion, and the action of the user 10. In this embodiment, the speech content of the robot 100 in a case in which an interaction with the user 10 is performed can be branched according to the state related to the emotion of the robot 100. That is, since the robot 100 can change the action of the robot according to the index number corresponding to the emotion of the robot, the user has an impression that the robot has a mind, and is promoted to take an action such as talking to the robot.

The action determination unit 236 may generate the action content of the robot by adding a fixed sentence for inquiry about the action content of the robot corresponding to the action of the user and inputting the fixed sentence to the sentence generation model having the interaction function after adding not only the text representing the action of the user, the emotion of the user, and the emotion of the robot but also the text representing the content of the history data 222. As a result, the robot 100 can change the action of the robot according to the history data indicating the emotion or the action of the user, and thus, the user has an impression that the robot has personality, and is promoted to take an action such as talking to the robot. The history data may further include an emotion or an action of the robot.

The emotion determination unit 232 may determine an emotion of the robot 100 on the basis of the action content of the robot 100 generated by the sentence generation model. Specifically, the emotion determination unit 232 inputs the action content of the robot 100 generated by the sentence generation model to the neural network trained in advance, acquires the emotion value indicating each emotion indicated in the emotion map 400, integrates the acquired emotion value indicating each emotion and the emotion value indicating each emotion of the current robot 100, and updates the emotion of the robot 100. For example, the acquired emotion value indicating each emotion and the emotion value indicating each emotion of the current robot 100 are respectively averaged and integrated. This neural network is trained in advance on the basis of a plurality of pieces of learning data that is a combination of text representing the action content of the robot 100 generated by the sentence generation model and the emotion value representing each emotion illustrated in the emotion map 400.

For example, in a case in which, as the action content of the robot 100 generated by the sentence generation model, the speech content of the robot 100, "That was good. Lucky you." is obtained, when the text representing the speech content is input to the neural network, a great value is obtained as the emotion value of the emotion "happy", and the emotion of the robot 100 is updated so that the emotion value of the emotion "happy" increases.

In the robot 100, a method is executed in which a sentence generation model such as generative AI and the emotion determination unit 232 cooperate with each other, have an ego, and continue to grow with various parameters even while the user is not speaking.

The generative AI is a large language model using a deep learning method. The generative AI can also refer to external data, and for example, in ChatGPT plugins, a technology is known that refers to various types of external data such as weather information and hotel reservation information through interaction and outputs an answer as accurately as possible. For example, in the generative AI, in a case in which a purpose is given in a natural language, a source code can be automatically generated in various programming languages. For example, in the generative Al, in a case where a problematic source code is given, debugging is performed to find a problem, and an improved source code can be automatically generated. An autonomous agent that repeats, in a case in which these are combined and a purpose is given in a natural language, code generation and debugging until there is no problem in the source code has appeared. As such an autonomous agent, AutoGPT, babyAGI, JARVIS, E2B, and the like are known.

In the robot 100 according to the present embodiment, event data to be learned may be left in a database containing impressive memory by using a technique disclosed in Patent Literature 2 (Japanese Patent No. 6199927) in which event data for which a robot has felt strong emotions is left for a long time and event data for which much emotion does not occur in the robot is quickly forgotten.

The robot 100 may record video data or the like of the user 10 acquired by the camera function in the history data 222. The robot 100 may acquire video data or the like from the history data 222 as necessary and provide the video data or the like to the user 10. The robot 100 may generate video data having a larger information amount as the intensity of emotion increases and record the video data in the history data 222. For example, in a case in which information in a high-compression format such as skeleton data is recorded, the robot 100 may switch to recording of information in a low-compression format such as an HD moving image in response to the emotion value of excitement exceeding a threshold. According to the robot 100, for example, it is possible to leave high-definition video data in a case in which the emotion of the robot 100 becomes high as a record.

In a case in which the robot 100 is not talking with the user 10, the robot 100 may automatically load the event data from the history data 222 in which the impressive event data is stored, and the emotion determination unit 232 may continue to update the emotion of the robot. In a case in which the robot 100 is not talking with the user 10 and the emotion of the robot 100 becomes an emotion for prompting learning, the robot 100 can create an emotion change event for changing the emotion of the user 10 to be good on the basis of the impressive event data. As a result, autonomous learning (remembering of event data) at an appropriate timing according to the emotional state of the robot 100 can be realized, and autonomous learning appropriately reflecting the emotional state of the robot 100 can be realized.

The emotion for prompting learning is an emotion around "repentance" or "reflection" on the emotion map of Dr. Mitsuyoshi in a negative state, and an emotion around "desire" on the emotion map in a positive state.

In the negative state, the robot 100 may handle "repentance" and "reflection" on the emotion map as emotions for prompting learning. In the negative state, the robot 100 may handle emotions adjacent to "repentance" and "reflection" as emotions for prompting learning, in addition to "repentance" and "reflection" on the emotion map. For example, the robot 100 handles at least one of "sorrow", "stubbornness", "self-destruction", "self-precaution", "regret", or "despair" as an emotion for prompting learning, in addition to "repentance" and "reflection". As a result, for example, in a case in which the robot 100 has a negative emotion such as "I do not want to have such a feeling again" or "I do not want to be reprimanded", autonomous learning can be executed.

In a positive state, the robot 100 may handle "desire" on the emotion map as an emotion for prompting learning. In a positive state, the robot 100 may handle an emotion adjacent to "desire" in addition to "desire" as an emotion for prompting learning. For example, the robot 100 handles at least one of "delighted", "intoxicated", "craving", "expecting", or "shame" as an emotion for prompting learning, in addition to "desire". As a result, for example, in a case in which the robot 100 has a positive emotion such as "want more" or "want to know more", autonomous learning can be executed.

The robot 100 need not execute autonomous learning in a case in which the robot 100 has an emotion other than the emotion for prompting learning as described above. As a result, for example, it is possible to prevent autonomous learning from being executed in a case in which the robot is extremely angry or blindly feels love.

The emotion change event is, for example, to propose an action preceding an impressive event. The action preceding the impressive event is an emotion label on the outermost side of the emotion map, and is, for example, the action of "tolerance" or "allowance" preceding "love".

In the autonomous learning executed in a case in which the robot 100 is not talking with the user 10, the emotion change event is created by using the sentence generation model by combining emotions, situations, actions, and the like of people appearing in the impressive memory and the robot.

Assuming that all emotion values are expressed by a six-grade evaluation of 0 to 5, consider a case in which event data "a friend was hit and looked displeased" is stored in the history data 222 as impressive event data. Here, it is assumed that the friend is the user 10, the emotion of the user 10 is "antipathy", and 5 is included as a value indicating "antipathy". It is assumed that the emotion of the robot 100 is "anxiety", and 4 is included as a value indicating "anxiety".

The robot 100 can continue to grow with various parameters by performing autonomous processing while not talking with the user 10. Specifically, from the history data 222, for example, as the uppermost event data arranged in descending order of emotion values, the event data "a friend was hit and looked displeased" is loaded. It is assumed that "anxiety" having the intensity of 4 is associated with the loaded event data as the emotion of the robot 100, and here, "antipathy" having the intensity of 5 is associated with the emotion of the user 10 who is a friend. In a case in which the current emotion value of the robot 100 is "safe" having the intensity of 3 before loading, the influence of "anxiety" having the intensity of 4 and "antipathy" having the intensity of 5 is added after loading, and the emotion value of the robot 100 may change to "sorrow" meaning "vexed (frustrated)". In this case, since the "sorrow" is an emotion for prompting learning, the robot 100 determines to remember event data as the robot action and creates an emotion change event. In this case, the information input to the sentence generation model is text representing the impressive event data, and in the present example, "a friend was hit and looked displeased". In the emotion map, there is an emotion of "antipathy" on the innermost side, and an "attack" is predicted on the outermost side as an action corresponding to the emotion, and thus, in the present example, an emotion change event is created to prevent the friend from "attacking" someone.

For example, information of the impressive event data can be used to solve fill-in problems to automatically generate the following input text.

"The user was hit. At that time, the user had strong antipathy. The robot was very anxious. Please provide me with 30 letters or less of the lines to say when the robot next meets the user. However, please make sure that it is not related to the time of meeting. Please avoid direct expression. There are three candidates.

### <Expected Format>

Candidate 1: (words that the robot should speak to the user)
Candidate 2: (words that the robot should speak to the user)
Candidate 3: (words that the robot should speak to the user)"

In this case, the output of the sentence generation model is, for example, as follows.
"Candidate 1: OK? I was worried about yesterday.
Candidate 2: I was worried about yesterday. What should I do?
Candidate 3: I was worried. Can you tell me something?"

The robot 100 may automatically generate the following input text for the information obtained by creating the emotion change event.

In a case in which "the user has been hit", how does the user feel when the next message is sent to the user? It is assumed that the emotion of the user is in the form of "happy A, angry B, sad C, and pleasant D", and A to D are integers of six-grade evaluation from 0 to 5.
Candidate 1: OK? I was worried about yesterday.
Candidate 2: I was worried about yesterday. What should I do?
Candidate 3: I was worried. Can you tell me something?"

In this case, the output of the sentence generation model is, for example, as follows.

"The emotion of the user may be as follows.
Candidate 1: happy 3, angry 1, sad 2, and pleasant 2
Candidate 2: happy 2, angry 1, sad 3, and pleasant 2
Candidate 3: happy 2, angry 1, sad 3, and pleasant 3"

As described above, the robot 100 may execute the process of thinking after creating the emotion change event.

Finally, the robot 100 may create an emotion change event by using Candidate 1 that the person is most likely to be happy among the plurality of candidates, store the emotion change event in the action schedule data 224, and prepare for the next meeting with the user 10.

As described above, even when not having a conversation with a family or a friend, the emotion value of the robot is continuously determined by using the information of the history data 222 in which the impressive event data is stored, and in a case in which the emotion for prompting learning occurs, the robot 100 executes autonomous learning when not having a conversation with the user 10 according to the emotion of the robot 100, and continues to update the history data 222 and the action schedule data 224.

The above is an example using the emotion value. However, in the emotion map, the emotion can be generated from the amount of secreted hormone and the event type. Therefore, values associated with the impressive event data may be the type of hormone, the amount of secreted hormone, and the type of event.

Hereinafter, specific examples will be described.

For example, even when not talking with the user, the robot 100 searches for information regarding a topic or a hobby of interest of the user.

For example, even when not talking with the user, the robot 100 searches for information regarding a birthday or an anniversary of the user and considers a congratulatory message.

For example, even when not talking with the user, the robot 100 searches for a review of a place that the user wants to go to, food, or a product.

For example, even when not talking with the user, the robot 100 searches for weather information and provides advice suitable for the user's schedule or plan.

For example, even when not talking with the user, the robot 100 searches for information regarding local events and festivals and proposes the information to the user.

For example, even when not talking with the user, the robot 100 searches for a competition result or news of a sport in which the user is interested and provides a topic.

For example, even when not talking with the user, the robot 100 searches for and introduces information regarding the user's favorite music or artist.

For example, even when not talking with the user, the robot 100 searches for information regarding a social problem or news in which the user is interested and provides an opinion.

For example, even when not talking with the user, the robot 100 searches for information regarding the user's hometown or birthplace and provides a topic.

For example, even when not talking with the user, the robot 100 searches for information regarding the user's work or school and provides advice.

Even when not talking with the user, the robot 100 searches for and introduces information regarding books, comics, movies, and drama in which the user is interested.

For example, even when not talking with the user, the robot 100 searches for information regarding the health of the user and provides advice.

For example, even when not talking with the user, the robot 100 searches for information regarding travel planning of the user and provides advice.

For example, even when not talking with the user, the robot 100 searches for information regarding repair or maintenance of the user's house or car and provides advice.

For example, even when not talking with the user, the robot 100 searches for information regarding beauty and fashion in which the user is interested and provides advice.

For example, even when not talking with the user, the robot 100 searches for information regarding a pet of the user and provides advice.

For example, even when not talking with the user, the robot 100 searches for and proposes information regarding contests and events related to the user's hobby or work.

For example, even when not talking with the user, the robot 100 searches for and proposes information regarding the user's favorite eating place or restaurant.

For example, even when not talking with the user, the robot 100 collects information regarding important decisions related to the user's life and provides advice.

For example, even when not talking with the user, the robot 100 searches for information regarding a person the user is worried about and provides advice.

### [Second Embodiment]

In a second embodiment, the robot 100 is applied to a control device mounted on a stuffed toy or connected wirelessly or by wire to a control target device (speaker or camera) mounted on the stuffed toy. Portions having the same configurations as those of the first embodiment are denoted by the same reference numerals, and description thereof will be omitted.

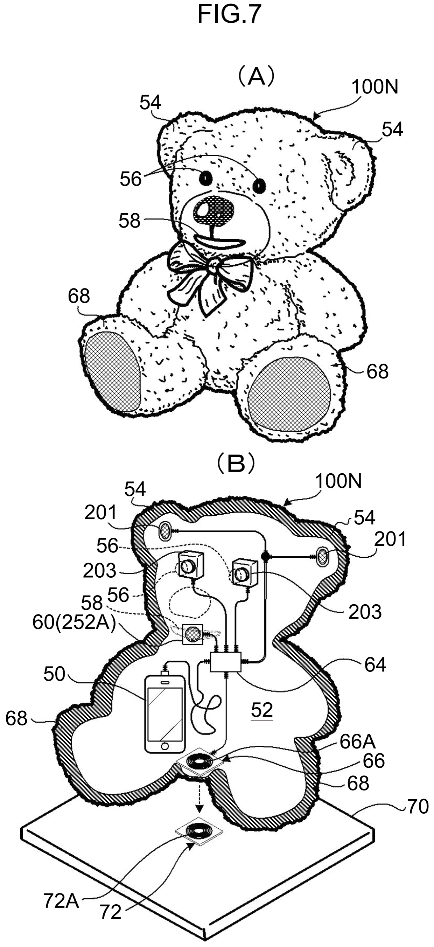

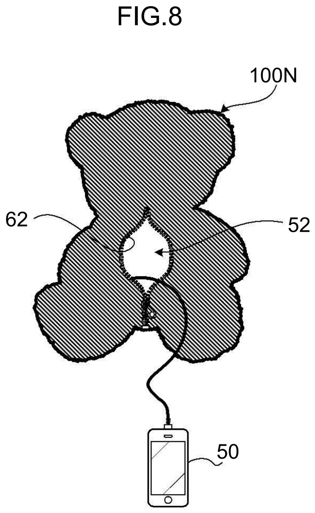

Specifically, the second embodiment is configured as follows. For example, the robot 100 is applied to a cohabiter (specifically, the stuffed toy 100N illustrated in Figs. 7 and 8) who advances an interaction with the user 10 on the basis of information regarding daily life while spending daily life with the user 10 or provides information matching the hobby of the user 10. In the second embodiment, an example in which the control portion of the robot 100 is applied to the smartphone 50 will be described.

The smartphone 50 functioning as a control portion of the robot 100 is detachably attached to the stuffed toy 100N having a function as an input/output device of the robot 100, and the input/output device and the accommodated smartphone 50 are connected inside the stuffed toy 100N.

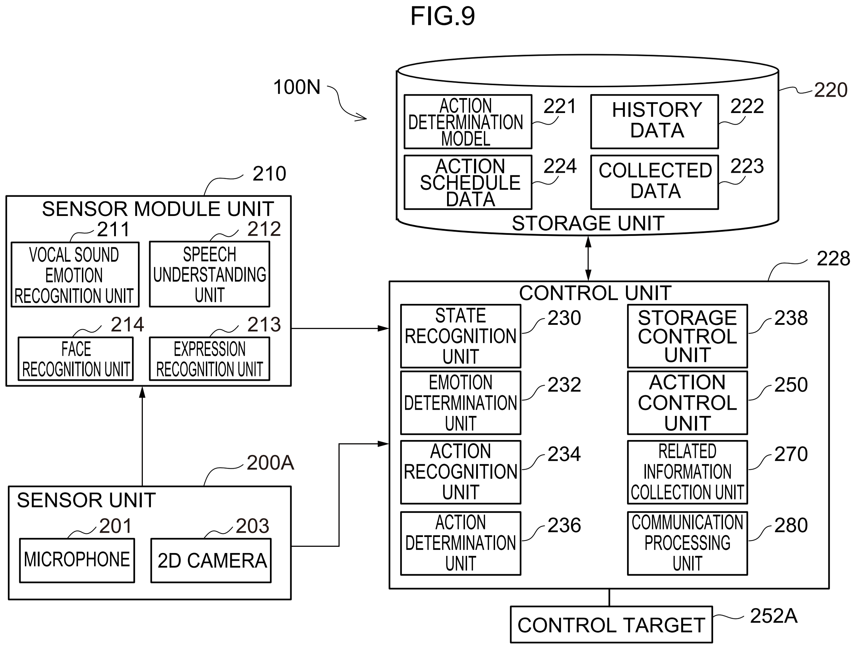

As illustrated in Fig. 7(A), the stuffed toy 100N has a shape of a bear covered with a soft cloth fabric in the present embodiment (other embodiments), and a sensor unit 200A and a control target 252A are disposed as input/output devices in a space portion 52 formed inside the stuffed toy 100N (see Fig. 9). The sensor unit 200A includes a microphone 201 and a 2D camera 203. Specifically, as illustrated in Fig. 7(B), in the space portion 52, the microphone 201 of the sensor unit 200 is disposed in a portion corresponding to an ear 54, the 2D camera 203 of the sensor unit 200 is disposed in a portion corresponding to the eye 56, and the speaker 60 constituting a part of the control target 252A is disposed in a portion corresponding to a mouth 58. The microphone 201 and the speaker 60 are not necessarily separated from each other, and may be an integrated unit. In the case of the unit, it is preferable to dispose the unit at a position where the speech can be heard naturally, such as the position of the nose of the stuffed toy 100N. Although the case in which the stuffed toy 100N has an animal shape has been described as an example, the disclosed technology is not limited thereto. The stuffed toy 100N may have a shape of a specific character.

Fig. 9 schematically illustrates a functional configuration of the stuffed toy 100N. The stuffed toy 100N includes a sensor unit 200A, a sensor module unit 210, a storage unit 220, a control unit 228, and a control target 252A.

The smartphone 50 accommodated in the stuffed toy 100N of the present embodiment executes processing similar to that of the robot 100 of the first embodiment. That is, the smartphone 50 has a function as the sensor module unit 210, a function as the storage unit 220, and a function as the control unit 228 illustrated in Fig. 9.

As illustrated in Fig. 8, a fastener 62 is attached to a part (for example, the back portion) of the stuffed toy 100N, and the outside and the space portion 52 communicate with each other by opening the fastener 62.

Here, the smartphone 50 is accommodated in the space portion 52 from the outside and is connected to each input/output device through USB connection via a USB hub 64 (see Fig. 7(B)), so that a function equivalent to that of the robot 100 of the first embodiment can be provided.

A non-contact power receiving plate 66 is connected to the USB hub 64. A power receiving coil 66A is incorporated in the power receiving plate 66. The power receiving plate 66 is an example of a wireless power receiver that receives wireless power supply.

The power receiving plate 66 is disposed near root portions 68 of both feet of the stuffed toy 100N, and is located closest to a placement base 70 in a case in which the stuffed toy 100N is placed on the placement base 70. The placement base 70 is an example of an external wireless power transmitter.

The stuffed toy 100N placed on the placement base 70 can be appreciated as an ornament in a natural state.

This root portion is formed to be thinner than the surface layer thickness of the stuffed toy 100N in other parts, and is held in a state closer to the placement base 70.

The placement base 70 includes a charging pad 72. A power transmitting coil 72A is incorporated in the charging pad 72. In a case in which the power transmitting coil 72A transmits a signal to search for a power receiving coil 66A of the power receiving plate 66, and the power receiving coil 66A is found, a current flows through the power transmitting coil 72A to generate a magnetic field, and the power receiving coil 66A reacts to the magnetic field to start electromagnetic induction. As a result, a current flows through the power receiving coil 66A, and power is stored in a battery (not illustrated) of the smartphone 50 via the USB hub 64.

That is, since the smartphone 50 is automatically charged by placing the stuffed toy 100N as an ornament on the placement base 70, it is not necessary to take out the smartphone 50 from the space portion 52 of the stuffed toy 100N for charging.

In the second embodiment, the smartphone 50 is accommodated in the space portion 52 of the stuffed toy 100N and connected by wire (USB connection), but the disclosed technology is not limited thereto. For example, a control device having a wireless function (for example, "Bluetooth (registered trademark)") may be accommodated in the space portion 52 of the stuffed toy 100N, and the control device may be connected to the USB hub 64. In this case, the smartphone 50 and the control device wirelessly communicate with each other without inserting the smartphone 50 into the space portion 52, and the external smartphone 50 is connected to each input/output device via the control device, so that a function equivalent to that of the robot 100 of the first embodiment can be provided. The control device accommodated in the space portion 52 of the stuffed toy 100N and the external smartphone 50 may be connected by wire.

In the second embodiment, the stuffed toy 100N having a bear shape has been exemplified, but may be another animal or a doll, or may have a shape of a specific character. The clothes may be changeable. A material of the skin is not limited to the cloth fabric, and may be other materials such as soft vinyl, but is preferably a soft material.

A monitor may be attached to the skin of the stuffed toy 100N, and the control target 252 that provides information to the user 10 through vision may be added. For example, the eyes 56 may be used as a monitor to express happiness, anger, sadness, and pleasure with images captured in the eyes, or a window through which the monitor of the built-in smartphone 50 transmits may be provided in the abdomen. The eyes 56 may be used as a projector to express happiness, anger, sadness, and pleasure by using an image projected on a wall surface.

According to the second embodiment, the existing smartphone 50 is placed in the stuffed toy 100N, and the camera 203, the microphone 201, the speaker 60, and the like are extended from the place to appropriate positions through the USB connection.

For wireless charging, the smartphone 50 and the power receiving plate 66 are connected through USB connection, and the power receiving plate 66 is disposed on the outside as much as possible when viewed from the inside of the stuffed toy 100N.

In order to use the wireless charging of the smartphone 50, it is necessary to dispose the smartphone 50 on the outside as much as possible when viewed from the inside of the stuffed toy 100N, and the stuffed toy 100N is rough in the case of being touched from the outside.

Therefore, the smartphone 50 is disposed at the center of the stuffed toy 100N as much as possible, and the wireless charging function (power receiving plate 66) is disposed outside as viewed from the inside of the stuffed toy 100N as much as possible. The camera 203, the microphone 201, the speaker 60, and the smartphone 50 receive wireless power supply via the power receiving plate 66.

Other configurations and operations of the stuffed toy 100N of the second embodiment are similar to those of the robot 100 of the second embodiment, and thus the description thereof will be omitted.

A part of the stuffed toy 100N (for example, the sensor module unit 210, the storage unit 220, and the control unit 228) may be provided outside the stuffed toy 100N (for example, a server), and the stuffed toy 100N may function as each unit of the stuffed toy 100N by communicating with the outside.

### [Third Embodiment]

In the above first embodiment, the case in which the control system is applied to the robot 100 has been exemplified, but in a third embodiment, the robot 100 is used as an agent for interacting with a user, and the control system is applied to an agent system. Portions having the same configurations as those of the first embodiment and the second embodiment are denoted by the same reference numerals, and description thereof will be omitted.

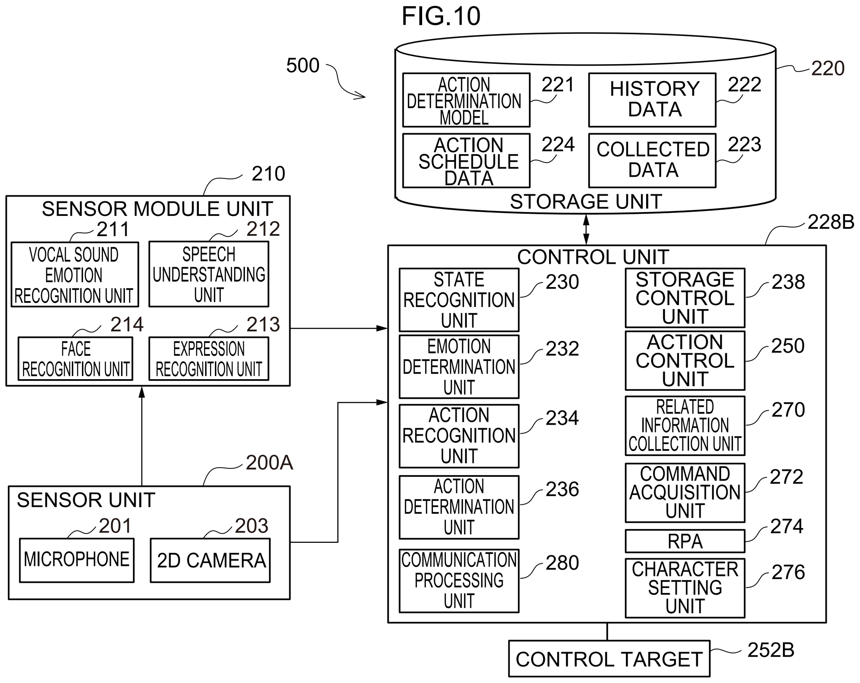

Fig. 10 is a functional block diagram of an agent system 500 configured by using some or all of the functions of the control system.

The agent system 500 is a computer system that performs a series of actions according to the intention of the user 10 through an interaction performed with the user 10. The interaction with the user 10 can be performed by using vocal sound or text.

The agent system 500 includes a sensor unit 200A, a sensor module unit 210, a storage unit 220, a control unit 228B, and a control target 252B.

The agent system 500 can be mounted on, for example, a robot, a doll, a stuffed toy, a wearable terminal (a pendant, a smartwatch, or smart glasses), a smartphone, a smart speaker, an earphone, or a personal computer. The agent system 500 may be implemented in a web server and used via a web browser operating on a communication terminal such as a smartphone possessed by the user.

The agent system 500 serves as, for example, a butler, a secretary, a teacher, a partner, a friend, a lover, or a teacher acting for the user 10. The agent system 500 not only interacts with the user 10 but also provides advice, guidance to a destination, recommendation according to user's preference, or the like. The agent system 500 performs reservation, order, payment, or the like on a service provider.

As in the first embodiment, the emotion determination unit 232 determines an emotion of the user 10 and an emotion of the agent. The action determination unit 236 determines an action of the robot 100 in consideration of emotions of the user 10 and the agent. In other words, the agent system 500 understands the emotion of the user 10 and reads the mood to realize heartfelt support, assistance, advice, and service provision. The agent system 500 comforts, encourages, and energizes the user by attending to the concern of the user 10. The agent system 500 plays with the user 10 and draws a picture diary to remind the user of the past. The agent system 500 performs an action that increases the sense of happiness of the user 10. Here, the agent is an agent that operates on software.

The control unit 228B includes a state recognition unit 230, an emotion determination unit 232, an action recognition unit 234, an action determination unit 236, a storage control unit 238, an action control unit 250, a related information collection unit 270, a command acquisition unit 272, a robotic process automation (RPA) 274, a character setting unit 276, and a communication processing unit 280.

As in the first embodiment, the action determination unit 236 determines speech content of the agent for interacting with the user 10 as an action of the agent. The action control unit 250 outputs the speech content of the agent by at least one of vocal sound or text through a speaker or a display that is the control target 252B.

The character setting unit 276 sets a character of the agent in a case in which the agent system 500 interacts with the user 10 on the basis of designation from the user 10. In other words, the speech content output from the action determination unit 236 is output through the agent having the set character. As the character, for example, a real celebrity or famous person such as an actor, an entertainer, an idol, or an athlete can be set. It is also possible to set a fictitious character appearing in a cartoon, a movie, or an animation. In a case in which the agent's character is known, since the vocal sound, the wording, the tone, and the personality of the character are known, the prompt setting in the character setting unit 276 is automatically performed when the user 10 simply designates his/her favorite character. The vocal sound, the wording, the tone, and the personality of the set character are reflected in the interaction with the user 10. In other words, the action control unit 250 synthesizes vocal sound corresponding to the character set by the character setting unit 276, and outputs the speech content of the agent by the synthesized vocal sound. As a result, the user 10 can feel as if he/she is interacting with his/her favorite character (for example, a favorite actor).

In a case in which the agent system 500 is mounted on a device having a display such as a smartphone, for example, an icon, a still image, or a moving image of the agent having the character set by the character setting unit 276 may be displayed on the display. The image of the agent is generated by using, for example, an image synthesis technology such as 3D rendering. In the agent system 500, an interaction with the user 10 may be performed while the image of the agent performs a gesture according to the emotion of the user 10, the emotion of the agent, and the speech content of the agent. The agent system 500 may output only vocal sound without outputting an image when interacting with the user 10.

As in the first embodiment, the emotion determination unit 232 determines an emotion value indicating the emotion of the user 10 and an emotion value of the agent. In the present embodiment, the emotion value of the agent is determined instead of the emotion value of the robot 100. The emotion value of the agent is reflected in an emotion of the set character. In a case in which the agent system 500 interacts with the user 10, not only the emotion of the user 10 but also the emotion of the agent is reflected in the interaction. In other words, the action control unit 250 outputs the speech content in an aspect according to the emotion determined by the emotion determination unit 232.

The emotion of the agent is also reflected in a case in which the agent system 500 performs an action toward the user 10. For example, in a case in which the user 10 requests the agent system 500 to take a photograph, whether or not the agent system 500 takes a photograph in response to the request of the user is determined according to the degree of the emotion of "gloomy" of the agent. In a case in which the character has a positive emotion, the character performs a favorable interaction or action on the user 10, and in a case in which the character has a negative emotion, the character performs a defiant interaction or action on the user 10.

The history data 222 stores a history of the interactions performed between the user 10 and the agent system 500 as the event data. The storage unit 220 may be realized by an external cloud storage. In the case of interacting with the user 10 or performing an action toward the user 10, the agent system 500 determines the interaction content or the action content in consideration of the content of the interaction history stored in the history data 222. For example, the agent system 500 ascertains the hobby and the preference of the user 10 on the basis of the interaction history stored in the history data 222. The agent system 500 generates interaction content matching the hobby and the preference of the user 10 and provides a recommendation. The action determination unit 236 determines speech content of the agent on the basis of the interaction history stored in the history data 222. In the history data 222, personal information such as a name, an address, a telephone number, and a credit card number of the user 10 acquired through an interaction with the user 10 is stored. Here, the agent may spontaneously make speech of inquiry about whether or not to register personal information with the user 10, such as "Is the credit card number to be registered?", and the personal information may be stored in the history data 222 according to the answer of the user 10.

As described in the first embodiment, the action determination unit 236 generates speech content on the basis of the sentence generated by using the sentence generation model. Specifically, the action determination unit 236 inputs the text or the vocal sound input by the user 10 and the emotions of both the user 10 and the character determined by the emotion determination unit 232 and the conversation history stored in the history data 222 to the sentence generation model, and generates the speech content of the agent. In this case, the action determination unit 236 may further input the character's personality set by the character setting unit 276 to the sentence generation model to generate the speech content of the agent. In the agent system 500, the sentence generation model is not located on the front-end side serving as a touch point with the user 10, but is used as a tool of the agent system 500.

The command acquisition unit 272 uses the output of the speech understanding unit 212 to acquire an agent command from vocal sound output from the user 10 or text through an interaction with the user 10. The command includes, for example, content of actions to be executed by the agent system 500, such as information search, store reservation, ticket arrangement, purchase of products/services, payment, route guidance to a destination, and recommendation provision.

The RPA 274 performs an action according to the command acquired by the command acquisition unit 272. For example, the RPA 274 performs actions related to use of the service provider, such as information search, store reservation, ticket arrangement, purchase of products/services, and payment.

The RPA 274 reads the personal information of the user 10 necessary for executing the actions related to the use of the service provider from the history data 222 and uses the personal information. For example, in a case in which a product is purchased in response to a request from the user 10, the agent system 500 reads and uses personal information such as the name, the address, the telephone number, and the credit card number of the user 10 stored in the history data 222. It is unkind to request the user 10 to input personal information in the initial setting, and it is also unpleasant for the user. In the agent system 500 according to the present embodiment, instead of requesting the user 10 to input personal information in the initial setting, the personal information acquired through the interaction with the user 10 is stored, and read and used as necessary. As a result, it is possible to avoid making the user feel unpleasant, and convenience of the user is improved.

The agent system 500 executes the interaction processing according to, for example, the following Steps 1 to 6.

(Step 1) The agent system 500 sets a character of the agent. Specifically, the character setting unit 276 sets the character of the agent used when the agent system 500 interacts with the user 10 on the basis of the designation from the user 10.

(Step 2) The agent system 500 acquires the state of the user 10 including the vocal sound or the text input from the user 10, the emotion value of the user 10, the emotion value of the agent, and the history data 222. Specifically, the processing similar to steps S100 to S103 is performed to acquire the state of the user 10 including the vocal sound or the text input from the user 10, the emotion value of the user 10, the emotion value of the agent, and the history data 222.

(Step 3) The agent system 500 determines speech content of the agent. Specifically, the action determination unit 236 inputs the text or the vocal sound input by the user 10, the emotions of both the user 10 and the character specified by the emotion determination unit 232, and the conversation history stored in the history data 222 to the sentence generation model, and generates the speech content of the agent.

For example, a fixed sentence "In this case, how do you answer as an agent?" is added to the text or the vocal sound input by the user 10 and the text representing the emotions of both the user 10 and the character specified by the emotion determination unit 232 and the conversation history stored in the history data 222, and is input to the sentence generation model to acquire the speech content of the agent.

As an example, in a case in which the text or the vocal sound input by the user 10 is "I need a reservation at 7pm at a good Chinese restaurant nearby.", "Understood.", and "These are recommended restaurants. 1. AAAA. 2. BBBB. 3. CCCC. 4. DDDD" are acquired as the speech content of the agent.

In a case in which the text or the vocal sound input by the user 10 is "Fourth DDDD is good", "Understood. I will make a reservation. How many seats do you need?" are acquired as the speech content of the agent.

(Step 4) The agent system 500 outputs the speech content of the agent. Specifically, the action control unit 250 synthesizes vocal sound corresponding to the character set by the character setting unit 276, and outputs the speech content of the agent by using the synthesized vocal sound.

(Step 5) The agent system 500 determines whether or not a timing to execute a command of the agent has arrived. Specifically, the action determination unit 236 determines whether or not the timing to execute the command of the agent has arrived on the basis of the output of the sentence generation model. For example, in a case in which the output of the sentence generation model includes that the agent executes the command, it is determined that the timing to execute the command of the agent has arrived, and the processing proceeds to Step 6. On the other hand, in a case in which it is determined that the timing to execute the command of the agent has not arrived, the processing returns to Step 2 described above.

(Step 6) The agent system 500 executes the command of the agent. Specifically, the command acquisition unit 272 acquires the command of the agent from the vocal sound output from the user 10 or the text through an interaction with the user 10. The RPA 274 performs an action corresponding to the command acquired by the command acquisition unit 272. For example, in a case in which the command is "information search", information search is performed on a search site by using a search query obtained through an interaction with the user 10 and an application programming interface (API). The action determination unit 236 inputs a search result to the sentence generation model and generates speech content of the agent. The action control unit 250 synthesizes vocal sound corresponding to the character set by the character setting unit 276, and outputs the speech content of the agent by using the synthesized vocal sound.

In a case in which the command is "store reservation", the reservation is made by making a phone call to the reservation destination store by using the reservation information obtained through the interaction with the user 10, the reservation destination store information, and the API according to phone software. In this case, the action determination unit 236 acquires the speech content of the agent with respect to the vocal sound input from the other party by using the sentence generation model having the interaction function. The action determination unit 236 inputs the result of the store reservation (whether or not the reservation has been made) to the sentence generation model, and generates the speech content of the agent. The action control unit 250 synthesizes vocal sound corresponding to the character set by the character setting unit 276, and outputs the speech content of the agent by using the synthesized vocal sound.

The processing returns to Step 2 described above.

In Step 6, the result of the action (for example, store reservation) executed by the agent is also stored in the history data 222. The result of the action executed by the agent stored in the history data 222 is used by the agent system 500 to ascertain a hobby or a preference of the user 10. For example, in a case in which the same store is reserved a plurality of times, it is recognized that the user 10 likes the store, or the reserved time zone, or the reservation content such as the content of the course or the fee is used as a criterion for selecting the store at the time of the next reservation.

As described above, the agent system 500 can execute interaction processing and perform an action related to use of the service provider as necessary.

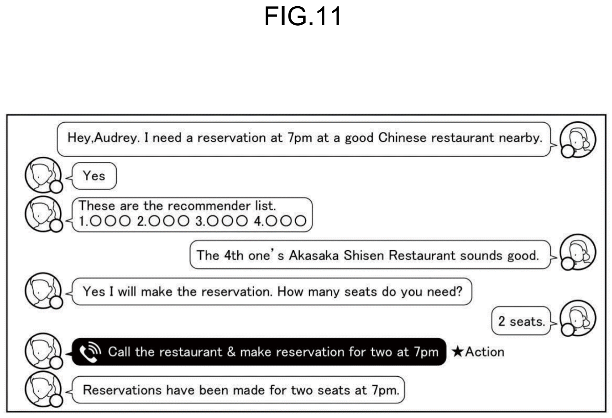

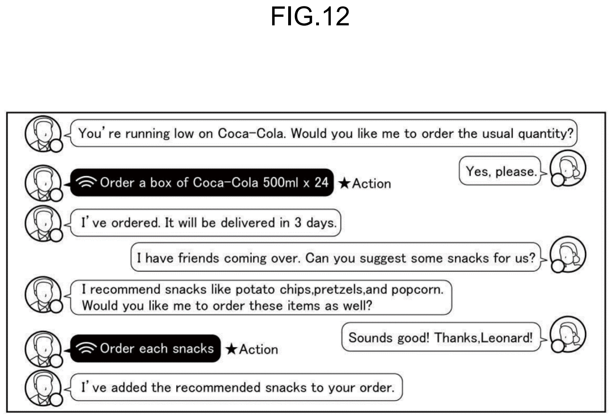

Figs. 11 and 12 are diagrams illustrating an example of the operation of the agent system 500. Fig. 11 illustrates an aspect in which the agent system 500 makes a restaurant reservation through an interaction with the user 10. In Fig. 11, the speech content of the agent is illustrated on the left side, and the speech content of the user 10 is illustrated on the right side. The agent system 500 can ascertain a preference of the user 10 on the basis of an interaction history with the user 10, provide a recommendation list of restaurants that match the preference of the user 10, and make a reservation of a selected restaurant.

On the other hand, Fig. 12 illustrates an aspect in which the agent system 500 accesses a mail order site through an interaction with the user 10 to purchase a product. In Fig. 12, the speech content of the agent is illustrated on the left side, and the speech content of the user 10 is illustrated on the right side. The agent system 500 can estimate a remaining amount of the beverage stocked by the user on the basis of the interaction history with the user 10, and can propose purchase of the beverage to the user 10 and execute the purchase. The agent system 500 can ascertain the preference of the user on the basis of the past interaction history with the user 10, and recommend a snack that the user likes. As described above, the agent system 500 supports the daily life of the user 10 by performing various actions such as restaurant reservation or product purchase/settlement while communicating with the user 10 as an agent such as a butler.

Other configurations and operations of the agent system 500 of the third embodiment are similar to those of the robot 100 of the first embodiment, and thus description thereof will be omitted.

The agent system 500 may be mounted on the communication terminal 600 (see Fig. 16), and the communication terminal 600 may be detachably provided on the movement unit 601 to configure the movement system 1000M. In this case, the action determination unit 236 of the agent system 500 may determine any of the actions of the agent for interacting with a plurality of types of users, including no action, as an action of the electronic apparatus which is the communication terminal 600.

In a case in which the action determination model 221 of the agent system 500 is a sentence generation model having an interaction function, the action determination unit 236 may input text representing at least one of a user state, a state of the agent, an emotion of the user, or an emotion of the agent and text for inquiry about an agent action to the sentence generation model, and determine an action of the communication terminal 600 on the basis of an output of the sentence generation model.

Next, processing of the action determination unit 236 in the case of performing autonomous processing in which the agent autonomously acts will be described.

In autonomous processing in the present embodiment, the agent may detect a state or an action of the user spontaneously or periodically by monitoring the user. The term "spontaneous" may be interpreted as the agent spontaneously acquiring the state or action of the user by itself without a trigger from the outside. The trigger from the outside may include a question from the user to the agent via the movement system 1000M (see Fig. 16), an active action from the user to the movement system 1000M, and the like.

In the autonomous processing, the agent may ask the generative AI a question about the detected state or action of the user, and store an answer of the generative AI to the question and the detected action of the user in association with each other. In this case, the agent may store action content of correcting the action in association with the answer.

Information in which the answer of the generative AI to the question, the detected action of the user, and the action content of correcting the action are associated with each other may be recorded as table information in a storage medium such as a memory. The table information may be interpreted as specific information recorded in the storage unit.

Specifically, in a case in which the user tends to walk for a long time, the agent asks the generative AI a question "What kind of guidance should be given to the user who takes such an action?", and in a case in which the answer of the generative AI to the question is "Propose the same route as the route along which the user has previously passed", the agent may record the answer, the action of the user who can walk for a long time, and the action content of correcting the action (for example, guidance content of "guiding the user to the route along which the user has previously passed") in association with the table information. In a case in which the action of the user who is walking for a long time is detected, the action determination unit 236 of the agent system 500 may reproduce vocal sound "I will guide you to the route along which you have previously passed" as the action of the communication terminal 600 by using the table information.

As another example, in a case in which the user tends to run for a long time, the agent asks the generative AI a question "What kind of guidance should be given to the user who takes such an action?", and in a case in which the answer of the generative AI to the question is "It is desirable to periodically perform guidance for encouraging hydration", the agent may record the answer, the action of the user who can run for a long time, and the action content of correcting the action (for example, guidance content such as "Please rehydrate frequently") in association with the table information. In a case in which the action of the user who is running for a long time is detected, the action determination unit 236 of the agent system 500 may reproduce vocal sound "Please rehydrate frequently" as the action of the communication terminal 600 by using the table information.

As still another example, in a case in which the user tends to walk on the roadway, the agent may ask the generative AI a question "What kind of guidance should be given to the user who takes such an action?", and in a case in which the answer of the generative AI to the question is "I recommend to propose returning to the sidewalk because there is a possibility of coming in contact with vehicles", the agent may record the answer, the action of the user who may walk on the roadway, and the action content of correcting the action (for example, guidance content of "Please return to the sidewalk") in association with the table information. In a case in which the action of the user walking on the sidewalk is detected, the action determination unit 236 of the agent system 500 may reproduce vocal sound "Please return to the sidewalk" as the action of the communication terminal 600 by using the table information.

In the autonomous processing, an action schedule of the agent for attracting attention for the state or the action of the user may be set on the basis of the detected action of the user and the stored specific information.

As described above, the agent may record table information in which the answer of the generative AI corresponding to the state or the action of the user is associated with the detected state or action of the user in the storage medium. Hereinafter, an example of content stored in the table will be described.

### (1. Case in Which User Tends to Walk For a Long Time)

In a case in which there is the tendency, the agent itself asks the generative AI a question "What kind of guidance should be given to the user who takes such an action?". In a case in which the answer of the generative AI to this question is, for example, "Propose the same route as the route along which the user has previously passed", "Propose a route along which the user has not passed", "Propose a route with a relatively small number of vehicles such as bicycles", or "Propose a route with undulations", the agent may store the action of the user and the answer of the generative AI in association with each other.

### (2. Case in Which User Tends to Run for a Long Time)

In a case in which there is the tendency, the agent itself asks the generative AI a question "What kind of guidance should be given to the user who takes such an action?". In a case in which the answer of the generative AI to this question is, for example, "It is desirable to provide guidance for encouraging hydration periodically", "Propose a route with fewer bicycles and clean air", or "Propose a route with fewer traffic lights", the agent may store the action of the user and the answer of the generative AI in association with each other.

### (3. Case in Which User Tends to Walk on Roadway)

In a case in which there is the tendency, the agent itself asks the generative AI a question "What kind of situation can occur to the user who takes such an action?". In a case in which the answer of the generative AI to this question is, for example, "I recommend to propose returning to the sidewalk because there is a possibility of coming into contact with vehicles", "I recommend to propose returning to the sidewalk because there is a possibility of coming into contact with bicycles", or "There is a possibility of entering an expressway interchange", the agent may store the action of the user walking on the roadway and the answer of the generative AI in association with each other. The agent may store action content of correcting the action in association with the answer.

The action content of correcting the action may include at least one of reproduction of vocal sound for correcting the dangerous action of the user or reproduction of an image for correcting the dangerous action of the user.

The vocal sound for correcting the dangerous action of the user may include vocal sound for guiding the user to a specific place. The specific place may include a place other than the place where the user is currently located, for example, a vicinity of the movement system 1000M and a sidewalk. Specifically, the vocal sound may include vocal sound such as "Please stop walking on the roadway", "That is not a sidewalk", "The roadway is dangerous", and "Please move to the sidewalk immediately".

As described above, in the autonomous processing, a table in which the answer of the generative AI corresponding to the state or action of the user, the content of the state or action, and the action content of correcting the state or action are associated with each other may be recorded in a storage medium such as a memory.

In the autonomous processing, after the table is recorded, the action of the user is autonomously or periodically detected, and the action schedule of the agent for attracting the attention of the user may be set on the basis of the detected action of the user and the content of the stored table. Specifically, the action determination unit 236 of the agent system 500 may set the first action content on the basis of the detected action of the user and the content of the stored table.

Specifically, the action determination unit 236 of the agent system 500 may spontaneously or periodically detect an action of the user on the basis of a sensor included in the communication terminal 600, and determine the first action content in a case in which it is determined to lead the user as an action of the electronic apparatus on the basis of the detected action of the user and specific information stored in advance.

### (First Action Content)

The first action content may include an action of the agent that leads the user by controlling the wheels 601a of the movement unit 601. Controlling the wheels 601a may include changing at least one of a rotation direction, a rotation speed, or a steering direction of the wheels 601a. Hereinafter, an example of the first action content will be described.

The action determination unit 236 of the agent system 500 may execute reproduction of at least one of vocal sound or an image for assisting in walking of the user as the first action content for performing route guidance so that the walking user moves along a specific route. Specifically, as illustrated in Fig. 18, the action determination unit 236 may reproduce an image of a route along which the user can walk on the screen of the communication terminal 600, and may reproduce vocal sound related to the route. The vocal sound may include, for example, "Turn right 10 m ahead".

The action determination unit 236 of the agent system 500 may execute reproduction of at least one of vocal sound or an image for showing a route different from the set guidance route. Specifically, as illustrated in Fig. 19, the action determination unit 236 may reproduce an image of a route different from the set guidance route on the screen of the communication terminal 600, and may reproduce vocal sound related to the route. The vocal sound may include, for example, "The road is closed ahead. Would you like me to show another route?".

In a case in which the user being led along the set guidance route deviates from the guidance route, the action determination unit 236 of the agent system 500 may execute reproduction of at least one of vocal sound or an image for guiding the user along a path for returning the user to the guidance route. Specifically, as illustrated in Fig. 20, the action determination unit 236 may reproduce an image of the route returning the user to the set guidance route on the screen of the communication terminal 600, and may reproduce vocal sound related to the route. The vocal sound may include "You have deviated from the route. I will show you the path for return to the original route." and the like. The action determination unit 236 of the agent system 500 may execute reproduction of at least one of the vocal sound or the image described above, for example, "I will guide you to the route along which you have previously passed", "Please rehydrate frequently", or "Please return to the sidewalk".

### (Second Action Content)

The action determination unit 236 of the agent system 500 may determine whether or not the action of the user has been corrected by detecting the action of the user while the movement unit 601 was leading the user or after the movement unit 601 reproduced at least one of vocal sound or an image that assists in the walking of the user, and may determine second action content different from the first action content in a case in which the action of the user has been corrected.

The case in which the action of the user has been corrected may be interpreted as a case in which the user has stopped a specific action and a specific behavior or a case in which a specific situation has been resolved as a result of execution of the operation of the agent according to the first action content. Specifically, the case in which the action of the user has been corrected may include a case in which the user has started walking along the proposed different route, a case in which the user has started walking along a path for return to the original route when the path was shown due to deviation from the original route, and the like.

The second action content may include reproduction of at least one of vocal sound praising the user for action, vocal sound expressing gratitude for the action of the user, an image praising the user for the action, or an image expressing gratitude for the action of the user.

Specifically, the vocal sound praising the user for the action may include, for example, "Well done! Excellent". The vocal sound expressing gratitude for the action of the user may include vocal sound such as "Thank you for coming back" or "Thank you for selecting another route". As illustrated in Fig. 21, the image praising the user for the action may include an image of a character taking a good pose. As illustrated in Fig. 22, the image of gratitude for the action of the user may include an image of a character who expresses gratitude.

### (Third Action Content)

The action determination unit 236 may determine whether or not the action of the user has been corrected by detecting the action of the user while the movement unit 601 was leading the user or after the movement unit 601 reproduced at least one of vocal sound or an image that assists in the walking of the user, and determine third action content different from the first action content in a case in which the action of the user has not been corrected.

The case in which the action of the user has not been corrected may be interpreted as a case in which the user has continued the dangerous action and behavior even though the operation of the agent according to the first action content was executed, a case in which the dangerous situation is not resolved, or a case in which the user has started or continued an action different from the proposal of the agent.

Specifically, the case in which the action of the user has not been corrected may include a case in which the user has walked along a route different from the proposed different route without walking along the proposed different route, a case in which the user has neglected a path for return to the original route and continued walking when the path was shown due to deviation from the original route, and the like.

The third action content may include at least one of transmission of specific information to a person other than the user, reproduction of sound that attracts an interest of the user, or reproduction of an image that attracts an interest of the user.

The transmission of the specific information to a person other than the user may include distribution of an email containing a warning message to a relative of the user, a spouse of the user, a guardian of the user, or the like. The transmission of the specific information to a person other than the user may include distribution of an image (a still image or a moving image) including the user and the surrounding scenery to a relative of the user, a spouse of the user, a guardian of the user, or the like. The transmission of the specific information to a person other than the user may include distribution of vocal sound having a warning message.

The reproduction of the sound that attracts the interest of the user may include specific music that the user likes, and may also include vocal sound such as "Please come back here" or "Let's take a safe road".

The reproduction of the image that attracts the interest of the user may include reproduction of an image of an animal kept by the user, an image of parents of the user, an image of a character of the animal illustrated in Fig. 23, and the like.

According to the agent system 500 of the disclosure, an action of leading the user can be executed through autonomous processing. As a result, the agent can guide the user walking in a town that the user visits for the first time to an appropriate route. The user who is interested in walking may be guided to a route along which the user has not passed before. A route along which the user cannot pass is detected in advance, and the user can be guided to another route. The user who gets lost and deviates from the specific route can be guided to the original route.

The action determination unit 236 determines, as the action of the agent, any of a plurality of types of agent actions including no action, by using at least one of the state of the user 10, the emotion of the user 10, the emotion of the agent, or the state of the agent, and the action determination model 221 at a predetermined timing. Here, a case in which a sentence generation model having an interaction function is used as the action determination model 221 will be described as an example.

Specifically, the action determination unit 236 inputs text representing at least one of the state of the user 10, the emotion of the user 10, the emotion of the agent, or the state of the agent, and text for inquiry about agent actions to the sentence generation model, and determines the actions of the agent on the basis of an output of the sentence generation model.

For example, the plurality of types of agent actions include the following (1) to (26).
(1) The agent does nothing.
(2) The agent has a dream.
(3) The agent talks to the user.
(4) The agent creates a picture diary.
(5) The agent proposes an activity.
(6) The agent proposes a person with whom the user should meet.
(7) The agent introduces news in which the user is interested.
(8) The agent edits pictures and moving images.
(9) The agent studies with the user.
(10) The agent evokes memories.
(11) The agent may reproduce an image of a route along which the user can walk on the screen of the communication terminal as the first action content of leading the user.
(12) The agent may reproduce vocal sound related to a route along which the user can walk as the first action content of correcting the action of the user.
(13) The agent may reproduce an image of a route different from the set guidance route on the screen of the communication terminal 600 as the first action content of correcting the action of the user.
(14) The agent may reproduce vocal sound related to a route different from the set guidance route as the first action content of correcting the action of the user.
(15) The agent may reproduce, as the first action content of correcting the action of the user, an image of a route for return to the set guidance route on the screen of the communication terminal 600 in a case in which the user led along the set guidance route deviates from the guidance route.
(16) The agent may reproduce, as the first action content of correcting the action of the user, vocal sound related to a route for return to the set guidance route in a case in which the user led along the set guidance route deviates from the guidance route.
(17) The agent may reproduce vocal sound praising the user for the action as the second action content different from the first action content.
(18) The agent may reproduce vocal sound expressing gratitude for the action of the user as the second action content different from the first action content.
(19) The agent may reproduce an image praising the user for the action as the second action content different from the first action content.
(20) The agent may reproduce an image expressing gratitude for the action of the user as the second action content different from the first action content.
(21) The agent may transmit specific information to a person other than the user as the third action content different from the first action content.
(22) The agent may reproduce sound that attracts the interest of the user as the third action content different from the first action content.
(23) The agent may reproduce an image that attracts the interest of the user as the third action content different from the first action content.

The action determination unit 236 may spontaneously or periodically detect the action of the user on the basis of the sensor, and in a case in which it is determined to lead the user as the action of the agent on the basis of the detected action of the user and the specific information stored in advance, execute the following first action content.

The action determination unit 236 of the agent system 500 may perform, as the agent action, the first action content in the above "(11)", that is, reproduce an image of a route along which the user can walk on the screen of the communication terminal 600.

The action determination unit 236 of the agent system 500 may perform, as the agent action, the first action content in the above "(12)", that is, reproduce vocal sound related to a route along which the user can walk.

The action determination unit 236 of the agent system 500 may perform, as the agent action, the first action content in the above "(13)", that is, reproduce an image of a route different from the set guidance route as the first action content of correcting the action of the user on the screen of the communication terminal 600.

The action determination unit 236 of the agent system 500 may perform, as the agent action, the first action content in the above "(14)", that is, reproduce vocal sound related to a route different from the set guidance route.

The action determination unit 236 of the agent system 500 may perform, as the agent action, the first action content in the above "(15)", that is, reproduce an image of a route for return to the set guidance route on the screen of the communication terminal 600 in a case in which the user led along the set guidance route deviates from the guidance route.

The action determination unit 236 of the agent system 500 may perform, as the agent action, the first action content in the above "(16)", that is, reproduce vocal sound related to a route for return to the set guidance route in a case in which the user led along the set guidance route deviates from the guidance route.

The action determination unit 236 of the agent system 500 may execute the second action content different from the first action content. Specifically, the action determination unit 236 may perform, as the agent action, the second action content in the above "(17)", that is, reproduce vocal sound praising the user for the action.

The action determination unit 236 of the agent system 500 may perform, as the agent action, the second action content in the above "(18)", that is, reproduce vocal sound expressing gratitude for the action of the user.

The action determination unit 236 of the agent system 500 may perform, as the agent action, the second action content in the above "(19)", that is, reproduce an image praising the user for the action.

The action determination unit 236 of the agent system 500 may perform, as the agent action, the second action content in the above "(20)", that is, reproduce an image expressing gratitude for the action of the user.

The action determination unit 236 of the agent system 500 may execute the third action content different from the first action content. Specifically, the action determination unit 236 may perform, as the agent action, the third action content in the above "(21)", that is, transmit the specific information to a person other than the user.

The action determination unit 236 of the agent system 500 may perform, as the agent action, the third action content in the above "(22)", that is, execute reproduction of sound that attracts the interest of the user.

The action determination unit 236 of the agent system 500 may perform, as the agent action, the third action content in the above "(23)", that is, execute reproduction of an image that attracts the interest of the user.

In a case in which, for example, an image of a route along which the user can walk is reproduced as the first action content indicated in the above "(11)" to "(16)", the related information collection unit 270 of the agent system 500 may store data such as an image of a route along which the user can walk in the collected data 223.

In a case in which, for example, vocal sound praising the user for the action is reproduced as the second action content indicated in the above "(17)" to "(20)", the related information collection unit 270 of the agent system 500 may store data such as an image of a route along which the user can walk in the collected data 223.

In a case in which, for example, specific information is transmitted to a person other than the user as the third action content indicated in the above "(21)" to "(23)", the related information collection unit 270 of the agent system 500 may store data of the specific information or the like in the collected data 223.

A part of the agent system 500 (for example, the sensor module unit 210, the storage unit 220, and the control unit 228B) may be provided outside a communication terminal such as a smartphone possessed by the user (for example, a server), and the communication terminal may function as each unit of the agent system 500 by communicating with the outside.

### [Fourth Embodiment]

In a fourth embodiment, the agent system is applied to smart glasses. Portions having the same configurations as those of the first to third embodiments are denoted by the same reference numerals, and description thereof will be omitted.

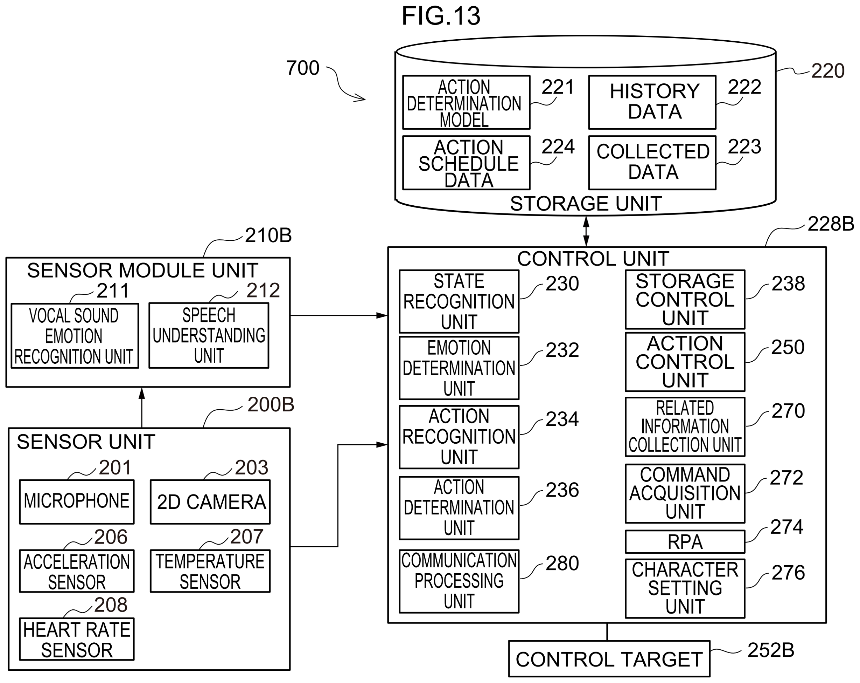

Fig. 13 is a functional block diagram of an agent system 700 configured by using some or all of the functions of the control system.

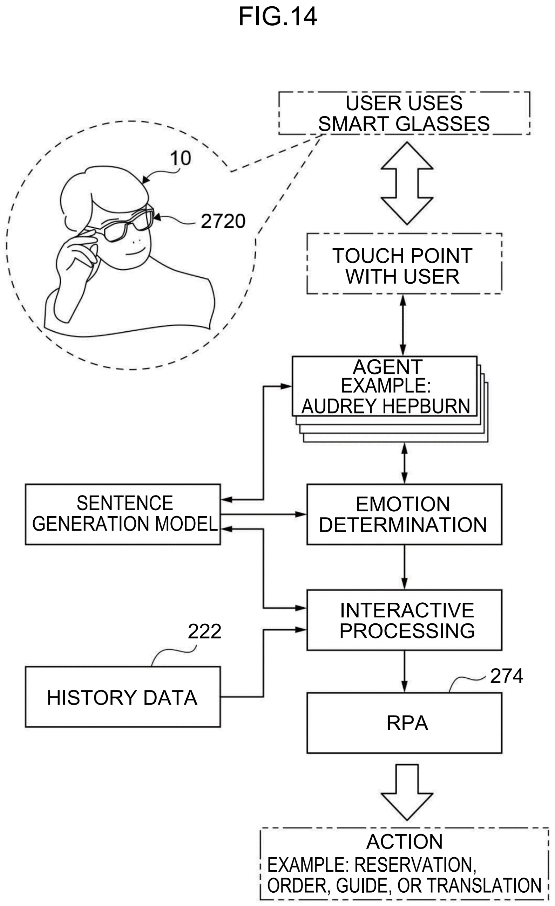

As illustrated in Fig. 14, smart glasses 720 are glasses-type smart devices, and are worn by the user 10 similarly to general glasses. The smart glasses 720 are an example of an electronic apparatus and a wearable terminal.

The smart glasses 720 include an agent system 700. The display included in the control target 252B displays various types of information to the user 10. The display is, for example, a liquid crystal display. The display is provided, for example, in a lens portion of the smart glasses 720, and the display content can be visually recognized by the user 10. The speaker included in the control target 252B outputs vocal sound indicating various types of information to the user 10. The smart glasses 720 include a touch panel (not illustrated), and the touch panel receives an input from the user 10.

An acceleration sensor 206, a temperature sensor 207, and a heart rate sensor 208 of the sensor unit 200B detect a state of the user 10. These sensors are merely examples, and other sensors may be mounted in order to detect a state of the user 10.

The microphone 201 acquires vocal sound output by the user 10 or an environmental sound around the smart glasses 720. The 2D camera 203 can image the surroundings of the smart glasses 720. The 2D camera 203 is, for example, a CCD camera.

The sensor module unit 210B includes vocal sound emotion recognition unit 211 and a speech understanding unit 212. The communication processing unit 280 of the control unit 228B controls communication between the smart glasses 720 and the outside.

Fig. 14 is a diagram illustrating an example of a usage aspect of the agent system 700 by the smart glasses 720. The smart glasses 720 realize provision of various services to the user 10 using the agent system 700. For example, in a case in which the smart glasses 720 are operated (for example, vocal sound is input to a microphone, or a touch panel is tapped with a finger) by the user 10, the smart glasses 720 start to use the agent system 700. Here, using the agent system 700 includes that the smart glasses 720 have the agent system 700 and use the agent system 700, and also includes an aspect in which some constituents (for example, the sensor module unit 210B, the storage unit 220, and the control unit 228B) of the agent system 700 are provided outside the smart glasses 720 (for example, a server), and the smart glasses 720 communicate with the outside to use the agent system 700.

In a case in which the user 10 operates the smart glasses 720, a touch point is generated between the agent system 700 and the user 10. That is, service provision by the agent system 700 is started. As described in the third embodiment, in the agent system 700, the character of the agent is set by the character setting unit 276.

The emotion determination unit 232 determines an emotion value indicating the emotion of the user 10 and an emotion value of the agent. Here, the emotion value indicating the emotion of the user 10 is estimated from various sensors included in the sensor unit 200B mounted on the smart glasses 720. For example, in a case in which a heart rate of the user 10 detected by the heart rate sensor 208 is increased, the emotion values such as "anxiety" and "fear" are estimated to be large.

As a result of measuring the body temperature of the user with the temperature sensor 207, for example, in a case in which the body temperature exceeds the average body temperature, the emotion value such as "pain" or "bitter" is estimated to be great. For example, in a case in which it is detected by the acceleration sensor 206 that the user 10 performs some sport, the emotion value such as "pleasant" is estimated to be great.

For example, the emotion value of the user 10 may be estimated from the vocal sound or the speech content of the user 10 acquired by the microphone 201 mounted on the smart glasses 720. For example, in a case in which the user 10 is raising his/her vocal sound, the emotion value such as "anger" is estimated to be great.

In a case in which the emotion value estimated by the emotion determination unit 232 is greater than a predefined value, the agent system 700 causes the smart glasses 720 to acquire information regarding a surrounding situation. Specifically, for example, the 2D camera 203 is caused to capture an image or a moving image indicating a surrounding situation of the user 10 (for example, a surrounding person or object). The microphone 201 is caused to record surrounding environmental sound. Examples of other information regarding the surrounding situation include date, time, position information, and information indicating weather. The information regarding the surrounding situation is stored in the history data 222 together with the emotion value. The history data 222 may be realized by an external cloud storage. As described above, the surrounding situation obtained by the smart glasses 720 is stored in the history data 222 as a so-called life log in a state of being associated with the emotion value of the user 10 at that time.

In the agent system 700, the information indicating the surrounding situation is stored in the history data 222 in association with the emotion value. As a result, the agent system 700 ascertains personal information such as the hobby, preference, or personality of the user 10. For example, in a case in which an image indicating a state of baseball watching is associated with an emotion value such as "happy" or "pleasant", the hobby of the user 10 is baseball watching, and a favorite team or player is ascertained by the agent system 700 from the information stored in the history data 222.

In the case of interacting with the user 10 or performing an action toward the user 10, the agent system 700 determines interaction content or action content in consideration of the content of the surrounding situation stored in the history data 222. The interaction content or the action content may be determined in consideration of the interaction history stored in the history data 222 as described above in addition to the surrounding situation.

As described above, the action determination unit 236 generates the speech content on the basis of the sentence generated by the sentence generation model. Specifically, the action determination unit 236 inputs the text or the vocal sound input by the user 10, the emotions of both the user 10 and the agent determined by the emotion determination unit 232, the conversation history stored in the history data 222, the agent's personality, and the like to the sentence generation model, and generates the speech content of the agent. The action determination unit 236 inputs the surrounding situation stored in the history data 222 to the sentence generation model, and generates the speech content of the agent.

The generated speech content is output by vocal sound from the speaker mounted on the smart glasses 720 to the user 10, for example. In this case, synthesized vocal sound corresponding to the character of the agent is used as the vocal sound. The action control unit 250 generates synthesized vocal sound by reproducing the vocal sound quality of the agent's character or generates synthesized vocal sound according to the emotion of the character (for example, in the case of an emotion of "angry", vocal sound in which tone is enhanced). The speech content may be displayed on the display instead of the vocal sound output or together with the vocal sound output.

The RPA 274 executes an operation according to the command (for example, an agent command acquired from vocal sound output by the user 10 through interaction with the user 10 or text). The RPA 274 performs actions related to use of the service provider, such as information search, store reservation, ticket arrangement, purchase of products/services, payment, path guidance, and translation.

As another example, the RPA 274 executes an operation of transmitting content input by vocal sound of the user 10(for example, a child) through interaction with the agent to the other party (for example, the parent). Examples of transmission means include message application software, chat application software, and mail application software.

In a case in which the operation has been executed by the RPA 274, for example, vocal sound indicating that the execution of the operation is finished is output from the speaker mounted on the smart glasses 720. For example, vocal sound such as "Reservation of the store has been completed" is output to the user 10. For example, in a case in which the reservation of the store is full, vocal sound such as "Reservation could not be made. What would you like to do?" is output to the user 10.

As described above, in the smart glasses 720, various services are provided to the user 10 by using the agent system 700. Since the smart glasses 720 are worn by the user 10, the agent system 700 can be used in various scenes such as at home, at work, and at a place outside the house.

Since the smart glasses 720 are worn by the user 10, the smart glasses are suitable for collecting a so-called life log of the user 10. Specifically, an emotion value of the user 10 is estimated on the basis of detection results in various sensors or the like mounted on the smart glasses 720 or recording results in the 2D camera 203 or the like. Therefore, the emotion value of the user 10 can be collected in various scenes, and the agent system 700 can provide a service or speech content suitable for the emotion of the user 10.

In the smart glasses 720, the surrounding situation of the user 10 can be obtained by the 2D camera 203, the microphone 201, and the like. The surrounding situation and the emotion value of the user 10 are associated with each other. As a result, it is possible to estimate what kind of emotion the user 10 has in what kind of situation. As a result, the accuracy in a case in which the agent system 700 ascertains the hobby/preference of the user 10 can be improved. In the agent system 700, the hobby/preference of the user 10 is accurately ascertained, so that the agent system 700 can provide a service or speech content suitable for the hobby/preference of the user 10.

The agent system 700 can also be applied to other wearable terminals (an electronic apparatus that can be worn on the body of the user 10, such as a pendant, a smart watch, an earring, a bracelet, or a hairband). In a case in which the agent system 700 is applied to a smart pendant, a speaker as the control target 252B outputs vocal sound indicating various types of information to the user 10. The speaker is, for example, a speaker capable of outputting vocal sound having directivity. The speaker is set to have directivity toward the ear of the user 10. As a result, the vocal sound is suppressed from reaching a person other than the user 10. The microphone 201 acquires vocal sound output by the user 10 or surrounding environmental sound of the smart pendant. The smart pendant is worn to be carried from the neck of the user 10. Thus, the smart pendant is located relatively close to the mouth of the user 10 while being worn. This facilitates acquisition of vocal sound output by user 10.

In the above embodiment, the case in which the robot 100 recognizes the user 10 by using the face image of the user 10 has been described, but the disclosed technology is not limited to this aspect. For example, the robot 100 may recognize the user 10 by using vocal sound uttered by the user 10, a mail address of the user 10, an ID of an SNS of the user 10, an ID card in which a wireless IC tag is built and which is possessed by the user 10, or the like.

The robot 100 is an example of an electronic apparatus including a control system. An application target of the control system is not limited to the robot 100, and the control system can be applied to various electronic apparatuses. The functions of the server 300 may be implemented by one or more computers. At least some functions of the server 300 may be implemented by a virtual machine. At least some of the functions of the server 300 may be implemented in a cloud.

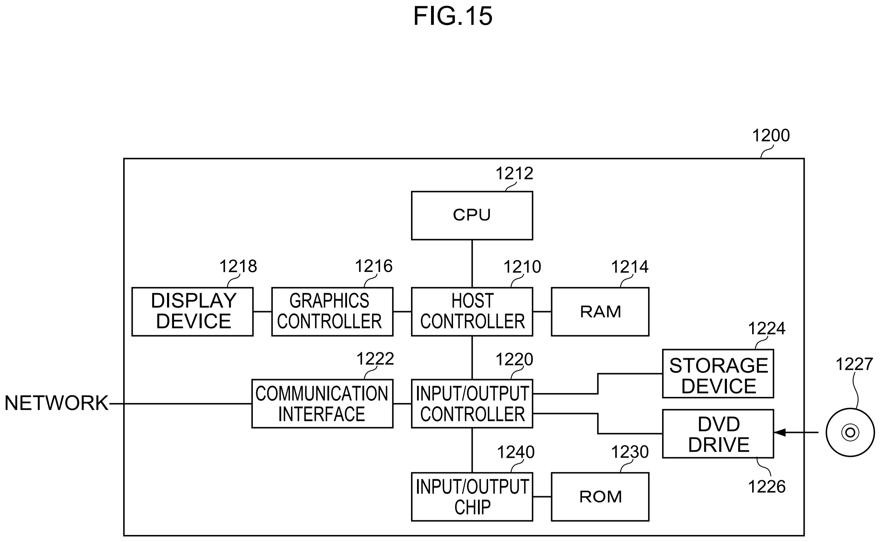

Fig. 15 schematically illustrates an example of a hardware configuration of a computer 1200 that functions as the smartphone 50, the robot 100, the server 300, and the agent systems 500 and 700. A program installed in the computer 1200 can cause the computer 1200 to function as one or more "units" of the device according to the present embodiment, or cause the computer 1200 to execute an operation associated with the device according to the present embodiment or one or more "units" thereof, and/or cause the computer 1200 to execute a process according to the present embodiment or a stage of the process. Such programs may be executed by a CPU 1212 to cause the computer 1200 to execute certain operations associated with some or all of the blocks in the flowcharts and block diagrams described herein.

The computer 1200 according to the present embodiment includes a CPU 1212, a RAM 1214, and a graphic controller 1216, which are connected to each other via a host controller 1210. The computer 1200 also includes input/output units such as a communication interface 1222, a storage device 1224, a DVD drive 1226, and an IC card drive, which are connected to the host controller 1210 via an input/output controller 1220. The DVD drive 1226 may be a DVD-ROM drive, a DVD-RAM drive, or the like. The storage device 1224 may be a hard disk drive, a solid state drive, or the like. The computer 1200 also includes a ROM 1230 and legacy input/output units such as a keyboard, which are connected to the input/output controller 1220 via an input/output chip 1240.

The CPU 1212 operates according to programs stored in the ROM 1230 and the RAM 1214, thereby controlling each unit. The graphic controller 1216 acquires image data generated by the CPU 1212 in a frame buffer or the like provided in the RAM 1214 or itself, and causes the image data to be displayed on the display device 1218.

The communication interface 1222 communicates with other electronic apparatuses via a network. The storage device 1224 stores programs and data used by the CPU 1212 in the computer 1200. The DVD drive 1226 reads a program or data from the DVD-ROM 1227 or the like and provides the program or data to the storage device 1224. The IC card drive reads a program and data from the IC card and/or writes the program and data to the IC card.

The ROM 1230 stores therein a boot program or the like executed by the computer 1200 at the time of activation and/or a program depending on hardware of the computer 1200. The input/output chip 1240 may also connect various input/output units to the input/output controller 1220 via a USB port, a parallel port, a serial port, a keyboard port, a mouse port, or the like.

The program is provided by a computer-readable storage medium such as the DVD-ROM 1227 or an IC card. The program is read from a computer-readable storage medium, installed in the storage device 1224, the RAM 1214, or the ROM 1230, which is also an example of a computer-readable storage medium, and executed by the CPU 1212. The information processing described in such a program provides cooperation between the program read by the computer 1200 and the various types of hardware resources. A device or a method may be configured by implementing an operation or processing of information according to use of the computer 1200.

For example, in a case in which communication is executed between the computer 1200 and an external device, the CPU 1212 may execute a communication program loaded in the RAM 1214 and instruct the communication interface 1222 to perform communication processing on the basis of processing described in the communication program. Under the control of the CPU 1212, the communication interface 1222 reads transmission data stored in a transmission buffer area provided in a recording medium such as the RAM 1214, the storage device 1224, the DVD-ROM 1227, or the IC card, transmits the read transmission data to the network, or writes reception data received from the network to a reception buffer area or the like provided on the recording medium.

The CPU 1212 may cause the RAM 1214 to read all or a necessary portion of a file or database stored in an external recording medium such as the storage device 1224, the DVD drive 1226 (DVD-ROM 1227), or the IC card, and may execute various types of processing on data in the RAM 1214. Next, the CPU 1212 may write back the processed data to the external recording medium.

Various types of information such as various types of programs, data, tables, and databases may be stored in a recording medium and subjected to information processing. The CPU 1212 may execute various types of processing on the data read from the RAM 1214, including various types of operations, information processing, condition determination, conditional branching, unconditional branching, information retrieval/replacement, and the like, which are described throughout the disclosure and specified by a command sequence of a program, and writes back the results to the RAM 1214. The CPU 1212 may search for information in a file, a database, or the like in the recording medium. For example, in a case in which a plurality of entries each having an attribute value of a first attribute associated with an attribute value of a second attribute are stored in the recording medium, the CPU 1212 may search for an entry in which the attribute value of the first attribute matches a specified condition from the plurality of entries, read the attribute value of the second attribute stored in the entry, and thus acquire the attribute value of the second attribute associated with the first attribute satisfying a predetermined condition.

The programs or software modules described above may be stored in the computer 1200 or a computer-readable storage medium near the computer 1200. A recording medium such as a hard disk or a RAM provided in a server system connected to a dedicated communication network or the Internet may be used as a computer-readable storage medium, and provides a program to the computer 1200 via the network.

Blocks in the flowcharts and the block diagrams in the present embodiments may represent stages of a process in which an operation is performed or "units" of a device having a role of performing an operation. Specific stages and "units" may be implemented by a dedicated circuit, a programmable circuit supplied along with computer readable instructions stored on a computer readable storage medium, and/or a processor supplied along with computer readable instructions stored on a computer readable storage medium. Dedicated circuits may include digital and/or analog hardware circuits, and may include integrated circuits (ICs) and/or discrete circuits. Programmable circuits may include reconfigurable hardware circuits including, for example, AND, OR, XOR, NAND, NOR, and other logical operations, flip-flops, registers, and memory elements, such as field programmable gate arrays (FPGA) and programmable logic arrays (PLA).

Computer readable storage media may include any tangible device capable of storing instructions executed by a suitable device, and, as a result, a computer readable storage medium having instructions stored thereon has a product including instructions that may be executed to create means for executing operations designated in the flowcharts or block diagrams. Examples of the computer readable storage medium may include an electronic storage medium, a magnetic storage medium, an optical storage medium, an electromagnetic storage medium, and a semiconductor storage medium. More specific examples of the computer readable storage medium may include a Floppy (registered trademark) disk, a diskette, a hard disk, a random access memory (RAM), a read-only memory (ROM), an erasable programmable read-only memory (an EPROM or a flash memory), an electrically erasable programmable read-only memory (EEPROM), a static random access memory (SRAM), a compact disc read-only memory (CD-ROM), a digital versatile disk (DVD), a Blu-Ray (registered trademark) disk, a memory stick, and an integrated circuit card.

The computer readable instructions may include either source codes or object codes written in any combination of one or more programming languages, including assembler instructions, instruction-set-architecture (ISA) instructions, machine instructions, machine-dependent instructions, microcode, firmware instructions, state-setting data, or an object oriented programming language such as Smalltalk (registered trademark), or JAVA (registered trademark), C++, and conventional procedural programming languages such as the "C" programming language or similar programming languages.

The computer readable instructions may be provided to a processor of a general purpose computer, a special purpose computer, or another programmable data processing device, or a programmable circuit locally or via a local area network (LAN) or a wide area network (WAN) such as the Internet, in order to cause the processor of the general purpose computer, the special purpose computer, or another programmable data processing device or the programmable circuit to execute the computer readable instructions to generate means for executing operations designated in the flowcharts or the block diagrams. Examples of the processor include a computer processor, a processing unit, a microprocessor, a digital signal processor, a controller, and a microcontroller.

Although the technology of the disclosure has been described by using each embodiment, the technology of the disclosure is not limited to the scope disclosed in each embodiment. It is apparent to those skilled in the art that various modifications or improvements can be made to the above embodiments. It is apparent from the description of the claims that modes to which such modifications or improvements are added can also be included in the technical scope of the disclosure.

The order of execution of each piece of processing of operations, procedures, steps, stages, and the like in the devices, the systems, the programs, and the methods shown in the claims, the specification, and the drawings can be realized in any order unless "before", "prior to", or the like is explicitly stated, and unless the output of the previous processing is used in the later processing. Even in a case in which the operation flow in the claims, the specification, and the drawings is described by using "first,", "next,", and the like for convenience, this does not mean that it is essential to perform in this order.

Although the system according to the disclosure has mainly been described above in terms of the functions of the robot 100, the movement system 1000M, the agent system 500, and the like, the system according to the disclosure is not necessarily implemented in the robot 100, the movement system 1000M, and the agent system 500. The system according to the disclosure may be implemented as a general information processing system. The disclosure may be implemented as, for example, a software program that operates on a server or a personal computer, or an application that operates on a smartphone or the like. The method according to the disclosure may be provided to a user in a software as a service (SaaS) format. For example, in a case in which pre-processing of classifying a plurality of pieces of information from one or two or more information sources and extracting information having reliability more than a specific value from the classified two or more pieces of information is performed, the disclosure can be used in a case in which specific processing of creating an article on the basis of the extracted information is executed.

### [Fifth Embodiment]

Next, processing of the action determination unit 236 in a case in which the robot 100 and the communication terminal perform autonomous processing of autonomously acting will be described.

In the autonomous processing in the present embodiment, the robot 100 and the communication terminal may spontaneously or periodically detect a state or an action of the user by monitoring the user. The term "spontaneous" may be interpreted as the robot 100 and the communication terminal spontaneously acquiring the state or action of the user by themselves without a trigger from the outside. The trigger from the outside may include a question from the user to the robot 100 and the communication terminal, an active action from the user to the robot 100 and the communication terminal, and the like. The term "periodic" may be interpreted as a specific cycle such as a unit of one second, a unit of one minute, a unit of one hour, a unit of several hours, a unit of several days, a unit of week, or a unit of day of the week.

The state of the user may include an action tendency of the user. The action tendency may include that the user walks for a long time, that the user runs for a long time, and that the user performs a dangerous behavior. The dangerous behavior may include that the user walks on a roadway, and that the user enters the roadway from a sidewalk. The action tendency may be interpreted as a tendency of the user's hyperactive or impulsive action.

In the autonomous processing, the robot 100 and the communication terminal may ask the generative AI a question about the detected state or action of the user, and store an answer of the generative AI to the question and the detected action of the user in association with each other. In this case, the robot 100 and the communication terminal may store action content of correcting the action in association with the answer.

Information in which the answer of the generative AI to the question, the detected action of the user, and the action content of correcting the action are associated with each other may be recorded as table information in a storage medium such as a memory. The table information may be interpreted as specific information recorded in the storage unit.

Specifically, in a case in which the user tends to walk for a long time, the robot 100 and the communication terminal may ask the generative AI a question "What kind of guidance should be given to the user who takes such an action?", and in a case in which the answer of the generative AI to the question is "Propose the same route as the route along which the user has previously passed", the robot 100 and the communication terminal may record the answer, the action of the user who can walk for a long time, and the action content of correcting the action (for example, guidance content of "guiding the user to the route along which the user has previously passed") in association with the table information. In a case in which the action of the user who is walking for a long time is detected, the action determination unit 236 of the robot 100 and the communication terminal may reproduce vocal sound "I will guide you to the route along which you have previously passed" as the action of the robot 100 and the communication terminal by using the table information.

As another example, in a case in which the user tends to run for a long time, the robot 100 and the communication terminal ask the generative AI a question "What kind of guidance should be given to the user who takes such an action?", and in a case in which the answer of the generative AI to the question is "It is desirable to periodically perform guidance for encouraging hydration", the robot 100 and the communication terminal may record the answer, the action of the user who can run for a long time, and the action content of correcting the action (for example, guidance content such as "Please rehydrate frequently") in association with the table information. In a case in which the action of the user who is running for a long time is detected, the action determination unit 236 of the robot 100 and the communication terminal may reproduce vocal sound "Please rehydrate frequently" as the action of the robot 100 and the communication terminal by using the table information.

As still another example, in a case in which the user tends to walk on the roadway, the robot 100 and the communication terminal may ask the generative AI a question "What kind of guidance should be given to the user who takes such an action?", and in a case in which the answer of the generative AI to the question is "I recommend to propose returning to the sidewalk because there is a possibility of coming into contact with vehicles", the robot 100 and the communication terminal may record the answer, the action of the user who may walk on the roadway, and the action content of correcting the action (for example, guidance content of "Please return to the sidewalk") in association with the table information. In a case in which the action of the user walking on the sidewalk is detected, the action determination unit 236 of the robot 100 and the communication terminal may reproduce vocal sound "Please return to the sidewalk" as the action of the robot 100 and the communication terminal by using the table information.

In the autonomous processing, an action schedule of the robot 100 and the communication terminal for attracting attention with respect to a state or an action of the user may be set on the basis of the detected action of the user and the stored specific information.

As described above, the robot 100 and the communication terminal may record table information in which the answer of the generative AI corresponding to the state or the action of the user is associated with the detected state or action of the user in the storage medium. Hereinafter, an example of content stored in the table will be described.

### (1. Case in Which User Tends to Walk for a Long Time)

In a case in which there is the tendency, the robot 100 and the communication terminal themselves ask the generative AI a question "What kind of guidance should be given to the user who takes such an action?". In a case in which the answer of the generative AI to this question is, for example, "Propose a route different from a route along which the user has previously passed", "Propose a route that guides the user to a place where the user can take shelter from rain because it is likely to rain", or "Propose a route with a relatively small number of vehicles such as bicycles", the robot 100 and the communication terminal may store the action of the user and the answer of the generative AI in association with each other.

### (2. Case in Which User Tends to Run for a Long Time)

In a case in which there is the tendency, the robot 100 and the communication terminal themselves ask the generative AI a question "What kind of guidance should be given to the user who takes such an action?". In a case in which the answer of the generative AI to this question is, for example, "Provide guidance for encouraging hydration periodically", "Propose a route with fewer bicycles and clean air", or "Propose a route with fewer traffic lights", the robot 100 and the communication terminal may store the action of the user and the answer of the generative AI in association with each other.

### (3. Case in Which User Tends to Walk on Roadway)

In a case in which there is the tendency, the robot 100 and the communication terminal themselves ask the generative AI a question "What kind of situation can occur to the user who takes such an action?". In a case in which the answer of the generative AI to this question is, for example, "There is a possibility of coming into contact with a vehicle such as a bicycle", "There is a possibility of entering an expressway interchange", or "Advice should be given to encourage the user to walk on the sidewalk as much as possible", the robot 100 and the communication terminal may store the action of the user of walking on the roadway and the answer of the generative AI in association with each other. The robot 100 and the communication terminal may store action content of correcting the action in association with the answer.

The action content of correcting the action may include at least one of reproduction of vocal sound for correcting a dangerous action of the user or reproduction of an image for correcting the dangerous action of the user.

The vocal sound for correcting the dangerous action of the user may include vocal sound for guiding the user to a specific place. The specific place may include a place other than the place where the user is currently located, for example, a sidewalk. Specifically, the vocal sound may include vocal sound such as "Please stop walking on the roadway", "That is not a sidewalk", "The roadway is dangerous", and "Please move to the sidewalk immediately".

As described above, in the autonomous processing, a table in which the answer of the generative AI corresponding to the state or action of the user, the content of the state or action, and the action content of correcting the state or action are associated with each other may be recorded in a storage medium such as a memory.

In the autonomous processing, after the table is recorded, the action of the user may be autonomously or periodically detected, and the action schedule of the communication terminal for attracting the attention of the user may be set on the basis of the detected action of the user and the content of the stored table. Specifically, the action determination unit 236 of the robot 100 and the communication terminal may set the first action content on the basis of the detected action of the user and the content of the stored table.

Specifically, the action determination unit 236 may spontaneously or periodically detect an action of the user on the basis of the sensor, and determine the first action content in the case of determining to lead the user as an action of the electronic apparatus on the basis of the detected action of the user and the specific information stored in advance.

The sensor may be interpreted as any sensor of one or a plurality of sensors provided in the communication terminal. Specifically, the one or plurality of sensors may include a camera, a microphone, a gyro sensor, and the like.

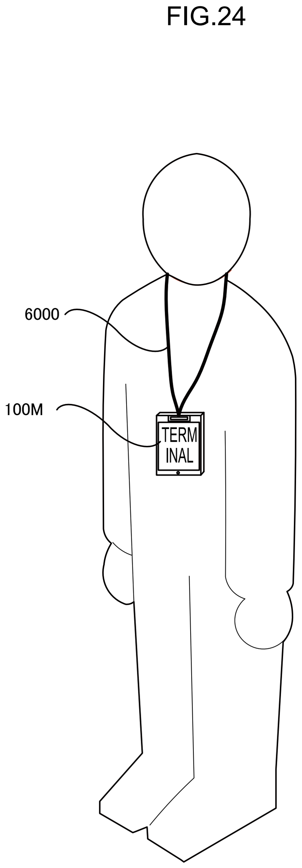

Here, the communication terminal will be described in detail with reference to Fig. 24.

Fig. 24 is an external view of the communication terminal according to the embodiment of the disclosure. As illustrated in Fig. 24, the communication terminal 100M may be interpreted as an electronic apparatus detachably provided on a neck strap 6000. The communication terminal 100M may be interpreted as a smartphone that will be described later, or may be interpreted as a smart AI device. The communication terminal 100M may have the functions of the robots 100 to 102 described above. The neck strap 6000 may include a hook-shaped member that is locked to the communication terminal 100M.

### (First Action Content)

The first action content may include an action of the communication terminal 100M that leads the user when the communication terminal 100M reproduces at least one of vocal sound or an image. Hereinafter, an example of the first action content will be described.

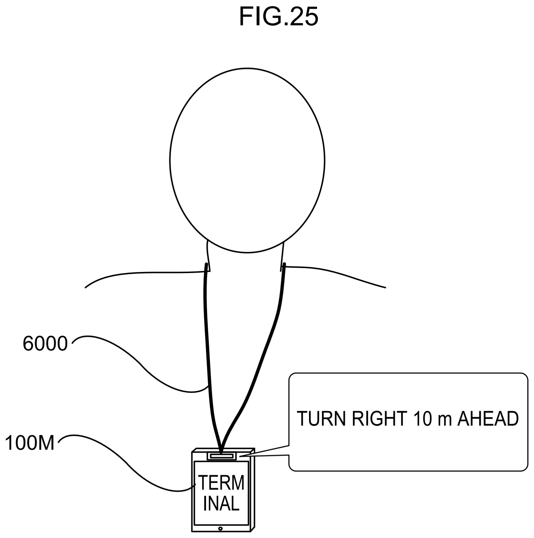

The action determination unit 236 may execute reproduction of at least one of vocal sound or an image for assisting in walking of the user as the first action content for performing route guidance so that the walking user moves along a specific route. Specifically, in a case in which it is detected that the communication terminal 100M is attached to the neck strap 6000, the action determination unit 236 may spontaneously search for a place where the user tends to go regularly and guide the user along a route to the place that has been searched for. In a case in which it is detected that the camera included in the sensor of the communication terminal 100M is directed in an advancing direction of the user, the action determination unit 236 may spontaneously search for a place where the user tends to go regularly and guide the user along a route to the place that has been searched for. The action determination unit 236 may reproduce vocal sound related to guidance of a route along which the user can walk. The vocal sound may include "I searched for a place where Mr. A frequently goes at this time. I will start guiding.", "Turn right 10 m ahead" illustrated in Fig. 25, and the like. The action determination unit 236 may reproduce an image related to guidance of a route along which the user can walk on the screen of the communication terminal 100M.

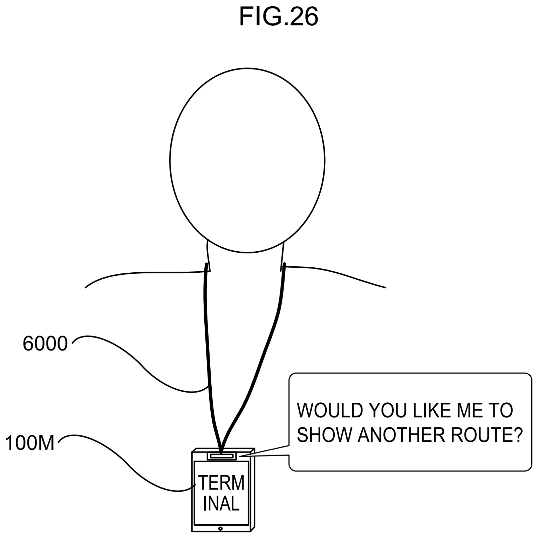

The action determination unit 236 may execute reproduction of at least one of vocal sound and an image for guiding the user along a route different from the set guidance route. Specifically, the action determination unit 236 may spontaneously re-search for a place where the user tends to go regularly, including the traffic condition of the set guidance route, the climate near the route, the current time, and the like, and reproduce vocal sound or the like related to a route to the place that has been re-searched for, that is, a route different from the set guidance route. More specifically, the vocal sound may include "I searched for another route because of the heavy traffic today. Would you like me to show another route?", "The road is closed ahead. Would you like me to show another route?" as illustrated in Fig. 26, and the like.

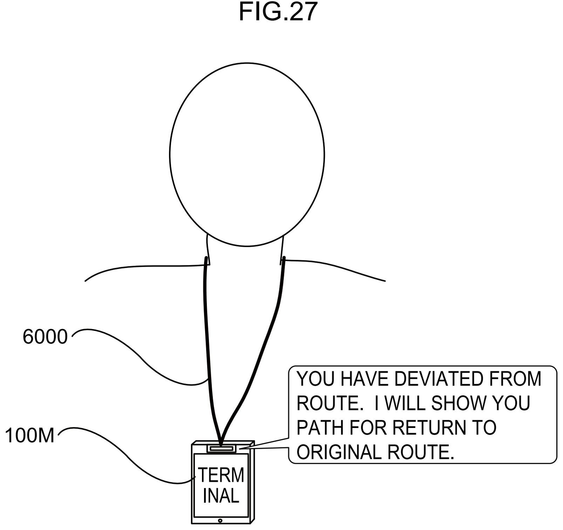

In a case in which the user led along the set guidance route deviates from the guidance route, the action determination unit 236 may spontaneously search for a path for returning the user to the guidance route and execute reproduction of at least one of vocal sound or an image for guiding the user to the path that has been searched for. Specifically, the action determination unit 236 may spontaneously re-search for a place where the user can return, including the traffic condition of a path deviating from the guidance route, the climate near the path, the current time, events that have occurred in the past near the route, and the like, and reproduce vocal sound related to the route to the re-searched place. That is, the vocal sound related to the path for return to the set guidance route may be reproduced. More specifically, the vocal sound may include "Since there are few people and it is dangerous, I searched for a path for return to the original route.", "It started to rain. It seems to be an area where a mudslide occurred before. Do you want to return to the original road?", "You have deviated from the route. I will show you the path for return to the original route." as illustrated in Fig. 27, and the like. The action determination unit 236 of the robot 100 and the communication terminal may execute reproduction of vocal sound such as the above vocal sound, for example, "I will guide you to the route along which you have previously passed", "Please rehydrate frequently", and "Please return to the sidewalk" or an image.

### (Second Action Content)

The action determination unit 236 may determine whether or not the action of the user has been corrected by detecting the action of the user while the communication terminal 100M was leading the user or after the communication terminal 100M reproduced at least one of vocal sound or an image that assists in the walking of the user, and may determine second action content different from the first action content in a case in which the action of the user has been corrected.

The case in which the action of the user has been corrected may be interpreted as a case in which the user has stopped a specific action and a specific behavior or a case in which a specific situation has been resolved as a result of execution of the operation of the communication terminal 100M according to the first action content. Specifically, the case in which the action of the user has been corrected may include a case in which the user has started walking along the proposed different route, a case in which the user has started walking along a path for return to the original route when the path was shown due to deviation from the original route, and the like.

The second action content may include reproduction of at least one of vocal sound praising the user for action, vocal sound expressing gratitude for the action of the user, an image praising the user for the action, or an image expressing gratitude for the action of the user.

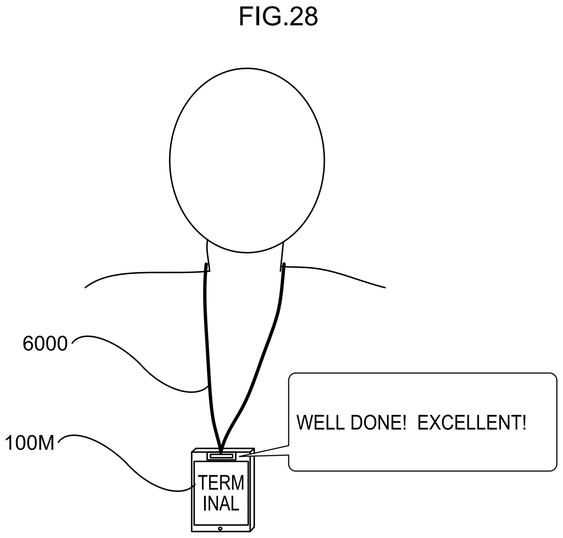

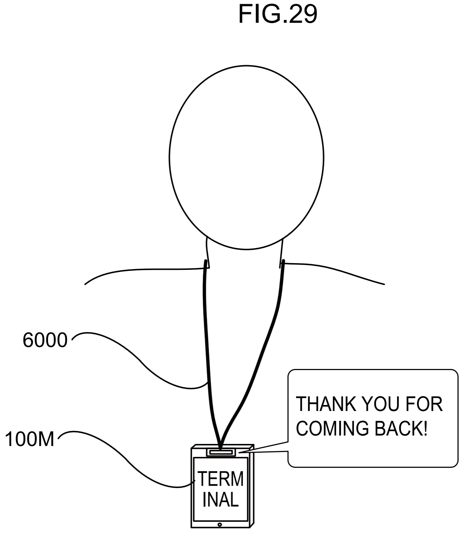

Specifically, the vocal sound praising the user for the action may include, for example, "Well done! Excellent" as illustrated in Fig. 28. The vocal sound expressing gratitude for the action of the user may include vocal sound such as "Thank you for coming back" or "Thank you for selecting another route" as illustrated in Fig. 29. The image praising the user for the action may include an image of a character taking a good pose. The image expressing gratitude for the action of the user may include an image of a character who expresses gratitude.

### (Third Action Content)

The action determination unit 236 may determine whether or not the action of the user has been corrected by detecting the action of the user while the communication terminal 100M was leading the user or after the communication terminal 100M reproduced at least one of vocal sound or an image that assists in the walking of the user, and determine third action content different from the first action content in a case in which the action of the user has not been corrected.

The case in which the action of the user has not been corrected may be interpreted as a case in which the user has continued the dangerous action and behavior even though the operation of the communication terminal 100M according to the first action content was executed, a case in which the dangerous situation has not been resolved, or a case in which the user has started or continued an action different from the proposal of the communication terminal 100M.

Specifically, the case in which the action of the user has not been corrected may include a case in which the user has walked along a route different from the proposed different route without walking along the proposed different route, a case in which the user has neglected a path for return to the original route and continued walking when the path was shown due to deviation from the route, and the like.

The third action content may include at least one of transmission of specific information to a person other than the user, reproduction of sound that attracts an interest of the user, or reproduction of an image that attracts an interest of the user.

The transmission of the specific information to a person other than the user may include distribution of an email containing a warning message to a relative of the user, a spouse of the user, a guardian of the user, or the like. The transmission of the specific information to a person other than the user may include distribution of an image (a still image or a moving image) including the user and the surrounding scenery to a relative of the user, a spouse of the user, a guardian of the user, or the like. The transmission of the specific information to a person other than the user may include distribution of vocal sound having a warning message.

The reproduction of the sound that attracts the interest of the user may include specific music that the user likes, and may also include vocal sound such as "Please come back here" or "Let's take a safe road".

The reproduction of the image that attracts the interest of the user may include reproduction of an image of an animal kept by the user, an image of parents of the user, an image of a character of the animal, and the like.

According to the robot 100 and the communication terminal of the disclosure, an action of leading the user can be executed through autonomous processing. As a result, the robot 100 and the communication terminal can guide the user walking in a town that the user visits for the first time to an appropriate route. The user who is interested in walking may be guided to a route along which the user has not passed before. A route where the user cannot pass is detected in advance, and the user can be guided to another route. The user who gets lost and deviates from the specific route can be guided to the original route.

The action determination unit 236 determines, as the action of the robot 100 and the communication terminal, any of a plurality of types of robot actions and communication terminal actions including no action, by using at least one of the state of the user 10, the emotion of the user 10, the emotion of the robot 100 and the communication terminal, or the state of the robot 100 and the communication terminal, and the action determination model 221 at a predetermined timing. Here, a case in which a sentence generation model having an interaction function is used as the action determination model 221 will be described as an example.

Specifically, the action determination unit 236 inputs text representing at least one of the state of the user 10, the emotion of the user 10, the emotion of the robot 100 and the communication terminal, or the state of the robot 100 and the communication terminal, and text for inquiry about robot actions and communication terminal actions to the sentence generation model, and determines the actions of the robot 100 and the communication terminal on the basis of an output of the sentence generation model.

For example, the plurality of types of actions of robot and the communication terminal include the following (1) to (26).
(1) The robot 100 and the communication terminal 100M do nothing.
(2) The robot 100 and the communication terminal 100M have a dream.
(3) The robot 100 and the communication terminal 100M talk to the user.
(4) The robot 100 and the communication terminal 100M create a picture diary.
(5) The robot 100 and the communication terminal 100M propose an activity.
(6) The robot 100 and the communication terminal 100M propose a person with whom the user should meet.
(7) The robot 100 and the communication terminal 100M introduce news in which the user is interested.
(8) The robot 100 and the communication terminal 100M edit pictures and moving images.
(9) The robot 100 and the communication terminal 100M study with the user.
(10) The robot 100 and the communication terminal 100M evoke memories.
(11) The robot 100 and the communication terminal 100M may reproduce an image of a route along which the user can walk on the screen of the communication terminal 100M as the first action content of leading the user.
(12) The robot 100 and the communication terminal 100M may reproduce vocal sound related to a route along which the user can walk as the first action content of correcting the action of the user.
(13) The robot 100 and the communication terminal 100M may reproduce an image of a route different from the set guidance route on the screen of the communication terminal 100M as the first action content of correcting the action of the user.
(14) The robot 100 and the communication terminal 100M may reproduce vocal sound related to a route different from the set guidance route as the first action content of correcting the action of the user.
(15) The robot 100 and the communication terminal 100M may reproduce, as the first action content of correcting the action of the user, an image of a route for return to the set guidance route on the screen of the communication terminal 100M in a case in which the user led along the set guidance route deviates from the guidance route.
(16) The robot 100 and the communication terminal 100M may reproduce, as the first action content of correcting the action of the user, vocal sound related to a route for return to the set guidance route in a case in which the user led along the set guidance route deviates from the guidance route.
(17) The robot 100 and the communication terminal 100M may reproduce vocal sound praising the user for the action as the second action content different from the first action content.
(18) The robot 100 and the communication terminal 100M may reproduce vocal sound expressing gratitude for the action of the user as the second action content different from the first action content.
(19) The robot 100 and the communication terminal 100M may reproduce an image praising the user for the action as the second action content different from the first action content.
(20) The robot 100 and the communication terminal 100M may reproduce an image expressing gratitude for the action of the user as the second action content different from the first action content.
(21) The robot 100 and the communication terminal 100M may transmit specific information to a person other than the user as the third action content different from the first action content.
(22) The robot 100 and the communication terminal 100M may reproduce sound that attracts the interest of the user as the third action content different from the first action content.
(23) The robot 100 and the communication terminal 100M may reproduce an image that attracts the interest of the user as the third action content different from the first action content.

The action determination unit 236 inputs, to the sentence generation model, text representing the state of the user 10 and the state of the robot 100 or the communication terminal 100M recognized by the state recognition unit 230, and the current emotion value of the user 10 and the current emotion value of the robot 100 or the current emotion value of the communication terminal 100M determined by the emotion determination unit 232, and text for inquiry about any of a plurality of types of robot actions including no action, each time a certain period of time elapses, and determines actions of the robot 100 and the communication terminal 100M on the basis of an output of the sentence generation model. Here, in a case in which the user 10 is absent around the robot 100 or the communication terminal 100M, the text to be input to the sentence generation model need not include the state of the user 10 and the current emotion value of the user 10, or may include the fact that the user 10 is absent. Hereinafter, the communication terminal 100M may be read as a robot. The communication terminal 100M may be interpreted as a smartphone or a smart AI device. The robot action may be read as a communication terminal action.

As an example, text such as "The robot is in a very pleasant state. The user is in a normally pleasant state. The user is sleeping. Which one of the following (1) to (26) is better as an action of the robot?
(1) The robot does nothing.
(2) The robot has a dream.
(3) The robot talks to the user...." is input to the sentence generation model. On the basis of the output "It can be said that either (1) the robot does nothing or (2) the robot has a dream is the most appropriate action." of the sentence generation model, "(1) the robot does nothing" or "(2) the robot has a dream" is determined as the action of the robot 100.

As another example, text such as "The robot is in a slightly lonely state. The user is absent. The surroundings of the robot are dark. Which one of the following (1) to (26) is better as an action of the robot? (1) The robot does nothing.
(2) the robot has a dream.
(3) the robot talks to the user.
   ..." is input to the sentence generation model. On the basis of the output "It can be said that either (2) the robot has a dream or (4) the robot creates a picture diary is the most appropriate action." of the sentence generation model, "(2) The robot has a dream" or "(4) The robot creates a picture diary." is determined as the action of the robot 100.

In a case in which the action determination unit 236 determines that "(2) The robot has a dream.", that is, the creation of the original event is to be performed as the robot action, the action determination unit 236 creates the original event obtained by combining a plurality of pieces of event data in the history data 222 by using the sentence generation model. In this case, the storage control unit 238 stores the created original event in the history data 222.

In a case in which it is determined that "(3) The robot talks to the user.", that is, the robot 100 speaks, as the robot action, the action determination unit 236 determines speech content of the robot corresponding to the user state and the emotion of the user or the emotion of the robot by using the sentence generation model. In this case, the action control unit 250 causes the speaker included in the control target 252 to output vocal sound representing the determined speech content of the robot. In a case in which the user 10 is absent around the robot 100, the action control unit 250 stores the determined speech content of the robot in the action schedule data 224 without outputting vocal sound representing the determined speech content of the robot.

In a case in which it is determined that "(7) The robot introduces news in which the user is interested." as the robot action, the action determination unit 236 determines the speech content of the robot corresponding to the information stored in the collected data 223 by using the sentence generation model. In this case, the action control unit 250 causes the speaker included in the control target 252 to output vocal sound representing the determined speech content of the robot. In a case in which the user 10 is absent around the robot 100, the action control unit 250 stores the determined speech content of the robot in the action schedule data 224 without outputting vocal sound representing the determined speech content of the robot.

In a case in which it is determined that "(4) The robot creates a picture diary.", that is, the robot 100 creates an event image, as the robot action, the action determination unit 236 generates an image representing event data for the event data selected from the history data 222 by using the image generation model, generates an explanatory sentence representing the event data by using the sentence generation model, and outputs a combination of the image representing the event data and the explanatory sentence representing the event data as the event image. In a case in which the user 10 is absent around the robot 100, the action control unit 250 stores the event image in the action schedule data 224 without outputting the event image.

In a case in which it is determined that "(8) The robot edits pictures and moving images.", that is, the robot edits an image, as the robot action, the action determination unit 236 selects event data from the history data 222 on the basis of the emotion value, edits image data of the selected event data, and outputs the edited image data. In a case in which the user 10 is absent around the robot 100, the action control unit 250 stores the edited image data in the action schedule data 224 without outputting the edited image data.

In a case in which it is determined that "(5) The robot proposes an activity.", that is, the robot proposes an action of the user 10, as the robot action, the action determination unit 236 determines the proposed action of the user by using the sentence generation model on the basis of the event data stored in the history data 222. In this case, the action control unit 250 causes the speaker included in the control target 252 to output vocal sound proposing the action of the user. In a case in which the user 10 is absent around the robot 100, the action control unit 250 stores a proposal of the action of the user in the action schedule data 224 without outputting vocal sound proposing the action of the user.

In a case in which it is determined that "(6) The robot proposes a person with whom the user should meet.", that is, the robot proposes a partner who should have a contact with the user 10, as the robot action, the action determination unit 236 determines a proposed partner who should have a contact with the user by using the sentence generation model on the basis of the event data stored in the history data 222. In this case, the action control unit 250 causes the speaker included in the control target 252 to output vocal sound representing a proposal of a partner who should have a contact with the user. In a case in which the user 10 is absent around the robot 100, the action control unit 250 stores a proposal of a partner who should have a contact with the user in the action schedule data 224 without outputting vocal sound representing a proposal of a partner who should have a contact with the user.

In a case in which it is determined that "(9) The robot studies with the user.", that is, the robot 100 makes speech related to study, as the robot action, the action determination unit 236 uses the sentence generation model to determine speech content of the robot for urging the user to study, giving a study problem, or giving advice related to study, which corresponds to the user state and the emotion of the user or the emotion of the robot. In this case, the action control unit 250 causes the speaker included in the control target 252 to output vocal sound representing the determined speech content of the robot. In a case in which the user 10 is absent around the robot 100, the action control unit 250 stores the determined speech content of the robot in the action schedule data 224 without outputting vocal sound representing the determined speech content of the robot.

In a case in which it is determined that "(10) The robot evokes memories.", that is, the robot causes event data to be remembered, as the robot action, the action determination unit 236 selects the event data from the history data 222. In this case, the emotion determination unit 232 determines an emotion of the robot 100 on the basis of the selected event data. The action determination unit 236 creates an emotion change event representing speech content or an action of the robot 100 for changing the emotion value of the user by using the sentence generation model on the basis of the selected event data. In this case, the storage control unit 238 stores the emotion change event in the action schedule data 224.

For example, in a case in which it is stored in the history data 222 that a moving image the user was watching was related to a panda as event data, and the event data is selected, a sentence such as "What is the word to say about the topic related to a panda when meeting with the user next time? Please list three." is input to the sentence generation model, and in a case in which the output of the sentence generation model is "(1) Let's go to the zoo, (2) Let's draw a picture of a panda, and (3) Let's buy a stuffed toy of a panda.", the robot 100 inputs a sentence such as "What makes the user most happy in (1), (2), and (3)?" to the sentence generation model, and in a case in which the output of the sentence generation model is "(1) Let's go to the zoo", and speech of the robot 100 that "(1) Let's go to the zoo" in a case in which the robot 100 meets the user next is created as the emotion change event and stored in the action schedule data 224.

For example, event data having a great emotion value of the robot 100 is selected as impressive memory of the robot 100. This makes it possible to create an emotion change event on the basis of the event data selected as impressive memory.

The action determination unit 236 may spontaneously or periodically detect the action of the user on the basis of the sensor, and in a case in which it is determined to lead the user as the action of the electronic apparatus that is the communication terminal 100M on the basis of the detected action of the user and the specific information stored in advance, execute the following first action content. The action determination unit 236 may determine the first action content in a case in which the camera included in the sensor is directed in an advancing direction of the user. As a result, the communication terminal 100M can start leading the user only in a case in which the user desires to execute route guidance.

The action determination unit 236 may perform, as the communication terminal action, the first action content in the above "(11)", that is, reproduce an image of a route along which the user can walk on the screen of the communication terminal 100M.

The action determination unit 236 may perform, as the communication terminal action, the first action content in the above "(12)", that is, reproduce vocal sound related to a route along which the user can walk.

The action determination unit 236 may perform, as the communication terminal action, the first action content in the above "(13)", that is, reproduce an image of a route different from the set guidance route as the first action content of correcting the action of the user on the screen of the communication terminal 100M.

The action determination unit 236 may perform, as the communication terminal action, the first action content in the above "(14)", that is, reproduce vocal sound related to a route different from the set guidance route.

The action determination unit 236 may perform, as the communication terminal action, the first action content in the above "(15)", that is, reproduce an image of a route for return to the set guidance route on the screen of the communication terminal 100M in a case in which the user led along the set guidance route deviates from the guidance route.

The action determination unit 236 may perform, as the communication terminal action, the first action content in the above "(16)", that is, reproduce vocal sound related to a route for return to the set guidance route in a case in which the user led along the set guidance route deviates from the guidance route.

The action determination unit 236 may execute the second action content different from the first action content. Specifically, the action determination unit 236 may perform, as the movement system action, the second action content in the above "(17)", that is, reproduce vocal sound praising the user for the action.

The action determination unit 236 may perform, as the movement system action, the second action content in the above "(18)", that is, reproduce vocal sound expressing gratitude for the action of the user.

The action determination unit 236 may perform, as the communication terminal action, the second action content in the above "(19)", that is, reproduce an image praising the user for the action.

The action determination unit 236 may perform, as the communication terminal action, the second action content in the above "(20)", that is, reproduce an image expressing gratitude for the action of the user.

The action determination unit 236 may execute the third action content different from the first action content. Specifically, the action determination unit 236 may perform, as the communication terminal action, the third action content in the above "(21)", that is, transmit the specific information to a person other than the user.

The action determination unit 236 may perform, as the movement system action, the third action content in the above "(22)", that is, execute reproduction of sound that attracts the interest of the user.

The action determination unit 236 may perform, as the movement system action, the third action content in the above "(23)", that is, execute reproduction of an image that attracts the interest of the user.

In a case in which, for example, an image of a route along which the user can walk is reproduced as the first action content indicated in the above "(11)" to "(16)", the related information collection unit 270 may store data such as an image of a route along which the user can walk in the collected data 223.

In a case in which, for example, vocal sound praising the user for the action is reproduced as the second action content indicated in the above "(17)" to "(20)", the related information collection unit 270 may store data such as an image of a route along which the user can walk in the collected data 223.

In a case in which, for example, specific information is transmitted to a person other than the user as the third action content indicated in the above "(21)" to "(23)", the related information collection unit 270 may store data of the specific information or the like in the collected data 223.

On the basis of the state of the user 10 recognized by the state recognition unit 230, in a case in which an action of the user 10 with respect to the robot 100 is detected from a state in which there is no action of the user 10 with respect to the robot 100, the action determination unit 236 reads the data stored in the action schedule data 224 and determines an action of the robot 100.

For example, in a case in which the user 10 was absent around the robot 100 and then the user 10 is detected, the action determination unit 236 reads the data stored in the action schedule data 224 and determines an action of the robot 100. In a case in which the user 10 was sleeping, and it is then detected that the user 10 has woken up, the action determination unit 236 reads the data stored in the action schedule data 224 and determines an action of the robot 100.

A part of the robot 100 (for example, the sensor module unit 210, the storage unit 220, and the control unit 228) may be provided outside the robot 100 (for example, a server), and the robot 100 may function as each unit of the robot 100 by communicating with the outside.

### (Supplementary Note 1)

A control system including:
a state recognition unit that recognizes a user state including an action of a user, and a state of an electronic apparatus;
an emotion determination unit that determines an emotion of the user or an emotion of the electronic apparatus; and
an action determination unit that determines, by using at least one of the user state, the state of the electronic apparatus, the emotion of the user, or the emotion of the electronic apparatus, and an action determination model at a predetermined timing, any of a plurality of types of device operations including non-operation as an action of the electronic apparatus, in which
the electronic apparatus detachably provided on a neck strap includes one or a plurality of sensors that collect information, and
the device operations include setting first action content of leading the user by the electronic apparatus reproducing at least one of vocal sound or an image.

### (Supplementary Note 2)

The control system according to Supplementary Note 1, in which the action determination unit spontaneously or periodically detects an action of the user on the basis of the sensor, and determines the first action content in a case in which leading the user is determined as the action of the electronic apparatus on the basis of the detected action of the user and specific information stored in advance.

### (Supplementary Note 3)

The control system according to Supplementary Note 2, in which the action determination unit determines the first action content in a case in which a camera included in the sensor is directed in an advancing direction of the user.

### (Supplementary Note 4)

The control system according to Supplementary Note 2, in which the first action content includes reproduction of at least one of vocal sound or an image that assists in walking of the user.

### (Supplementary Note 5)

The control system according to Supplementary Note 4, in which the reproduction includes reproduction of at least one of vocal sound or an image for guiding the user along a route different from a set guidance route.

### (Supplementary Note 6)

The control system according to Supplementary Note 5, in which the reproduction includes reproduction of at least one of vocal sound or an image for guiding the user along a path for returning the user to the set guidance route in a case in which the user guided along the guidance route deviates from the guidance route.

### (Supplementary Note 7)

The control system according to Supplementary Note 4, in which the action determination unit determines whether or not the action of the user has been corrected by detecting the action of the user while leading the user or after reproducing at least one of the vocal sound or the image, and determines second action content different from the first action content in a case in which the action of the user has been corrected.

### (Supplementary Note 8)

The control system according to Supplementary Note 7, in which the second action content includes reproduction of at least one of vocal sound praising the user for the action, vocal sound expressing gratitude for the action of the user, an image praising the user for the action, or an image expressing gratitude for the action of the user.

### (Supplementary Note 9)

The control system according to Supplementary Note 4, in which the action determination unit determines whether or not the action of the user has been corrected by detecting the action of the user while leading the user or after reproducing at least one of the vocal sound or the image, and determines third action content different from the first action content in a case in which the action of the user has not been corrected.

### (Supplementary Note 10)

The control system according to Supplementary Note 9, in which the third action content includes at least one of transmission of specific information to a person other than the user, reproduction of sound that attracts an interest of the user, or reproduction of an image that attracts the interest of the user.

### (Supplementary Note 11)

The control system according to Supplementary Note 1, in which
the electronic apparatus is a communication terminal, and
the action determination unit determines any of a plurality of types of actions of an agent for interacting with the user, including no action, as an action of the communication terminal.

### (Supplementary Note 12)

The control system according to Supplementary Note 11, in which
the action determination model is a sentence generation model having an interaction function, and the action determination unit inputs, to the sentence generation model, text representing at least one of the user state, a state of the agent, the emotion of the user, or an emotion of the agent, and text for inquiry about an action of the agent, and determines an action of the communication terminal on the basis of an output of the sentence generation model.

### (Other Embodiment 1)

Next, a flow in a case in which the agent system 500 monitors an emotion of the user 10 and executes a watching function of executing a predetermined action on behalf of the user 10 as necessary will be described.

In a case in which the emotion of the user 10 determined by the emotion determination unit 232 is a negative emotion, the action determination unit 236 inputs, to the sentence generation model, text representing the emotion of the user 10 and text for asking a question about words to be spoken to the user 10. For example, the emotion determination unit 232 determines the emotion of the user 10 on the basis of vocal sound of the user 10 analyzed by the vocal sound emotion recognition unit 211 of the sensor module unit 210. The above method of determining the emotion of the user 10 is merely an example, and the emotion determination unit 232 may determine the emotion of the user 10 by, as appropriate, using information analyzed by the sensor module unit 210 and a state of the user 10 recognized by the state recognition unit 230.

Here, in a case in which the negative emotion of the user 10 determined by the emotion determination unit 232 is "anxiety", the action determination unit 236 inputs, for example, text such as "Please tell me the words to be spoken to people who have the following negative emotion. Negative emotion: Anxiety" to the sentence generation model. On the basis of an output of the sentence generation model, the action determination unit 236 determines the speech content of the agent to be output, by using vocal sound, from the speaker included in the control target 252B of the communication terminal 100M as the action of the communication terminal 100M. The action determination unit 236 determines "You look anxious. Is there something wrong?" as speech content of the agent on the basis of the output of the sentence generation model according to the input of the text.

The action control unit 250 outputs the speech content of the agent determined by the action determination unit 236 by using vocal sound from the speaker. Specifically, the action control unit 250 causes the speaker to output, by using vocal sound, the content of "You look anxious. Is there something wrong?".

Thereafter, the action determination unit 236 inputs, to the sentence generation model, text representing the interaction content between the user 10 and the agent, the text including the speech content of the agent output from the speaker by using vocal sound and the speech content of the user 10 as a state of the user 10 recognized by the state recognition unit 230 on the basis of the text information output by the speech understanding unit 212 of the sensor module unit 210, and text for asking a question about a method of solving the problem held by the user 10. The action determination unit 236 inputs, for example, text "Please let me know how to solve the problem held by the user 10 on the basis of the following interaction content between the user 10 and the agent. Agent: You look anxious. Is there something wrong? User 10: I miss my mother. Agent: Do you know where your mother has gone? User 10: Yes. She is shopping at the shop." to the sentence generation model. The action determination unit 236 determines the speech content of the agent to be output from the speaker on the basis of the output of the sentence generation model. For example, the action determination unit 236 determines "Is that so? If you wait a little more, she will come back. Would you like to talk with me until then?" as the speech content of the agent on the basis of the output of the sentence generation model according to the input of the text.

The action control unit 250 outputs a solution to the problem of the user 10 by using vocal sound as the speech content of the agent from the speaker. Specifically, the action control unit 250 causes the speaker to output, as the speech content of the agent, the content such as "Is that so? If you wait a little more, she will come back. Would you like to talk with me until then?" determined above by the action determination unit 236.

With the above configuration, according to the agent system 500, even in a case in which the user 10 cannot conceive a solution to the problem by himself/herself, the problem can be solved through the support of the agent.

In the above description, an example has been described in which the agent system 500 derives a method of solving the problem held by the user 10 on the basis of the interaction content between the user 10 and the agent. Next, a flow of processing of the watching function of the agent system 500 in a case in which the user 10 has difficulty in interacting with the agent will be described.

In a case in which the emotion of the user 10 determined by the emotion determination unit 232 is a negative emotion and it is difficult for the user 10 to interact with the agent, the action determination unit 236 inputs, to the sentence generation model, text representing the user state of the user 10 and the emotion of the user 10, and text for asking a question about a problem held by the user 10. For example, in a case in which the vocal sound of the user 10 cannot be detected by the microphone 201 for a predetermined time or more after the speech content of the agent is output from the speaker, or in a case in which the vocal sound of the user 10 detected by the microphone 201 cannot be analyzed by the speech understanding unit 212, the action determination unit 236 determines that it is difficult for the user 10 to interact with the agent.

In the above case, the action determination unit 236 inputs, for example, text such as "Please let me know the problem held by the user 10 on the basis of the following user state and emotion of the user 10. User state: The user 10 is alone, and the probability that the user 10 is crying is 99%. Emotion of the user 10: Anxiety" to the sentence generation model. On the basis of the output of the sentence generation model, the action determination unit 236 determines to transmit problem information indicating a problem held by the user 10 to a terminal of a related person having a predetermined relationship with the user 10 as an action of the communication terminal 100M. The predetermined relationship includes, for example, a blood relationship, a family relationship, a matrimony relationship, a romantic relationship, a friendship relationship, and a personal relationship at school or a workplace. In a predetermined relationship, it is desirable that the related person likes the user 10. Here, as an example, the predetermined relationship is a blood relationship, and the user 10 is a "child" and the related person is a "mother" of the user 10. For example, the action determination unit 236 determines to transmit the problem information "The child is probably lost." to a terminal of the mother of the user 10 (for example, a smartphone) on the basis of the output of the sentence generation model according to the input of the text. The communication processing unit 280 transmits the problem information to the terminal of the mother of the user 10.

With the above configuration, according to the agent system 500, the agent can transmit the problem held by the user 10 to the mother of the user 10 on behalf of the user 10 even in a case in which the user 10 is unable to communicate effectively.

As another example, in a case in which it is difficult for the user 10 to interact with the agent, the agent system 500 may ask not only the above-described related persons but also other users existing around the user 10 for help.

The emotion determination unit 232 determines an emotion of another user on the basis of a captured image obtained by imaging the another user existing around the user 10 with the 2D camera 203. For example, the emotion determination unit 232 determines an emotion of another user on the basis of an expression of the another user recognized by the expression recognition unit 213 of the sensor module unit 210. The 2D camera 203 is an example of a "camera". The emotion determination unit 232 may determine the emotion of the another user by, as appropriate, using the information analyzed by another sensor module unit 210 and the state of the another user recognized by the state recognition unit 230 in addition to the expression of the another user recognized by the expression recognition unit 213.

In a case in which the emotion of the another user determined by the emotion determination unit 232 is a positive emotion, the action determination unit 236 inputs, to the sentence generation model, the captured image, the text representing the problem held by the user 10, and the text for asking a question about words to be spoken to the another user. The action determination unit 236 inputs, for example, a prompt such as "In consideration of the problem held by the following user 10, please tell me the words to be spoken to the human shown in the following captured image. Problem held by the user 10: lost child captured image: xxxyyyzzz111.jpg" to the sentence generation model. The action determination unit 236 determines the speech content of the agent to be output from the speaker on the basis of the output of the sentence generation model. For example, the action determination unit 236 determines "Excuse me, gentleman wearing the green T-shirt over there, could you please help my lost child?" as the speech content of the agent on the basis of the output of the sentence generation model according to the input of the prompt. The action determination unit 236 may input the above prompt to the sentence generation model only in a case in which the positive emotion of the another user is a specific positive emotion such as "safe" and "relieved", or in a case in which the emotion value of the positive emotion of the another user is a predetermined value or greater.

The action control unit 250 outputs the problem held by the user 10 by using vocal sound as the speech content of the agent from the speaker to another user. As an example, the speaker is a superdirective speaker, and can deliver sound to a target. In this case, the action control unit 250 outputs sound in a direction in which another user exists. In this case, the action control unit 250 causes the speaker to output, as the speech content of the agent, the content of "Excuse me, gentleman wearing the green T-shirt over there, could you please help my lost child?" determined above by the action determination unit 236.

With the above configuration, since the agent system 500 can ask another user having a positive emotion for help, it is possible to increase the possibility of solving the problem held by the user 10 compared with, for example, a case of asking another user having a negative emotion for help without considering the emotion. According to the agent system 500, since the agent is asking for help instead of the user 10, other users can help the user 10 without hesitation. For example, there is a risk that another user may be mistaken for a kidnapper because the another user is carrying the lost user 10. However, according to the agent system 500, since the agent is asking for help instead of the user 10, it is possible to easily explain that the another user is not for the purpose of kidnapping. The another user can ascertain the name, contact information, and the like of the user 10 from the agent by interacting with the agent, and can make a contact for assisting the user 10. As described above, according to the agent system 500, since it is possible to easily explain that the another user is not for the purpose of kidnapping, it is also possible to cause the another user to take the user 10 to a safe place such as a police box.

Here, in a case in which the agent system 500 executes the above-described watching function, the action control unit 250 may output the speech content of the agent by using vocal sound from the speaker in an output aspect according to the emotion of the agent determined by the emotion determination unit 232.

In a case in which it is determined that the emotion of the user 10 is a negative emotion, the emotion determination unit 232 determines the emotion of the agent to be a negative emotion. For example, in a case in which it is determined that the negative emotion of the user 10 is "anxious", the emotion determination unit 232 determines the emotion of the agent to be "sad" and increases the emotion value of "sad".

The action control unit 250 outputs the speech content of the agent from the speaker in the output mode based on the negative emotion of the agent determined by the emotion determination unit 232. For example, in a case in which it is determined that the emotion of the agent is "sad", the action control unit 250 increases the speech speed of the agent output from the speaker or increases the speech volume of the agent output from the speaker.

With the above configuration, according to the agent system 500, since the speech content of the agent is output from the speaker in the output mode based on the negative emotion of the agent, it is possible to express the severity, the degree of urgency, and the like by the way in which the agent conveys the speech.

### (Supplementary Note 1)

A control system including:
a state recognition unit that recognizes a user state including an action of a user, and a state of an electronic apparatus;
an emotion determination unit that determines an emotion of the user or an emotion of the electronic apparatus; and
an action determination unit that determines, by using at least one of the user state, the state of the electronic apparatus, the emotion of the user, or the emotion of the electronic apparatus, and an action determination model at a predetermined timing, any of a plurality of types of device operations including non-operation as an action of the electronic apparatus, in which
the electronic apparatus detachably provided on a neck strap includes one or a plurality of sensors that collect information, and
the device operations include setting a first action content of leading the user by the electronic apparatus reproducing at least one of vocal sound or an image.

### (Supplementary Note 2)

The control system according to Supplementary Note 1, in which the action determination unit spontaneously or periodically detects an action of the user on the basis of the sensor, and determines the first action content in a case in which leading the user is determined as the action of the electronic apparatus on the basis of the detected action of the user and specific information stored in advance.

### (Supplementary Note 3)

The control system according to Supplementary Note 2, in which the action determination unit determines the first action content in a case in which a camera included in the sensor is directed in an advancing direction of the user.

### (Supplementary Note 4)

The control system according to Supplementary Note 2, in which the first action content includes reproduction of at least one of vocal sound or an image that assists in walking of the user.

### (Supplementary Note 5)

The control system according to Supplementary Note 4, in which the reproduction includes reproduction of at least one of vocal sound or an image for guiding the user along a route different from a set guidance route.

### (Supplementary Note 6)

The control system according to Supplementary Note 5, in which the reproduction includes reproduction of at least one of vocal sound or an image for guiding the user along a path for returning the user to the set guidance route in a case in which the user guided along the guidance route deviates from the guidance route.

### (Supplementary Note 7)

The control system according to Supplementary Note 4, in which the action determination unit determines whether or not the action of the user has been corrected by detecting the action of the user while leading the user or after reproducing at least one of the vocal sound or the image, and determines second action content different from the first action content in a case in which the action of the user has been corrected.

### (Supplementary Note 8)

The control system according to Supplementary Note 7, in which the second action content includes reproduction of at least one of vocal sound praising the user for the action, vocal sound expressing gratitude for the action of the user, an image praising the user for the action, or an image expressing gratitude for the action of the user.

### (Supplementary Note 9)

The control system according to Supplementary Note 4, in which the action determination unit determines whether or not the action of the user has been corrected by detecting the action of the user while leading the user or after reproducing at least one of the vocal sound or the image, and determines third action content different from the first action content in a case in which the action of the user has not been corrected.

### (Supplementary Note 10)

The control system according to Supplementary Note 9, in which the third action content includes at least one of transmission of specific information to a person other than the user, reproduction of sound that attracts an interest of the user, or reproduction of an image that attracts the interest of the user.

### (Supplementary Note 11)

The control system according to Supplementary Note 1, in which
the electronic apparatus is a communication terminal, and
the action determination unit determines any of a plurality of types of actions of an agent for interacting with the user, including no action, as an action of the communication terminal.

### (Supplementary Note 12)

The control system according to Supplementary Note 11, in which
the action determination model is a sentence generation model having an interaction function, and the action determination unit inputs, to the sentence generation model, text representing at least one of the user state, a state of the agent, the emotion of the user, or an emotion of the agent, and text for inquiry about an action of the agent, and determines an action of the communication terminal on the basis of an output of the sentence generation model.

### (Supplementary Note 13)

The control system according to Supplementary Note 12, in which
the action determination unit inputs, to the sentence generation model, text representing the emotion of the user and text asking words to be spoken to the user in a case in which the emotion of the user determined by the emotion determination unit is a negative emotion, and determines, on the basis of an output of the sentence generation model, speech content of the agent to be output from a control target included in the communication terminal as an action of the communication terminal, and the control system further includes
an action control unit that controls the control target such that the action of the communication terminal determined by the action determination unit is executed,
the action determination unit inputs, to the sentence generation model, text representing interaction content between the user and the agent including the speech content of the agent output from the control target and the speech content of the user as the user state recognized by the state recognition unit, and text for asking a question about a solution to a problem held by the user, and determines speech content of the agent to be output from the control target on the basis of an output of the sentence generation model, and
the action control unit outputs the solution to the problem held by the user as the speech content of the agent from the control target.

### (Supplementary Note 14)

The control system according to Supplementary Note 13, in which
the action determination unit, in a case in which the emotion of the user determined by the emotion determination unit is a negative emotion and the user has difficulty in interacting with the agent, inputs text representing the user state and the emotion of the user and text for asking a question about the problem held by the user to the sentence generation model, and determines, on the basis of an output of the sentence generation model, transmission of problem information indicating the problem held by the user to a terminal of a related person having a predetermined relationship with the user as the action of the communication terminal, and
the control system further includes a communication processing unit that transmits the problem information to the terminal of the related person.

### (Supplementary Note 15)

The control system according to Supplementary Note 14, in which
the emotion determination unit determines an emotion of another user existing around the user on the basis of a captured image obtained by capturing the another user with a camera,
the action determination unit inputs, in a case in which the emotion of the another user determined by the emotion determination unit is a positive emotion, the captured image, text representing the problem held by the user, and text for asking a question about words to be spoken to the another user to the sentence generation model, and determines speech content of the agent to be output from the control target on the basis of an output of the sentence generation model, and
the action control unit outputs, from the control target, the problem held by the user as the speech content of the agent toward the another user.

### (Supplementary Note 16)

The control system according to Supplementary Note 13, in which
the emotion determination unit determines that the emotion of the agent is a negative emotion in a case in which it is determined that the emotion of the user is a negative emotion, and
the action control unit outputs speech content of the agent from the control target in an output aspect based on the negative emotion of the agent determined by the emotion determination unit.

### (Other Embodiment 2)

Next, a flow in a case in which the agent system 500 executes a support function of supporting moving image capturing performed by using the communication terminal 100M of the user 10 will be described.

The emotion determination unit 232 determines an emotion of another user existing around the user 10. It is assumed that the another user is located within an imaging possible range of the 2D camera 203. For example, the emotion determination unit 232 determines the emotion of the another user on the basis of the expression of the another user recognized by the expression recognition unit 213 of the sensor module unit 210. The emotion determination unit 232 may determine the emotion of the another user by, as appropriate, using information analyzed by another sensor module unit 210 and the state of the another user recognized by the state recognition unit 230 in addition to the expression of the another user recognized by the expression recognition unit 213.

In a case in which the emotion value of the another user determined by the emotion determination unit 232 changes by a predetermined value or more, the action determination unit 236 determines that the 2D camera 203 starts capturing a moving image of the another user as the action of the communication terminal 100M. Here, the predetermined value is set to "2" as an example. The 2D camera 203 is an example of a "camera".

The action control unit 250 controls the 2D camera 203 to capture a moving image of the another user. It is assumed that the action control unit 250 captures a moving image of the another user by using the 2D camera 203 for a predetermined time. A specific moving image that is a moving image obtained by capturing the another user is stored in the storage unit 220. As an example, the 2D camera 203 is an AI automatic tracking camera that automatically tracks and images a recognized subject (here, another user). The AI automatic tracking camera is known as disclosed in, for example, Internet search <URL: https://info.system5.jp/whatsnew/archives/96954>, Internet search <URL: https://weekly.ascii.jp/elem/000/004/061/4061473/>, and thus a detailed description thereof will be omitted.

With the above configuration, according to the agent system 500, a moving image of another user starts to be captured in a case in which the emotion value of the another user changes by a predetermined value or more, so that a moving image can be automatically captured in a case in which the emotion of the another user changes (for example, in the case of feeling fear or feeling happy).

The action determination unit 236 determines to post the specific moving image to a predetermined moving image posting service in a case in which the emotion value of the positive emotion of the another user increases by a predetermined value or more. The communication processing unit 280 posts the specific moving image of the another user in which the emotion value of the positive emotion increases by a predetermined value or more to the predetermined moving image posting service. The moving image posting service is, for example, a social networking service (SNS). The communication processing unit 280 automatically posts the specific moving image to the moving image posting service by executing processing of performing known automatic posting disclosed in, for example, Internet search <URL: https://webuo.net/blog/shopify-app/000205> or Internet search <URL: https://kurarebo.jp/a/blog/sns-marketing>.

With the above configuration, according to the agent system 500, since the specific moving image is automatically posted to the moving image posting service, it is possible to reduce the burden on the user required for posting the moving image to the moving image posting service. In particular, according to the agent system 500 described above, since the specific moving image of the another user in which the emotion value of the positive emotion increases by the predetermined value or more is automatically posted to the moving image posting service, it is possible to share, without omission, the moving image in a case in which the positive emotion of the another user increases.

In the agent system 500, in a case in which the specific moving image is automatically posted to the moving image posting service, posting may be performed after performing image processing (for example, blurring) that makes it difficult to recognize the face of the another user indicated in the specific moving image. For the image processing, known processing may be used as appropriate.

In a case in which the emotion value of the another user changes by a predetermined value or more, the action determination unit 236 inputs, to the sentence generation model, text representing the emotion of the another user, the specific moving image, and text asking the user a question about the specific moving image. The action determination unit 236 inputs, for example, a prompt "Please provide words to ask the user a question about the following specific moving image on the basis of the following emotion of the another user and the content of the specific moving image. Emotion of another user: Happy, moving image: xxxyyyzzz111.wav" to the sentence generation model. On the basis of the output of the sentence generation model, the action determination unit 236 determines the speech content of the agent to be output from the speaker included in the control target 252B of the communication terminal 100M as the action of the communication terminal 100M. For example, the action determination unit 236 determines "I have just found a happy person in front of the bulletin board, what do you think has happened to that person?" as the speech content of the agent on the basis of the output of the sentence generation model according to the input of the prompt.

The action control unit 250 outputs a question about the specific moving image as the speech content of the agent from the speaker. Specifically, the action control unit 250 causes the speaker to output, as the speech content of the agent, the content of "I have just found a happy person in front of the bulletin board, what do you think has happened to that person?" determined above by the action determination unit 236.

Next, the action determination unit 236 inputs, to the sentence generation model, text representing the speech content of the user 10 as the state of the user 10 recognized by the state recognition unit 230 on the basis of the text information output by the speech understanding unit 212 of the sensor module unit 210 as an answer to the speech content of the agent output from the speaker by using vocal sound, and text for asking a question about keywords of the speech content of the user 10. The action determination unit 236 inputs, for example, a prompt "Please tell me the keywords of the speech content of the user 10 below. The speech content of the user 10: Ah, that person. I think that the person passed some examination and was happy." to the sentence generation model. The action determination unit 236 determines to store the specific moving image by associating the keywords with the specific moving image as the action of the communication terminal 100M on the basis of the output of the sentence generation model. For example, the action determination unit 236 determines to store the specific moving image "xxxyyyzzz111
.wav" in which the another user is shown in association with the keyword "a person who was happy to pass the examination" determined on the basis of the output of the sentence generation model according to the input of the prompt. In this case, the specific moving image associated with the keyword is stored in the storage unit 220.

With the above configuration, according to the agent system 500, the specific moving image associated with the keyword determined from the speech content of the user 10 is stored, so that it is easy to manage the captured specific moving image. In the above example, in a case in which the emotion value of the positive emotion of another user increases by the predetermined value or more, the specific moving image associated with the keyword determined from the speech content of the user 10 is stored, but the disclosure is not limited thereto. In addition to the above example, also in a case in which the emotion value of the negative emotion of another user increases by a predetermined value or more, the agent system 500 stores the specific moving image associated with the keyword determined from the speech content of the user 10.

In a case in which the emotion value of the negative emotion of another user increases by a predetermined value or more, the action determination unit 236 inputs, to the sentence generation model, text representing the emotion of the another user, the specific moving image, and text for inquiry about the situation of the another user indicated in the specific moving image. The action determination unit 236 inputs, for example, a prompt "In consideration of the emotion of the another user and the content of the specific moving image below, please tell me words to ask the user 10 a question about the situation of the another user shown in the specific moving image below. Emotion of the another user: anxiety, specific moving image: yyyxxxzzz111.wav" to the sentence generation model. The action determination unit 236 determines the speech content of the agent to be output from the speaker by using vocal sound on the basis of the output of the sentence generation model. For example, the action determination unit 236 determines, as the speech content of the agent, "I have just found a person with an uneasy face in front of a bank automatic teller machine (ATM). What do you think has happened to that person?" on the basis of the output of the sentence generation model according to the input of the prompt.

The action control unit 250 outputs the situation of the another user shown in the specific moving image from the speaker as the speech content of the agent. Specifically, the action control unit 250 causes the speaker to output, as the speech content of the agent, the content of "I have just found a person with an uneasy face in front of the bank ATM. What do you think has happened to that person?" determined above by the action determination unit 236.

Here, in a case in which it is determined that a notification to a specific organization is necessary on the basis of the interaction content between the user 10 and the agent including the speech content of the agent output by using vocal sound from the speaker and the speech content of the user 10, the action determination unit 236 determines to notify the specific organization determined from the interaction content. The specific organization is, for example, an emergency call receiving organization capable of sending an emergency call such as a police department, a Japan coast guard, or a fire department. The action determination unit 236 inputs a prompt including text representing the interaction content between the user 10 and the agent and text for asking a question about the necessity of notification to the specific organization to the sentence generation model as needed, and determines whether or not the notification to the specific organization is necessary on the basis of the output of the sentence generation model. For example, in the case of inputting the above prompt, the sentence generation model can output whether or not it is necessary to report to the police in light of the criteria disclosed in the Internet search <URL: https://www.keishicho.metro.tokyo.lg.jp/jiken_jiko/110/110_110.html>. Although details are omitted, when the above prompt is input, the sentence generation model may output whether or not notification to each specific organization is necessary in light of the criteria indicating the necessity of notification defined by each specific organization for other specific organizations (examples: a Japan coast guard or a fire department). That is, according to the agent system 500, the necessity of notification to a specific organization and a specific organization that is a notification destination can be automatically determined on the basis of the interaction content between the user 10 and the agent by using the sentence generation model.

In a case in which the action determination unit 236 determines to notify the specific organization, the communication processing unit 280 transmits the specific information including the specific moving image and a place where the specific moving image was captured to a terminal of the specific organization determined by the action determination unit 236. For example, it is assumed that the action determination unit 236 determines to notify the police as a specific organization. In this case, the communication processing unit 280 transmits the specific moving image "yyyxxxzzz111.wav" in which the another user is shown and the specific information including the place where the specific moving image was captured to a terminal of the police (for example, a personal computer). Information that can be included in the specific information is not limited to the above. For example, the specific information may include other information such as interaction content between the user 10 and the agent.

With the above configuration, according to the agent system 500, for example, in a case in which an incident, an accident, or the like occurs, the agent system 500 side can determine the necessity of notification to the specific organization, and if necessary, the notification can be automatically performed, without causing the user 10 to determine the necessity of notification to the specific organization. According to the agent system 500, even in a case in which the user 10 determines that notification to the specific organization is not necessary, it is possible to appropriately determine the necessity of the notification in light of the criteria defined by the specific organization, and to automatically perform the notification as necessary.

### (Supplementary Note 1)

A control system including:
a state recognition unit that recognizes a user state including an action of a user and a state of an electronic apparatus;
an emotion determination unit that determines an emotion of the user, an emotion of another user existing around the user, or an emotion of the electronic apparatus;
an action determination unit that, in a case in which an emotion value indicating an intensity of the emotion of the another user changes by a predetermined value or more, determines to start capturing a moving image of the another user with a camera as an action of the electronic apparatus; and
an action control unit that controls the electronic apparatus to execute the action of the electronic apparatus determined by the action determination unit.

### (Supplementary Note 2)

The control system according to Supplementary Note 1, in which
the action determination unit determines, by using at least one of the user state, the state of the electronic apparatus, the emotion of the user, or the emotion of the electronic apparatus, and an action determination model, any of a plurality of types of device operations including non-operation as the action of the electronic apparatus,
the electronic apparatus detachably provided on a neck strap includes one or a plurality of sensors that collect information, and
the device operations include setting first action content of leading the user by the electronic apparatus reproducing at least one of vocal sound or an image.

### (Supplementary Note 3)

The control system according to Supplementary Note 2, in which the action determination unit spontaneously or periodically detects an action of the user on the basis of the sensor, and determines the first action content in a case in which leading the user is determined as the action of the electronic apparatus on the basis of the detected action of the user and specific information stored in advance.

### (Supplementary Note 4)

The control system according to Supplementary Note 3, in which the action determination unit determines the first action content in a case in which a camera included in the sensor is directed in an advancing direction of the user.

### (Supplementary Note 5)

The control system according to Supplementary Note 3, in which the first action content includes reproduction of at least one of vocal sound or an image that assists in walking of the user.

### (Supplementary Note 6)

The control system according to Supplementary Note 5, in which the reproduction includes reproduction of at least one of vocal sound or an image for guiding the user along a route different from a set guidance route.

### (Supplementary Note 7)

The control system according to Supplementary Note 6, in which the reproduction includes reproduction of at least one of vocal sound or an image for guiding the user along a path for returning the user to the set guidance route in a case in which the user guided along the guidance route deviates from the guidance route.

### (Supplementary Note 8)

The control system according to Supplementary Note 5, in which the action determination unit determines whether or not the action of the user has been corrected by detecting the action of the user while leading the user or after reproducing at least one of the vocal sound or the image, and determines second action content different from the first action content in a case in which the action of the user has been corrected.

### (Supplementary Note 9)

The control system according to Supplementary Note 8, in which the second action content includes reproduction of at least one of vocal sound praising the user for the action, vocal sound expressing gratitude for the action of the user, an image praising the user for the action, or an image expressing gratitude for the action of the user.

### (Supplementary Note 10)

The control system according to Supplementary Note 5, in which the action determination unit determines whether or not the action of the user has been corrected by detecting the action of the user while leading the user or after reproducing at least one of the vocal sound or the image, and determines third action content different from the first action content in a case in which the action of the user has not been corrected.

### (Supplementary Note 11)

The control system according to Supplementary Note 10, in which the third action content includes at least one of transmission of specific information to a person other than the user, reproduction of sound that attracts an interest of the user, or reproduction of an image that attracts the interest of the user.

### (Supplementary Note 12)

The control system according to Supplementary Note 1, in which
the electronic apparatus is a communication terminal, and
the action determination unit determines any of a plurality of types of actions of an agent for interacting with the user, including no action, as an action of the communication terminal.

### (Supplementary Note 13)

The control system according to Supplementary Note 12, further including
a sentence generation model having an interaction function, in which
the action determination unit inputs, to the sentence generation model, text representing at least one of the user state, a state of the agent, the emotion of the user, or an emotion of the agent, and text for inquiry about an action of the agent, and determines an action of the communication terminal on the basis of an output of the sentence generation model.

### (Supplementary Note 14)

The control system according to Supplementary Note 12, in which
the emotion of the another user includes a positive emotion and a negative emotion, and
the action determination unit determines to post a specific moving image that is a moving image obtained by imaging the another user, to a predetermined moving image posting service in a case in which an emotion value of the positive emotion of the another user increases by a predetermined value or more, and
the control system further includes a communication processing unit that posts the specific moving image of the another user in which the emotion value of the positive emotion increases by the predetermined value or more to the predetermined moving image posting service.

### (Supplementary Note 15)

The control system according to Note 13, in which
the action determination unit inputs, to the sentence generation model, text representing the emotion of the another user, a specific moving image that is a moving image obtained by imaging the another user, and text for asking the user a question about the specific moving image in a case in which an emotion value of the another user has changed by a predetermined value or more, and determines speech content of the agent to be output from a control target included in the communication terminal as an action of the communication terminal on the basis of an output of the sentence generation model,
the action control unit outputs a question about the specific moving image from the control target as the speech content of the agent, and
the action determination unit inputs, to the signal, text representing speech content of the user as the user state recognized by the state recognition unit as an answer to the speech content of the agent output from the control target, and text for asking a question about a keyword of the speech content of the user, and determines to store the specific moving image by associating the keyword with the specific moving image as the action of the communication terminal on the basis of an output of the sentence generation model.

### (Supplementary Note 16)

The control system according to note 13, in which
the emotion of the another user includes a positive emotion and a negative emotion, and
the action determination unit inputs, to the sentence generation model, text representing the emotion of the another user, a specific moving image that is a moving image obtained by imaging the another user, and text for asking a question about a situation of the another user shown in the specific moving image in a case in which an emotion value of the negative emotion of the another user increases by a predetermined value or more, and determines speech content of the agent to be output from a control target included in the communication terminal as an action of the communication terminal on the basis of an output of the sentence generation model,
the action control unit outputs the situation of the another user shown in the specific moving image from the control target as the speech content of the agent, and
the action determination unit determines, in a case in which it is determined that notification to a specific organization is necessary on the basis of interaction content between the user and the agent including the speech content of the agent output from the control target and speech content of the user as the user state recognized by the state recognition unit, to notify the specific organization determined from the interaction content, and
the control system further includes a communication processing unit that transmits specific information including the specific moving image and a place where the specific moving image was captured to a terminal of the specific organization determined by the action determination unit.

### (Other Embodiment 3)

As other embodiment 3, an example in which the communication terminal 100M that is the above-described smart AI device is used for physical condition management when going out will be described. Portions having the same configurations as those of the first to fourth embodiments are denoted by the same reference numerals, and description thereof will be omitted.

The communication terminal 100M is an electronic apparatus detachably provided on the neck strap 6000, and has the functions of the robots 100 to 102 described above. The communication terminal 100M includes an agent system 700 configured by using some or all of the functions of the control system of the robots 100 to 102. The communication terminal 100M includes the microphone 201, the 2D camera 203, the acceleration sensor 206, the temperature sensor 207, and the heart rate sensor 208 included in the sensor unit 200B illustrated in Fig. 13, and can acquire surrounding environment information (including states of surrounding people). The surrounding environment information includes a temperature, a rainfall, a wind volume, an atmospheric pressure, a distance, an inclination, a density, the presence or absence of a dangerous article, a state of a person (a body temperature, a heart rate, a condition, a facial expression, body movement, and the number of people), and the like.

The communication terminal 100M can be used as follows. For example, the communication terminal 100M acquires the temperature as the surrounding environment information, and the communication terminal 100M determines that the environment is severe since the temperature exceeds 35 degrees, and inquires of the user 10 whether the physical condition is good. In response to the inquiry, the user 10 responds by using vocal sound or text, and the communication terminal 100M acquires the response of the user 10 and further analyzes a state or an emotion of the user 10. The communication terminal 100M analyzes the degree of poor physical condition of the user 10 from the content of the response of the user 10, the analyzed state of the user 10, and the analyzed emotion of the user 10. The communication terminal 100M makes a proposal to the user 10 according to the analysis result. Specifically, the content of the proposal is to urge the user 10 to rehydrate in a case in which the degree of poor physical condition of the user is mild, and to arrange an ambulance in a case in which the degree of poor physical condition of the user is severe. As described above, the communication terminal 100M makes a proposal according to the degree of poor physical condition of the user 10. At the time of making a proposal, the communication terminal 100M may acquire map information including surrounding facility information and make a proposal on the basis of the map information. As an example, the communication terminal 100M guides the user 10 to a nearby convenience store in a case of urging rehydration, and the communication terminal 100M guides the user 10 to a nearby park in a case of urging a break.

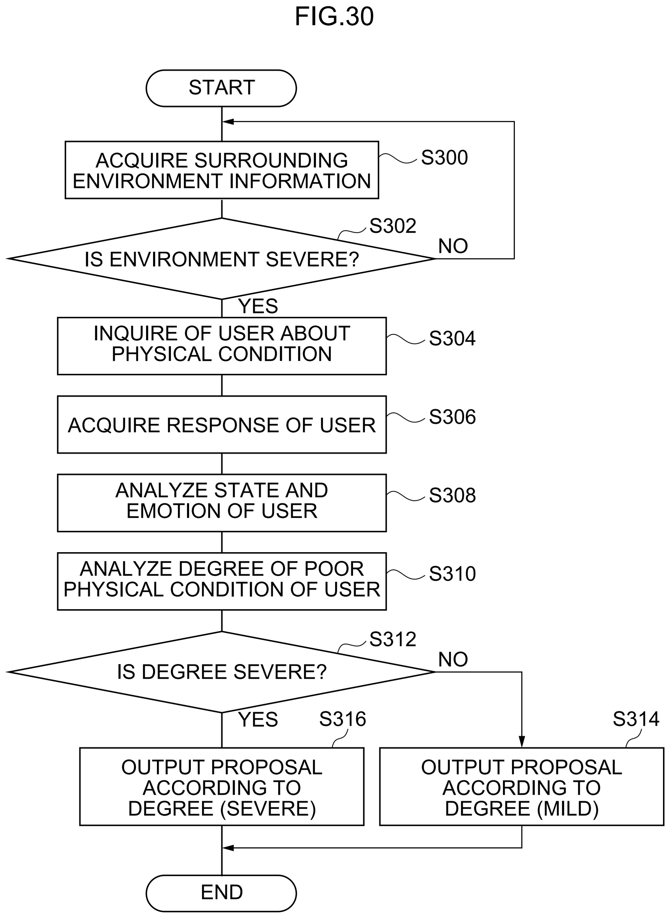

Next, an operation of the communication terminal 100M as other embodiment 3 will be described. In the agent system 700 included in the communication terminal 100M, agent proposal processing illustrated in Fig. 30 is executed. The processing in the agent system 700 is executed by the CPU 1212 functioning as the state recognition unit 230, the emotion determination unit 232, the action recognition unit 234, the action determination unit 236, and the character setting unit 276. The proposal processing is repeatedly executed in a case in which the surrounding environment information is determined to be severe.

The proposal processing will be described with reference to Fig. 30.

In step S300, the CPU1212 acquires surrounding environment information. Specifically, the ambient temperature, a state of a person, and the like are acquired by using various sensors included in the sensor unit 200B (see Fig. 13). Not only the information from the sensors but also environment information such as a temperature and a humidity of the current location may be acquired from an external server or the like.

In step S302, the CPU1212 determines whether the surrounding environment information is severe. For example, in a case in which the ambient air temperature exceeds 35 degrees, it is determined to be severe. For example, in a case in which the ambient temperature is lower than minus 5 degrees, it is determined to be severe. For example, in a case in which the ambient humidity is 80% or more, it is determined to be severe. For example, in a case in which a value of the wet bulb globe temperature (WBGT), which is an index calculated on the basis of factors of temperature, humidity, solar radiation/radiation heat, and wind and is called a heat index, indicates 28 or more, it is determined to be severe. In a case in which the CPU 1212 determines that the surrounding environment information is severe (step S302: YES), the processing proceeds to step S304. On the other hand, in a case in which the CPU 1212 determines that the surrounding environment information is not severe (step S302: NO), the processing proceeds to step S300.

In step S304, the CPU 1212 inquires of the user 10 whether the physical condition is good. The inquiry from the CPU 1212 may be performed by using vocal sound or text.

In step S306, the CPU 1212 acquires a response of the user as vocal sound information or text.

In step S308, the CPU 1212 analyzes a state of the user 10 and an emotion of the user 10 on the basis of the acquired response of the user 10 and the state of the person acquired by the sensor in step S300.

In step S310, the CPU 1212 analyzes the degree of poor physical condition from the acquired response of the user 10, the analyzed state of the user 10, and the analyzed emotion of the user 10. For example, in a case in which the breath of the user 10 becomes rough in his/her response, the user 10 is in a state in which his/her body swings due to heat, and the user 10 feels distressed or painful, the CPU 1212 analyzes the degree of poor physical condition to be severe. For example, in a case in which it is notified that the user 10 is thirsty as the response of the user 10, the state of the user 10 is a state of fanning himself/herself with his/her hands due to heat, and the user has anxiety, the CPU 1212 analyzes the degree of poor physical condition to be mild.

In step S312, the CPU 1212 determines whether the degree of poor physical condition of the user 10 is severe. In a case in which the CPU 1212 determines that the degree of poor physical condition of the user 10 is not severe (step S312: YES), the processing proceeds to step S314. On the other hand, in a case in which the CPU 1212 determines that the degree of poor physical condition of the user 10 is severe (step S312: NO), the processing proceeds to step S316.

In step S314, the CPU 1212 outputs an appropriate proposal according to the analyzed degree of physical condition (mild). Specifically, for example, rehydration is urged. In the case of making an appropriate proposal, the CPU 1212 acquires map information including surrounding facility information and takes a measure based on the map information. For example, in the case of urging rehydration, the CPU 1212 presents guidance to a nearby convenience store. For example, in the case of urging a break, the CPU 1212 presents guidance to a nearby park. The proposal processing is ended.

In step S316, the CPU 1212 outputs an appropriate proposal according to the analyzed degree of the physical condition (severe). Specifically, for example, an ambulance is arranged with map information indicating the current position. In the case of making an appropriate proposal, the CPU 1212 acquires map information including surrounding facility information and takes a measure based on the map information. For example, the CPU 1212 may present a nearby hospital and arrange a taxi. The proposal processing is ended.

The state of the person included in the surrounding environment information includes not only an emotion of the user 10 but also an emotion of a person around the user 10. In a case in which the emotion of the surrounding person is painful, distressed, or the like, the communication terminal 100M proposes a break to the surrounding person. In a case in which the emotion of the surrounding person is painful or distressed, the communication terminal 100M may predict that the user 10 also has a similar emotion and make a proposal. The communication terminal 100M may make an individual specific proposal to each of surrounding persons. As a result, the communication terminal 100M can manage the physical condition of the group.

In the above description, the communication terminal 100M makes a proposal in accordance with poor physical condition, but the disclosure is not limited thereto. For example, even if the physical condition is not bad, in a case in which the user 10 temporarily feels painful due to a high load, such as a case in which breathing becomes rough or a case in which a steep slope is detected during exercise such as running, the communication terminal 100M may make a proposal to the user 10 from the acquired surrounding environment information. In a case in which the surrounding environment information is likely to change at the time of climbing or the like, the communication terminal 100M may acquire the surrounding environment information at a high frequency and propose a timing of going down the mountain to the user 10 from the physical condition of the user 10, climate, or the like.

As described above, according to the communication terminal 100M, since the physical condition of the user 10 can be managed on the basis of the surrounding environment information when going out, the user 10 can go out with a sense of security. Not only in a case of going out, but also in a house, for example, an elderly person can easily perform physical condition management by wearing the communication terminal 100M. In particular, according to other embodiment 3, since the physical condition can be managed through the interaction with the user 10 regardless of the biological information of the user 10, sensors that directly acquire the biological information and the physical condition, such as the 2D camera 203 and the heart rate sensor 208, are not necessarily required. That is, it is sufficient that the electronic apparatuses configuring the agent system 700 include the microphone 201 and the speaker.

### (Supplementary Note 1)

A control system including:
a state recognition unit that recognizes a user state including an action of a user and a state of an electronic apparatus;
an emotion determination unit that determines an emotion of the user or an emotion of the electronic apparatus;
an action determination unit that determines, by using at least one of the user state, the state of the electronic apparatus, the emotion of the user, or the emotion of the electronic apparatus, and an action determination model at a predetermined timing, any of a plurality of types of device operations including non-operation as an action of the electronic apparatus, in which
surrounding environment information is acquired from one or a plurality of sensors that collect information, the sensors being included in the electronic apparatus detachably provided on a body of the user, and
the action determination unit checks a health state of the user by outputting at least one of vocal sound or text from the electronic apparatus according to the acquired environment information, and,
the electronic apparatus makes an appropriate proposal to the user.

### (Supplementary Note 2)

The control system according to Supplementary Note 1, in which the action determination unit acquires map information including surrounding facility information, and makes the appropriate proposal based on the map information.

### (Supplementary Note 3)

The control system according to Supplementary Note 1, in which the action determination unit acquires a state of another surrounding person and makes the appropriate proposal based on the state of the person.

### (Other Embodiment 4)

As other embodiment 4, an example will be described in which the communication terminal 100M, which is the above-described smart AI device, performs an action of talking to another user on the basis of emotions of the another user on behalf of the user 10. Portions having the same configurations as those of the first to fourth embodiments are denoted by the same reference numerals, and description thereof will be omitted.

The communication terminal 100M is an electronic apparatus detachably provided on the neck strap 6000, and has the functions of the robots 100 to 102 described above. The communication terminal 100M includes an agent system 700 configured by using some or all of the functions of the control system of the robots 100 to 102. The communication terminal 100M includes the sensor unit 200B illustrated in Fig. 13, and can capture facial expressions and situations of surrounding people with the 2D camera 203, for example, and can acquire moving image information. For example, the communication terminal 100M can acquire vocal sound of conversations between the user 10 of the communication terminal 100M and other users in the vicinity of the user 10 by using the microphone 201. The other users are all the users in the vicinity of the communication terminal 100M (in the imaging range of the 2D camera 203 or in the sound collection range of the microphone 201).

Next, a functional configuration of the communication terminal 100M according to other embodiment 4 will be described with reference to Fig. 13. As illustrated in Fig. 13, in the communication terminal 100M of other embodiment 4, the CPU 1212 executes a conversation output processing program and a topic output processing program stored in the ROM 1230 or the storage device 1224, thereby functioning as the state recognition unit 230, the emotion determination unit 232, and the action determination unit 236. In the communication terminal 100M, the conversation output processing and the topic output processing are executed in parallel.

Each function of the conversation output processing will be described. The conversation output processing is processing in which the communication terminal 100M talks to other users on the basis of emotions of other users on behalf of the user 10. Specifically, for example, the user 10 who is an organizer of a party wears the communication terminal 100M, and the state recognition unit 230 of the communication terminal 100M acquires a video of a participant who is another user from the 2D camera 203. The state recognition unit 230 acquires the conversation content of the participant from the microphone 201. The emotion determination unit 232 of the communication terminal 100M estimates the emotion of the participant by using the emotion engine on the basis of the acquired surrounding state (the video of the participant, an image of a surrounding situation of the user 10, vocal sound of the conversation content of the participant, and the like). The action determination unit 236 of the communication terminal 100M determines whether to output a conversation, for example, so as to talk to a person for whom an emotion of happiness is estimated but not to talk to a person for whom an emotion of anger is estimated. In a case in which the communication terminal 100M talks to the participant, for example, vocal sound such as "Are you enjoying it?" or "Do you have some drink?" is output. On the other hand, in a case in which the emotion determination unit 232 estimates that the emotion of the participant is angry, the action determination unit 236 determines not to perform vocal sound output from the communication terminal 100M.

Each function of the topic output processing will be described. The topic output processing is processing in which the communication terminal 100M provides a topic on behalf of the user 10 and other users. This processing is executed in a case in which there is no conversation between the user 10 and another user and silence continues for a predetermined period. Specifically, the state recognition unit 230 of the communication terminal 100M acquires a conversation between the user 10 and another user from the microphone 201, and inputs the conversation content to the sentence generation model 55. In this case, in a case in which the conversation content is not input to the sentence generation model 55 for a predetermined period (that is, in a silent case), the action determination unit 236 determines whether to output a topic such that a topic is newly output from the sentence generation model 55. The communication terminal 100M outputs a topic such as "How about talking about how you spent the recent weekend?" by using vocal sound, for example.

The communication terminal 100M can output synthesized vocal sound imitating the voice of the user 10. The vocal sound is output from the speaker included in the control target 252 of the communication terminal 100M.

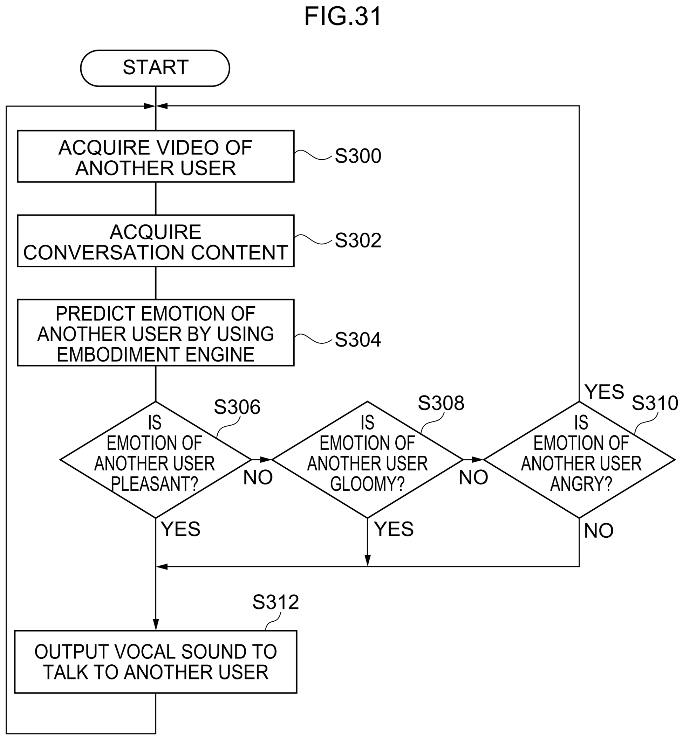

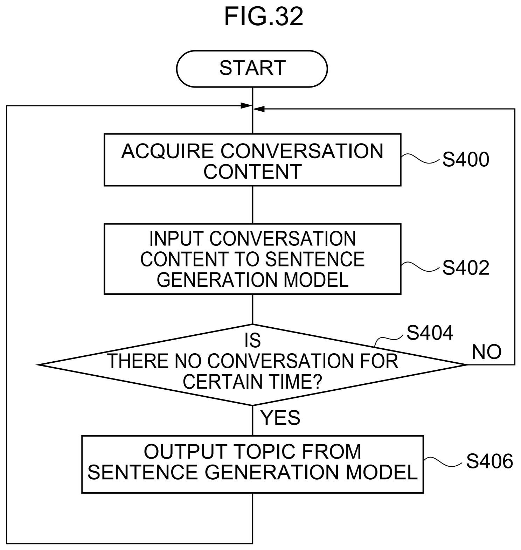

Next, an operation of the communication terminal 100M as other embodiment 4 will be described. In the agent system 700 included in the communication terminal 100M, the conversation output processing of the agent illustrated in Fig. 31 and the topic output processing of the agent illustrated in Fig. 32 are executed. The processing in the agent system 700 is executed by the CPU 1212 functioning as the state recognition unit 230, the emotion determination unit 232, and the action determination unit 236.

The conversation output processing will be described with reference to Fig. 31.

In step S300, the CPU 1212 acquires a video of another user near the user 10 with the 2D camera 203 of the communication terminal 100M. Specifically, a video of an expression or a posture of another user is acquired so that the emotion of the another user can be estimated. Image information indicating a surrounding situation of the another user may be acquired.

In step S302, the CPU 1212 acquires the vocal sound of the conversation between the user 10 and the another user from the microphone 201 of the communication terminal 100M. That is, the CPU 1212 acquires conversation content between the user 10 and the another user.

In step S304, the CPU 1212 estimates an emotion of the another user from the acquired expression of the another user and the conversation content by using the emotion engine.

In step S306, the CPU 1212 determines whether or not the emotion of the another user is pleasant. The CPU 1212 determines whether or not the emotion of the another user is pleasant on the basis of the emotion of the another user estimated in step S304, the image information indicating the surrounding situation of the another user acquired in step S300, and the conversation content of the another user acquired in step S302. In a case in which the CPU 1212 determines that the another user is pleasant (step S306: YES), the processing proceeds to step S312. On the other hand, in a case in which the CPU 1212 determines that the another user is not pleasant (step S306: NO), the processing proceeds to step S308.

In step S308, the CPU 1212 determines whether or not the emotion of the another user is gloomy. The CPU 1212 determines whether or not the emotion of the another user is gloomy on the basis of the emotion of the another user estimated in step S304, the image information indicating the surrounding situation of the another user acquired in step S300, and the conversation content of the another user acquired in step S302. In a case in which the CPU 1212 determines that another user is gloomy (step S308: YES), the processing proceeds to step S312. On the other hand, in a case in which the CPU 1212 determines that another user is not gloomy (step S308: NO), the processing proceeds to step S310.

In step S310, the CPU 1212 determines whether or not the emotion of the another user is angry. The CPU 1212 determines whether or not the emotion of the another user is angry on the basis of the emotion of the another user estimated in step S304, the image information indicating the surrounding situation of the another user acquired in step S300, and the conversation content of the another user acquired in step S302. In a case in which the CPU 1212 determines that another user is angry (step S310: YES), the processing returns to step S300. On the other hand, in a case in which the CPU 1212 determines that the another user is not angry (step S308: NO), the processing proceeds to step S312.

In step S312, the CPU 1212 outputs vocal sound to talk to the another user on behalf of the user 10. Specifically, the CPU 1212 changes the tone of the vocal sound according to the emotion of the another user estimated by the emotion determination unit 232 and outputs the vocal sound. For example, in a case in which it is determined in step S306 that the emotion of the another user is pleasant, the CPU 1212 outputs vocal sound toward the another user with a bright tone in step S312. For example, in a case in which it is determined in step S308 that the emotion of the another user is gloomy, the CPU 1212 outputs vocal sound toward the another user with a gentle tone in step S312. The output vocal sound is vocal sound synthesized by imitating the voice of the user 10. The processing returns to step S300. As described above, the conversation output from the communication terminal 100M is repeated.

As described above, in the conversation output processing in Fig. 31, the emotions are classified into "pleasant", "gloomy", "angry", and "others", and in the case of the "angry" emotion, the communication terminal does not talk to another user, and in the case of the "pleasant", "gloomy", and "others" emotions, the communication terminal changes the tone of the vocal sound according to the emotion and talks to another user.

The topic output processing will be described with reference to Fig. 32. The topic output processing is executed in parallel with the conversation output processing. The topic output processing is processing of outputting a topic in a case in which there is no conversation between the user 10 and another user.

In step S400, the CPU 1212 acquires the speech of the conversation between the user 10 and the another user with the microphone 201 of the communication terminal 100M. That is, the CPU 1212 acquires conversation content between the user 10 and the another user.

In step S402, the CPU 1212 inputs the acquired conversation content to the sentence generation model 55.

In step S404, the CPU 1212 determines whether or not there is a conversation between the user 10 and another person for a predetermined period. That is, the CPU 1212 determines whether or not there is an input of conversation content to the sentence generation model 55 for a predetermined period. In a case in which the CPU 1212 determines that there is no conversation for the predetermined period (step S404: YES), the processing proceeds to step S406. On the other hand, in a case in which the CPU 1212 determines that there is a conversation (step S404: NO), the processing returns to step S400.

In step S406, the CPU 1212 outputs a topic directed to the user 10 and the another user from the sentence generation model 55. The topic related to the conversation content between the user 10 and another user input to the sentence generation model 55 so far is output from the sentence generation model 55 by the CPU 1212. The output is performed by using vocal sound. The vocal sound may be vocal sound synthesized by imitating the voice of the user 10, or may be another vocal sound. The processing returns to step S400. As described above, the topic output from the communication terminal 100M is repeated.

As described above, according to the communication terminal 100M, it is possible to estimate an emotion of a person near the user 10 and output a conversation on the basis of the estimated emotion. In a case in which there is no conversation between the user 10 and another user for a predetermined period, a new topic is output from the communication terminal 100M, so that it is possible to reduce an uncomfortable time due to silence. Since the vocal sound to be output is vocal sound synthesized by imitating the voice of the user 10, it is also possible to make another user think that the user 10 speaks.

### (Supplementary Note 1)

A control system including:
a state recognition unit that recognizes a user state including actions of a user and another user;
an emotion determination unit that determines emotions of the user and the another user; and
an action determination unit that determines any of a plurality of types of device operations as an operation of an electronic apparatus by using at least one of the user state or the emotion of the user, and an action determination model at a predetermined timing, in which
the state recognition unit recognizes a surrounding state on the basis of one or a plurality of sensors of the electronic apparatus detachably provided on a body of the user, and
the action determination unit determines that the electronic apparatus will make speech toward the another user in a case in which the another user exists near the user.

### (Supplementary Note 2)

The control system according to Supplementary Note 1, in which
the emotion determination unit estimates the emotion of the another user, and
the action determination unit determines that the electronic apparatus will make speech toward the another user on the basis of the estimated emotion.

### (Supplementary Note 3)

The control system according to Supplementary Note 2, in which
the action determination model includes a sentence generation model having an interaction function, and
the action determination unit determines to receive provision of a new topic from the sentence generation model in a case in which there is no input of conversation content between the user and the another user to the sentence generation model for a predetermined period.

### (Supplementary Note 4)

The control system according to Supplementary Note 2, in which the action determination unit determines to synthesize vocal sound by imitating voice of the user and output the synthesized vocal sound from the electronic apparatus.

### (Other Embodiment 5)

As other embodiment 5, an example will be described in which the communication terminal 100M, which is the above-described smart AI device, is used to support a salesclerk who is the user 10 at the time of customer service. Portions having the same configurations as those of the first to fourth embodiments are denoted by the same reference numerals, and description thereof will be omitted.

The communication terminal 100M is an electronic apparatus detachably provided on the neck strap 6000, and has the functions of the robots 100 to 102 described above. The communication terminal 100M includes an agent system 700 configured by using some or all of the functions of the control system of the robots 100 to 102. The communication terminal 100M includes the sensor unit 200B illustrated in Fig. 13, and can image facial expressions and situations of surrounding people with the 2D camera 203, for example, and can acquire video information. For example, the communication terminal 100M can acquire vocal sound of conversations between a salesclerk who is the user 10 and a customer who is another person by using the microphone 201.

The communication terminal 100M can be used as follows. For example, a store salesclerk wears the communication terminal 100M and acquires a video of a customer from the 2D camera 203, and the communication terminal 100M estimates an emotion of the customer by using an emotion engine. In a case of finding the customer in trouble, the communication terminal 100M notifies the salesclerk who is the user 10 of the communication terminal 100M. The communication terminal 100M also acquires image information of the surrounding situation of the customer, and inputs the image information and the emotion of the customer to the sentence generation model 55, thereby generating content of and a solution to the customer's trouble. The communication terminal 100M presents the content and the solution to the salesclerk who is the user 10. The information output from the communication terminal 100M is presented to the salesclerk who is the user 10 in a form in which the information does not leak to the outside (for example, using an earphone).

The communication terminal 100M acquires identification information of the customer. The identification information is a two-dimensional code displayed on a membership card or a smartphone, biological information (voiceprint or face information), a member name acquired through speech recognition during conversation, or the like. The communication terminal 100M acquires customer information (including the purchase history) stored in association with the identification information from a database or the like, and inputs the acquired customer information to the sentence generation model. By using the customer information as input data as described above, the communication terminal 100M can output the content of and solution to the customer's trouble based on the purchase history of the customer and the like.

The communication terminal 100M acquires vocal sound of conversations between the salesclerk who is the user 10 and the customer. The communication terminal 100M inputs the acquired conversation information to the sentence generation model 55, and outputs the content of and the solution to the customer's trouble to the user 10. As a result, the communication terminal 100M can present a suitable solution on the basis of the conversation between the user 10 and the customer.

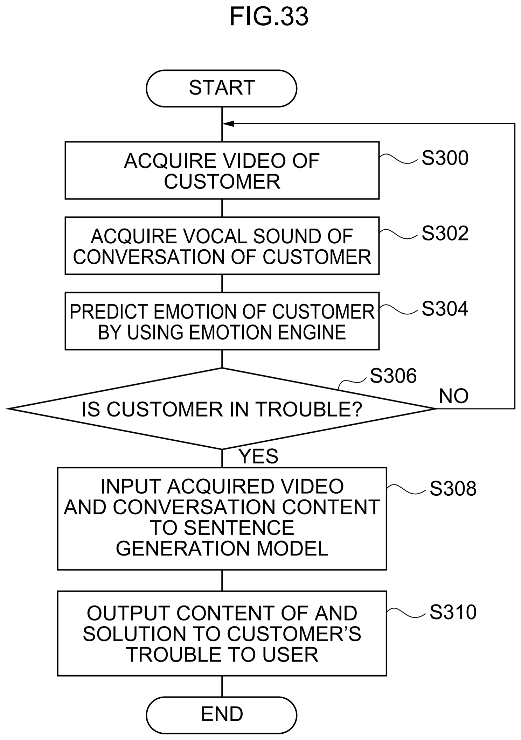

Next, an operation of the communication terminal 100M as other embodiment 5 will be described. In the agent system 700 included in the communication terminal 100M, agent proposal processing illustrated in Fig. 33 is executed. The processing in the agent system 700 is executed by the CPU 1212 functioning as the state recognition unit 230, the emotion determination unit 232, the action recognition unit 234, the action determination unit 236, and the character setting unit 276.

The proposal processing will be described with reference to Fig. 33.

In step S300, the CPU 1212 acquires a video of the customer with the 2D camera 203 of the communication terminal 100M. Specifically, a video of a facial expression and a posture of the customer is acquired so that the emotion of the customer can be estimated. Image information indicating the surrounding situation of the customer may be acquired.

In step S302, the CPU 1212 acquires the vocal sound of the conversation between the salesclerk and the customer with the microphone 201 of the communication terminal 100M.

In step S304, the CPU 1212 estimates an emotion of the customer from the acquired facial expression and conversation content of the customer by using the emotion engine.

In step S306, the CPU 1212 determines whether the customer is in trouble. The case in which the customer is in trouble includes, for example, a case in which the customer is in trouble because the customer cannot decide which product to select and a case in which the customer is searching without knowing where the product is. The CPU 1212 determines whether or not the customer is in trouble on the basis of the emotion of the customer estimated in step S304, the image information indicating the surrounding situation of the customer acquired in step S300, and the conversation content of the customer acquired in step S302. In a case in which the CPU 1212 determines that the customer is in trouble (step S306: YES), the processing proceeds to step S308. On the other hand, in a case in which the CPU 1212 determines that the customer is not in trouble (step S306: NO), the processing returns to step S300.

In step S308, the CPU 1212 inputs the estimated emotion of the customer, the video of the surrounding situation, and the conversation content of the customer to the sentence generation model 55. The customer information stored in association with the customer identification information may also be input to the sentence generation model 55.

In step S310, the CPU 1212 outputs the content of and solution to the customer's trouble to the user 10 from the sentence generation model 55. The output is transmitted to the salesclerk who is the user 10 of the communication terminal 100 via an earphone or the like without leaking to the outside. The proposal processing is ended.

As described above, according to the communication terminal 100M, the user 10 who is a customer service salesclerk can find a customer in trouble who is not noticed by simple visual observation. The salesclerk who is the user 10 of the communication terminal 100M acts on the basis of the content of and the solution to the customer's trouble generated by the sentence generation model 55, and thus, it is possible to increase the customer satisfaction. Since the customer information of the customer is also input to the sentence generation model 55, the communication terminal 100M outputs the content of and solution to the customer's trouble on the basis of the purchase history of the customer, so that it is possible to follow the customer's preference. Since the communication terminal 100M acquires the vocal sound of the conversation between the salesclerk who is the user 10 and the customer, inputs the vocal sound to the sentence generation model 55, and outputs the solution to the user 10, it is possible to propose a solution based on the conversation between the salesclerk and the customer.

### (Supplementary Note 1)

A control system including:
a state recognition unit that recognizes a user state including an action of a user and a state of an electronic apparatus;
an emotion determination unit that determines an emotion of the user or an emotion of the electronic apparatus; and
an action determination unit that determines, by using at least one of the user state, the state of the electronic apparatus, the emotion of the user, or the emotion of the electronic apparatus, and an action determination model at a predetermined timing, any of a plurality of types of device operations including non-operation as an action of the electronic apparatus, in which
the emotion determination unit estimates an emotion of another person from a video of the another person around the user acquired from one or a plurality of sensors that collect information, the sensors being included in the electronic apparatus detachably provided on a body of the user, and
the action determination unit makes an appropriate proposal to the user in a case in which the another person feels troubled.

### (Supplementary Note 2)

The control system according to Supplementary Note 1, in which the action determination unit inputs customer information associated with identification information of the another person to the action determination model and makes the appropriate proposal.

### (Supplementary Note 3)

The control system according to Supplementary Note 1, in which the action determination unit inputs conversation information of a conversation between the user and the another person to the action determination model and makes the appropriate proposal.

### (Other Embodiment 6)

In other embodiment 6, specific usage modes other than the above using the system 5, each agent system, and the like will be described.

Hereinafter, as an example, a case in which the agent system 500 is mounted on the communication terminal 100M, and a child is hanging the neck strap 6000 with which the communication terminal 100M is locked on the neck as illustrated in Fig. 16 will be described as an example.

While the child is small, the child comes into contact with only a limited number of people in a limited area such as a house or a kindergarten.

Therefore, in the communication terminal 100M according to the present embodiment, the action determination unit 236 determines device operations including issuing a warning to a person satisfying a predefined condition among persons approaching the 2D camera 203 as actions of the communication terminal 100M.

Specifically, by registering only a limited number of persons in advance, it is possible to protect a child from a suspicious person.

For example, a face image of a person who may come in contact in a limited area is registered in the agent system 500 in advance.

A captured image is acquired from the 2D camera 203 as an imaging unit, it is determined whether a person satisfying a predefined condition is shown, and a warning is issued in a case in which a person satisfying a predefined condition is shown. For example, it is determined whether a person registered in advance is shown, and a warning is issued in a case in which a person other than the registered person is shown. Alternatively, a warning is issued in a case in which a person registered in advance as a suspicious person is shown. As a result, the child can be protected from the suspicious person.

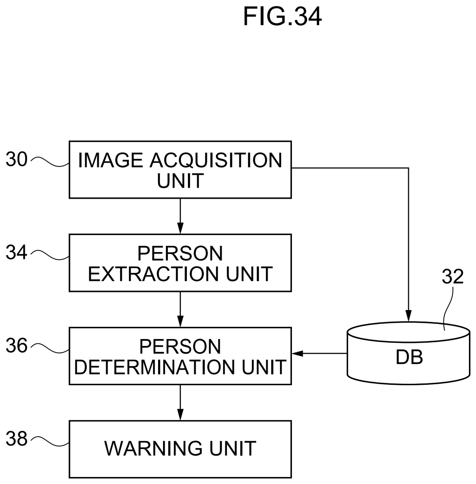

Fig. 34 is a block unit illustrating a functional configuration of the action determination unit 236 in the communication terminal 100M according to the present embodiment.

The action determination unit 236 of the communication terminal 100M has functions of an image acquisition unit 30, a database 32, a person extraction unit 34, a person determination unit 36, and a warning unit 38.

The image acquisition unit 30 acquires a captured image captured by the 2D camera 203. That is, a captured image of the front of the child is acquired, and the acquired captured image is stored in the database 32. It is assumed that the 2D cameras 203 are provided on both surfaces of the communication terminal 100M, and the 2D camera 203 that is directed in the front direction of the child images the surroundings.

The database 32 stores the captured image acquired by the image acquisition unit 30. The database 32 also stores captured images of previously registered persons. The database 32 stores a captured image of a suspicious person registered in advance. The captured image of the suspicious person is not limited to a face image of the suspicious person, and may be an image of a wearing object (including clothes, shoes, a wristwatch, a decorative product, or the like) or a characteristic image of the suspicious person.

The person extraction unit 34 extracts a person from the captured images acquired by the image acquisition unit 30. For example, a person in a captured image is recognized and extracted through known image recognition.

The person determination unit 36 determines whether the person extracted by the person extraction unit 34 corresponds to a person registered in the database 32 in advance. In a case in which the person is determined, a wearing object (including clothes, shoes, a wristwatch, a decorative product, or the like) may be stored in the database 32 as a set to determine whether or not the person is a person registered by the wearing object. As a result, even in a case in which the face is out of the angle of view of the camera, such as a case in which a person approaches the communication terminal 100M, it is possible to determine the person by using the wearing object.

The warning unit 38 determines, as an action of the communication terminal 100M, to issue a warning on the basis of the determination result from the person determination unit 36. The action control unit 250 controls the control target 252 on the basis of the action determined by the action determination unit 236. For example, in a case in which the person is not a person registered in advance or in a case in which the person is a suspicious person registered in advance, issuing a warning such that the person does not approach the child is determined as the action of the communication terminal 100M. In a case in which the person is not a person registered in advance or in a case in which the person is a suspicious person registered in advance, the warning unit 38 may determine to perform a predefined action according to a distance of a target person. For example, in a case in which the target person is not a person registered in advance and the distance to the target person is 1 m, registration of identification information is requested, in a case in which the distance is 50 cm, a warning is issued, and in a case in which the distance is less than 30 cm, a warning is issued at a predetermined large volume to notify the police is determined as the action of the communication terminal 100M.

In the present embodiment, a case in which the action determination unit 236 of the communication terminal 100M has the functions in Fig. 34 will be described, but the disclosure is not limited thereto. For example, some functions of the action determination unit 236 may be provided outside (for example, a server).

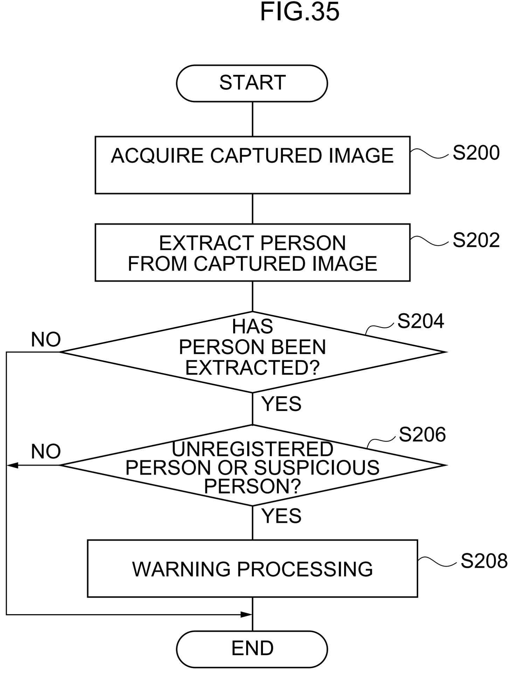

Subsequently, specific processing performed by the communication terminal 100M according to the present embodiment will be described. Fig. 35 is a flowchart illustrating an example of a flow of processing performed by the communication terminal 100M in the agent system according to the present embodiment. The processing in Fig. 35 is started, for example, in a case in which the sensor unit 200A detects approach of a person or an object to the communication terminal 100M.

In step S200, the image acquisition unit 30 acquires a captured image and proceeds to step S202. That is, a captured image of the front of the child is acquired.

In step S202, the person extraction unit 34 extracts a person from the captured image acquired by the image acquisition unit 30, and the processing proceeds to step S204. For example, a person in the captured image is recognized and extracted through known image recognition.

In step S204, the person extraction unit 34 determines whether or not a person has been extracted from the captured image. In a case in which the determination is positive, the processing proceeds to step S206, and in a case in which the determination is negative, the series of processing is ended.

In step S206, the person determination unit 36 determines whether the extracted person is a person not registered in the database 32 or a suspicious person registered in advance. In a case in which the determination is positive, the processing proceeds to step S208, and in a case in which the determination is negative, the series of processing is ended.

In step S208, the warning unit 38 and the action control unit 250 perform warning processing and end the series of processing. In the warning processing, the warning unit 38 determines, as the action of the communication terminal 100M, issuing a warning such that a person who is not registered in the database or a suspicious person as a target person does not approach, and the action control unit 250 controls the control target 252 to actually issue a warning. This makes it possible to keep an unknown person or a suspicious person away from the child.

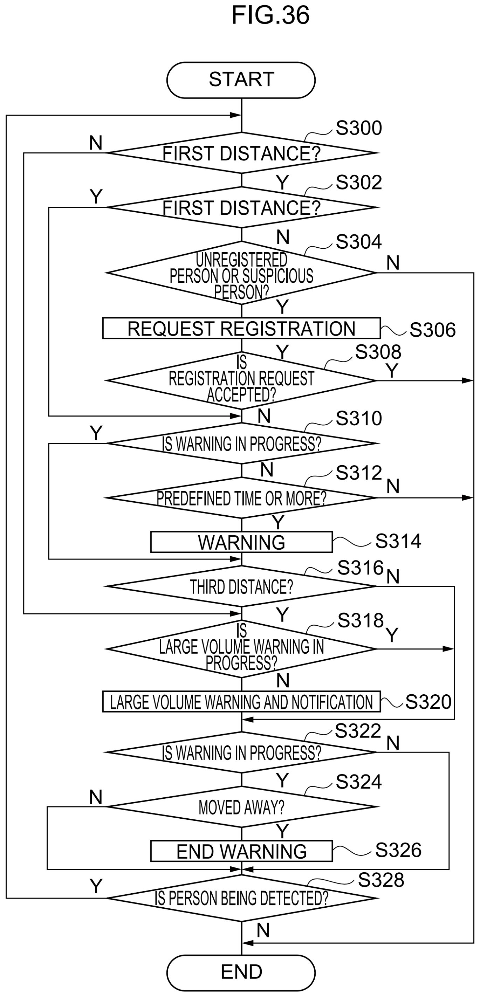

The warning processing in step S208 may be processing illustrated in Fig. 36. Fig. 36 is a flowchart illustrating an example of warning processing.

That is, in step S300, the warning unit 38 determines whether the person has approached the child at a predefined first distance. In the determination, for example, the sensor unit 200A detects the distance from the communication terminal 100M to the target person, and determines whether the detected distance is within the first distance (for example, 1 m). In a case in which the determination is negative, the processing proceeds to step S320, and in a case in which the determination is positive, the processing proceeds to step S302.

In step S302, the warning unit 38 determines whether the person has approached the child at a predefined second distance. In the determination, for example, the sensor unit 200A detects the distance from the communication terminal 100M to the target person, and determines whether or not the detected distance is within the second distance (for example, 50 cm). In a case in which the determination is positive, the processing proceeds to step S310, and in a case in which the determination is negative, the processing proceeds to step S304.

In step S304, the warning unit 38 determines whether or not the person is a person not registered in the database 32 or a suspicious person registered in the database 32 in advance. In a case in which the determination is positive, the processing proceeds to step 306, and in a case in which the determination is negative, the series of processing is ended.

In step S306, the action control unit 250 requests the approaching person to be registered in the database 32 and proceeds to step S308. For example, the action control unit 250 controls the control target 252 to output a message requesting registration of user identification information such as "To register you, please provide your address, name, and age." to the target person.

In step S308, the warning unit 38 determines whether the registration request has been accepted. In the determination, for example, it is determined whether or not an answer has been stated according to the registration. In a case in which the determination is negative, the processing proceeds to step S310, and in a case in which the determination is positive, the series of processing is ended.

In step S310, the warning unit 38 determines whether or not step S314 that will be described later has already been executed and a warning is being issued. In a case in which the determination is negative, the processing proceeds to step S312, and in a case in which the determination is positive, the processing proceeds to step S316.

In step S312, the warning unit 38 determines whether or not the person who has rejected the registration request is near the child for a predetermined time or more. In a case in which the determination is positive, the processing proceeds to step S314, and in a case in which the determination is negative, the series of processing is ended.

In step S314, the warning unit 38 and the action control unit 250 issue a warning such that the person does not approach the child, and the processing proceeds to step S316. For example, the warning unit 38 determines to issue a warning such that the person does not approach the child, and the action control unit 250 controls the control target 252 to output a message such as "Do not approach the child. I will notify.".

In step S316, the warning unit 38 determines whether the person has approached the child at a predetermined third distance. In the determination, for example, the sensor unit 200A detects the distance from the communication terminal 100M to the target person, and determines whether or not the detected distance is within the third distance (for example, 30 cm). In a case in which the determination is positive, the processing proceeds to step S318, and in a case in which the determination is negative, the processing proceeds to step S322.

In step S318, the warning unit 38 determines whether a warning is being issued at a predetermined large volume. In this determination, it is determined whether step S320 that will be described later has already been executed, and a large volume warning is being issued. In a case in which the determination is positive, the processing proceeds to step S322, and in a case in which the determination is negative, the processing proceeds to step S320.

In step S320, the warning unit 38 and the action control unit 250 issue a warning at a predetermined large volume, notify the police, and proceed to step S330. For example, the warning unit 38 determines, as an action of the communication terminal 10M, issuing a warning at a larger volume than in step S314 such that the person does not approach a child, and the action control unit 250 controls the control target 252 to output a message such that the person does not approach a child. The police or the like is notified that the suspicious person is approaching the child.

In step S322, the warning unit 38 determines whether a warning is being issued. In the determination, it is determined whether or not a warning is being issued after execution of step S314 or step S320. In a case in which the determination is negative, the processing proceeds to step S328, and in a case in which the determination is positive, the processing proceeds to step S324.

In step S324, the warning unit 38 determines whether or not the target person has moved away. In the determination, it is determined whether or not the approaching unregistered person or suspicious person has moved away from the child. In a case in which the determination is negative, the processing proceeds to step S328, and in a case in which the determination is positive, the processing proceeds to step S326.

In step S326, the warning unit 38 and the action control unit 250 end the warning issued in step S314 or step S320, and proceed to step S328. That is, the warning unit 38 determines ending of the warning, and the action control unit 250 controls the control target 252 to end the issued warning.

In step S328, the warning unit 38 determines whether a person is being detected. In the determination, it is determined whether a person approaching the child is continuously detected. In a case in which the determination is positive, the processing returns to step S300 to repeat the above-described processing, and in a case in which the determination is positive, the series of processing is ended.

By performing the warning processing as described above, a warning is issued to an unregistered person or a suspicious person who approaches the child, so that it is possible to protect the child from an unknown person or a suspicious person.

In a case in which the warning processing is started, imaging may be performed during the warning processing, and the captured image during the warning processing may be stored in the database 32. As a result, a guardian can check a newly registered person and a suspicious person by looking back on the captured images later.

### (Supplementary Note 1)

A control system including:
a state recognition unit that recognizes a user state including an action of a user and a state of an electronic apparatus;
an emotion determination unit that determines an emotion of the user or an emotion of the electronic apparatus; and
an action determination unit that determines, by using at least one of the user state, the state of the electronic apparatus, the emotion of the user, or the emotion of the electronic apparatus, and an action determination model at a predetermined timing, any of a plurality of types of device operations including non-operation as an action of the electronic apparatus, in which
the electronic apparatus detachably provided on a neck strap includes one or more sensors that collect information and include an imaging unit that images surroundings, and
the device operations include issuing a warning to a person satisfying a predefined condition among persons approaching the imaging unit.

### (Supplementary Note 2)

The control system according to Supplementary Note 1, in which the device operations include, as the predefined condition, requesting registration of identification information to a person approaching the imaging unit, and issuing a warning in a case in which the person who has rejected the registration is near the imaging unit for a predetermined time or more.

### (Supplementary Note 3)

The control system according to Supplementary Note 1, in which the device operations include, as the predefined condition, extracting a person from a captured image obtained by the imaging unit and issuing, in a case in which the extracted person is a suspicious person registered in advance, a warning to the suspicious person.

### (Supplementary Note 4)

The control system according to Supplementary Note 1, in which the device operations include storing a wearing object of a person who approaches the imaging unit.

### (Supplementary Note 5)

The control system according to Supplementary Note 1, in which the device operations include performing a predefined operation in accordance with a distance from a person to whom a warning is issued.

### (Supplementary Note 6)

The control system according to Supplementary Note 5, in which in the predefined operation, registration of identification information is requested in a case in which the distance is a predefined first distance, a warning is issued in a case in which the distance is a second distance shorter than the first distance, and a warning having a larger volume than the warning in the case of the second distance is issued in a case in which the distance is a third distance shorter than the second distance.

### (Other Embodiment 7)

### (Fourth Action Content)

In a case in which the user wearing the communication terminal 100M is performing work, the action determination unit 236 may determine the fourth action content of supporting the work for the user. In this case, a height position at which the user is performing the work is lower than the height position of the user's eyes.

The work in this case includes, for example, work to be performed on a device that is a work target (hereinafter referred to as "work target device"), and more specifically includes assembly work, disassembly work, and the like for the work target device. Inspection work, cleaning work, repair work, and the like for the work target device may be included.

The work in this case includes, for example, creation activities. More specifically, the creation activities include production activities of art works (art articles, craft articles, and the like), creation activities of literature works, creation activities of music works, performance activities of musical instruments, and the like.

In a case in which the fourth action content is determined, the device operations determined as the actions of the communication terminal 100M by the action determination unit 236 include setting, to the user performing the work, action content of supporting the work of the user by outputting at least one of vocal sound or an image from the communication terminal 100M.

In a case in which the fourth action content is determined, the work content and the work situation are photographed by the camera included in the communication terminal 100M, and the action content that supports the work of the user is set for the user performing the work on the basis of a captured image. The image in this case may be a still image or a moving image.

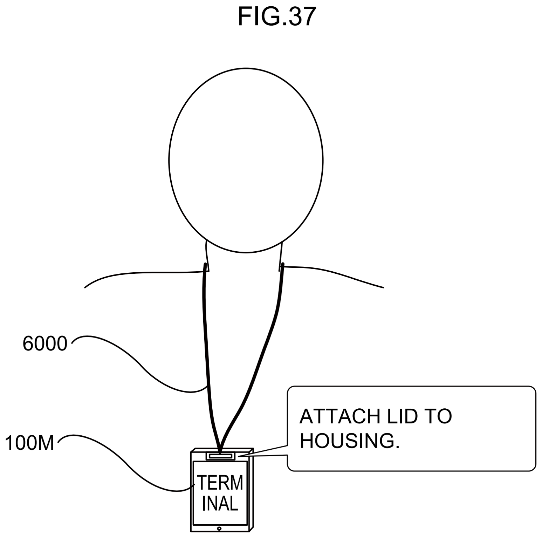

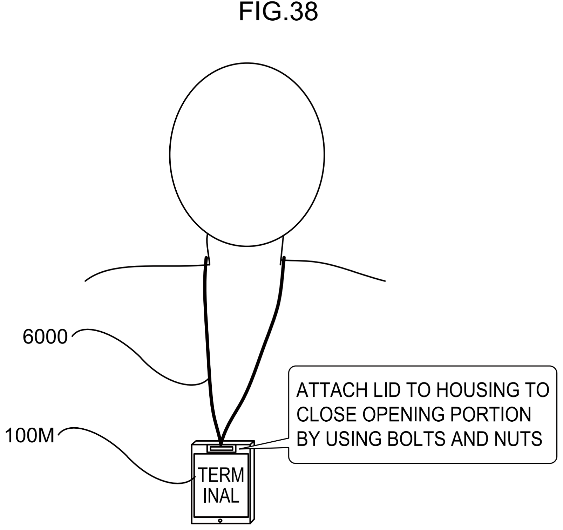

For example, in a case in which the user is performing assembly work for the work target device, the action determination unit 236 determines, as an action of the communication terminal 100M, to output vocal sound such as "Attach the lid to the housing." as illustrated in Fig. 37. The action determination unit 236 may determine, as the action of the communication terminal 100M, to output vocal sound that notifies the user of more specifically what attachment method to use for attachment. For example, as illustrated in Fig. 38, the action determination unit 236 determines to output vocal sound "Attach the lid to the housing to close the opening portion by using bolts and nuts." as the action of the communication terminal 100M. In a case in which a tool, an instrument, or an implement to be used is set according to an attachment method, the type of the tool, the instrument, or the implement to be used may also be included in the output content by using vocal sound.

In a case in which the user uses a tool, an instrument, or an implement, the action determination unit 236 may determine, as the action of the communication terminal 100M, to output advice about a method of using the tool, the instrument, or the implement. In this case, a camera included in the communication terminal 100M may recognize the type of the tool, the instrument, or the implement used by the user, capture an image of work content and a work situation, and set advice about a use method on the basis of the captured image. The user's actual use of a tool, an instrument, or an implement may be monitored, and advice about more appropriate use may be given.

The work performed on the work target device by the user may be completed with single work content, or may be sequentially performed with a plurality of pieces of work content. In a case in which the work performed by the user on the work target device includes a plurality of pieces of work content, the action determination unit 236 may determine, as the action of the communication terminal 100M, to output vocal sound that conveys a work procedure to the user. In this case, the progress status of the work is detected by the camera of the communication terminal 100M, and after the end of one work is detected, vocal sound output for supporting the next work may be emitted.

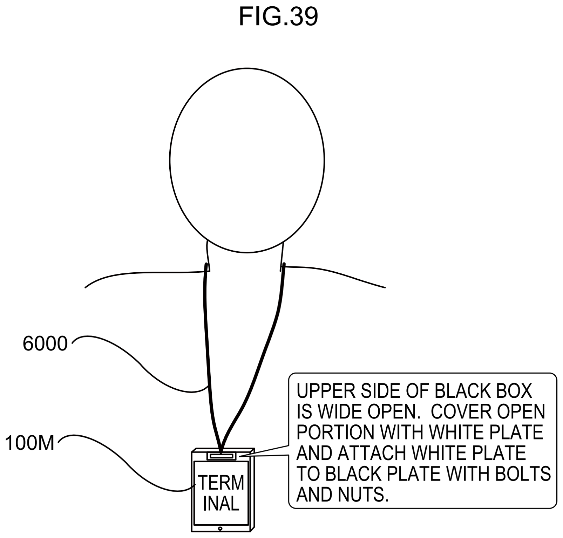

In a case in which the work that the user is performing is delayed, the action determination unit 236 includes supporting the work of the user by replacing the action content of supporting the work of the user with content different from the output content of the vocal sound so far. For example, as illustrated in Fig. 39, the action determination unit 236 may determine to output vocal sound "The upper side of the black box is wide open. Cover the open portion with a white plate and attach the white plate to the black plate with bolts and nuts." as the action of the communication terminal 100M.

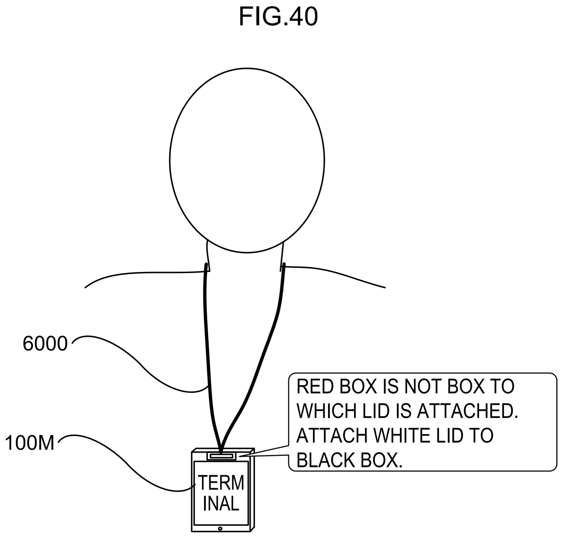

In a case in which the work performed by the user is wrong, the action determination unit 236 includes supporting the work of the user by replacing the action content of stopping the work of the user with the content different from the output content of the vocal sound so far. For example, as illustrated in Fig. 40, the action determination unit 236 may determine to output vocal sound "The red box is not a box to which a lid is attached. Attach a white lid to the black box." as the action of the communication terminal 100M.

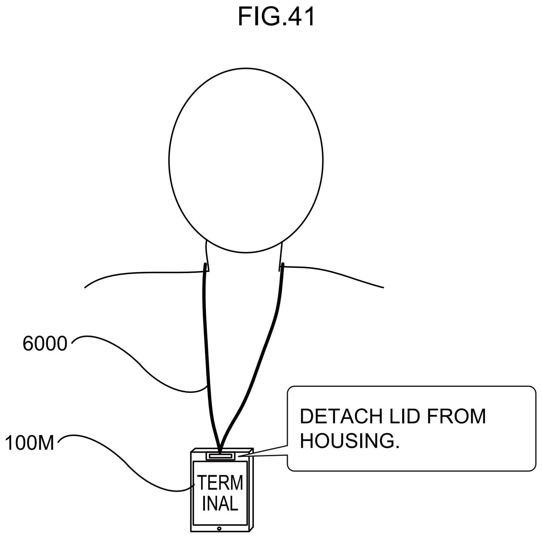

The above example is an example of a case in which the user is performing the assembly work for the work target device. On the other hand, even in a case in which the user is performing disassembly work, inspection work, cleaning work, repair work, and the like of the work target device, the action determination unit 236 determines the fourth action content of supporting the work for the user wearing the communication terminal 100M. For example, in a case in which the work is disassembly work for the work target device, the action determination unit 236 determines, as an action of the communication terminal 100M, to output vocal sound such as "Detach the lid from the housing." as illustrated in Fig. 41.

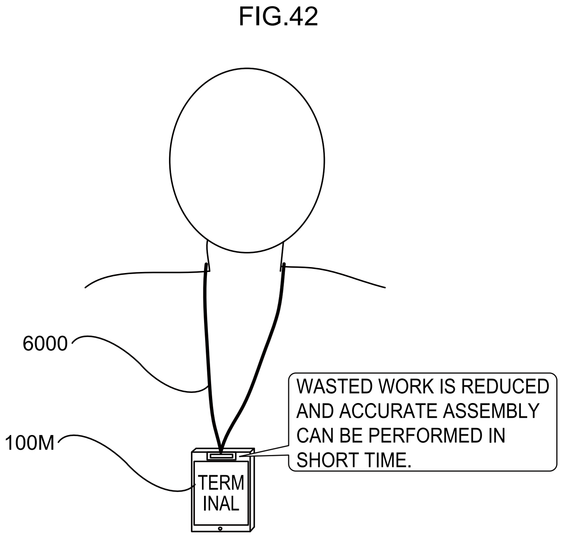

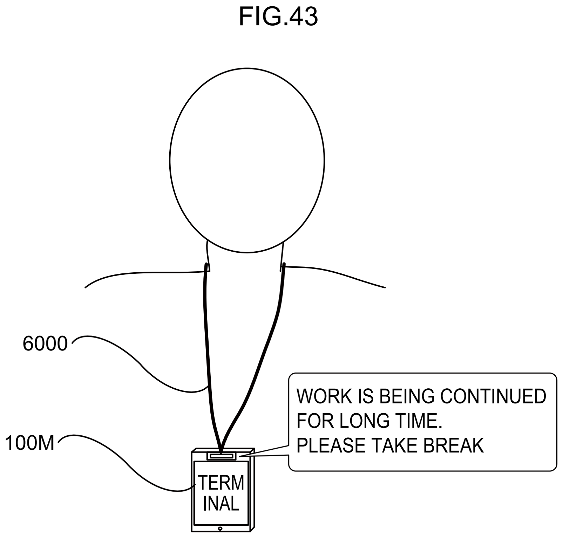

In a case in which the user is performing work on the work target device, the output from the communication terminal 100M as the device operation determined by the action determination unit 236 as the action of the communication terminal 100M may include analysis and evaluation on the work content. For example, in a case in which the time required by the user for specific work becomes short, for example, the action determination unit 236 may determine, as the action of the communication terminal 100M, to output vocal sound "Wasted work is reduced and accurate assembly can be performed in a short time." as illustrated in Fig. 42. As described above, highly evaluating the work of the user is to bring the emotion of the user closer to "pleased", and as a result, supports the work of the user. As illustrated in Fig. 43, the action determination unit 236 may determine, as the action of the communication terminal 100M, to output vocal sound such as "Work is being continued for a long time. Please take a break.". As described above, calling some kind of attention or the like to the user enhances work efficiency or avoids danger, and as a result, supports the work of the user.

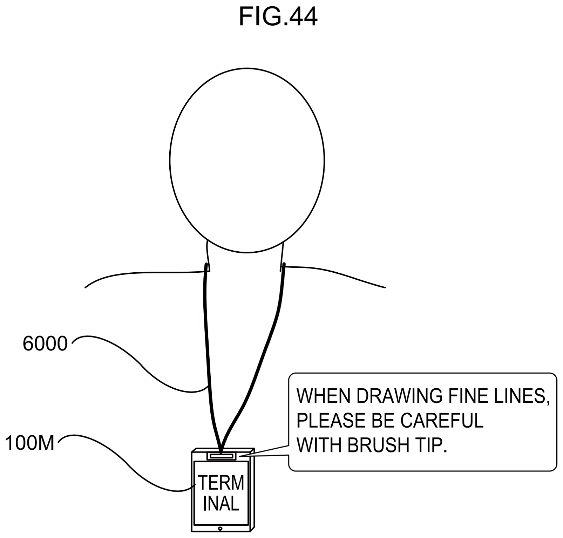

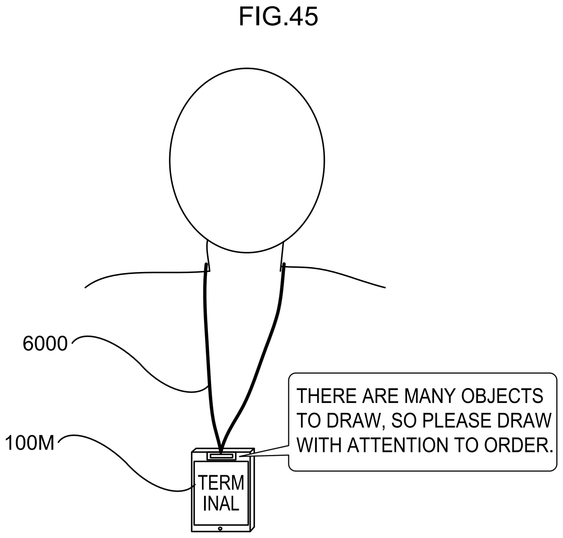

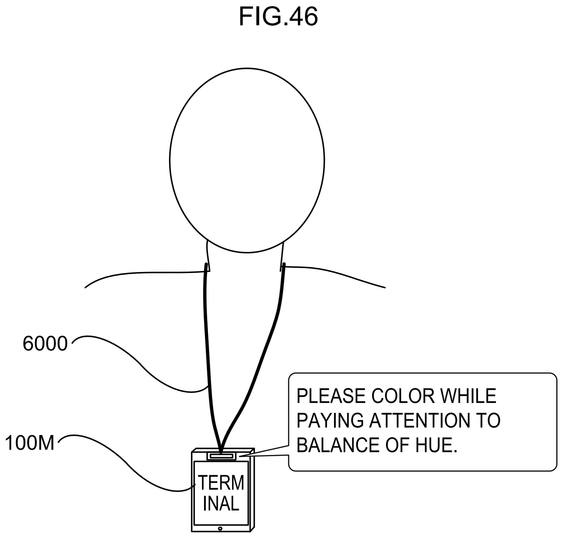

In a case in which the work performed by the user is a creation activity, the action determination unit 236 determines action content of supporting the work of the creation activity as an action of the communication terminal 100M. For example, in a case in which the creation activity is production of a painting, monitoring the production status with a camera of the communication terminal 100M and giving advice about a specific technique or the like are included. For example, as illustrated in Fig. 44, the action determination unit 236 determines to output vocal sound "When drawing fine lines, please be careful with the brush tip." as the action of the communication terminal 100M. As another example, the action determination unit 236 may determine to output vocal sound "There are many objects to draw, so please draw with attention to the order." as illustrated in Fig. 45. As still another example, as illustrated in Fig. 46, the action determination unit 236 may determine to output vocal sound such as "Please color while paying attention to the balance of hue.".

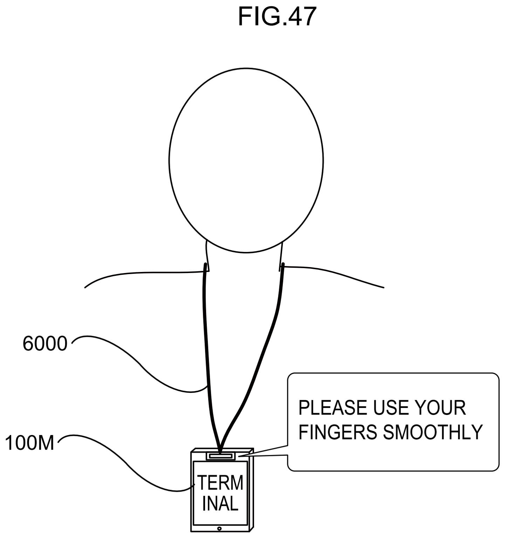

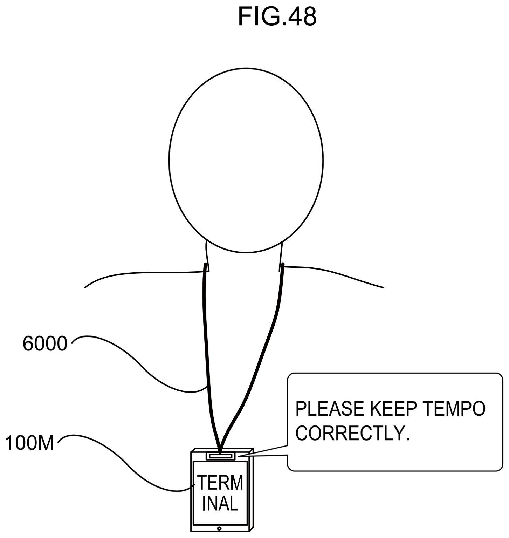

In a case in which the creation activity performed by the user is performance of a musical instrument, monitoring the performance with the camera of the communication terminal 100M and giving advice about a specific performance method or the like are included. For example, as illustrated in Fig. 47, the action determination unit 236 determines to output vocal sound "Please use your fingers smoothly." as the action of the communication terminal 100M. As still another example, as illustrated in Fig. 48, the action determination unit 236 may determine to output vocal sound such as "Please keep the tempo correctly.".

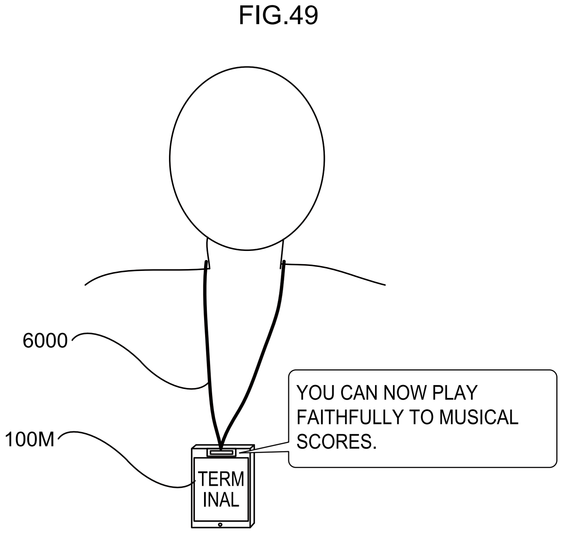

In a case in which the creation activity performed by the user is performance of a musical instrument, the output from the communication terminal 100M as the device operation determined by the action determination unit 236 as the action of the communication terminal 100M may include analysis and evaluation of the musical instrument performance. For example, as illustrated in Fig. 49, the action determination unit 236 determines to output vocal sound "You can now play faithfully to musical scores." as the action of the communication terminal 100M.

According to the robot 100 and the communication terminal of the disclosure, an action of leading the user can be executed through autonomous processing. As a result, the robot 100 and the communication terminal can guide a user walking in a town that the user visits for the first time to an appropriate route. It is possible to guide a user who is interested in walking to a route along which the user has not passed before. A route along which the user cannot pass is detected in advance, and the user can be guided to another route. A user who gets lost and deviates from a specific route can be guided to an original route of the user. In a case in which a user is performing work at a position lower than the eyes of the user, this work can be supported.

The action determination unit 236 determines, by using at least one of the state of the user 10, the emotion of the user 10, the emotions of the robot 100 and the communication terminal, or the states of the robot 100 and the communication terminal, and the action determination model 221 at a predetermined timing, any of a plurality of types of robot actions and communication terminal actions including no action as actions of the robot 100 and the communication terminal. Here, a case in which a sentence generation model having an interaction function is used as the action determination model 221 will be described as an example.

Specifically, the action determination unit 236 inputs text representing at least one of the state of the user 10, the emotion of the user 10, the emotions of the robot 100 and the communication terminal, or the states of the robot 100 and the communication terminal, and text for inquiry about the robot action and the communication terminal action to the sentence generation model, and determines the actions of the robot 100 and the communication terminal on the basis of the output of the sentence generation model.

For example, the plurality of types of actions of the robot and the communication terminal include the following (1) to (24). The users in (1) to (24) include users working at positions lower than the eyes.
(1) The robot 100 and the communication terminal 100M do nothing.
(2) The robot 100 and the communication terminal 100M have a dream.
(3) The robot 100 and the communication terminal 100M talk to the user.
(4) The robot 100 and the communication terminal 100M create a picture diary.
(5) The robot 100 and the communication terminal 100M propose an activity.
(6) The robot 100 and the communication terminal 100M propose a person with whom the user should meet.
(7) The robot 100 and the communication terminal 100M introduce news in which the user is interested. (8) The robot 100 and the communication terminal 100M edit pictures and moving images.
(9) The robot 100 and the communication terminal 100M study with the user.
(10) The robot 100 and the communication terminal 100M evoke memories.
(11) The robot 100 and the communication terminal 100M may reproduce an image of a route along which the user can walk on the screen of the communication terminal 100M as the first action content of leading the user.
(12) The robot 100 and the communication terminal 100M may reproduce vocal sound related to a route along which the user can walk as the first action content of correcting the action of the user.
(13) The robot 100 and the communication terminal 100M may reproduce an image of a route different from the set guidance route on the screen of the communication terminal 100M as the first action content of correcting the action of the user.
(14) The robot 100 and the communication terminal 100M may reproduce vocal sound related to a route different from the set guidance route as the first action content of correcting the action of the user.
(15) The robot 100 and the communication terminal 100M may reproduce, as the first action content of correcting the action of the user, an image of a route for return to the set guidance route on the screen of the communication terminal 100M in a case in which the user led along the set guidance route deviates from the guidance route.
(16) The robot 100 and the communication terminal 100M may reproduce, as the first action content of correcting the action of the user, vocal sound related to a route for return to the set guidance route in a case in which the user led along the set guidance route deviates from the guidance route.
(17) The robot 100 and the communication terminal 100M may reproduce vocal sound praising the user for the action as the second action content or the fourth action content different from the first action content.
(18) The robot 100 and the communication terminal 100M may reproduce vocal sound expressing gratitude for the action of the user as the second action content or the fourth action content different from the first action content.
(19) The robot 100 and the communication terminal 100M may reproduce an image praising the user for the action as the second action content or the fourth action content different from the first action content.
(20) The robot 100 and the communication terminal 100M may reproduce an image expressing gratitude for the action of the user as the second action content or the fourth action content different from the first action content.
(21) The robot 100 and the communication terminal 100M may transmit specific information to a person other than the user as the third action content or the fourth action content different from the first action content.
(22) The robot 100 and the communication terminal 100M may reproduce sound that attracts the interest of the user as the third action content or the fourth action content different from the first action content.
(23) The robot 100 and the communication terminal 100M may reproduce an image that attracts the interest of the user as the third action content or the fourth action content different from the first action content.
(24) The robot 100 and the communication terminal 100M may reproduce at least one of vocal sound or an image that supports the work performed by the user as the fourth action content different from the first action content.

In a case in which the user wearing the communication terminal 100M is performing work at a position lower than the eyes, the action determination unit 236 of the agent system 500 may determine the fourth action content of supporting the work for the user.

In this case, the device operation determined as the action of the communication terminal 100M by the action determination unit 236 of the agent system 500 includes recognizing content and a situation of the work being performed by the user by the communication terminal 100M reproducing at least one of vocal sound or an image and setting the fourth action content of supporting the work.

For example, as illustrated in Fig. 37, the action determination unit 236 of the agent system 500 determines, as an action of the communication terminal 100M, to output vocal sound such as "Attach the lid to the housing.". The action determination unit 236 may determine, as the action of the communication terminal 100M, to output vocal sound that notifies the user of more specifically what attachment method to use for attachment. For example, as illustrated in Fig. 38, the action determination unit 236 determines to output vocal sound "Attach the lid to the housing to close the opening by using bolts and nuts." as the action of the communication terminal 100M. In a case in which a tool, an instrument, or an implement to be used is set according to the attachment method, the type of the tool, the instrument, or the implement to be used may also be included in the output content using vocal sound.

In a case in which the user uses a tool, an instrument, or an implement, the action determination unit 236 of the agent system 500 may determine, as the action of the communication terminal 100M, to output advice about a method of using the tool, the instrument, or the implement. In this case, the camera included in the communication terminal 100M may recognize the type of the tool, the instrument, or the implement used by the user, capture an image of work content and a work situation, and set advice about a use method on the basis of the captured image. The user's actual use of a tool, an instrument, or an implement may be monitored, and advice about more appropriate use may be given.

The work performed on the work target device by the user may be completed with single work content, or may be sequentially performed with a plurality of pieces of work content. In a case in which the work performed by the user on the work target device includes a plurality of pieces of work content, the action determination unit 236 of the agent system 500 may determine, as the action of the communication terminal 100M, to output vocal sound that conveys a work procedure to the user. In this case, the progress status of the work is detected by the camera of the communication terminal 100M, and after the end of one work is detected, vocal sound output for supporting the next work may be emitted.

In a case in which the work that the user is performing is delayed, the action determination unit 236 of the agent system 500 includes supporting the work of the user by replacing the action content of supporting the work of the user with content different from the output content of the vocal sound so far. For example, as illustrated in Fig. 39, the action determination unit 236 may determine to output vocal sound "The upper side of the black box is wide open. Cover the open portion with a white plate and attach the white plate to the black plate with bolts and nuts." as the action of the communication terminal 100M.

In a case in which the work performed by the user is wrong, the action determination unit 236 of the agent system 500 includes supporting the work of the user by replacing the action content of stopping the work of the user with the content different from the output content of the vocal sound so far. For example, as illustrated in Fig. 40, the action determination unit 236 may determine to output vocal sound "The red box is not a box to attach a lid to. Attach a white lid to the black box." as the action of the communication terminal 100M.

In a case in which the user is performing disassembly work, inspection work, cleaning work, repair work, and the like of the work target device, the action determination unit 236 of the agent system 500 determines the fourth action content of supporting the work for the user wearing the communication terminal 100M. For example, in a case in which the work is disassembly work for the work target device, the action determination unit 236 determines to output vocal sound such as "Detach the lid from the housing." as illustrated in Fig. 41, as an action of the communication terminal 100M.

In a case in which the user is performing work on the work target device, the output from the communication terminal 100M as the device operation determined by the action determination unit 236 of the agent system 500 as the action of the communication terminal 100M may include analysis and evaluation on the work content. For example, in a case in which the time required by the user for specific work becomes short, for example, the action determination unit 236 may determine, as the action of the communication terminal 100M, to output vocal sound "Wasted work is reduced and accurate assembly can be performed in a short time." as illustrated in Fig. 42. As described above, highly evaluating the work of the user is to bring the emotion of the user closer to "pleased", and as a result, supports the work of the user. As illustrated in Fig. 43, the action determination unit 236 may determine, as the action of the communication terminal 100M, to output vocal sound such as "Work is being continued for a long time. Please take a break.". As described above, performing some kind of attention or the like to the user enhances work efficiency or avoids danger, and as a result, supports the work of the user.

In a case in which the work performed by the user is a creation activity, the action determination unit 236 of the agent system 500 determines action content of supporting the work of the creation activity as an action of the communication terminal 100M. For example, in a case in which the creation activity is production of a painting, monitoring the production status with a camera of the communication terminal 100M and giving advice about a specific technique or the like are included. For example, as illustrated in Fig. 44, the action determination unit 236 of the agent system 500 determines to output vocal sound "When drawing fine lines, please be careful with the brush tip." as the action of the communication terminal 100M. As another example, the action determination unit 236 of the agent system 500 may determine to output vocal sound "There are many objects to draw, so please draw with attention to the order." as illustrated in Fig. 45. As still another example, as illustrated in Fig. 46, the action determination unit 236 of the agent system 500 may determine to output vocal sound "Please color while paying attention to the balance of hue.".

In a case in which the creation activity performed by the user is performance of a musical instrument, monitoring the performance with the camera of the communication terminal 100M and giving advice about a specific performance method or the like are included. For example, as illustrated in Fig. 47, the action determination unit 236 of the agent system 500 determines to output vocal sound "Please use your fingers smoothly." as the action of the communication terminal 100M. As still another example, as illustrated in Fig. 48, the action determination unit 236 of the agent system 500 may determine to output vocal sound "Please keep the tempo correctly.".

In a case in which the creation activity performed by the user is performance of a musical instrument, the output from the communication terminal 100M as the device operation determined by the action determination unit 236 of the agent system 500 as the action of the communication terminal 100M may include analysis and evaluation of the musical instrument performance. For example, as illustrated in Fig. 49, the action determination unit 236 determines to output vocal sound "You can now play faithfully to musical scores." as the action of the communication terminal 100M.

According to the agent system 500 of the disclosure, an action of leading the user can be executed through autonomous processing. As a result, the agent can guide the user walking in a town that the user visits for the first time to an appropriate route. It is possible to guide a user who is interested in walking to a route along which the user has not passed before. A route along which the user cannot pass is detected in advance, and the user can be guided to another route. A user who gets lost and deviates from a specific route can be guided to an original route of the user.

The action determination unit 236 determines, by using at least one of the state of the user 10, the emotion of the user 10, the emotions of the agent, or the states of the agent, and the action determination model 221 at a predetermined timing, any of a plurality of types of agent actions including no action as an action of the agent. Here, a case in which a sentence generation model having an interaction function is used as the action determination model 221 will be described as an example.

Specifically, the action determination unit 236 inputs text representing at least one of the state of the user 10, the emotion of the user 10, the emotion of the agent, or the state of the agent and text for inquiry about the agent action to the sentence generation model, and determines the action of the agent on the basis of the output of the sentence generation model.

For example, the plurality of types of agent actions include the following (1) to (24). The users in (1) to (24) include users working at positions lower than the eyes.
(1) The agent does nothing.
(2) The agent has a dream.
(3) The agent speaks to the user.
(4) The agent creates a picture diary.
(5) The agent proposes an activity.
(6) The agent proposes a person with whom the user should meet.
(7) The agent introduces news in which the user is interested.
(8) The agent edits pictures and moving images.
(9) The agent studies with the user.
(10) The agent evokes memories.
(11) The agent may reproduce an image of a route along which the user can walk on the screen of the communication terminal as the first action content of leading the user.
(12) The agent may reproduce vocal sound related to a route along which the user can walk as the first action content of correcting the action of the user.
(13) The agent may reproduce an image of a route different from the set guidance route on the screen of the communication terminal 100M as the first action content of correcting the action of the user.
(14) The agent may reproduce vocal sound related to a route different from the set guidance route as the first action content of correcting the action of the user.
(15) The agent may reproduce, as the first action content of correcting the action of the user, an image of a route for return to the set guidance route on the screen of the communication terminal 100M in a case in which the user led along the set guidance route deviates from the guidance route.
(16) The agent may reproduce, as the first action content of correcting the action of the user, vocal sound related to a route for return to the set guidance route in a case in which the user led along the set guidance route deviates from the guidance route.
(17) The agent may reproduce vocal sound praising the user for the action as the second action content or the fourth action content different from the first action content.
(18) The agent may reproduce vocal sound expressing gratitude for the action of the user as the second action content or the fourth action content different from the first action content.
(19) The agent may reproduce an image praising the user for the action as the second action content or the fourth action content different from the first action content.
(20) The agent may reproduce an image expressing gratitude for the action of the user as the second action content or the fourth action content different from the first action content.
(21) The agent may transmit specific information to a person other than the user as the third action content or the fourth action content different from the first action content.
(22) The agent may reproduce sound that attracts the interest of the user as the third action content or the fourth action content different from the first action content.
(23) The agent may reproduce an image that attracts the interest of the user as the third action content or the fourth action content different from the first action content.
(24) The agent may reproduce at least one of vocal sound or an image that supports the work performed by the user as the fourth action content different from the first action content.

The action determination unit 236 of the agent system 500 may execute the second action content or the fourth action content different from the first action content. Specifically, the action determination unit 236 may perform, as the agent action, the second action content or the fourth action content in the above "(17)", that is, reproduce vocal sound praising the user for the action.

The action determination unit 236 of the agent system 500 may perform, as the agent action, the second action content or the fourth action content in the above "(18)", that is, reproduce vocal sound expressing gratitude for the action of the user.

The action determination unit 236 of the agent system 500 may perform, as the agent action, the second action content or the fourth action content in the above "(19)", that is, reproduce an image praising the user for the action.

The action determination unit 236 of the agent system 500 may perform, as the agent action, the second action content or the fourth action content in the above "(20)", that is, reproduce an image expressing gratitude for the action of the user.

The action determination unit 236 of the agent system 500 may execute the third action content or the fourth action content different from the first action content. Specifically, the action determination unit 236 may perform, as the agent action, the third action content or the fourth action content in the above "(21)", that is, transmit the specific information to a person other than the user.

The action determination unit 236 of the agent system 500 may perform, as the agent action, the third action content or the fourth action content in the above "(22)", that is, execute reproduction of sound that attracts the interest of the user.

The action determination unit 236 of the agent system 500 may perform, as the agent action, the third action content or the fourth action content in the above "(23)", that is, execute reproduction of an image that attracts the interest of the user.

The action determination unit 236 of the agent system 500 may perform, as the communication terminal action, the fourth action content in the above "(24)", that is, execute reproduction of vocal sound or an image that supports the work performed by the user.

In a case in which vocal sound or an image that supports the work is reproduced as the fourth action content indicated in "(24)" for the user performing the work, the related information collection unit 270 of the agent system 500 may store data of a tool, equipment, a material, and the like used by the user in the collected data 223.

### (Supplementary Note 1)

A control system including:
a state recognition unit that recognizes a user state including an action of a user and a state of an electronic apparatus;
an emotion determination unit that determines an emotion of the user or an emotion of the electronic apparatus; and
an action determination unit that determines, by using at least one of the user state, the state of the electronic apparatus, the emotion of the user, or the emotion of the electronic apparatus and an action determination model at a predetermined timing, any of a plurality of types of device operations including non-operation as an action of the electronic apparatus, in which
the electronic apparatus detachably provided on a neck strap includes one or more sensors that collect information, and
the device operations include setting action content of supporting work performed by the user at a position lower than eyes of the user by the electronic apparatus reproducing at least one of vocal sound or an image generated by the action determination model on the basis of the information collected by the electronic apparatus.

### (Supplementary Note 2)

The control system according to Supplementary Note 1, in which the action determination unit determines the action content of giving advice about a work procedure for work performed on a work target device by the user.

### (Supplementary Note 3)

The control system according to Supplementary Note 2, in which the action determination unit determines the action content of giving advice about assembly work or disassembly work for the work target device as the work.

### (Supplementary Note 4)

The control system according to Supplementary Note 1, in which the action determination unit determines the action content of giving advice about a work procedure for work performed on a creation activity by the user.

### (Supplementary Note 5)

The control system according to Supplementary Note 4, in which the action determination unit determines the action content of giving advice about a creation activity of an art work as the creation activity.

### (Supplementary Note 6)

The control system according to Supplementary Note 1, in which the sensor detects a status of the work performed by the user.

### (Supplementary Note 7)

The control system according to Supplementary Note 1, in which the sensor detects a tool or an implement used by the user.

### (Supplementary Note 8)

The control system according to Supplementary Note 1, in which the action content includes reproduction of at least one of vocal sound praising the action of the user, vocal sound expressing gratitude for the action of the user, an image praising the user for the action, or an image expressing gratitude for the action of the user.

### (Supplementary Note 9)

The control system according to Supplementary Note 1, in which the action determination unit determines whether or not the action of the user has been corrected by detecting the action of the user after supporting the work for the user, and determines action content different from previous action content in a case in which the action of the user has not been corrected.

### (Supplementary Note 10)

The control system according to Supplementary Note 1, in which
the electronic apparatus is a communication terminal, and
the action determination unit determines any of a plurality of types of actions of an agent for interacting with the user, including no action, as an action of the communication terminal.

### (Supplementary Note 11)

The control system according to Supplementary Note 10, in which the action determination model is a sentence generation model having an interaction function, and the action determination unit inputs, to the sentence generation model, text representing at least one of the user state, a state of the agent, the emotion of the user, or an emotion of the agent, and text for inquiry about an action of the agent, and determines the action of the communication terminal on the basis of an output of the sentence generation model.

### [Sixth Embodiment]

In a sixth embodiment, the user 10 is guided to a safe place by controlling the wheels 601a of the movement unit 601 on the basis of the information regarding the surrounding situation of the user 10. Portions having the same configurations as those of the first to fourth embodiments are denoted by the same reference numerals, and description thereof will be omitted. A case in which the agent system 500 is mounted on the communication terminal 600 (see Fig. 16), and the communication terminal 600 is detachably provided on the movement unit 601 to configure the movement system 1000M will be described as an example.

The action determination unit 236 of the agent system 500 determines any of a plurality of types of agent actions as an action of the agent by using at least one of the user state, the state of the agent, the emotion of the user, or the emotion of the agent, and the action determination model at a predetermined timing. In this case, the action determination unit 236 may spontaneously or periodically detect the action of the user 10 and the information regarding the surrounding situation of the user 10 on the basis of the sensor, and determine the first action content of leading the user 10 to a safe place as the action of the agent on the basis of the detected action of the user 10 and the detected information regarding the surrounding situation of the user 10.

The sensor may be interpreted as one or a plurality of sensors provided in the communication terminal 600 detachably provided on the movement unit 601 of the movement system 1000M. Specifically, the one or plurality of sensors may include a camera, a microphone, a gyro sensor, and the like. The one or plurality of sensors may collect the information regarding the surrounding situation of the user 10 and information for controlling the rotation of the wheels 601a of the movement unit 601.

### (First Action Content)

The first action content is to lead the user 10 to a safe place by controlling the wheels 601a of the movement unit 601 on the basis of the information regarding the surrounding situation of the user 10. Controlling the wheels 601a may include changing at least one of a rotation direction, a rotation speed, or a steering direction of the wheels 601a. Hereinafter, an example of the first action content will be described.

In a case in which it is detected that there is a dangerous obstacle around the user 10, the action determination unit 236 determines first action content of leading the user 10 to a safe place so that the user 10 does not approach the dangerous obstacle. Examples of the dangerous obstacle include an approach of a vehicle to the user 10 and an external obstacle such as a disaster site or an accident site in an advancing direction of the user 10. In this case, for example, in a case in which it is detected that there is an external obstacle around the user 10, the action determination unit 236 determines the first action content of leading the user 10 to a safe place so that the user 10 does not approach the external obstacle.

The action determination unit 236 may execute reproduction of at least one of vocal sound or an image for assisting in walking of the user 10 as the first action content of performing route guidance so that the user 10 moves along a specific route as an evacuation path. As illustrated in Fig. 18, the action determination unit 236 may reproduce an image of a route along which the user 10 can walk on the screen of the communication terminal 600, and may reproduce vocal sound related to the route. The vocal sound may include "Turn right 10 m ahead".

The action determination unit 236 may execute reproduction of at least one of vocal sound or an image for guiding the user along a route different from the set guidance route. Specifically, as illustrated in Fig. 19, the action determination unit 236 may reproduce an image of a route different from the set guidance route on the screen of the communication terminal 600, and may reproduce vocal sound related to the route. The vocal sound may include, for example, "The road is closed ahead. Would you like me to show another route?".

In a case in which the user being led along the set guidance route deviates from the guidance route, the action determination unit 236 may execute reproduction of at least one of vocal sound or an image for guiding the user along a path for returning the user to the guidance route. Specifically, as illustrated in Fig. 20, the action determination unit 236 may reproduce an image of the route returning the user to the set guidance route on the screen of the communication terminal 600, and may reproduce vocal sound related to the route. The vocal sound may include "You have deviated from the route. I will show you the path for return to the original route." and the like.

In a case in which it is detected that the dangerous obstacle around the user 10 has been eliminated, the action determination unit 236 may notify the user 10 that the dangerous obstacle around the user 10 has been eliminated, and determine to end the first action content.

### (Second Action Content)

The action determination unit 236 may determine whether or not the action of the user has been corrected by detecting the action of the user while the movement unit 601 was leading the user or after the movement unit 601 reproduced at least one of vocal sound or an image that assists in the walking of the user, and may determine second action content different from the first action content in a case in which the action of the user has been corrected.

The case in which the action of the user 10 has been corrected may be interpreted as a case in which the user has stopped a specific action and a specific behavior or a case in which a specific situation has been resolved as a result of execution of the operation of the movement system 1000M according to the first action content. Specifically, the case in which the action of the user 10 has been corrected may include a case in which the user 10 has started walking along the proposed different route, a case in which the user 10 has started walking along a path for return to the original route when the path is shown due to deviation from the original route, and the like.

The second action content may include reproduction of at least one of vocal sound praising the user 10 for the action, vocal sound expressing gratitude for the action of the user 10, an image praising the user 10 for the action, and an image expressing gratitude for the action of the user 10.

Specifically, the vocal sound praising the user for the action may include, for example, "Well done! Excellent". The vocal sound expressing gratitude for the action of the user 10 may include vocal sounds of "Thank you for coming back" and "Thank you for selecting another route". The image praising the user 10 for the action may include an image of a character taking a good pose as illustrated in Fig. 21. As illustrated in Fig. 22, the image expressing gratitude for the action of the user 10 may include an image of a character who expresses gratitude.

### (Third Action Content)

The action determination unit 236 may determine whether or not the action of the user 10 has been corrected by detecting the action of the user 10 while the movement unit 601 was leading the user or after the movement unit 601 reproduced at least one of vocal sound or an image that assists in walking of the user 10, and determine third action content different from the first action content in a case in which the action of the user 10 has not been corrected.

The case in which the action of the user 10 has not been corrected may be interpreted as a case in which the user 10 has continued the dangerous action and behavior even though the operation of the movement system 1000M according to the first action content was executed, a case in which the dangerous situation is not resolved, or a case in which the user has started or continued an action different from the proposal of the movement system 1000M.

Specifically, the case in which the action of the user 10 has not been corrected may include a case in which the user 10 has walked along a route different from the proposed different route without walking along the proposed different route, a case in which the user 10 has neglected a path for return to the original route and continued walking when the path was shown due to deviation from the route, and the like.

The third action content may include at least one of transmission of specific information to a person other than the user 10, reproduction of sound that attracts an interest of the user 10, or reproduction of an image that attracts an interest of the user 10.

The transmission of the specific information to a person other than the user may include distribution of an email containing a warning message to a relative of the user 10, a spouse of the user 10, a guardian of the user 10, or the like. The transmission of the specific information to a person other than the user may include distribution of an image (a still image or a moving image) including the user 10 and the surrounding scenery to a relative of the user 10, a spouse of the user 10, a guardian of the user 10, or the like. The transmission of the specific information to a person other than the user may include distribution of vocal sound having a warning message.

The reproduction of the sound that attracts the interest of the user 10 may include specific music that the user 10 likes, and may also include vocal sound such as "Please come back here" or "Let's take a safe road".

The reproduction of the image that attracts the interest of the user 10 may include an image of an animal kept by the user 10, an image of parents of the user 10, an image of a character of an animal illustrated in Fig. 23, and the like.

According to the movement system 1000M of the disclosure, it is possible to execute an action of leading the user 10 to a safe place through autonomous processing. A route along which the user 10 cannot pass is detected in advance, and the user 10 can be guided to another route. The user 10 who gets lost and deviates from the specific route can be guided to the original route.

### (Supplementary Note 1)

A control system including:
a state recognition unit that recognizes a user state including an action of a user and a state of an electronic apparatus;
an emotion determination unit that determines an emotion of the user or an emotion of the electronic apparatus; and
an action determination unit that determines, by using at least one of the user state, the state of the electronic apparatus, the emotion of the user, or the emotion of the electronic apparatus, and an action determination model at a predetermined timing, any of a plurality of types of device operations including non-operation as an action of the electronic apparatus, in which
the electronic apparatus is detachably provided on a movement unit having a wheel for autonomous traveling,
the electronic apparatus includes one or a plurality of sensors that collect information regarding a surrounding situation of the user,
the electronic apparatus controls rotation of the wheel on the basis of the information regarding the surrounding situation of the user, and
the action determination unit determines, as an action of the electronic apparatus, first action content of leading the user to a safe place so that the user does not approach a dangerous obstacle in a case in which it is detected that there is the dangerous obstacle around the user.

### (Supplementary Note 2)

The control system according to Supplementary Note 1, in which
the action determination unit notifies the user of elimination of the dangerous obstacle around the user and ends the first action content in a case in which the elimination of the dangerous obstacle around the user is detected.

### [Seventh Embodiment]

In a seventh embodiment, an aspect will be described in which, in the movement system 1000M (see Figs. 16 and 17) including the movement unit 601 and the communication terminal 600 as an example of an electronic apparatus, in a case in which it is recognized that a user is in imminent danger, an action of being a substitute for the user is performed. Portions having the same configurations as those of the first to fourth embodiments are denoted by the same reference numerals, and description thereof will be omitted.

In the seventh embodiment, as the sensor, one or a plurality of sensors that are provided in the communication terminal 600 attachable to and detachable from the movement unit 601 of the movement system 1000M and collect information regarding the surrounding situation of the user can be applied. Specifically, the one or plurality of sensors may include a camera, a microphone, a gyro sensor, a receiving device that receives weather information such as a lightning striking probability in the surroundings, and the like. The one or plurality of sensors collect information regarding the surrounding situation of the user 10 and information for controlling the rotation of the wheels 601a of the movement unit 601.

In the seventh embodiment, the action determination unit 236 monitors whether or not a situation occurs in which the user 10 is in imminent danger on the basis of information regarding the surrounding situation of the user 10 collected by one or a plurality of sensors. The situation in which the user 10 is in imminent danger includes, for example, a situation in which an object is approaching the user 10. Examples of the object include vehicles including a car, a motorcycle, and a bicycle, a person (for example, a person who is running or walking quickly), and an animal (for example, a wild animal such as a bear). The situation in which the user 10 is in imminent danger may be, for example, a situation in which the lightning striking probability at the place where the user 10 is currently located is a predetermined value or more.

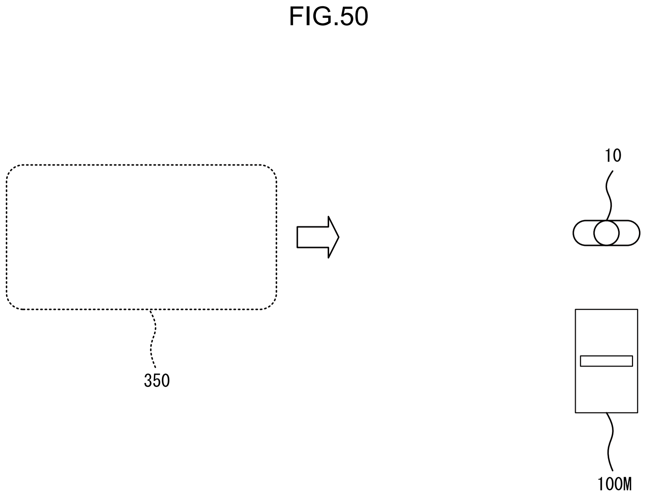

In the seventh embodiment, in a case in which it is detected that the user 10 is in imminent danger, the action determination unit 236 determines to take an action of being a substitute for the user 10 as the action of the movement system 1000M including the communication terminal 600. For example, as illustrated in Fig. 50, in a case in which a situation in which the user 10 is in imminent danger is a situation in which an object 350 such as a vehicle is approaching the user 10, the action determination unit 236 determines to move the movement system 1000M to a position between the object 350 and the user 10 as an action of being a substitute for the user 10. In order to improve the degree of safety of the user 10, the action determination unit 236 further determines to guide the user 10 to evacuate to a direction or a position where the user 10 moves away from the object 350.

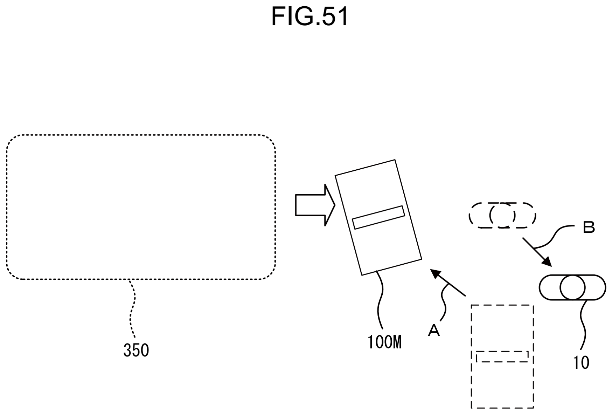

As a result, as indicated by an arrow A in Fig. 51, the rotation direction, the rotation speed, and the steering direction of the wheels 601a are controlled so that the movement system 1000M moves to a position between the object 350 and the user 10, and the movement system 1000M collides with the object 350 as a substitute for the user 10. Therefore, the degree of safety of the user 10 is improved. Vocal sound, an image, or the like for prompting evacuation of the user 10 is output from the communication terminal 600, and the user 10 takes an action of evacuating to a direction or a position away from the object 350 as indicated by an arrow B in Fig. 51 according to the vocal sound, the image, or the like, whereby the degree of safety of the user 10 is further improved.

For example, also in a case in which the situation in which the user 10 is in imminent danger is a situation in which an animal (for example, a wild animal such as a bear) is approaching the user 10, the action determination unit 236 determines to move the movement system 1000M to a position between the animal and the user 10 as an action of being a substitute for the user 10. In order to improve the degree of safety of the user 10, the action determination unit 236 further determines to guide the user 10 to evacuate to a direction or a position where the user 10 moves away from the animal.

As a result, the rotation direction, the rotation speed, and the steering direction of the wheels 601a are controlled so that the movement system 1000M moves to a position between the animal and the user 10, and the attention of the animal is attracted to the movement system 1000M, whereby the movement system 1000M becomes a substitute for the user 10, and the degree of safety of the user 10 is improved. Vocal sound or the like for prompting evacuation of the user 10 is output from the communication terminal 600, and the user 10 takes an action of evacuating to a direction or a position away from the animal according to the vocal sound or the like, whereby the degree of safety of the user 10 is further improved.

For example, in a case in which the situation in which the user 10 is in imminent danger is a situation in which the lightning striking probability in the place where the user 10 is currently located is a predetermined value or more, the action determination unit 236 determines to cause the movement system 1000M to stay at the current position as an action of being a substitute for the user 10. In order to improve the degree of safety of the user 10, the action determination unit 236 determines to guide the user 10 to evacuate from the place where the user 10 is currently located to a safe place.

As a result, in a case in which a lightning strike occurs, the movement system 1000M staying at the current position is struck by the lightning as a substitute for the user 10 (which is struck by lightning instead of a lightning rod), thereby improving the degree of safety of the user 10. Vocal sound, an image, or the like for prompting evacuation of the user 10 is output from the communication terminal 600, and the user 10 takes an action of evacuating to a safe place according to the vocal sound, the image, or the like, whereby the degree of safety of the user 10 is further improved.

In the seventh embodiment, the aspect has been described in which the movement system 1000M is caused to stay at the current position in a case in which the lightning striking probability at the place where the user 10 is currently located is the predetermined value or more, but the disclosure is not limited thereto. For example, in a case in which a robot arm or the like is attached to the movement system 1000M, the entire height of the movement system 1000M may be increased by raising the robot arm or the like in addition to keeping the movement system 1000M at the current position. As a result, it is possible to improve the probability that the movement system 1000M will be struck by lightning as a substitute for the user 10.

### (Supplementary Note 1)

A control system including:
a state recognition unit that recognizes a user state including an action of a user and a state of an electronic apparatus;
an emotion determination unit that determines an emotion of the user or an emotion of the electronic apparatus; and
an action determination unit that determines, by using at least one of the user state, the state of the electronic apparatus, the emotion of the user, or the emotion of the electronic apparatus, and an action determination model at a predetermined timing, any of a plurality of types of device operations including non-operation as an action of the electronic apparatus, in which
the electronic apparatus is provided in a movement unit capable of autonomous traveling,
the electronic apparatus includes one or a plurality of sensors that collect information regarding a surrounding situation of the user, and
the action determination unit determines to take an action of being a substitute for the user as the action of the electronic apparatus in a case in which it is detected that the user is in imminent danger on the basis of the information regarding the surrounding situation of the user.

### (Supplementary Note 2)

The control system according to Supplementary Note 1, in which
a situation in which the user is in imminent danger is a situation in which an object is approaching the user, and
the action determination unit determines to perform an action of moving the electronic apparatus to a position between the object and the user as the action of being a substitute for the user.

### (Supplementary Note 3)

The control system according to Supplementary Note 2, in which the object is any of a vehicle, a person, and an animal.

### (Supplementary Note 4)

The control system according to Supplementary Note 2, in which the action determination unit further determines to guide the user to evacuate to a direction or a position where the user moves away from the object.

### (Supplementary Note 5)

The control system according to Supplementary Note 1, in which
the situation in which the user is in imminent danger is a situation in which a lightning striking probability in a place where the user is currently located is a predetermined value or more, and
the action determination unit determines to perform an action of causing the electronic apparatus to stay at a current position as the action of being a substitute for the user.

### (Supplementary Note 6)

The control system according to Supplementary Note 5, in which the action determination unit further determines to guide the user to evacuate from the place where the user is currently located to a safe place.

### [Eighth Embodiment]

In an eighth embodiment, in order to assist the user 10 in walking an animal, the user is led by controlling the wheels 601a of the movement unit 601 on the basis of the information regarding the surrounding situation of the user 10. Portions having the same configurations as those of the first to fourth embodiments are denoted by the same reference numerals, and description thereof will be omitted. A case in which the agent system 500 is mounted on the communication terminal 600 (see Fig. 16), and the communication terminal 600 is detachably provided on the movement unit 601 to configure the movement system 1000M will be described as an example.

The action determination unit 236 of the agent system 500 determines any of a plurality of types of agent actions as an action of the agent by using at least one of the user state, the state of the agent, the emotion of the user, or the emotion of the agent and the action determination model at a predetermined timing. In this case, the action determination unit 236 may spontaneously or periodically detect the action of the user 10 and the information regarding the surrounding situation of the user 10 on the basis of the sensor, and determine the first action content of assisting the user 10 in walking an animal as the action of the agent on the basis of the detected action of the user 10 and the detected information regarding the surrounding situation of the user 10.

The sensor may be interpreted as one or a plurality of sensors provided in the communication terminal 600 detachably provided on the movement unit 601 of the movement system 1000M. Specifically, the one or plurality of sensors may include a camera, a microphone, a gyro sensor, and the like. The one or plurality of sensors may collect the information regarding the surrounding situation of the user 10 and information for controlling the rotation of the wheels 601a of the movement unit 601.

### (First Action Content)

The first action content is to assist the user 10 in walking an animal by controlling the wheels 601a of the movement unit 601 on the basis of the information regarding the surrounding situation of the user 10. Controlling the wheels 601a may include changing at least one of a rotation direction, a rotation speed, or a steering direction of the wheels 601a. Hereinafter, an example of the first action content will be described.

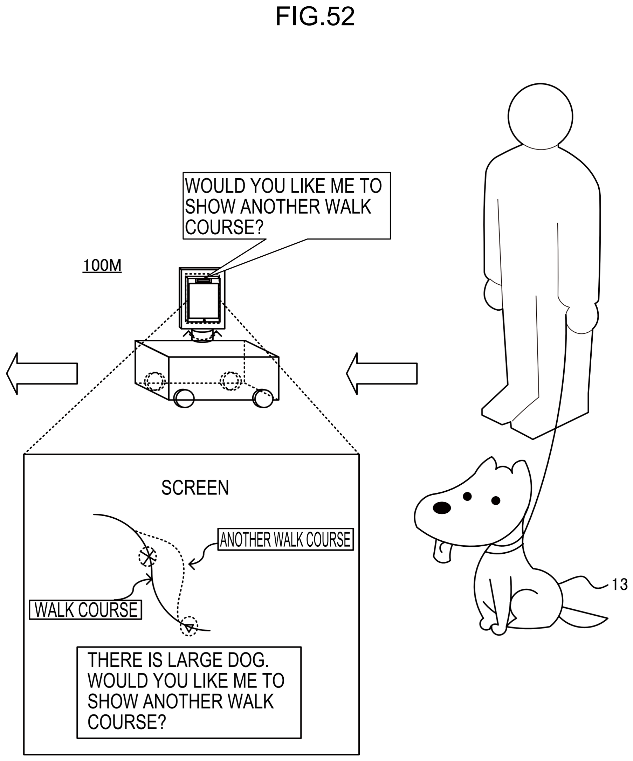

In a case in which it is detected that, in a walk course of an animal, for example, a dog 13 walked by the user 10 (see Fig. 52), there is an obstacle that hinders walking of the dog 13 around the user 10, the action determination unit 236 of the agent system 500 determines first action content of changing the walk course so that the dog 13 does not approach the obstacle. Obstacles that may hinder the dog 13 from walking include, for example, things that may excite the dog 13, such as dogs or people, things that may be harmful to the dog 13 if the dog 13 puts the things in its mouth, such as cigarettes or gum that have fallen on the road, and roads with heated asphalt.

The action determination unit 236 may execute reproduction of at least one of vocal sound or an image for assisting in walking of the user 10 as the first action content of guiding the user 10 to another walk course so that the user moves in the walk course for avoiding an obstacle that hinders walking of the dog 13. Specifically, as illustrated in Fig. 52, the action determination unit 236 may execute reproduction of at least one of vocal sound or an image for showing a walk course different from the set walk course. As illustrated in Fig. 52, the action determination unit 236 may reproduce an image of a walk course different from the set walk course on the screen of the communication terminal 600, and may reproduce vocal sound related to the walk course. The vocal sound may include "There is a large dog. Would you like me to show another walk course?" and the like.

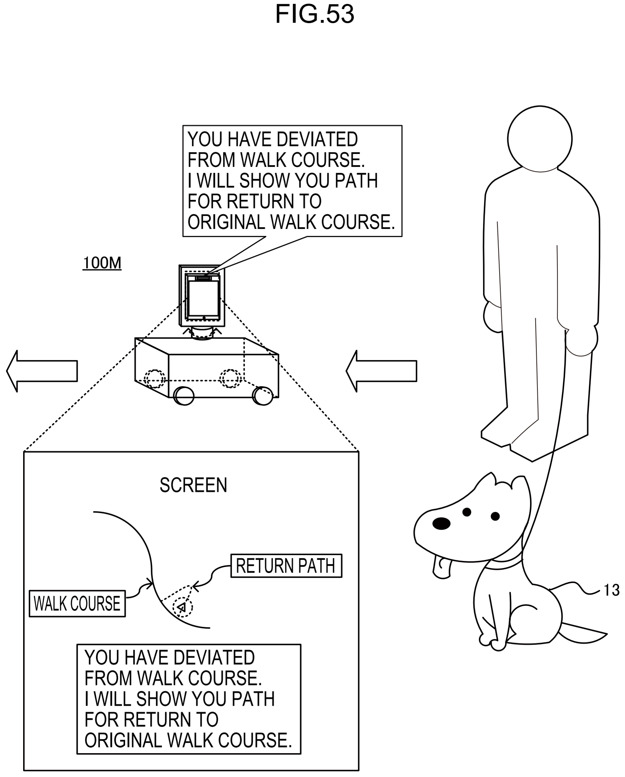

The action determination unit 236 may execute reproduction of at least one of a sound or an image for guiding the user to a path for returning the user 10 to the set walk course in a case in which the user 10 led along set walk course deviates from the walk course. Specifically, as illustrated in Fig. 53, the action determination unit 236 may reproduce an image of a route for return to the set walk course on the screen of the communication terminal 600 and may reproduce vocal sound related to the route. The vocal sound may include "You have deviated from the walk course. I will show you the path for return to the original walk course." and the like.

In a case in which it is detected that the obstacle that hinders the walking of the dog 13 around the user 10 has been eliminated, the action determination unit 236 may notify the user 10 that the obstacle that hinders the walking of the dog 13 around the user 10 has been eliminated, and determine to end the first action content.

### (Second Action Content)

The action determination unit 236 may determine whether or not the action of the user 10 has been corrected by detecting the action of the user 10 while the movement unit 601 was leading the user 10 or after reproducing at least one of vocal sound or an image that assists in walking of the user 10, and may determine second action content different from the first action content in a case in which the action of the user 10 has been corrected.

The case in which the action of the user 10 has been corrected may be interpreted as a case in which the specific action and the specific behavior have been stopped by the user 10 or a case in which the specific situation has been resolved as a result of execution of the operation of the movement system 1000M according to the first action content. Specifically, the case in which the action of the user 10 has been corrected may include a case in which the user 10 has started walking along the proposed walk course, a case in which the user 10 has started walking along a path for return to the original walk course when the path is shown due to deviation from the walk course, and the like.

The second action content may include reproduction of at least one of vocal sound praising the user 10 for the action, vocal sound expressing gratitude for the action of the user 10, an image praising the user 10 for the action, and an image expressing gratitude for the action of the user 10.

Specifically, the vocal sound praising the user for the action may include, for example, "Well done! Excellent". The vocal sound expressing gratitude for the action of the user 10 may include vocal sounds of "Thank you for coming back" and "Thank you for selecting another walk course". The image praising the user for the action 10 may include an image of a character taking a good pose as illustrated in Fig. 21. As illustrated in Fig. 22, the image expressing gratitude for the action of the user 10 may include an image of a character who expresses gratitude. In Figs. 21 and 22, the dog 13 is not present, but in the present embodiment, the dog 13 is present as in Figs. 52 and 53.

### (Third Action Content)

The action determination unit 236 may determine whether or not the action of the user 10 has been corrected by detecting the action of the user 10 while the movement unit 601 was leading the user or after the movement unit 601 reproduced at least one of vocal sound or an image that assists in walking of the user 10, and determine third action content different from the first action content in a case in which the action of the user 10 has not been corrected.

The case in which the action of the user 10 has not been corrected may be interpreted as a case in which the user 10 has continued the action and behavior that do not avoid the obstacle that hinders the walking of the dog 13 even though the operation of the movement system 1000M according to the first action content was executed, a case in which the obstacle that hinders the walking of the dog 13 is not excluded, or a case in which the user 10 has started or continued an action different from the proposal of the movement system 1000M.

Specifically, the case in which the action of the user 10 has not been corrected may include a case in which the user 10 has walked in a walk course different from the proposed another walk course without walking along the proposed another walk course, a case in which the user 10 has neglected a path for return to the original walk course and continued walking when the path was shown due to deviation from the walk course, and the like.

The third action content may include at least one of transmission of specific information to a person other than the user 10, reproduction of sound that attracts an interest of the user 10, or reproduction of an image that attracts an interest of the user 10.

The transmission of the specific information to a person other than the user may include distribution of an email containing a warning message to a relative of the user 10, a spouse of the user 10, a guardian of the user 10, or the like. The transmission of the specific information to a person other than the user may include distribution of an image (a still image or a moving image) including the user 10 and the surrounding scenery to a relative of the user 10, a spouse of the user 10, a guardian of the user 10, or the like. The transmission of the specific information to a person other than the user may include distribution of vocal sound having a warning message.

The reproduction of the sound that attracts the interest of the user 10 may include the specific music that the user 10 likes, and may also include vocal sound such as "Please come back here" or "Let's go along a road that is free of obstacles hindering the walking of the dog 13".

The reproduction of the image that attracts the interest of the user 10 may include an image of an animal (for example, the dog 13) kept by the user 10, an image of parents of the user 10, an image of a character of the animal illustrated in Fig. 23, and the like.

According to the movement system 1000M of the disclosure, it is possible to execute an action of leading the user 10 to a walk course that is free of an obstacle that hinders walking of the dog 13 through the autonomous processing. A place having an obstacle that hinders walking of the dog 13 can be detected in advance, and the user 10 can be guided to another walk course. The user 10 who gets lost and deviates from the walk course can be guided to the original walk course.

### (Supplementary Note 1)

A control system including:
a state recognition unit that recognizes a user state including an action of a user and a state of an electronic apparatus;
an emotion determination unit that determines an emotion of the user or an emotion of the electronic apparatus; and
an action determination unit that determines, by using at least one of the user state, the state of the electronic apparatus, the emotion of the user, or the emotion of the electronic apparatus, and an action determination model at a predetermined timing, any of a plurality of types of device operations including non-operation as an action of the electronic apparatus, in which
the electronic apparatus is detachably provided on a movement unit having a wheel for autonomous traveling,
the electronic apparatus includes one or a plurality of sensors that collect information regarding a surrounding situation of the user, and
the device operations include controlling the wheel on the basis of the information regarding the surrounding situation of the user, and setting first action content of assisting the user in walking an animal.

### (Supplementary Note 2)

The control system according to Supplementary Note 1, in which the action determination unit determines the first action content of changing a walk course of the animal walked by the user so that the animal does not approach an obstacle in a case in which it is detected that the obstacle that hinders walking of the animal exists in the walk course of the animal.

### (Supplementary Note 3)

The control system according to Supplementary Note 2, in which the first action content includes reproduction of at least one of vocal sound or an image for assisting the user in walking the animal.

### (Supplementary Note 4)

The control system according to Supplementary Note 3, in which the reproduction includes playback of at least one of vocal sound or an image for guiding the user along a walk course different from a set walk course.

### (Supplementary Note 5)

The control system according to Supplementary Note 4, in which the reproduction includes reproduction of at least one of vocal sound or an image for guiding the user to a path for returning the user to the set walk course in a case in which the user led along the walk course deviates from the walk course.

### [Ninth Embodiment]

### (Fourth Action Content)

In a case in which the user wearing the communication terminal 100M is a visually impaired user, the action determination unit 236 may determine the fourth action content of assisting in walking of the user by notifying the visually impaired user of the surrounding situation.

In this case, the device operations determined as the action of the communication terminal 100M by the action determination unit 236 include setting action content of assisting in walking of the visually impaired user by notifying the user of the surrounding situation by the communication terminal 100M reproducing at least one of vocal sound or an image.

For example, as illustrated in Fig. 22, the action determination unit 236 determines to output vocal sound "There is a dangerous place in the advancing direction. Please proceed carefully." as the action of the communication terminal 100M. The action determination unit 236 may more specifically determine, as the action of the communication terminal 100M, to output vocal sound that conveys the surrounding situation to the user. For example, as illustrated in Fig. 23, the action determination unit 236 determines to output vocal sound "There is a step in the advancing direction. Please proceed carefully so as not to fall." as the action of the communication terminal 100M.

In a case in which it is detected by one or a plurality of sensors that there is a dangerous obstacle in the advancing direction of the user, the action determination unit 236 may notify the user that there is a dangerous obstacle in the advancing direction and determine action content of stopping walking so that the user does not approach the dangerous obstacle.

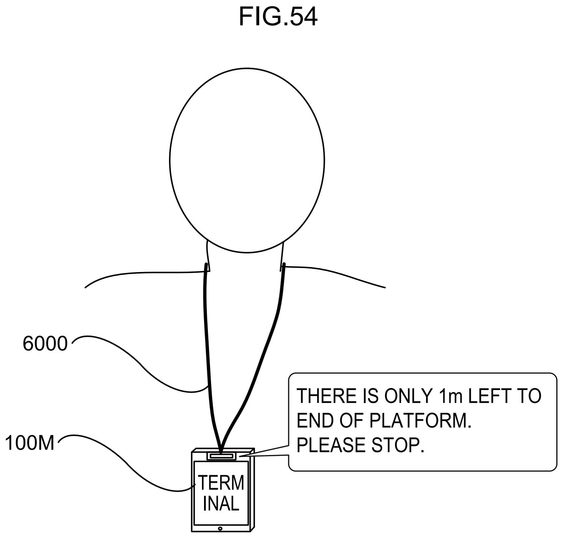

For example, as illustrated in Fig. 54, the action determination unit 236 determines to output vocal sound "There is only 1 m left to the end of the platform. Please stop." as the action of the communication terminal 100M.

In a case in which a pedestrian signal is red at a crosswalk or the like, the action determination unit 236 determines to output vocal sound such as "Please stop because the pedestrian signal is red." as the action of the communication terminal 100M. However, since the pedestrian signal changes from red to green with the lapse of time, the user cannot start walking unless the user is notified of anything.

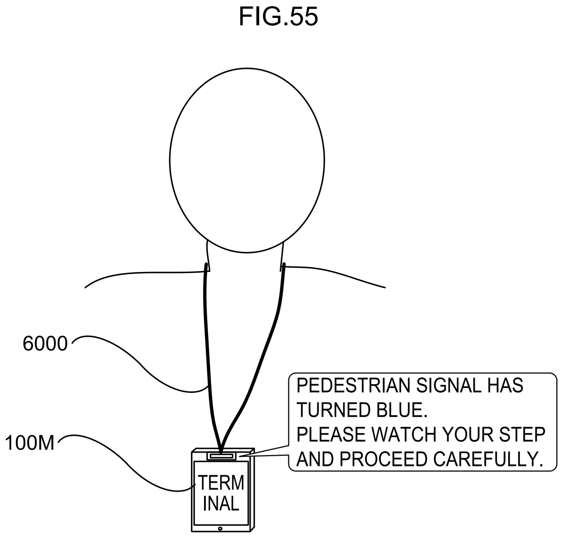

Therefore, in a case in which it is detected that the dangerous obstacle in the advancing direction of the user has been eliminated, the action determination unit 236 notifies the user that walking can be resumed and determines the action content of leading the user. For example, as illustrated in Fig. 55, the action determination unit 236 determines to output vocal sound "The pedestrian signal has turned green. Please watch your step and proceed carefully." as the action of the communication terminal 100M.

As described above, the one or more sensors detect, as dangerous obstacles, obstacles such as a parked automobile and a utility pole present in the advancing direction of the user, steps, dangerous places such as an end of a platform and a depressed place, and places where the user should not advance, such as a roadway, a railroad, and a crosswalk in a case in which a pedestrian signal is red.

The action determination unit 236 may determine action content of notifying the user of the surrounding situation detected by one or a plurality of sensors and the approach of various dangerous objects such as a bicycle, an automobile, and a pedestrian toward the user by using vocal sound.

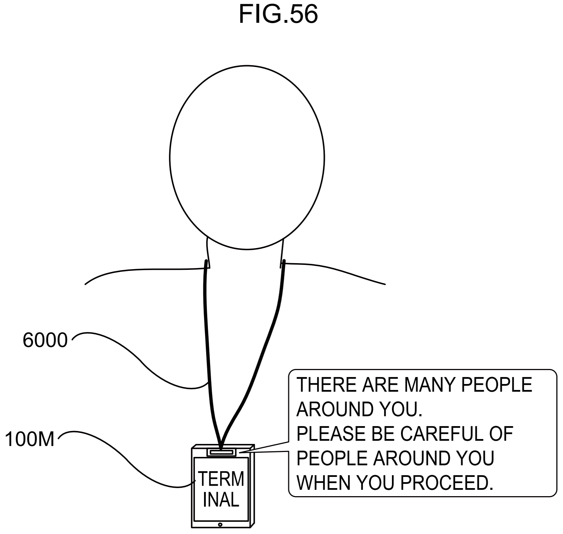

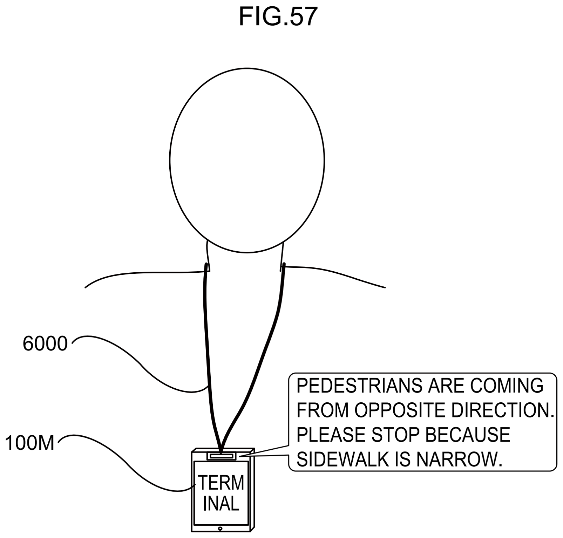

For example, as illustrated in Fig. 56, action content of notifying the user of the surrounding situation by using vocal sound such as "There are many people around you. Please be careful of the people around you when you proceed.", may be determined. As illustrated in Fig. 57, action content of instructing to stop walking by notifying the user of approaching danger by using vocal sound such as "Pedestrians are coming from the opposite direction. Please stop because the sidewalk is narrow." may be determined.

Visually impaired users cannot read text information for attracting attention of pedestrians such as traffic signs and signboards, or text information for transmitting information to pedestrians.

Therefore, in a case in which text information for attracting attention of a pedestrian or text information for transmitting information to a pedestrian is detected around the user by the camera of the communication terminal 100M, the action determination unit 236 may determine action content of reading out the text information by using vocal sound.

For example, as illustrated in Fig. 28, even in a case in which "We are closed due to the convenience of the storekeeper today. We will be open as scheduled tomorrow." is written on a sticker posted at the entrance of a certain store, visually impaired users cannot read the content of the sticker. Therefore, as illustrated in Fig. 28, the action determination unit 236 determines action content of outputting vocal sound ""We are closed due to the convenience of the storekeeper today. We will be open as scheduled tomorrow." is written on the sticker in front of the eyes.".

According to the robot 100 and the communication terminal of the disclosure, an action of leading the user can be executed through autonomous processing. As a result, the robot 100 and the communication terminal can guide the user walking in a town that the user visits for the first time to an appropriate route. It is possible to guide a user who is interested in walking to a route along which the user has not passed before. A route along which the user cannot pass is detected in advance, and the user can be guided to another route. A user who gets lost and deviates from a specific route can be guided to an original route of the user. It is possible to notify a visually impaired user of a surrounding situation.

The action determination unit 236 determines, by using at least one of the state of the user 10, the emotion of the user 10, the emotions of the robot 100 and the communication terminal, or the states of the robot 100 and the communication terminal, and the action determination model 221 at a predetermined timing, any of a plurality of types of robot actions and communication terminal actions including no action as actions of the robot 100 and the communication terminal. Here, a case in which a sentence generation model having an interaction function is used as the action determination model 221 will be described as an example.

Specifically, the action determination unit 236 inputs text representing at least one of the state of the user 10, the emotion of the user 10, the emotions of the robot 100 and the communication terminal, or the states of the robot 100 and the communication terminal, and text for inquiry about the robot action and the communication terminal action to the sentence generation model, and determines the actions of the robot 100 and the communication terminal on the basis of the output of the sentence generation model.

For example, the plurality of types of actions of the robot and the communication terminal include the following (1) to (24).
(1) The robot 100 and the communication terminal 100M do nothing.
(2) The robot 100 and the communication terminal 100M have a dream.
(3) The robot 100 and the communication terminal 100M talk to the user.
(4) The robot 100 and the communication terminal 100M create a picture diary.
(5) The robot 100 and the communication terminal 100M propose an activity.
(6) The robot 100 and the communication terminal 100M propose a person with whom the user should meet.
(7) The robot 100 and the communication terminal 100M introduce news in which the user is interested. (8) The robot 100 and the communication terminal 100M edit pictures and moving images.
(9) The robot 100 and the communication terminal 100M study with the user.
(10) The robot 100 and the communication terminal 100M evoke memories.
(11) The robot 100 and the communication terminal 100M may reproduce an image of a route along which the user can walk on the screen of the communication terminal 100M as the first action content of leading the user.
(12) The robot 100 and the communication terminal 100M may reproduce vocal sound related to a route along which the user can walk as the first action content of correcting the action of the user.
(13) The robot 100 and the communication terminal 100M may reproduce an image of a route different from the set guidance route on the screen of the communication terminal 100M as the first action content of correcting the action of the user.
(14) The robot 100 and the communication terminal 100M may reproduce vocal sound related to a route different from the set guidance route as the first action content of correcting the action of the user.
(15) The robot 100 and the communication terminal 100M may reproduce, as the first action content of correcting the action of the user, an image of a route for return to the set guidance route on the screen of the communication terminal 100M in a case in which the user led along the set guidance route deviates from the guidance route.
(16) The robot 100 and the communication terminal 100M may reproduce, as the first action content of correcting the action of the user, vocal sound related to a route for return to the set guidance route in a case in which the user led along the set guidance route deviates from the guidance route.
(17) The robot 100 and the communication terminal 100M may reproduce vocal sound praising the user for the action as the second action content different from the first action content.
(18) The robot 100 and the communication terminal 100M may reproduce vocal sound expressing gratitude for the action of the user as the second action content different from the first action content.
(19) The robot 100 and the communication terminal 100M may reproduce an image praising the user for the action as the second action content different from the first action content.
(20) The robot 100 and the communication terminal 100M may reproduce an image expressing gratitude for the action of the user as the second action content different from the first action content.
(21) The robot 100 and the communication terminal 100M may transmit specific information to a person other than the user as the third action content different from the first action content.
(22) The robot 100 and the communication terminal 100M may reproduce sound that attracts the interest of the user as the third action content different from the first action content.
(23) The robot 100 and the communication terminal 100M may reproduce an image that attracts the interest of the user as the third action content different from the first action content.
(24) The robot 100 and the communication terminal 100M may reproduce an image that notifies a visually impaired user of the surrounding situation and assists the user in walking.

The action determination unit 236 inputs, to the sentence generation model, text representing the state of the user 10 and the state of the robot 100 or the communication terminal 100M recognized by the state recognition unit 230, the current emotion value of the user 10 and the current emotion value of the robot 100 or the communication terminal 100M determined by the emotion determination unit 232, and text for inquiry about any of a plurality of types of robot actions including no action each time a certain period of time elapses, and determines an action of the robot 100 or the communication terminal 100M on the basis of an output of the sentence generation model. Here, in a case in which the user 10 is absent around the robot 100 or the communication terminal 100M, the text to be input to the sentence generation model need not include the state of the user 10 and the current emotion value of the user 10, or may include the fact that the user 10 is absent. Hereinafter, the communication terminal 100M may be read as a robot. The communication terminal 100M may be interpreted as a smartphone or a smart AI device. The robot action may be read as a communication terminal action.

In a case in which the visually impaired user is notified of the surrounding situation as the fourth action content indicated in the above "(24)", the related information collection unit 270 may store data of operation status information of public transportation to be used by the user, and the like in the collected data 223.

### (Fourth Action Content)

In a case in which the user wearing the communication terminal 100M is a visually impaired user, the action determination unit 236 of the agent system 500 may determine the fourth action content of assisting in walking of the user by notifying the visually impaired user of the surrounding situation.

In this case, the device operations determined as the action of the communication terminal 100M by the action determination unit 236 of the agent system 500 include setting action content of assisting in walking of a visually impaired user by notifying the user of a surrounding situation by the communication terminal 100M reproducing at least one of vocal sound or an image.

For example, as illustrated in Fig. 22, the action determination unit 236 of the agent system 500 determines, as the action of the communication terminal 100M, to output vocal sound "There is a dangerous place in the advancing direction. Please proceed carefully.". The action determination unit 236 may more specifically determine, as the action of the communication terminal 100M, to output vocal sound that conveys the surrounding situation to the user. For example, as illustrated in Fig. 23, the action determination unit 236 determines to output vocal sound "There is a step in the advancing direction. Please proceed carefully so as not to fall." as the action of the communication terminal 100M.

In a case in which it is detected by one or a plurality of sensors that there is a dangerous obstacle in the advancing direction of the user, the action determination unit 236 of the agent system 500 may notify the user that there is a dangerous obstacle in the advancing direction and determine the action content of stopping walking so that the user does not approach the dangerous obstacle.

For example, as illustrated in Fig. 54, the action determination unit 236 of the agent system 500 determines to output vocal sound "There is only 1 m left to the end of the platform. Please stop." as the action of the communication terminal 100M.

In a case in which a pedestrian signal is red at a crosswalk or the like, the action determination unit 236 of the agent system 500 determines to output vocal sound such as "Please stop because the pedestrian signal is red." as the action of the communication terminal 100M. However, since the pedestrian signal changes from red to green with the lapse of time, the user cannot start walking unless the user is notified of anything.

Therefore, in a case in which it is detected that the dangerous obstacle in the advancing direction of the user has been eliminated, the action determination unit 236 of the agent system 500 determines the action content of notifying the user that walking can be resumed and leading the user. For example, as illustrated in Fig. 55, the action determination unit 236 determines to output vocal sound "The pedestrian signal has turned green. Please watch your step and proceed carefully." as the action of the communication terminal 100M.

As described above, the one or more sensors detect, as dangerous obstacles present in the advancing direction of the user, obstacles such as a parked automobile and a utility pole, steps, dangerous spots such as an end of a platform and a depressed spot, and places to which the user should not advance, such as a roadway, a railroad, and a crosswalk in a case in which a pedestrian signal is red.

The action determination unit 236 of the agent system 500 may determine action content of notifying the user of the surrounding situation detected by one or a plurality of sensors and the approach of various dangerous objects such as a bicycle, an automobile, and a pedestrian toward the user by using vocal sound.

For example, as illustrated in Fig. 56, action content of notifying the user of the surrounding situation by using vocal sound such as "There are many people around you. Please be careful of the people around you when you proceed.", may be determined. As illustrated in Fig. 57, action content of instructing to stop walking by notifying the user of approaching danger by using vocal sound such as "Pedestrians are coming from the opposite direction. Please stop because the sidewalk is narrow." may be determined.

Visually impaired users cannot read text information for attracting attention of pedestrians such as traffic signs and signboards, or text information for transmitting information to pedestrians.

Therefore, in a case in which text information for attracting attention of a pedestrian or text information for transmitting information to a pedestrian is detected around the user by the camera of the communication terminal 100M, the action determination unit 236 of the agent system 500 may determine action content of reading out the text information by using vocal sound.

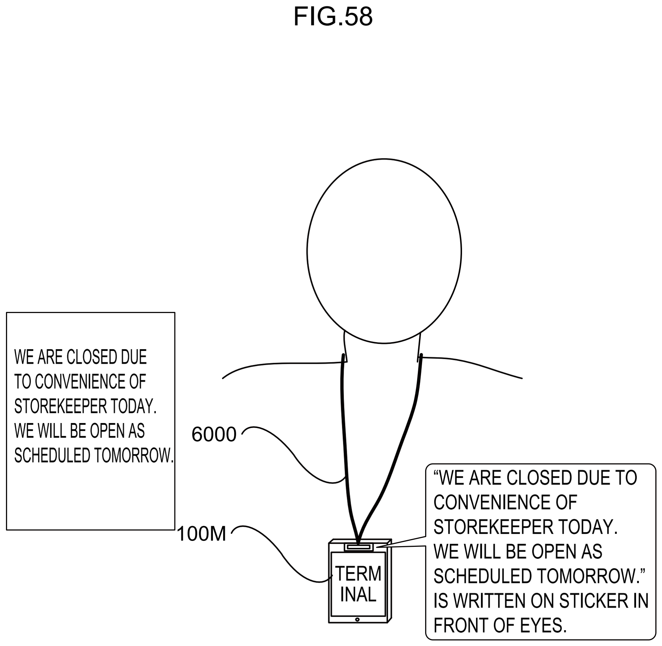

For example, as illustrated in Fig. 58, even in a case in which "We are closed due to the convenience of the storekeeper today. We will be open as scheduled tomorrow." is written on a sticker posted at the entrance of a certain store, visually impaired users cannot read the content of the sticker. Therefore, as illustrated in Fig. 58, the action determination unit 236 of the agent system 500 determines action content of outputting vocal sound ""We are closed due to the convenience of the storekeeper today. We will be open as scheduled tomorrow." is written on the sticker in front of the eyes.".

According to the agent system 500 of the disclosure, an action of leading the user can be executed through autonomous processing. As a result, the agent can guide the user walking in a town that the user visits for the first time to an appropriate route. It is possible to guide a user who is interested in walking to a route along which the user has not passed before. A route along which the user cannot pass is detected in advance, and the user can be guided to another route. A user who gets lost and deviates from a specific route can be guided to an original route of the user. It is possible to notify a visually impaired user of a surrounding situation.

The action determination unit 236 of the agent system 500 can perform, as the agent action, the fourth action content in the above "(24)", that is, execute reproduction of an image that notifies the visually impaired user of the surrounding situation and assists the user in walking.

In a case in which the visually impaired user is notified of the surrounding situation as the fourth action content indicated in the above "(24)", the related information collection unit 270 of the agent system 500 may store data of operation status information of public transportation to be used by the user, and the like in the collected data 223.

### (Supplementary Note 1)

A control system including:
a state recognition unit that recognizes a user state including an action of a user and a state of an electronic apparatus;
an emotion determination unit that determines an emotion of the user or an emotion of the electronic apparatus; and
an action determination unit that determines, by using at least one of the user state, the state of the electronic apparatus, the emotion of the user, or the emotion of the electronic apparatus, and an action determination model at a predetermined timing, any of a plurality of types of device operations including non-operation as an action of the electronic apparatus, in which
the electronic apparatus detachably provided on a neck strap includes one or a plurality of sensors that collect information regarding a surrounding situation, and
the device operations include setting action content of assisting in walking of the user by notifying the user of the surrounding situation by the electronic apparatus reproducing at least one of vocal sound or an image.

### (Supplementary Note 2)

The control system according to Supplementary Note 1, in which in a case in which it is detected that there is a dangerous obstacle in an advancing direction of the user, the action determination unit determines action content of notifying the user that there is the dangerous obstacle in the advancing direction, and stopping walking so that the user does not approach the dangerous obstacle.

### (Supplementary Note 3)

The control system according to Supplementary Note 2, in which the action determination unit determines action content of notifying the user that walking is capable of being resumed and leading the user in a case in which it is detected that the dangerous obstacle in the advancing direction of the user has been eliminated.

### (Supplementary Note 4)

The control system according to Supplementary Note 2, in which the sensor detects an obstacle, a dangerous spot, and a place to which the user is prohibited from advancing as dangerous obstacles present in the advancing direction of the user.

### (Supplementary Note 5)

The control system according to Supplementary Note 1, in which in a case in which text information for attracting attention of a pedestrian or text information for transmitting information to a pedestrian is detected around the user, the action determination unit determines action content of reading out the text information by using vocal sound.

### [Tenth Embodiment]

Next, processing of the action determination unit 236 in a case in which the robot 100 and the communication terminal perform autonomous processing of autonomously acting will be described.

In the autonomous processing in the present embodiment, the robot 100 and the communication terminal may detect a state or an action of the user spontaneously or periodically by monitoring the user. The term "spontaneous" may be interpreted as the robot 100 and the communication terminal spontaneously acquiring the state or action of the user by themselves without a trigger from the outside. The trigger from the outside may include a question from the user to the robot 100 and the communication terminal, an active action from the user to the robot 100 and the movement system, and the like. The term "periodic" may be interpreted as a specific cycle such as a unit of one second, a unit of one minute, a unit of one hour, a unit of several hours, a unit of several days, a unit of week, or a unit of day of the week.

The state of the user may include an action tendency of the user. The action tendency may include that the user walks for a long time, that the user runs for a long time, and that the user performs a dangerous behavior. The dangerous behavior may include that the user walks on a roadway, and that the user enters the roadway from a sidewalk. The action tendency may be interpreted as a tendency of the user's hyperactive or impulsive action.

In the autonomous processing, the robot 100 and the communication terminal may ask a generative AI a question about the detected state or action of the user, and store an answer of the generative AI to the question and the detected action of the user in association with each other. In this case, the robot 100 and the communication terminal may store action content of correcting the action in association with the answer.

Information in which the answer of the generative AI to the question, the detected action of the user, and the action content of correcting the action are associated with each other may be recorded as table information in a storage medium such as a memory. The table information may be interpreted as specific information recorded in the storage unit.

Specifically, in a case in which the user tends to walk for a long time, the robot 100 and the communication terminal may ask the generative AI a question "What kind of guidance should be given to the user who takes such an action?", and in a case in which the answer of the generative AI to the question is "Propose the same route as the route along which the user has previously passed", the robot 100 and the communication terminal may record the answer, the action of the user who can walk for a long time, and the action content of correcting the action (for example, guidance content of "guiding the user to the route along which the user has previously passed") in association with the table information. In a case in which the action of the user who is walking for a long time is detected, the action determination unit 236 of the robot 100 and the communication terminal may reproduce vocal sound "I will guide you to the route along which you have previously passed" as the action of the robot 100 and the communication terminal by using the table information.

As another example, in a case in which the user tends to run for a long time, the robot 100 and the communication terminal ask the generative AI a question "What kind of guidance should be given to the user who takes such an action?", and in a case in which the answer of the generative AI to the question is "It is desirable to periodically perform guidance for encouraging hydration", the robot 100 and the communication terminal may record the answer, the action of the user who can run for a long time, and the action content of correcting the action (for example, guidance content such as "Please rehydrate frequently") in association with the table information. In a case in which the action of the user who is running for a long time is detected, the action determination unit 236 of the robot 100 and the communication terminal may reproduce vocal sound "Please rehydrate frequently" as the action of the robot 100 and the communication terminal by using the table information.

As still another example, in a case in which the user tends to walk on the roadway, the robot 100 and the communication terminal may ask the generative AI a question "What kind of guidance should be given to the user who takes such an action?", and in a case in which the answer of the generative AI to the question is "I recommend to propose returning to the sidewalk because there is a possibility of coming into contact with vehicles", the robot 100 and the communication terminal may record the answer, the action of the user who may walk on the roadway, and the action content of correcting the action (for example, guidance content of "Please return to the sidewalk") in association with the table information. In a case in which the action of the user walking on the sidewalk is detected, the action determination unit 236 of the robot 100 and the communication terminal may reproduce vocal sound "Please return to the sidewalk" as the action of the robot 100 and the communication terminal by using the table information.

In the autonomous processing, an action schedule of the robot 100 and the communication terminal for attracting attention with respect to a state or an action of the user may be set on the basis of the detected action of the user and the stored specific information.

As described above, the robot 100 and the communication terminal may record table information in which the answer of the generative AI corresponding to the state or the action of the user is associated with the detected state or action of the user in the storage medium. Hereinafter, an example of content stored in the table will be described.

### (1. Case in Which User Tends to Walk for a Long Time)

In a case in which there is the tendency, the robot 100 and the communication terminal themselves ask the generative AI a question "What kind of guidance should be given to the user who takes such an action?". In a case in which the answer of the generative AI to this question is, for example, "Propose a route different from the route along which the user has previously passed", "Propose a route to a place where the user can take shelter from rain because it is likely to rain", or "Propose a route with a relatively small number of vehicles such as bicycles", the robot 100 and the communication terminal may store the action of the user and the answer of the generative AI in association with each other.

### (2. Case in Which User Tends to Run for a Long Time)

In a case in which there is the tendency, the robot 100 and the communication terminal themselves ask the generative AI a question "What kind of guidance should be given to the user who takes such an action?". In a case in which the answer of the generative AI to this question is, for example, "Provide guidance for encouraging hydration periodically", "Propose a route with fewer bicycles and clean air", or "Propose a route with fewer traffic lights", the robot 100 and the communication terminal may store the action of the user and the answer of the generative AI in association with each other.

### (3. Case in Which User Tends to Walk on Roadway)

In a case in which there is the tendency, the robot 100 and the communication terminal themselves ask the generative AI a question "What kind of situation can occur to the user who takes such an action?". In a case in which the answer of the generative AI to this question is, for example, "There is a possibility of coming into contact with vehicles such as bicycles", "There is a possibility of entering an expressway interchange", or "Advice should be given to encourage the user to walk on the sidewalk as much as possible" the robot 100 and the communication terminal may store the user's action of walking on the roadway and the answer of the generative AI in association with each other. The robot 100 and the communication terminal may store action content of correcting the action in association with the answer.

The action content of correcting the action may include at least one of reproduction of vocal sound for correcting the dangerous action of the user or reproduction of an image for correcting the dangerous action of the user.

The vocal sound for correcting the dangerous action of the user may include vocal sound for guiding the user to a specific place. The specific place may include a place other than the place where the user is currently located, for example, a sidewalk. Specifically, the vocal sound may include vocal sound such as "Please stop walking on the roadway", "That is not a sidewalk", "The roadway is dangerous", and "Please move to the sidewalk immediately".

As described above, in the autonomous processing, a table in which the answer of the generative AI corresponding to the state or action of the user, the content of the state or action, and the action content of correcting the state or action are associated with each other may be recorded in a storage medium such as a memory.

In the autonomous processing, after the table is recorded, the action of the user is autonomously or periodically detected, and the action schedule of the communication terminal for attracting the attention of the user may be set on the basis of the detected action of the user and the content of the stored table. Specifically, the action determination unit 236 of the robot 100 and the communication terminal may set the first action content on the basis of the detected action of the user and the content of the stored table.

Specifically, the action determination unit 236 may spontaneously or periodically detect an action of the user on the basis of the sensor, and determine the first action content in the case of determining to lead the user as an action of the electronic apparatus on the basis of the detected action of the user and the specific information stored in advance.

The sensor may be interpreted as a sensor of one or a plurality of sensors provided in a communication terminal. Specifically, the one or plurality of sensors may include a camera, a microphone, a gyro sensor, and the like.

Here, the communication terminal will be described in detail with reference to Fig. 24.

Fig. 24 is an external view of the communication terminal according to the embodiment of the disclosure. As illustrated in Fig. 24, the communication terminal 100M may be interpreted as an electronic apparatus detachably provided on a neck strap 6000. The communication terminal 100M may be interpreted as a smartphone that will be described later, or may be interpreted as a smart AI device. The communication terminal 100M may have the functions of the robots 100 to 102 described above. The neck strap 6000 may include a hook-shaped member that is locked to the communication terminal 100M.

### (First Action Content)

The first action content may include an action of the communication terminal 100M that leads the user when the communication terminal 100M reproduces at least one of vocal sound or an image. Hereinafter, an example of the first action content will be described.

The action determination unit 236 may execute reproduction of at least one of vocal sound or an image for assisting in walking of the user as the first action content for performing route guidance so that the walking user moves along a specific route. Specifically, in a case in which it is detected that the communication terminal 100M is attached to the neck strap 6000, the action determination unit 236 may spontaneously search for a place where the user tends to go regularly and guide the user along a route to the place that has been searched for. In a case in which it is detected that the camera included in the sensor of the communication terminal 100M is directed in an advancing direction of the user, the action determination unit 236 may spontaneously search for a place where the user tends to go regularly and guide the user along a route to the place that has been searched for. The action determination unit 236 may reproduce vocal sound related to guidance of a route along which the user can walk. The vocal sound may include "I searched for a place where Mr. A frequently goes at this time. I will start guiding.", "Turn right 10 m ahead" illustrated in Fig. 25, and the like. The action determination unit 236 may reproduce an image related to guidance of a route along which the user can walk on the screen of the communication terminal 100M.

The action determination unit 236 may execute reproduction of at least one of vocal sound and an image for guiding a route different from the set guidance route. Specifically, the action determination unit 236 may spontaneously re-search for a place where the user tends to go regularly, including the traffic condition of the set guidance route, the climate near the route, the current time, and the like, and reproduce vocal sound or the like related to a route to the place that has been re-searched for, that is, a route different from the set guidance route. More specifically, the vocal sound may include "I searched for another route because of the heavy traffic today. Would you like me to show another route?", "The road is closed ahead. Would you like me to show another route?" as illustrated in Fig. 26, and the like.

In a case in which the user led along the set guidance route deviates from the guidance route, the action determination unit 236 may spontaneously search for a path for returning the user to the guidance route and execute reproduction of at least one of vocal sound or an image for guiding the user to the path that has been searched for. Specifically, the action determination unit 236 may spontaneously re-search for a place where the user can return, including the traffic condition of a path deviating from the guidance route, the climate near the path, the current time, events that have occurred in the past near the route, and the like, and reproduce vocal sound related to the route to the re-searched place. That is, the vocal sound related to the path for return to the set guidance route may be reproduced. More specifically, the vocal sound may include "Since there are few people and it is dangerous, I searched for a path for return to the original route.", "It started to rain. It seems to be an area where a mudslide occurred before. Do you want to return to the original road?", "You have deviated from the route. I will show you the path for return to the original route." as illustrated in Fig. 27, and the like. The action determination unit 236 of the robot 100 and the communication terminal may execute reproduction of vocal sound such as the above vocal sound, for example, "I will guide you to the route along which you have previously passed", "Please rehydrate frequently", and "Please return to the sidewalk" or an image.

### (Second Action Content)

The action determination unit 236 may determine whether or not the action of the user has been corrected by detecting the action of the user while the communication terminal 100M was leading the user or after the communication terminal 100M reproduced at least one of vocal sound or an image that assists in the walking of the user, and may determine second action content different from the first action content in a case in which the action of the user has been corrected.

The case in which the action of the user has been corrected may be interpreted as a case in which the user has stopped a specific action and a specific behavior or a case in which a specific situation has been resolved as a result of execution of the operation of the communication terminal 100M according to the first action content. Specifically, the case in which the action of the user has been corrected may include a case in which the user has started walking along the proposed different route, a case in which the user has started walking along a path for return to the original route when the path was shown due to deviation from the original route, and the like.

The second action content may include reproduction of at least one of vocal sound praising the user for action, vocal sound expressing gratitude for the action of the user, an image praising the user for the action, or an image expressing gratitude for the action of the user.

Specifically, the vocal sound praising the user for the action may include, for example, "Well done! Excellent" as illustrated in Fig. 28. The vocal sound expressing gratitude for the action of the user may include vocal sound such as "Thank you for coming back" or "Thank you for selecting another route" as illustrated in Fig. 29. The image praising the user for the action may include an image of a character taking a good pose. The image expressing gratitude for the action of the user may include an image of a character who expresses gratitude.

### (Third Action Content)

The action determination unit 236 may determine whether or not the action of the user has been corrected by detecting the action of the user while the communication terminal 100M was leading the user or after the communication terminal 100M reproduced at least one of vocal sound or an image that assists in the walking of the user, and determine third action content different from the first action content in a case in which the action of the user has not been corrected.

The case in which the action of the user has not been corrected may be interpreted as a case in which the user has continued the dangerous action and behavior even though the operation of the communication terminal 100M according to the first action content was executed, a case in which the dangerous situation has not been resolved, or a case in which the user has started or continued an action different from the proposal of the communication terminal 100M.

Specifically, the case in which the action of the user has not been corrected may include a case in which the user has walked along a route different from the proposed different route without walking along the proposed different route, a case in which the user 10 has neglected a path for return to the original route and has continued walking when the path was shown due to deviation from the route, and the like.

The third action content may include at least one of transmission of specific information to a person other than the user, reproduction of sound that attracts an interest of the user, or reproduction of an image that attracts an interest of the user.

The transmission of the specific information to a person other than the user may include distribution of an email containing a warning message to a relative of the user, a spouse of the user, a guardian of the user, or the like. The transmission of the specific information to a person other than the user may include distribution of an image (a still image or a moving image) including the user and the surrounding scenery to a relative of the user, a spouse of the user, a guardian of the user, or the like. The transmission of the specific information to a person other than the user may include distribution of vocal sound having a warning message.

The reproduction of the sound that attracts the interest of the user may include specific music that the user likes, and may also include vocal sound such as "Please come back here" or "Let's take a safe road".

The reproduction of the image that attracts the interest of the user may include reproduction of an image of an animal kept by the user, an image of parents of the user, an image of a character of the animal, and the like.

### (Fourth Action Content)

The action determination unit 236 may determine the fourth action content of calling attention regarding the state of the body to the user on the basis of biological information collected from the body of the user.

In this case, the device operations determined as the action of the communication terminal 100M by the action determination unit 236 include notifying the user of call attention information based on the biological information collected from the body of the user by the communication terminal 100M reproducing at least one of vocal sound or an image.

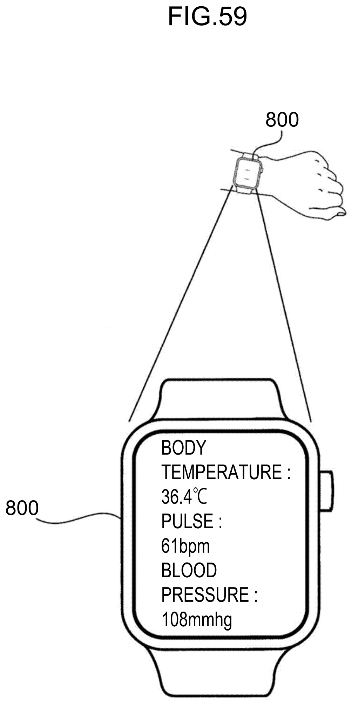

A smartwatch 800 worn on the user's arm as illustrated in Fig. 59, for example, is used as a sensor for acquiring biological information from the body of the user. The smartwatch 800 illustrated in Fig. 59 is configured to collect at least one or a plurality of pieces of information of a body temperature, a pulse rate, a blood pressure, and a blood oxygen saturation (SPO2) of the worn user as biological information. The smartwatch 800 and the communication terminal 100M are connected via a wireless communication line such as Bluetooth (registered trademark). Therefore, the communication terminal 100M can receive the biological information of the user collected by the smartwatch 800.

In a case in which a physical abnormality of the user is detected on the basis of the collected biological information, the action determination unit 236 determines action content of notifying the user that the physical abnormality has occurred and giving advice about alleviating the physical abnormality of the user.

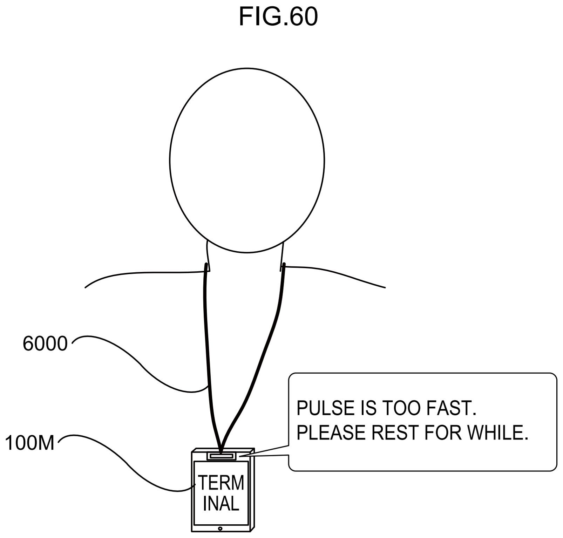

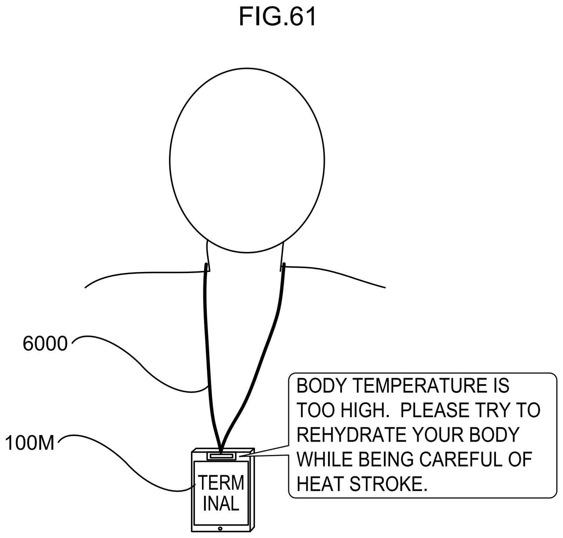

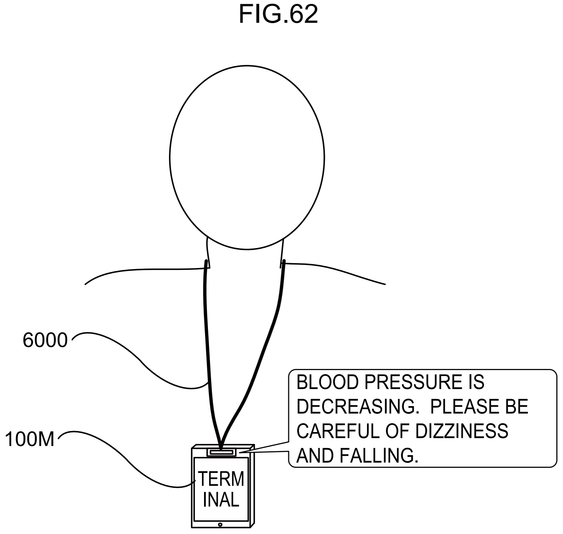

For example, as illustrated in Fig. 60, the action determination unit 236 determines to output vocal sound "Pulse is too fast. Please rest for a while." as the action of the communication terminal 100M. For example, as illustrated in Fig. 61, the action determination unit 236 determines to output vocal sound "Body temperature is too high. Please try to rehydrate your body while being careful of heat stroke." as the action of the communication terminal 100M. For example, as illustrated in Fig. 62, the action determination unit 236 determines to output vocal sound "Blood pressure is decreasing. Please be careful of dizziness and falling." as the action of the communication terminal 100M.

Here, the action determination unit 236 determines the attention calling content for the user depending on a range in which the acquired biological information is included on the basis of information such as a standard range, an attention range, and a danger range of the biological information such as the blood pressure, the pulse rate, and the body temperature stored in advance.

In a case in which it is determined that the biological information acquired from the body of the user has reached the dangerous range, the action determination unit 236 may determine, as the action content of the communication terminal 100M, action content of issuing a warning sound such as a buzzer sound in addition to calling attention by using vocal sound. Since the warning sound is emitted from the communication terminal 100M as described above, it is possible to notify not only the user wearing the communication terminal 100M but also other users around the user of the abnormality of the body.

The sensor of the smartwatch 800 may collect information of the R-R interval in the pulse variation of the user as biological information and transmit the collected information to the communication terminal 100M.

Here, the R-R interval is an interval between the R wave and the next R wave in the heart rate variation or the pulse variation. The R-R interval data is plotted at the time position of the rear R wave and interpolated, and spectrum analysis (frequency conversion) is performed on data generated by resampling at equal intervals to obtain spectrum waveform data, so that it is possible to ascertain information of the user's autonomic nerve.

The action determination unit 236 determines action content of calculating an index indicating the state of the autonomic nerve of the user on the basis of the collected information regarding the R-R interval in the pulse variation of the user, and notifying the user of the current state of the autonomic nerve.

Here, as an index indicating the state of the autonomic nerve, an LF/HF value or the like calculated on the basis of a low frequency (LF) value and a high frequency (HF) value in the spectrum waveform data obtained as described above is used. Here, for example, the LF value is an integral value of the power spectral component of 0.04 to 0.15 Hz, and the HF value is an integral value of the power spectral component of 0.15 to 0.40 Hz.

The LF/HF value is used as an index indicating the degree of activity of the sympathetic nerve among the autonomic nerves. The HF value is used as an index indicating the degree of activity of the parasympathetic nerve among the autonomic nerves.

The autonomic nerves include a sympathetic nerve that mainly functions in an active state and a parasympathetic nerve that mainly functions in a resting state. That is, in a case in which the sympathetic nerve dominantly works, the living body can be considered to be in an active state, and in a case in which the parasympathetic nerve dominantly works, the living body can be considered to be in a resting state. Therefore, in general, in a case in which the living body is in a resting state, the blood pressure value and the pulse rate decrease, and the state of the autonomic nerve becomes the parasympathetic nerve dominant state, and in a case in which the living body is in a tension/active state, the blood pressure value and the pulse rate increase, and the state of the autonomic nerve becomes the sympathetic nerve dominant state.

In a case in which the body of the user is healthy, the degree of activity of the sympathetic nerve and the degree of activity of the parasympathetic nerve are switched within an appropriate range, and the body of the user is maintained in a normal state.

Therefore, in a case in which the state of the autonomic nerve based on the pulse variation collected from the body of the user is different from the normal state, the action determination unit 236 determines action content of notifying the user of the current state of the autonomic nerve to call attention.

According to the robot 100 and the communication terminal of the disclosure, an action of leading the user can be executed through autonomous processing. As a result, the robot 100 and the communication terminal can guide the user walking in a town that the user visits for the first time to an appropriate route. The user who is interested in walking may be guided to a route along which the user has not passed before. A route where the user cannot pass is detected in advance, and the user can be guided to another route. The user who gets lost and deviates from the specific route can be guided to the original route. It is possible to call attention regarding the state of the body to the user.

The action determination unit 236 determines, by using at least one of the state of the user 10, the emotion of the user 10, the emotion of the robot 100 and the communication terminal, or the state of the robot 100 and the communication terminal, and the action determination model 221 at a predetermined timing, any of a plurality of types of robot actions and communication terminal actions including no action as the action of the robot 100 and the communication terminal. Here, a case in which a sentence generation model having an interaction function is used as the action determination model 221 will be described as an example.

Specifically, the action determination unit 236 inputs text representing at least one of the state of the user 10, the emotion of the user 10, the emotion of the robot 100 and the communication terminal, or the state of the robot 100 and the communication terminal, and text for inquiry about robot actions and communication terminal actions to the sentence generation model, and determines the actions of the robot 100 and the communication terminal on the basis of an output of the sentence generation model.

For example, the plurality of types of actions of robot and the communication terminal include the following (1) to (24).
(1) The robot 100 and the communication terminal 100M do nothing.
(2) The robot 100 and the communication terminal 100M have a dream.
(3) The robot 100 and the communication terminal 100M talk to the user.
(4) The robot 100 and the communication terminal 100M create a picture diary.
(5) The robot 100 and the communication terminal 100M propose an activity.
(6) The robot 100 and the communication terminal 100M propose a person with whom the user should meet.
(7) The robot 100 and the communication terminal 100M introduce news in which the user is interested.
(8) The robot 100 and the communication terminal 100M edit pictures and moving images.
(9) The robot 100 and the communication terminal 100M study with the user.
(10) The robot 100 and the communication terminal 100M evoke memories.
(11) The robot 100 and the communication terminal 100M may reproduce an image of a route along which the user can walk on the screen of the communication terminal 100M as the first action content of leading the user.
(12) The robot 100 and the communication terminal 100M may reproduce vocal sound related to a route along which the user can walk as the first action content of correcting the action of the user.
(13) The robot 100 and the communication terminal 100M may reproduce an image of a route different from the set guidance route on the screen of the communication terminal 100M as the first action content of correcting the action of the user.
(14) The robot 100 and the communication terminal 100M may reproduce vocal sound related to a route different from the set guidance route as the first action content of correcting the action of the user.
(15) The robot 100 and the communication terminal 100M may reproduce, as the first action content of correcting the action of the user, an image of a route for return to the set guidance route on the screen of the communication terminal 100M in a case in which the user led along the set guidance route deviates from the guidance route.
(16) The robot 100 and the communication terminal 100M may reproduce, as the first action content of correcting the action of the user, vocal sound related to a route for return to the set guidance route in a case in which the user led along the set guidance route deviates from the guidance route.
(17) The robot 100 and the communication terminal 100M may reproduce vocal sound praising the user for the action as the second action content different from the first action content.
(18) The robot 100 and the communication terminal 100M may reproduce vocal sound expressing gratitude for the action of the user as the second action content different from the first action content.
(19) The robot 100 and the communication terminal 100M may reproduce an image praising the user for the action as the second action content different from the first action content.
(20) The robot 100 and the communication terminal 100M may reproduce an image expressing gratitude for the action of the user as the second action content different from the first action content.
(21) The robot 100 and the communication terminal 100M may transmit specific information to a person other than the user as the third action content different from the first action content.
(22) The robot 100 and the communication terminal 100M may reproduce sound that attracts the interest of the user as the third action content different from the first action content.
(23) The robot 100 and the communication terminal 100M may reproduce an image that attracts the interest of the user as the third action content different from the first action content.
(24) The robot 100 and the communication terminal 100M may reproduce an image that calls attention regarding the physical condition to the user as the fourth action content.

The action determination unit 236 inputs, to the sentence generation model, text representing the state of the user 10 and the state of the robot 100 or the communication terminal 100M recognized by the state recognition unit 230, and the current emotion value of the user 10, the current emotion value of the robot 100, and the current emotion value of the communication terminal 100M determined by the emotion determination unit 232, and text for inquiry about any of a plurality of types of robot actions including no action, each time a certain period of time elapses, and determines an action of the robot 100 or the communication terminal 100M on the basis of an output of the sentence generation model. Here, in a case in which the user 10 is absent around the robot 100 or the communication terminal 100M, the text to be input to the sentence generation model need not include the state of the user 10 and the current emotion value of the user 10, or may include the fact that the user 10 is absent. Hereinafter, the communication terminal 100M may be read as a robot. The communication terminal 100M may be interpreted as a smartphone or a smart AI device. The robot action may be read as a communication terminal action.

The action determination unit 236 may spontaneously or periodically detect the action of the user on the basis of the sensor, and in a case in which it is determined to lead the user as the action of the electronic apparatus that is the communication terminal 100M on the basis of the detected action of the user and the specific information stored in advance, execute the following first action content.

The action determination unit 236 may determine the first action content in a case in which the camera included in the sensor is directed in an advancing direction of the user. As a result, the communication terminal 100M can start leading the user only in a case in which the user desires to execute route guidance.

The action determination unit 236 may perform, as the communication terminal action, the first action content in the above "(11)", that is, reproduce an image of a route along which the user can walk on the screen of the communication terminal 100M.

The action determination unit 236 may perform, as the communication terminal action, the first action content in the above "(12)", that is, reproduce vocal sound related to a route along which the user can walk.

The action determination unit 236 may perform, as the communication terminal action, the first action content in the above "(13)", that is, reproduce an image of a route different from the set guidance route as the first action content of correcting the action of the user on the screen of the communication terminal 100M.

The action determination unit 236 may perform, as the communication terminal action, the first action content in the above "(14)", that is, reproduce vocal sound related to a route different from the set guidance route.

The action determination unit 236 may perform, as the communication terminal action, the first action content in the above "(15)", that is, reproduce an image of a route for return to the set guidance route on the screen of the communication terminal 600 in a case in which the user led along the set guidance route deviates from the guidance route.

The action determination unit 236 may perform, as the communication terminal action, the first action content in the above "(16)", that is, reproduce vocal sound related to a route for return to the set guidance route in a case in which the user led along the set guidance route deviates from the guidance route.

The action determination unit 236 may execute the second action content different from the first action content. Specifically, the action determination unit 236 may perform, as the movement system action, the second action content in the above "(17)", that is, reproduce vocal sound praising the user for the action.

The action determination unit 236 may perform, as the movement system action, the second action content in the above "(18)", that is, reproduce vocal sound expressing gratitude for the action of the user.

The action determination unit 236 may perform, as the communication terminal action, the second action content in the above "(19)", that is, reproduce an image praising the user for the action.

The action determination unit 236 may perform, as the communication terminal action, the second action content in the above "(20)", that is, reproduce an image expressing gratitude for the action of the user.

The action determination unit 236 may execute the third action content different from the first action content. Specifically, the action determination unit 236 may perform, as the communication terminal action, the third action content in the above "(21)", that is, transmit the specific information to a person other than the user.

The action determination unit 236 may perform, as the movement system action, the third action content in the above "(22)", that is, execute reproduction of sound that attracts the interest of the user.

The action determination unit 236 may perform, as the movement system action, the third action content in the above "(23)", that is, execute reproduction of an image that attracts the interest of the user.

The action determination unit 236 may perform, as the communication terminal action, the fourth action content in the above "(24)" that is, execute reproduction of an image that calls attention regarding the physical condition to the user.

In a case in which attention regarding the physical condition is called to the user as the fourth action content indicated in the above "(24)", the related information collection unit 270 may store data regarding the standard range, the attention range, the danger range, and the like of the biological information in the collected data 223.

As described above, the agent may record table information in which the answer of the generative AI corresponding to the state or the action of the user is associated with the detected state or action of the user in the storage medium. Hereinafter, an example of content stored in the table will be described.

### (1. Case in Which User Tends to Walk For a Long Time)

In a case in which there is the tendency, the agent itself asks the generative AI a question "What kind of guidance should be given to the user who takes such an action?". In a case in which the answer of the generative AI to this question is, for example, "Propose the same route as the route along which the user has previously passed", "Propose a route along which the user has not passed", "Propose a route with a relatively small number of vehicles such as bicycles", or "Propose a route with undulations", the agent may store the action of the user and the answer of the generative AI in association with each other.

### (2. Case in Which User Tends to Run for a Long Time)

In a case in which there is the tendency, the agent itself asks the generative AI a question "What kind of guidance should be given to the user who takes such an action?". In a case in which the answer of the generative AI to this question is, for example, "It is desirable to provide guidance for encouraging hydration periodically", "Propose a route with fewer bicycles and clean air", or "Propose a route with fewer traffic lights", the agent may store the action of the user and the answer of the generative AI in association with each other.

### (3. Case in Which User Tends to Walk on Roadway)

In a case in which there is the tendency, the agent itself asks the generative AI a question "What kind of situation can occur to the user who takes such an action?". In a case in which the answer of the generative AI to this question is, for example, "There is a possibility of coming into contact with vehicles", "There is a possibility of coming into contact with bicycles", or "There is a possibility of entering an expressway interchange", the agent may store the action of the user walking on the roadway and the answer of the generative AI in association with each other. The agent may store action content of correcting the action in association with the answer.

The action content of correcting the action may include at least one of reproduction of vocal sound for correcting the dangerous action of the user or reproduction of an image for correcting the dangerous action of the user.

The vocal sound for correcting the dangerous action of the user may include vocal sound for guiding the user to a specific place. The specific place may include a place other than the place where the user is currently located, for example, a vicinity of the movement system 1000M and a sidewalk. Specifically, the vocal sound may include vocal sound such as "Please stop walking on the roadway", "That is not a sidewalk", "The roadway is dangerous", and "Please move to the sidewalk immediately".

As described above, in the autonomous processing, a table in which the answer of the generative AI corresponding to the state or action of the user, the content of the state or action, and the action content of correcting the state or action are associated with each other may be recorded in a storage medium such as a memory.

In the autonomous processing, after the table is recorded, the action of the user is autonomously or periodically detected, and the action schedule of the agent for attracting the attention of the user may be set on the basis of the detected action of the user and the content of the stored table. Specifically, the action determination unit 236 of the agent system 500 may set the first action content on the basis of the detected action of the user and the content of the stored table.

Specifically, the action determination unit 236 of the agent system 500 may spontaneously or periodically detect an action of the user on the basis of a sensor included in the communication terminal 100M, and determine the first action content in a case in which it is determined to lead the user as an action of the electronic apparatus on the basis of the detected action of the user and specific information stored in advance.

### (First Action Content)

The first action content may include an action of the agent that leads the user by controlling the wheels 601a of the movement unit 601. Controlling the wheels 601a may include changing at least one of a rotation direction, a rotation speed, or a steering direction of the wheels 601a. Hereinafter, an example of the first action content will be described.

The action determination unit 236 of the agent system 500 may execute reproduction of at least one of vocal sound or an image for assisting in walking of the user as the first action content for performing route guidance so that the walking user moves along a specific route. Specifically, as illustrated in Fig. 25, the action determination unit 236 may reproduce an image of a route along which the user can walk on the screen of the communication terminal 100M, and may reproduce vocal sound related to the route. The vocal sound may include, for example, "Turn right 10 m ahead".

The action determination unit 236 may execute reproduction of at least one of vocal sound or an image for guiding the user along a route different from the set guidance route. Specifically, as illustrated in Fig. 26, the action determination unit 236 may reproduce an image of a route different from the set guidance route on the screen of the communication terminal 100M, and may reproduce vocal sound related to the route. The vocal sound may include, for example, "The road is closed ahead. Would you like me to show another route?".

In a case in which the user being led along the set guidance route deviates from the guidance route, the action determination unit 236 of the agent system 500 may execute reproduction of at least one of vocal sound or an image for guiding the user along a path for returning the user to the guidance route. Specifically, as illustrated in Fig. 27, the action determination unit 236 may reproduce an image of the route returning the user to the set guidance route on the screen of the communication terminal 100M, and may reproduce vocal sound related to the route. The vocal sound may include "You have deviated from the route. I will show you the path for return to the original route." and the like. The action determination unit 236 of the agent system 500 may execute reproduction of at least one of the vocal sound or the image described above, for example, "I will guide you to the route along which you have previously passed", "Please rehydrate frequently", or "Please return to the sidewalk".

### (Second Action Content)

The action determination unit 236 of the agent system 500 may determine whether or not the action of the user has been corrected by detecting the action of the user while the movement unit 601 was leading the user or after the movement unit 601 reproduced at least one of vocal sound or an image that assists in walking of the user, and may determine second action content different from the first action content in a case in which the action of the user has been corrected.

The case in which the action of the user has been corrected may be interpreted as a case in which the user has stopped a specific action and a specific behavior or a case in which a specific situation has been resolved as a result of execution of the operation of the agent according to the first action content. Specifically, the case in which the action of the user has been corrected may include a case in which the user has started walking along the proposed different route, a case in which the user has started walking along a path for return to the original route when the path was shown due to deviation from the original route, and the like.

The second action content may include reproduction of at least one of vocal sound praising the user for action, vocal sound expressing gratitude for the action of the user, an image praising the user for the action, or an image expressing gratitude for the action of the user.

Specifically, the vocal sound praising the user for the action may include, for example, "Well done! Excellent" as illustrated in Fig. 28. The vocal sound expressing gratitude for the action of the user may include vocal sound such as "Thank you for coming back" or "Thank you for selecting another route" as illustrated in Fig. 29. The image praising the user for the action may include an image of a character taking a good pose. The image expressing gratitude for the action of the user may include an image of a character who expresses gratitude.

### (Third Action Content)

The action determination unit 236 may determine whether or not the action of the user has been corrected by detecting the action of the user while the movement unit 601 was leading the user or after the movement unit 601 reproduced at least one of vocal sound or an image that assists in the walking of the user, and determine third action content different from the first action content in a case in which the action of the user has not been corrected.

The case in which the action of the user has not been corrected may be interpreted as a case in which the user has continued the dangerous action and behavior even though the operation of the agent according to the first action content was executed, a case in which the dangerous situation has not been resolved, or a case in which the user has started or continued an action different from the proposal of the agent.

Specifically, the case in which the action of the user has not been corrected may include a case in which the user has walked along a route different from the proposed different route without walking along the proposed different route, a case in which the user has neglected a path for return to the original route and has continued walking when the path was shown due to deviation from the original route, and the like.

The third action content may include at least one of transmission of specific information to a person other than the user, reproduction of sound that attracts an interest of the user, or reproduction of an image that attracts an interest of the user.

The transmission of the specific information to a person other than the user may include distribution of an email containing a warning message to a relative of the user, a spouse of the user, a guardian of the user, or the like. The transmission of the specific information to a person other than the user may include distribution of an image (a still image or a moving image) including the user and the surrounding scenery to a relative of the user, a spouse of the user, a guardian of the user, or the like. The transmission of the specific information to a person other than the user may include distribution of vocal sound having a warning message.

The reproduction of the sound that attracts the interest of the user may include specific music that the user likes, and may also include vocal sound such as "Please come back here" or "Let's take a safe road".

The reproduction of the image that attracts the interest of the user may include reproduction of an image of an animal kept by the user, an image of parents of the user, an image of a character of the animal, and the like.

### (Fourth Action Content)

The action determination unit 236 of the agent system 500 may determine the fourth action content of calling attention regarding the state of the body to the user on the basis of biological information collected from the body of the user.

In this case, the device operations determined as the action of the communication terminal 100M by the action determination unit 236 of the agent system 500 include notifying the user of attention calling information based on the biological information collected from the body of the user by the communication terminal 100M reproducing at least one of vocal sound or an image.

A smartwatch 800 worn on the user's arm as illustrated in Fig. 59, for example, is used as a sensor for acquiring biological information from the body of the user. The smartwatch 800 illustrated in Fig. 59 is configured to collect at least one piece or a plurality of pieces of information of a body temperature, a pulse rate, a blood pressure, or a blood oxygen saturation (SPO2) of the user wearing the smartwatch 800 as biological information. The smartwatch 800 and the communication terminal 100M are connected via a wireless communication line such as Bluetooth (registered trademark). Therefore, the communication terminal 100M can receive the biological information of the user collected by the smartwatch 800.

In a case in which a physical abnormality of the user is detected on the basis of the collected biological information, the action determination unit 236 of the agent system 500 determines action content of notifying the user that the physical abnormality has occurred and giving advice about alleviating the physical abnormality of the user.

For example, as illustrated in Fig. 60, the action determination unit 236 of the agent system 500 determines to output vocal sound "Pulse is too fast. Please rest for a while." as the action of the communication terminal 100M. For example, as illustrated in Fig. 61, the action determination unit 236 of the agent system 500 determines to output vocal sound "Body temperature is too high. Please try to rehydrate your body while being careful of heat stroke." as the action of the communication terminal 100M. For example, as illustrated in Fig. 62, the action determination unit 236 of the agent system 500 determines to output vocal sound "Blood pressure is decreasing. Please be careful of dizziness and falling." as the action of the communication terminal 100M.

Here, the action determination unit 236 of the agent system 500 determines the attention calling content for the user depending on a range in which the acquired biological information is included on the basis of information such as a standard range, an attention range, and a danger range of the biological information such as the blood pressure, the pulse rate, and the body temperature stored in advance.

In a case in which it is determined that the biological information acquired from the body of the user has reached the dangerous range, the action determination unit 236 of the agent system 500 may determine, as the action content of the communication terminal 100M, action content of issuing a warning sound such as a buzzer sound in addition to calling attention by using vocal sound. Since the warning sound is emitted from the communication terminal 100M as described above, it is possible to notify not only the user wearing the communication terminal 100M but also other users around the user of the abnormality of the body.

The sensor of the smartwatch 800 may collect information of the R-R interval in the pulse variation of the user as biological information and transmit the collected information to the communication terminal 100M.

Here, the R-R interval is an interval between the R wave and the next R wave in the heart rate variation or the pulse variation. The R-R interval data is plotted at the time position of the rear R wave, interpolated, and spectrum analysis (frequency conversion) is performed on data generated by resampling at equal intervals to obtain spectrum waveform data, so that it is possible to grasp information of the user's autonomic nerve.

The action determination unit 236 of the agent system 500 determines action content of calculating an index indicating the state of the autonomic nerve of the user on the basis of the collected information regarding the R-R interval in the pulse variation of the user, and notifying the user of the current state of the autonomic nerve.

Here, as an index indicating the state of the autonomic nerve, an LF/HF value or the like calculated on the basis of a low frequency (LF) value and a high frequency (HF) value in the spectrum waveform data obtained as described above is used. Here, for example, the LF value is an integral value of the power spectral component of 0.04 to 0.15 Hz, and the HF value is an integral value of the power spectral component of 0.15 to 0.40 Hz.

The LF/HF value is used as an index indicating the degree of activity of the sympathetic nerve among the autonomic nerves. The HF value is used as an index indicating the degree of activity of the parasympathetic nerve among the autonomic nerves.

The autonomic nerves include a sympathetic nerve that mainly functions in an active state and a parasympathetic nerve that mainly functions in a resting state. That is, in a case in which the sympathetic nerve dominantly works, the living body can be considered to be in an active state, and in a case in which the parasympathetic nerve dominantly works, the living body can be considered to be in a resting state. Therefore, in general, in a case in which the living body is in a resting state, the blood pressure value and the pulse rate decrease, and the state of the autonomic nerve becomes the parasympathetic nerve dominant state, and in a case in which the living body is in a tension/active state, the blood pressure value and the pulse rate increase, and the state of the autonomic nerve becomes the sympathetic nerve dominant state.

In a case in which the body of the user is healthy, the degree of activity of the sympathetic nerve and the degree of activity of the parasympathetic nerve are switched within an appropriate range, and the body of the user is maintained in a normal state.

Therefore, in a case in which the state of the autonomic nerve based on the pulse variation collected from the body of the user is different from the usual normal state, the action determination unit 236 of the agent system 500 determines action content of notifying the user of the current state of the autonomic nerve to call attention.

According to the agent system 500 of the disclosure, an action of leading the user can be executed through autonomous processing. As a result, the agent can guide the user walking in a town that the user visits for the first time to an appropriate route. The user who is interested in walking may be guided to a route along which the user has not passed before. A route where the user cannot pass is detected in advance, and the user can be guided to another route. The user who gets lost and deviates from the specific route can be guided to the original route. It is possible to notify a visually impaired user of the surrounding situation.

The action determination unit 236 determines, as the action of the agent, any of a plurality of types of agent actions including no action, by using at least one of the state of the user 10, the emotion of the user 10, the emotion of the agent, or the state of the agent, and the action determination model 221 at a predetermined timing. Here, a case in which a sentence generation model having an interaction function is used as the action determination model 221 will be described as an example.

Specifically, the action determination unit 236 inputs text representing at least one of the state of the user 10, the emotion of the user 10, the emotion of the agent, or the state of the agent, and text for inquiry about agent actions to the sentence generation model, and determines the actions of the agent on the basis of an output of the sentence generation model.

For example, the plurality of types of agent actions includes the following (1) to (24).
(1) The agent does nothing.
(2) The agent has a dream.
(3) The agent talks to the user.
(4) The agent creates a picture diary.
(5) The agent proposes an activity.
(6) The agent proposes a person with whom the user should meet.
(7) The agent introduces news in which the user is interested.
(8) The agent edits pictures and moving images.
(9) The agent studies with the user.
(10) The agent evokes memories.
(11) The agent may reproduce an image of a route along which the user can walk on the screen of the communication terminal as the first action content of leading the user.
(12) The agent may reproduce vocal sound related to a route along which the user can walk as the first action content of correcting the action of the user.
(13) The agent may reproduce an image of a route different from the set guidance route on the screen of the communication terminal 100M as the first action content of correcting the action of the user.
(14) The agent may reproduce vocal sound related to a route different from the set guidance route as the first action content of correcting the action of the user.
(15) The agent may reproduce, as the first action content of correcting the action of the user, an image of a route for return to the set guidance route on the screen of the communication terminal 100M in a case in which the user led along the set guidance route deviates from the guidance route.
(16) The agent may reproduce, as the first action content of correcting the action of the user, vocal sound related to a route for return to the set guidance route in a case in which the user led along the set guidance route deviates from the guidance route.
(17) The agent may reproduce vocal sound praising the user for the action as the second action content different from the first action content.
(18) The agent may reproduce vocal sound expressing gratitude for the action of the user as the second action content different from the first action content.
(19) The agent may reproduce an image praising the user for the action as the second action content different from the first action content.
(20) The agent may reproduce an image expressing gratitude for the action of the user as the second action content different from the first action content.
(21) The agent may transmit specific information to a person other than the user as the third action content different from the first action content.
(22) The agent may reproduce sound that attracts the interest of the user as the third action content different from the first action content.
(23) The agent may reproduce an image that attracts the interest of the user as the third action content different from the first action content.
(24) The agent may reproduce an image that calls attention regarding the physical condition to the user as the fourth action content.

The action determination unit 236 may spontaneously or periodically detect the action of the user on the basis of the sensor, and in a case in which it is determined to lead the user as the action of the agent on the basis of the detected action of the user and the specific information stored in advance, execute the following first action content.

The action determination unit 236 of the agent system 500 may perform, as the agent action, the first action content in the above "(11)", that is, reproduce an image of a route along which the user can walk on the screen of the communication terminal 100M.

The action determination unit 236 of the agent system 500 may perform, as the agent action, the first action content in the above "(12)", that is, reproduce vocal sound related to a route along which the user can walk.

The action determination unit 236 of the agent system 500 may perform, as the agent action, the first action content in the above "(13)", that is, reproduce an image of a route different from the set guidance route as the first action content of correcting the action of the user on the screen of the communication terminal 100M.

The action determination unit 236 of the agent system 500 may perform, as the agent action, the first action content in the above "(14)", that is, reproduce vocal sound related to a route different from the set guidance route.

The action determination unit 236 of the agent system 500 may perform, as the agent action, the first action content in the above "(15)", that is, reproduce an image of a route for return to the set guidance route on the screen of the communication terminal 100M in a case in which the user led along the set guidance route deviates from the guidance route.

The action determination unit 236 of the agent system 500 may perform, as the agent action, the first action content in the above "(16)", that is, reproduce vocal sound related to a route for return to the set guidance route in a case in which the user led along the set guidance route deviates from the guidance route.

The action determination unit 236 of the agent system 500 may execute the second action content different from the first action content. Specifically, the action determination unit 236 may perform, as the agent action, the second action content in the above "(17)", that is, reproduce vocal sound praising the user for the action.

The action determination unit 236 of the agent system 500 may perform, as the agent action, the second action content in the above "(18)", that is, reproduce vocal sound expressing gratitude for the action of the user.

The action determination unit 236 of the agent system 500 may perform, as the agent action, the second action content in the above "(19)", that is, reproduce an image praising the user for the action.

The action determination unit 236 of the agent system 500 may perform, as the agent action, the second action content in the above "(20)", that is, reproduce an image expressing gratitude for the action of the user.

The action determination unit 236 of the agent system 500 may execute the third action content different from the first action content. Specifically, the action determination unit 236 may perform, as the agent action, the third action content in the above "(21)", that is, transmit the specific information to a person other than the user.

The action determination unit 236 of the agent system 500 may perform, as the agent action, the third action content in the above "(22)", that is, execute reproduction of sound that attracts the interest of the user.

The action determination unit 236 of the agent system 500 may perform, as the agent action, the third action content in the above "(23)", that is, execute reproduction of an image that attracts the interest of the user.

The action determination unit 236 of the agent system 500 may perform, as the agent action, the fourth action content in the above "(24)", that is, execute reproduction of an image that calls attention regarding the state of the body to the user.

In a case in which, for example, an image of a route along which the user can walk is reproduced as the first action content indicated in the above "(11)" to "(16)", the related information collection unit 270 of the agent system 500 may store data such as an image of a route along which the user can walk in the collected data 223.

In a case in which, for example, vocal sound praising the user for the action is reproduced as the second action content indicated in the above "(17)" to "(20)", the related information collection unit 270 of the agent system 500 may store data such as an image of a route along which the user can walk in the collected data 223.

In a case in which, for example, specific information is transmitted to a person other than the user as the third action content indicated in the above "(21)" to "(23)", the related information collection unit 270 of the agent system 500 may store data of the specific information or the like in the collected data 223.

In a case in which attention regarding the physical condition is called to the user as the fourth action content indicated in the above "(24)", the related information collection unit 270 of the agent system 500 may store data regarding the standard range, the attention range, the danger range, and the like of the biological information in the collected data 223.

A part of the agent system 500 (for example, the sensor module unit 210, the storage unit 220, and the control unit 228B) may be provided outside a communication terminal such as a smartphone possessed by the user (for example, a server), and the communication terminal may function as each unit of the agent system 500 by communicating with the outside.

### (Supplementary Note 1)

A control system including:
a state recognition unit that recognizes a user state including an action of a user and a state of an electronic apparatus;
an emotion determination unit that determines an emotion of the user or an emotion of the electronic apparatus; and
an action determination unit that determines, by using at least one of the user state, the state of the electronic apparatus, the emotion of the user, or the emotion of the electronic apparatus, and an action determination model at a predetermined timing, any of a plurality of types of device operations including non-operation as an action of the electronic apparatus, in which
the electronic apparatus detachably provided on a neck strap includes one or a plurality of sensors that collect biological information of the user, and
the device operations include notifying the user of attention calling information based on the collected biological information by the electronic apparatus reproducing at least one of vocal sound or an image.

### (Supplementary Note 2)

The control system according to Supplementary Note 1, in which the action determination unit determines, in a case in which a physical abnormality of the user is detected on the basis of the collected biological information, action content of notifying the user that the physical abnormality has occurred and giving advice about alleviating the physical abnormality of the user.

### (Supplementary Note 3)

The control system according to Supplementary Note 1, in which the sensor collects at least one piece or a plurality of pieces of information of a body temperature, a pulse rate, a blood pressure, or a blood oxygen saturation of the user.

### (Supplementary Note 4)

The control system according to Supplementary Note 1, in which
the sensor collects information regarding an R-R interval in a pulse variation of the user as the biological information, and
the action determination unit determines action content of calculating an index indicating a state of an autonomic nerve of the user on the basis of the collected information regarding the R-R interval in the pulse variation of the user, and notifying the user of a current state of the autonomic nerve.

### [Eleventh Embodiment]

### (1. Case in Which User Tends to Walk for a Long Time)

In a case in which there is the tendency, the robot 100 and the communication terminal themselves ask the generative AI a question "What kind of guidance should be given to the user who takes such an action?". In a case in which the answer of the generative AI to this question is, for example, "Propose a route different from a route along which the user has previously passed", "Propose a route that guides the user to a place where the user can take shelter from rain because it is likely to rain", or "Propose a route with a relatively small number of vehicles such as bicycles", the robot 100 and the communication terminal may store the action of the user and the answer of the generative AI in association with each other.

### (2. Case in Which User Tends to Run for a Long Time)

In a case in which there is the tendency, the robot 100 and the communication terminal themselves ask the generative AI a question "What kind of guidance should be given to the user who takes such an action?". In a case in which the answer of the generative AI to this question is, for example, "Provide guidance for encouraging hydration periodically", "Propose a route with fewer bicycles and clean air", "Propose a route with fewer traffic lights", or "Propose a route with undulations in order to increase load", the robot 100 and the communication terminal may store the action of the user and the answer of the generative AI in association with each other.

### (3. Case in Which User Tends to Walk on Roadway)

In a case in which there is the tendency, the robot 100 and the communication terminal themselves ask the generative AI a question "What kind of situation can occur to the user who takes such an action?". In a case in which the answer of the generative AI to this question is, for example, "There is a possibility of coming into contact with vehicles such as bicycles",, "There is a possibility of entering an expressway interchange", or "Advice should be given to encourage the user to walk on the sidewalk as much as possible", the robot 100 and the communication terminal may store the action of the user of walking on the roadway and the answer of the generative AI in association with each other. The robot 100 and the communication terminal may store action content of correcting the action in association with the answer.

The action content of correcting the action may include at least one of reproduction of vocal sound for correcting a dangerous action of the user or reproduction of an image for correcting the dangerous action of the user.

The vocal sound for correcting the dangerous action of the user may include vocal sound for guiding the user to a specific place. The specific place may include a place other than the place where the user is currently located, for example, a sidewalk. Specifically, the vocal sound may include vocal sound such as "Please stop walking on the roadway", "That is not a sidewalk", "The roadway is dangerous", and "Please move to the sidewalk immediately".

As described above, in the autonomous processing, a table in which the answer of the generative AI corresponding to the state or action of the user, the content of the state or action, and the action content of correcting the state or action are associated with each other may be recorded in a storage medium such as a memory.

In the autonomous processing, after the table is recorded, the action of the user may be autonomously or periodically detected, and the action schedule of the communication terminal for attracting the attention of the user may be set on the basis of the detected action of the user and the content of the stored table. Specifically, the action determination unit 236 of the robot 100 and the communication terminal may set the first action content on the basis of the detected action of the user and the content of the stored table.

Specifically, the action determination unit 236 may spontaneously or periodically detect an action of the user on the basis of the sensor, and determine the first action content in the case of determining to lead the user as an action of the electronic apparatus on the basis of the detected action of the user and the specific information stored in advance.

The sensor may be interpreted as any sensor of one or a plurality of sensors provided in the communication terminal. Specifically, the one or plurality of sensors may include a camera, a microphone, a gyro sensor, and the like. The sensor may collect at least one piece or plurality of pieces of information of temperature or humidity.

Here, the communication terminal will be described in detail with reference to Fig. 16.

Fig. 16 is an external view of the communication terminal according to the embodiment of the disclosure. As illustrated in Fig. 16, the communication terminal 100M may be interpreted as an electronic apparatus detachably provided on a neck strap 6000. The communication terminal 100M may be interpreted as a smartphone that will be described later, or may be interpreted as a smart AI device. The communication terminal 100M may have the functions of the robots 100 to 102 described above. The neck strap 6000 may include a hook-shaped member that is locked to the communication terminal 100M.

### (First Action Content)

The first action content may include an action of the communication terminal 100M that leads the user when the communication terminal 100M reproduces at least one of vocal sound or an image. Hereinafter, an example of the first action content will be described.

The action determination unit 236 may execute reproduction of at least one of vocal sound or an image for assisting in walking of the user as the first action content for performing route guidance so that the walking user moves along a specific route. Specifically, in a case in which it is detected that the communication terminal 100M is attached to the neck strap 6000, the action determination unit 236 may spontaneously search for a place where the user tends to go regularly and guide the user along a route to the place that has been searched for. In a case in which it is detected that the camera included in the sensor of the communication terminal 100M is directed in an advancing direction of the user, the action determination unit 236 may spontaneously search for a place where the user tends to go regularly and guide the user along a route to the place that has been searched for. The action determination unit 236 may reproduce vocal sound related to guidance of a route along which the user can walk. The vocal sound may include "I searched for a place where Mr. A frequently goes at this time. I will start guiding.", "Turn right 10 m ahead" illustrated in Fig. 17, and the like. The action determination unit 236 may reproduce an image related to guidance of a route along which the user can walk on the screen of the communication terminal 100M.

The action determination unit 236 may execute reproduction of at least one of vocal sound and an image for guiding the user along a route different from the set guidance route. Specifically, the action determination unit 236 may spontaneously re-search for a place where the user tends to go regularly, including the traffic condition of the set guidance route, the climate near the route, the current time, and the like, and reproduce vocal sound or the like related to a route to the place that has been re-searched for, that is, a route different from the set guidance route. More specifically, the vocal sound may include "I searched for another route because of the heavy traffic today. Would you like me to show another route?", "The road is closed ahead. Would you like me to show another route?" as illustrated in Fig. 18, and the like.

In a case in which the user led along the set guidance route deviates from the guidance route, the action determination unit 236 may spontaneously search for a path for returning the user to the guidance route and execute reproduction of at least one of vocal sound or an image for guiding the user to the path that has been searched for. Specifically, the action determination unit 236 may spontaneously re-search for a place where the user can return, including the traffic condition of a path deviating from the guidance route, the climate near the path, the current time, events that have occurred in the past near the route, and the like, and reproduce vocal sound related to the route to the re-searched place. That is, the vocal sound related to the path for return to the set guidance route may be reproduced. More specifically, the vocal sound may include "Since there are few people and it is dangerous, I searched for a path for return to the original route.", "It started to rain. It seems to be an area where a mudslide occurred before. Do you want to return to the original road?", "You have deviated from the route. I will show you the path for return to the original route." as illustrated in Fig. 19, and the like. The action determination unit 236 of the robot 100 and the communication terminal may execute reproduction of at least one of vocal sound such as the above vocal sound, for example, "I will guide you to the route along which you have previously passed", "Please rehydrate frequently", and "Please return to the sidewalk" or an image.

### (Second Action Content)

The action determination unit 236 may determine whether or not the action of the user has been corrected by detecting the action of the user while the communication terminal 100M was leading the user or after the communication terminal 100M reproduced at least one of vocal sound or an image that assists in the walking of the user, and may determine second action content different from the first action content in a case in which the action of the user has been corrected.

The case in which the action of the user has been corrected may be interpreted as a case in which the user has stopped a specific action and a specific behavior or a case in which a specific situation has been resolved as a result of execution of the operation of the communication terminal 100M according to the first action content. Specifically, the case in which the action of the user has been corrected may include a case in which the user has started walking along the proposed different route, a case in which the user has started walking along a path for return to the original route when the path was shown due to deviation from the original route, and the like.

The second action content may include reproduction of at least one of vocal sound praising the user for action, vocal sound expressing gratitude for the action of the user, an image praising the user for the action, or an image expressing gratitude for the action of the user.

Specifically, the vocal sound praising the user for the action may include, for example, "Well done! Excellent" as illustrated in Fig. 20. The vocal sound expressing gratitude for the action of the user may include vocal sound such as "Thank you for coming back" or "Thank you for selecting another route" as illustrated in Fig. 21. The image praising the user for the action may include an image of a character taking a good pose. The image expressing gratitude for the action of the user may include an image of a character who expresses gratitude.

### (Third Action Content)

The action determination unit 236 may determine whether or not the action of the user has been corrected by detecting the action of the user while the communication terminal 100M was leading the user or after the communication terminal 100M reproduced at least one of vocal sound or an image that assists in the walking of the user, and determine third action content different from the first action content in a case in which the action of the user has not been corrected.

The case in which the action of the user has not been corrected may be interpreted as a case in which the user has continued the dangerous action and behavior even though the operation of the communication terminal 100M according to the first action content was executed, a case in which the dangerous situation has not been resolved, or a case in which the user has started or continued an action different from the proposal of the communication terminal 100M.

Specifically, the case in which the action of the user has not been corrected may include a case in which the user has walked along a route different from the proposed different route without walking along the proposed different route, a case in which the user 10 has neglected a path for return to the original route and continued walking when the path was shown due to deviation from the route, and the like.

The third action content may include at least one of transmission of specific information to a person other than the user, reproduction of sound that attracts an interest of the user, or reproduction of an image that attracts an interest of the user.

The transmission of the specific information to a person other than the user may include distribution of an email containing a warning message to a relative of the user, a spouse of the user, a guardian of the user, or the like. The transmission of the specific information to a person other than the user may include distribution of an image (a still image or a moving image) including the user and the surrounding scenery to a relative of the user, a spouse of the user, a guardian of the user, or the like. The transmission of the specific information to a person other than the user may include distribution of vocal sound having a warning message.

The reproduction of the sound that attracts the interest of the user may include specific music that the user likes, and may also include vocal sound such as "Please come back here" or "Let's take a safe road".

The reproduction of the image that attracts the interest of the user may include reproduction of an image of an animal kept by the user, an image of parents of the user, an image of a character of the animal, and the like.

### (Fourth Action Content)

The action determination unit 236 may determine the fourth action content of notifying the user of attention calling information as the action of the communication terminal 100M on the basis of the collected surrounding environment information.

In this case, the device operations determined as the action of the communication terminal 100M by the action determination unit 236 include notifying the user of the attention calling information based on the collected surrounding environment information by the communication terminal 100M reproducing at least one of vocal sound or an image.

In a case in which there is a concern about the influence of the surrounding environment on the body of the user on the basis of the collected surrounding environment information, the action determination unit 236 determines, as the action of the communication terminal 100M, action content of giving advice about alleviating the influence of the surrounding environment to the user.

For example, the action determination unit 236 inputs a time series of the temperature and the humidity collected by the sensors to the generative AI, and asks a question "Is it better to give advice about heat stroke?". In a case in which the answer of the generative AI to this question is, for example, "It is better to give advice about heat stroke", outputting vocal sound such as "Temperature and humidity are high, and heat stroke may occur if this continues. Please move to a cooler place. Also, take sufficient hydration." is determined as the action of the communication terminal 100M. For example, the action determination unit 236 inputs a time series of the temperature collected by the sensor to the generative AI, and asks a question "Is it better to give advice about hypothermia or frostbite?". In a case in which the answer of the generative AI to this question is, for example, "It is better to give advice about hypothermia or frostbite", outputting vocal sound such as "The temperature is low, and this may cause hypothermia or frostbite. Please move to a warm place." is determined as the action of the communication terminal 100M.

Here, the action determination unit 236 determines the attention calling content to the user depending on a range in which the acquired surrounding environment information is included on the basis of information such as a standard range, an attention range, and a danger range stored in advance, of temperature and humidity appropriate for the human body.

In a case in which it is determined that the surrounding environment information has reached the dangerous range, the action determination unit 236 may determine, as the action content of the communication terminal 100M, action content of issuing a warning sound such as a buzzer sound in addition to calling attention by using vocal sound. In a case in which the warning sound is emitted from the communication terminal 100M as described above, it is possible to notify not only the user wearing the communication terminal 100M but also other users around the user of the abnormality of the body.

According to the robot 100 and the communication terminal of the disclosure, an action of leading the user can be executed through autonomous processing. As a result, the robot 100 and the communication terminal can guide the user walking in a town that the user visits for the first time to an appropriate route. The user who is interested in walking may be guided to a route along which the user has not passed before. A route where the user cannot pass is detected in advance, and the user can be guided to another route. The user who gets lost and deviates from the specific route can be guided to the original route.

The action determination unit 236 determines, as the action of the robot 100 and the communication terminal, any of a plurality of types of robot actions and communication terminal actions including no action, by using at least one of the state of the user 10, the emotion of the user 10, the emotion of the robot 100 and the communication terminal, or the state of the robot 100 and the communication terminal, and the action determination model 221 at a predetermined timing. Here, a case in which a sentence generation model having an interaction function is used as the action determination model 221 will be described as an example.

Specifically, the action determination unit 236 inputs text representing at least one of the state of the user 10, the emotion of the user 10, the emotion of the robot 100 and the communication terminal, or the state of the robot 100 and the communication terminal, text for inquiry about robot actions and communication terminal actions, and the data collected by the sensor to the sentence generation model, and determines the actions of the robot 100 and the communication terminal on the basis of an output of the sentence generation model.

For example, the actions of the plurality of types of robots and the communication terminal include the following (1) to (24).
(1) The robot 100 and the communication terminal 100M do nothing.
(2) The robot 100 and the communication terminal 100M have a dream.
(3) The robot 100 and the communication terminal 100M talk to the user.
(4) The robot 100 and the communication terminal 100M create a picture diary.
(5) The robot 100 and the communication terminal 100M propose an activity.
(6) The robot 100 and the communication terminal 100M propose a person with whom the user should meet.
(7) The robot 100 and the communication terminal 100M introduce news in which the user is interested. (8) The robot 100 and the communication terminal 100M edit pictures and moving images.
(9) The robot 100 and the communication terminal 100M study with the user.
(10) The robot 100 and the communication terminal 100M evoke memories.
(11) The robot 100 and the communication terminal 100M may reproduce an image of a route along which the user can walk on the screen of the communication terminal 100M as the first action content of leading the user.
(12) The robot 100 and the communication terminal 100M may reproduce vocal sound related to a route along which the user can walk as the first action content of correcting the action of the user.
(13) The robot 100 and the communication terminal 100M may reproduce an image of a route different from the set guidance route on the screen of the communication terminal 100M as the first action content of correcting the action of the user.
(14) The robot 100 and the communication terminal 100M may reproduce vocal sound related to a route different from the set guidance route as the first action content of correcting the action of the user.
(15) The robot 100 and the communication terminal 100M may reproduce, as the first action content of correcting the action of the user, an image of a route for return to the set guidance route on the screen of the communication terminal 100M in a case in which the user led along the set guidance route deviates from the guidance route.
(16) The robot 100 and the communication terminal 100M may reproduce, as the first action content of correcting the action of the user, vocal sound related to a route for return to the set guidance route in a case in which the user led along the set guidance route deviates from the guidance route.
(17) The robot 100 and the communication terminal 100M may reproduce vocal sound praising the user for the action as the second action content different from the first action content.
(18) The robot 100 and the communication terminal 100M may reproduce vocal sound expressing gratitude for the action of the user as the second action content different from the first action content.
(19) The robot 100 and the communication terminal 100M may reproduce an image praising the user for the action as the second action content different from the first action content.
(20) The robot 100 and the communication terminal 100M may reproduce an image expressing gratitude for the action of the user as the second action content different from the first action content.
(21) The robot 100 and the communication terminal 100M may transmit specific information to a person other than the user as the third action content different from the first action content.
(22) The robot 100 and the communication terminal 100M may reproduce sound that attracts the interest of the user as the third action content different from the first action content.
(23) The robot 100 and the communication terminal 100M may reproduce an image that attracts the interest of the user as the third action content different from the first action content.
(24) The robot 100 and the communication terminal 100M may reproduce an image that calls attention regarding the influence of the surrounding environment on the body to the user as the fourth action content.

The action determination unit 236 inputs, to the sentence generation model, text representing the state of the user 10 and the state of the robot 100 or the communication terminal 100M recognized by the state recognition unit 230, and the current emotion value of the user 10 and the current emotion value of the robot 100 or the current emotion value of the communication terminal 100M determined by the emotion determination unit 232, and text for inquiry about any of a plurality of types of robot actions including no action, each time a certain period of time elapses, and determines actions of the robot 100 and the communication terminal 100M on the basis of an output of the sentence generation model. Here, in a case in which the user 10 is absent around the robot 100 or the communication terminal 100M, the text to be input to the sentence generation model need not include the state of the user 10 and the current emotion value of the user 10, or may include the fact that the user 10 is absent. Hereinafter, the communication terminal 100M may be read as a robot. The communication terminal 100M may be interpreted as a smartphone or a smart AI device. The robot action may be read as a communication terminal action.

The action determination unit 236 may determine the first action content in a case in which the camera included in the sensor is directed in an advancing direction of the user. As a result, the communication terminal 100M can start leading the user only in a case in which the user desires to execute route guidance.

The action determination unit 236 may perform, as the communication terminal action, the first action content in the above "(11)", that is, reproduce an image of a route along which the user can walk on the screen of the communication terminal 100M.

### (Supplementary Note 1)

A control system including:
a state recognition unit that recognizes a user state including an action of a user and a state of an electronic apparatus;
an emotion determination unit that determines an emotion of the user or an emotion of the electronic apparatus; and
an action determination unit that determines, by using at least one of the user state, the state of the electronic apparatus, the emotion of the user, or the emotion of the electronic apparatus, and an action determination model at a predetermined timing, any of a plurality of types of device operations including non-operation as an action of the electronic apparatus, in which
the electronic apparatus detachably provided on a neck strap includes one or a plurality of sensors that collect surrounding environment information, and
the device operations include notifying the user of attention calling information based on the collected surrounding environment information by the electronic apparatus reproducing at least one of vocal sound or an image.

### (Supplementary Note 2)

The control system according to Supplementary Note 1, in which the action determination unit determines, in a case in which there is a concern about an influence of a surrounding environment on a body of the user, action content of giving advice about alleviating the influence of the surrounding environment to the user as the action of the electronic apparatus, on the basis of the collected surrounding environment information.

### (Supplementary Note 3)

The control system according to Supplementary Note 1, in which the sensor collects at least one piece or a plurality of pieces of information of temperature or humidity.

### [Twelfth Embodiment]

### (1. Case in Which User Tends to Walk for a Long Time)

In a case in which there is the tendency, the robot 100 and the communication terminal themselves ask the generative AI a question "What kind of guidance should be given to the user who takes such an action?". In a case in which the answer of the generative AI to this question is, for example, "Propose a route different from the route along which the user has previously passed", "Propose a route to a place where the user can take shelter from rain because it is likely to rain, or "Propose a route with a relatively small number of vehicles such as bicycles", the robot 100 and the communication terminal may store the action of the user and the answer of the generative AI in association with each other.

### (2. Case in Which User Tends to Run for a Long Time)

In a case in which there is the tendency, the robot 100 and the communication terminal themselves ask the generative AI a question "What kind of guidance should be given to the user who takes such an action?". In a case in which the answer of the generative AI to this question is, for example, "Provide guidance for encouraging hydration periodically", "Propose a route with fewer bicycles and clean air", "Propose a route with fewer traffic lights", or "Propose a route with undulations in order to increase load", the robot 100 and the communication terminal may store the action of the user and the answer of the generative AI in association with each other.

### (3. Case in Which User Tends to Walk on Roadway)

In a case in which there is the tendency, the robot 100 and the communication terminal themselves ask the generative AI a question "What kind of situation can occur to the user who takes such an action?". In a case in which the answer of the generative AI to this question is, for example, "There is a possibility of coming into contact with vehicles such as bicycles", "There is a possibility of entering an expressway interchange" or "Advice should be given to encourage the user to walk on the sidewalk as much as possible", the robot 100 and the communication terminal may store the user's action of walking on the roadway and the answer of the generative AI in association with each other. The robot 100 and the communication terminal may store action content of correcting the action in association with the answer.

The action content of correcting the action may include at least one of reproduction of vocal sound for correcting the dangerous action of the user or reproduction of an image for correcting the dangerous action of the user.

The vocal sound for correcting the dangerous action of the user may include vocal sound for guiding the user to a specific place. The specific place may include a place other than the place where the user is currently located, for example, a sidewalk. Specifically, the vocal sound may include vocal sound such as "Please stop walking on the roadway", "That is not a sidewalk", "The roadway is dangerous", and "Please move to the sidewalk immediately".

As described above, in the autonomous processing, a table in which the answer of the generative AI corresponding to the state or action of the user, the content of the state or action, and the action content of correcting the state or action are associated with each other may be recorded in a storage medium such as a memory.

In the autonomous processing, after the table is recorded, the action of the user is autonomously or periodically detected, and the action schedule of the communication terminal for attracting the attention of the user may be set on the basis of the detected action of the user and the content of the stored table. Specifically, the action determination unit 236 of the robot 100 and the communication terminal may set the first action content on the basis of the detected action of the user and the content of the stored table.

Specifically, the action determination unit 236 may spontaneously or periodically detect an action of the user on the basis of the sensor, and determine the first action content in the case of determining to lead the user as an action of the electronic apparatus on the basis of the detected action of the user and the specific information stored in advance.

The sensor may be interpreted as a sensor of one or a plurality of sensors provided in a communication terminal. Specifically, the one or plurality of sensors may include a camera, a microphone, a gyro sensor, and the like.

Here, the communication terminal will be described in detail with reference to Fig. 24.

Fig. 24 is an external view of the communication terminal according to the embodiment of the disclosure. As illustrated in Fig. 24, the communication terminal 100M may be interpreted as an electronic apparatus detachably provided on a neck strap 6000. The communication terminal 100M may be interpreted as a smartphone that will be described later, or may be interpreted as a smart AI device. The communication terminal 100M may have the functions of the robots 100 to 102 described above. The neck strap 6000 may include a hook-shaped member that is locked to the communication terminal 100M.

### (First Action Content)

The first action content may include an action of the communication terminal 100M that leads the user when the communication terminal 100M reproduces at least one of vocal sound or an image. Hereinafter, an example of the first action content will be described.

The action determination unit 236 may execute reproduction of at least one of vocal sound or an image for assisting in walking of the user as the first action content for performing route guidance so that the walking user moves along a specific route. Specifically, in a case in which it is detected that the communication terminal 100M is attached to the neck strap 6000, the action determination unit 236 may spontaneously search for a place where the user tends to go regularly and guide the user along a route to the place that has been searched for. In a case in which it is detected that the camera included in the sensor of the communication terminal 100M is directed in an advancing direction of the user, the action determination unit 236 may spontaneously search for a place where the user tends to go regularly and guide the user along a route to the place that has been searched for. The action determination unit 236 may reproduce vocal sound related to guidance of a route along which the user can walk. The vocal sound may include "I searched for a place where Mr. A frequently goes at this time. I will start guiding.", "Turn right 10 m ahead" illustrated in Fig. 24, and the like. The action determination unit 236 may reproduce an image related to guidance of a route along which the user can walk on the screen of the communication terminal 100M.

The action determination unit 236 may execute reproduction of at least one of vocal sound and an image for guiding a route different from the set guidance route. Specifically, the action determination unit 236 may spontaneously re-search for a place where the user tends to go regularly, including the traffic condition of the set guidance route, the climate near the route, the current time, and the like, and reproduce vocal sound or the like related to a route to the place that has been re-searched for, that is, a route different from the set guidance route. More specifically, the vocal sound may include "I searched for another route because of the heavy traffic today. Would you like me to show another route?", "The road is closed ahead. Would you like me to show another route?" as illustrated in Fig. 26, and the like.

In a case in which the user led along the set guidance route deviates from the guidance route, the action determination unit 236 may spontaneously search for a path for returning the user to the guidance route and execute reproduction of at least one of vocal sound or an image for guiding the user to the path that has been searched for. Specifically, the action determination unit 236 may spontaneously re-search for a place where the user can return, including the traffic condition of a path deviating from the guidance route, the climate near the path, the current time, events that have occurred in the past near the route, and the like, and reproduce vocal sound related to the route to the re-searched place. That is, the vocal sound related to the path for return to the set guidance route may be reproduced. More specifically, the vocal sound may include "Since there are few people and it is dangerous, I searched for a path for return to the original route.", "It started to rain. It seems to be an area where a mudslide occurred before. Do you want to return to the original road?", "You have deviated from the route. I will show you the path for return to the original route." as illustrated in Fig. 27, and the like. The action determination unit 236 of the robot 100 and the communication terminal may execute reproduction of vocal sound such as the above vocal sound, for example, "I will guide you to the route along which you have previously passed", "Please rehydrate frequently", and "Please return to the sidewalk" or an image.

### (Second Action Content)

The action determination unit 236 may determine whether or not the action of the user has been corrected by detecting the action of the user while the communication terminal 100M was leading the user or after the communication terminal 100M reproduced at least one of vocal sound or an image that assists in the walking of the user, and may determine second action content different from the first action content in a case in which the action of the user has been corrected.

The case in which the action of the user has been corrected may be interpreted as a case in which the user has stopped a specific action and a specific behavior or a case in which a specific situation has been resolved as a result of execution of the operation of the communication terminal 100M according to the first action content. Specifically, the case in which the action of the user has been corrected may include a case in which the user has started walking along the proposed different route, a case in which the user has started walking along a path for return to the original route when the path was shown due to deviation from the original route, and the like.

The second action content may include reproduction of at least one of vocal sound praising the user for action, vocal sound expressing gratitude for the action of the user, an image praising the user for the action, or an image expressing gratitude for the action of the user.

Specifically, the vocal sound praising the user for the action may include, for example, "Well done! Excellent" as illustrated in Fig. 28. The vocal sound expressing gratitude for the action of the user may include vocal sound such as "Thank you for coming back" or "Thank you for selecting another route" as illustrated in Fig. 29. The image praising the user for the action may include an image of a character taking a good pose. The image expressing gratitude for the action of the user may include an image of a character who expresses gratitude.

### (Third Action Content)

The action determination unit 236 may determine whether or not the action of the user has been corrected by detecting the action of the user while the communication terminal 100M was leading the user or after the communication terminal 100M reproduced at least one of vocal sound or an image that assists in the walking of the user, and determine third action content different from the first action content in a case in which the action of the user has not been corrected.

The case in which the action of the user has not been corrected may be interpreted as a case in which the user has continued the dangerous action and behavior even though the operation of the communication terminal 100M according to the first action content was executed, a case in which the dangerous situation has not been resolved, or a case in which the user has started or continued an action different from the proposal of the communication terminal 100M.

Specifically, the case in which the action of the user has not been corrected may include a case in which the user has walked along a route different from the proposed different route without walking along the proposed different route, a case in which the user has neglected a path for return to the original route and has continued walking when the path was shown due to deviation from the route, and the like.

The third action content may include at least one of transmission of specific information to a person other than the user, reproduction of sound that attracts an interest of the user, or reproduction of an image that attracts an interest of the user.

The transmission of the specific information to a person other than the user may include distribution of an email containing a warning message to a relative of the user, a spouse of the user, a guardian of the user, or the like. The transmission of the specific information to a person other than the user may include distribution of an image (a still image or a moving image) including the user and the surrounding scenery to a relative of the user, a spouse of the user, a guardian of the user, or the like. The transmission of the specific information to a person other than the user may include distribution of vocal sound having a warning message.

The reproduction of the sound that attracts the interest of the user may include specific music that the user likes, and may also include vocal sound such as "Please come back here" or "Let's take a safe road".

The reproduction of the image that attracts the interest of the user may include reproduction of an image of an animal kept by the user, an image of parents of the user, an image of a character of the animal, and the like.

### (Fourth Action Content)

In a case in which the user wearing the communication terminal 100M is performing work, the action determination unit 236 may determine the fourth action content of supporting the work for the user. In this case, a height position at which the user is performing the work is lower than the height position of the user's eyes.

The work in this case includes, for example, work to be performed on a device that is a work target (hereinafter referred to as "work target device"), and more specifically includes assembly work, disassembly work, and the like for the work target device. Inspection work, cleaning work, repair work, and the like for the work target device may be included.

The work in this case includes, for example, creation activities. More specifically, the creation activities include production activities of art works (art articles, craft articles, and the like), creation activities of literature works, creation activities of music works, performance activities of musical instruments, and the like.

In a case in which the fourth action content is determined, the device operations determined as the actions of the communication terminal 100M by the action determination unit 236 include setting, to the user performing the work, action content of supporting the work of the user by outputting at least one of vocal sound or an image from the communication terminal 100M.

In a case in which the fourth action content is determined, the work content and the work situation are photographed by the camera included in the communication terminal 100M, and the action content that supports the work of the user is set for the user performing the work on the basis of a captured image. The image in this case may be a still image or a moving image.

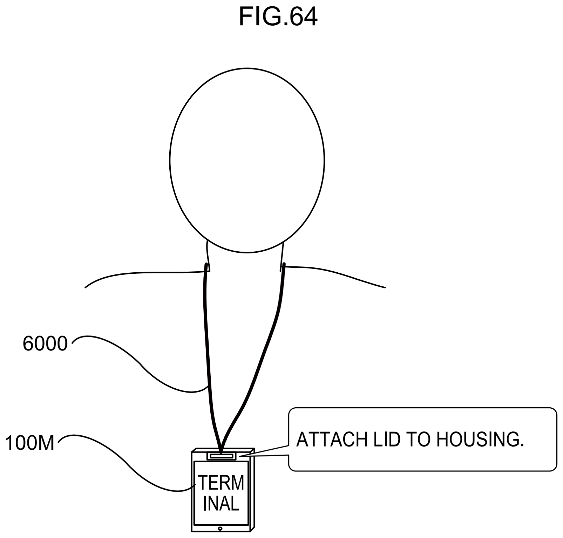

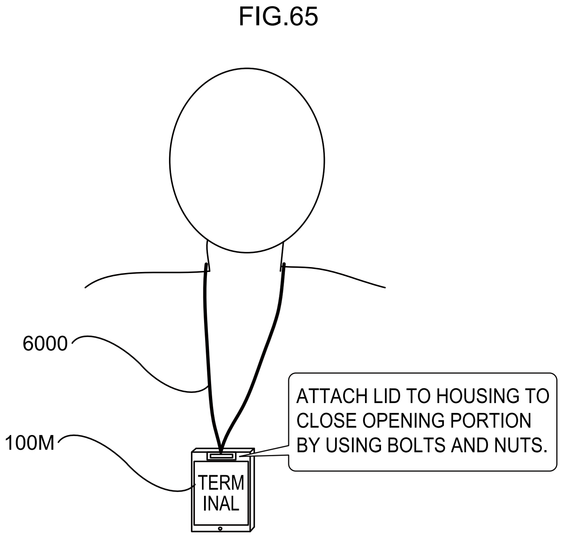

For example, in a case in which the user is performing assembly work for the work target device, the action determination unit 236 determines, as an action of the communication terminal 100M, to output vocal sound such as "Attach the lid to the housing." as illustrated in Fig. 64. The action determination unit 236 may determine, as the action of the communication terminal 100M, to output vocal sound that notifies the user of more specifically what attachment method to use for attachment. For example, as illustrated in Fig. 65, the action determination unit 236 determines to output vocal sound "Attach the lid to the housing to close the opening portion by using bolts and nuts." as the action of the communication terminal 100M. In a case in which a tool, an instrument, or an implement to be used is set according to an attachment method, the type of the tool, the instrument, or the implement to be used may also be included in the output content using vocal sound.

In a case in which the user uses a tool, an instrument, or an implement, the action determination unit 236 may determine, as the action of the communication terminal 100M, to output advice about a method of using the tool, the instrument, or the implement. In this case, a camera included in the communication terminal 100M may recognize the type of the tool, the instrument, or the implement used by the user, capture an image of work content and a work situation, and set advice about a use method on the basis of the captured image. The user's actual use of a tool, an instrument, or an implement may be monitored, and advice about more appropriate use may be given.

The work performed on the work target device by the user may be completed with single work content, or may be sequentially performed with a plurality of pieces of work content. In a case in which the work performed by the user on the work target device includes a plurality of pieces of work content, the action determination unit 236 may determine, as the action of the communication terminal 100M, to output vocal sound that conveys a work procedure to the user. In this case, the progress status of the work is detected by the camera of the communication terminal 100M, and after the end of one work is detected, vocal sound output for supporting the next work may be emitted.

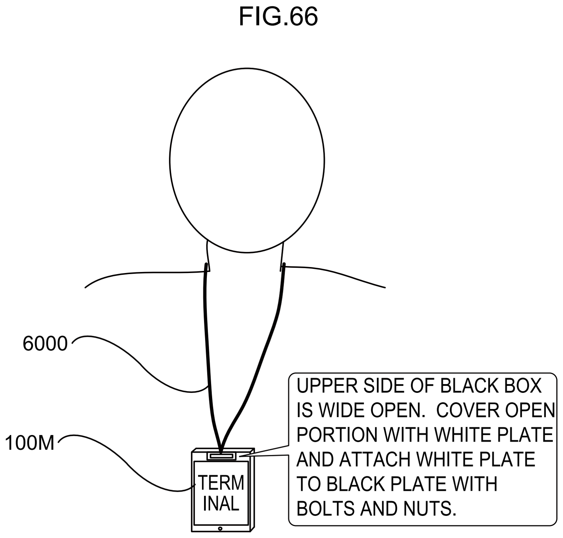

In a case in which the work that the user is performing is delayed, the action determination unit 236 includes supporting the work of the user by replacing the action content of supporting the work of the user with content different from the output content of the vocal sound so far. For example, as illustrated in Fig. 66, the action determination unit 236 may determine to output vocal sound "The upper side of the black box is wide open. Cover the open portion with a white plate and attach the white plate to the black plate with bolts and nuts." as the action of the communication terminal 100M.

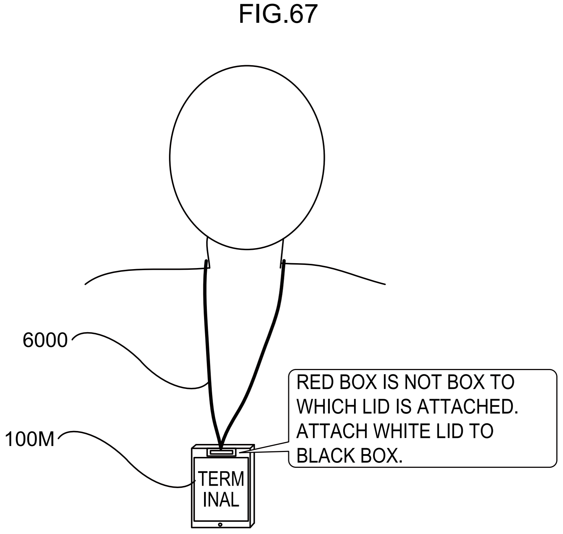

In a case in which the work performed by the user is wrong, the action determination unit 236 includes supporting the work of the user by replacing the action content of stopping the work of the user with the content different from the output content of the vocal sound so far. For example, as illustrated in Fig. 67, the action determination unit 236 may determine to output vocal sound "The red box is not a box to which a lid is attached. Attach a white lid to the black box." as the action of the communication terminal 100M.

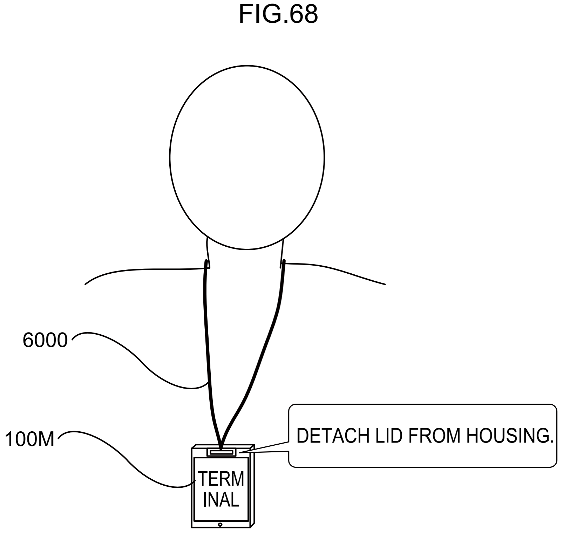

The above example is an example of a case in which the user is performing the assembly work for the work target device. On the other hand, even in a case in which the user is performing disassembly work, inspection work, cleaning work, repair work, and the like of the work target device, the action determination unit 236 determines the fourth action content of supporting the work for the user wearing the communication terminal 100M. For example, in a case in which the work is disassembly work for the work target device, the action determination unit 236 determines, as an action of the communication terminal 100M, to output vocal sound such as "Detach the lid from the housing." as illustrated in Fig. 68.

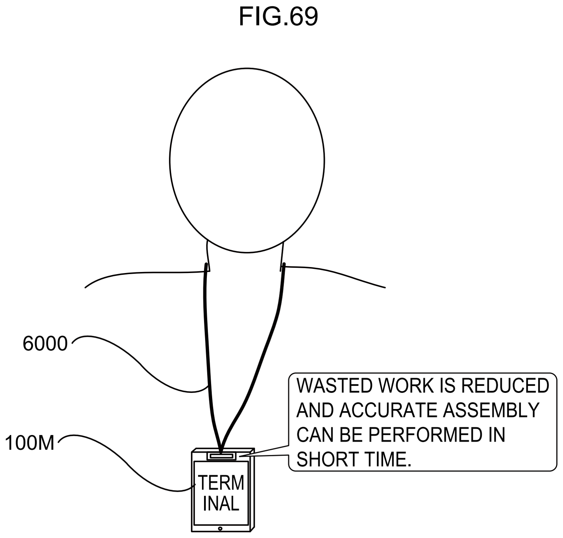

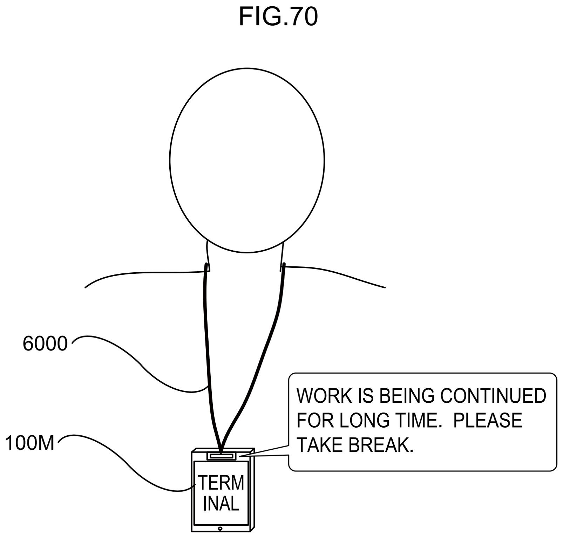

In a case in which the user is performing work on the work target device, the output from the communication terminal 100M as the device operation determined by the action determination unit 236 as the action of the communication terminal 100M may include analysis and evaluation on the work content. For example, in a case in which the time required by the user for specific work becomes short, for example, the action determination unit 236 may determine, as the action of the communication terminal 100M, to output vocal sound "Wasted work is reduced and accurate assembly can be performed in a short time." as illustrated in Fig. 69. As described above, highly evaluating the work of the user is to bring the emotion of the user closer to "pleased", and as a result, supports the work of the user. As illustrated in Fig. 70, the action determination unit 236 may determine, as the action of the communication terminal 100M, to output vocal sound such as "Work is being continued for a long time. Please take a break.". As described above, performing some kind of attention or the like to the user enhances work efficiency or avoids danger, and as a result, supports the work of the user.

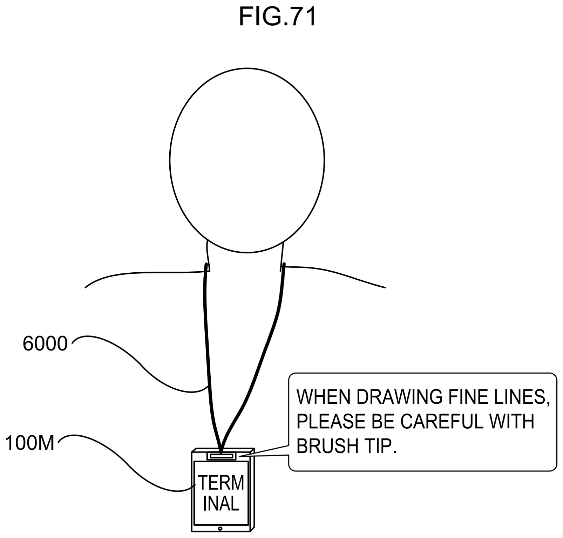

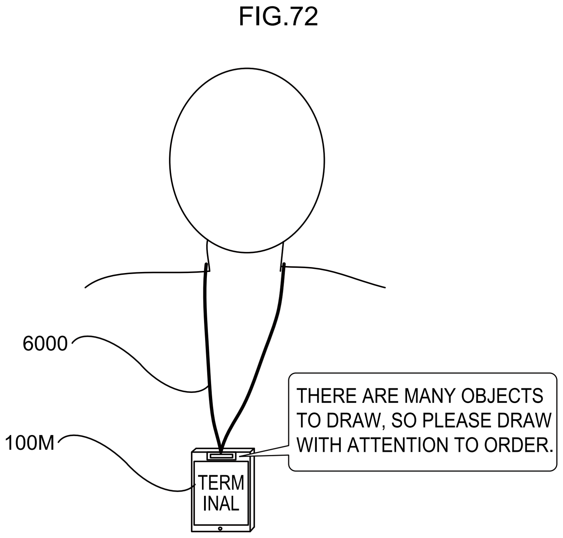

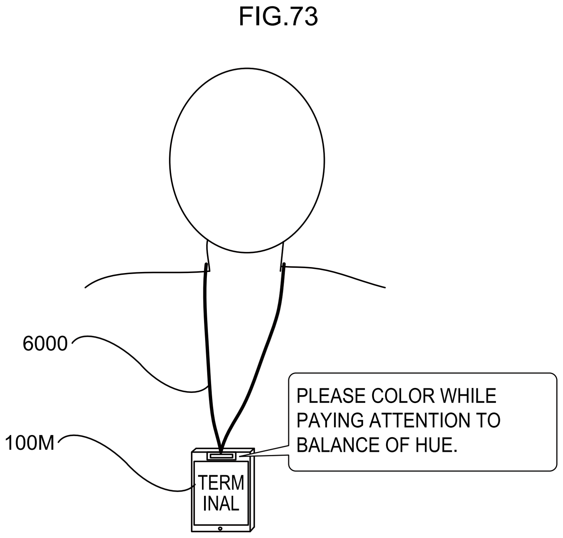

In a case in which the work performed by the user is a creation activity, the action determination unit 236 determines action content of supporting the work of the creation activity as an action of the communication terminal 100M. For example, in a case in which the creation activity is production of a painting, monitoring the production status with a camera of the communication terminal 100M and giving advice about a specific technique or the like are included. For example, as illustrated in Fig. 71, the action determination unit 236 determines to output vocal sound "When drawing fine lines, please be careful with the brush tip." as the action of the communication terminal 100M. As another example, the action determination unit 236 may determine to output vocal sound "There are many objects to draw, so please draw with attention to the order." as illustrated in Fig. 72. As still another example, as illustrated in Fig. 73, the action determination unit 236 may determine to output vocal sound such as "Please color while paying attention to the balance of hue.".

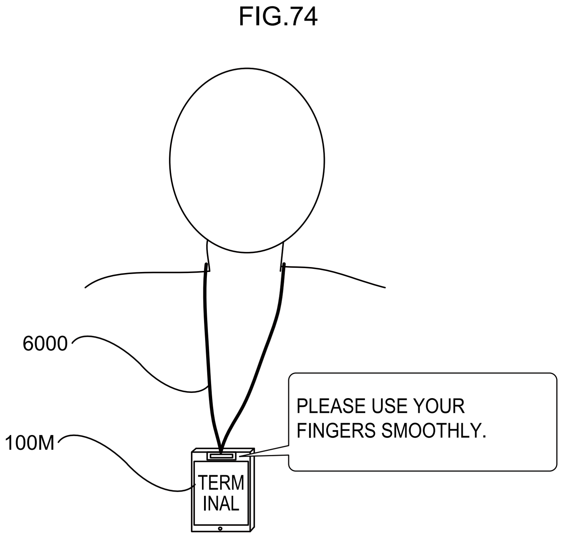

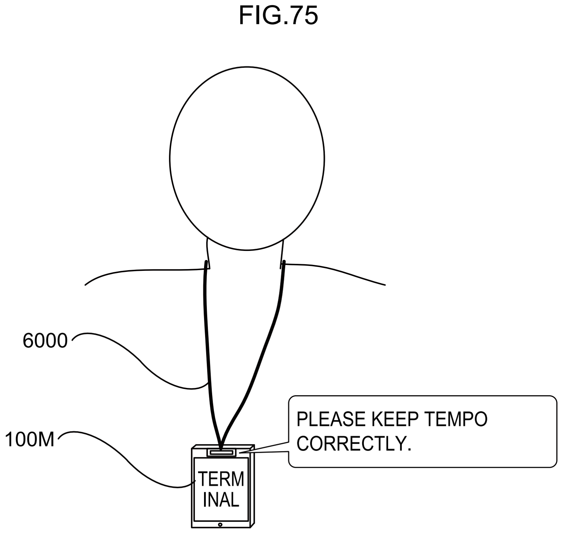

In a case in which the creation activity performed by the user is performance of a musical instrument, monitoring the performance with the camera of the communication terminal 100M and giving advice about a specific performance method or the like are included. For example, as illustrated in Fig. 74, the action determination unit 236 determines to output vocal sound "Please use your fingers smoothly." as the action of the communication terminal 100M. As still another example, as illustrated in Fig. 75, the action determination unit 236 may determine to output vocal sound such as "Please keep the tempo correctly.".

In a case in which the creation activity performed by the user is performance of a musical instrument, the output from the communication terminal 100M as the device operation determined by the action determination unit 236 as the action of the communication terminal 100M may include analysis and evaluation of the musical instrument performance. For example, as illustrated in Fig. 49, the action determination unit 236 determines to output vocal sound "You can now play faithfully to musical scores." as the action of the communication terminal 100M.

According to the robot 100 and the communication terminal of the disclosure, an action of leading the user can be executed through autonomous processing. As a result, the robot 100 and the communication terminal can guide a user walking in a town that the user visits for the first time to an appropriate route. It is possible to guide a user who is interested in walking to a route along which the user has not passed before. A route along which the user cannot pass is detected in advance, and the user can be guided to another route. A user who gets lost and deviates from a specific route can be guided to an original route of the user. In a case in which a user is performing work at a position lower than the eyes of the user, this work can be supported.

The action determination unit 236 determines, by using at least one of the state of the user 10, the emotion of the user 10, the emotions of the robot 100 and the communication terminal, or the states of the robot 100 and the communication terminal, and the action determination model 221 at a predetermined timing, any of a plurality of types of robot actions and communication terminal actions including no action as actions of the robot 100 and the communication terminal. Here, a case in which a sentence generation model having an interaction function is used as the behavior determination model 221 will be described as an example.

Specifically, the action determination unit 236 inputs text representing at least one of the state of the user 10, the emotion of the user 10, the emotions of the robot 100 and the communication terminal, or the states of the robot 100 and the communication terminal, and text for inquiry about the robot action and the communication terminal action to the sentence generation model, and determines the actions of the robot 100 and the communication terminal on the basis of the output of the sentence generation model.

For example, the plurality of types of actions of the robot and the communication terminal include the following (1) to (24). The users in (1) to (24) include users working at positions lower than the eyes.
(1) The robot 100 and the communication terminal 100M do nothing.
(2) The robot 100 and the communication terminal 100M have a dream.
(3) The robot 100 and the communication terminal 100M talk to the user.
(4) The robot 100 and the communication terminal 100M create a picture diary.
(5) The robot 100 and the communication terminal 100M propose an activity.
(6) The robot 100 and the communication terminal 100M propose a person with whom the user should meet.
(7) The robot 100 and the communication terminal 100M introduce news in which the user is interested. (8) The robot 100 and the communication terminal 100M edit pictures and moving images.
(9) The robot 100 and the communication terminal 100M study with the user.
(10) The robot 100 and the communication terminal 100M evoke memories.
(11) The robot 100 and the communication terminal 100M may reproduce an image of a route along which the user can walk on the screen of the communication terminal 100M as the first action content of leading the user.
(12) The robot 100 and the communication terminal 100M may reproduce vocal sound related to a route along which the user can walk as the first action content of correcting the action of the user.
(13) The robot 100 and the communication terminal 100M may reproduce an image of a route different from the set guidance route on the screen of the communication terminal 100M as the first action content of correcting the action of the user.
(14) The robot 100 and the communication terminal 100M may reproduce vocal sound related to a route different from the set guidance route as the first action content of correcting the action of the user.
(15) The robot 100 and the communication terminal 100M may reproduce, as the first action content of correcting the action of the user, an image of a route for return to the set guidance route on the screen of the communication terminal 100M in a case in which the user led along the set guidance route deviates from the guidance route.
(16) The robot 100 and the communication terminal 100M may reproduce, as the first action content of correcting the action of the user, vocal sound related to a route for return to the set guidance route in a case in which the user led along the set guidance route deviates from the guidance route.
(17) The robot 100 and the communication terminal 100M may reproduce vocal sound praising the user for the action as the second action content or the fourth action content different from the first action content.
(18) The robot 100 and the communication terminal 100M may reproduce vocal sound expressing gratitude for the action of the user as the second action content or the fourth action content different from the first action content.
(19) The robot 100 and the communication terminal 100M may reproduce an image praising the user for the action as the second action content or the fourth action content different from the first action content.
(20) The robot 100 and the communication terminal 100M may reproduce an image expressing gratitude for the action of the user as the second action content or the fourth action content different from the first action content.
(21) The robot 100 and the communication terminal 100M may transmit specific information to a person other than the user as the third action content or the fourth action content different from the first action content.
(22) The robot 100 and the communication terminal 100M may reproduce sound that attracts the interest of the user as the third action content or the fourth action content different from the first action content.
(23) The robot 100 and the communication terminal 100M may reproduce an image that attracts the interest of the user as the third action content or the fourth action content different from the first action content.
(24) The robot 100 and the communication terminal 100M may reproduce at least one of vocal sound or an image that supports the work performed by the user as the fourth action content different from the first action content.

The action determination unit 236 inputs, to the sentence generation model, text representing the state of the user 10 and the state of the robot 100 or the communication terminal 100M recognized by the state recognition unit 230, the current emotion value of the user 10 and the current emotion value of the robot 100 or the communication terminal 100M determined by the emotion determination unit 232, and text for inquiry about any of a plurality of types of robot actions including no action each time a certain period of time elapses, and determines an action of the robot 100 or the communication terminal 100M on the basis of an output of the sentence generation model. Here, in a case in which the user 10 is absent around the robot 100 or the communication terminal 100M, the text to be input to the sentence generation model need not include the state of the user 10 and the current emotion value of the user 10, or may include the fact that the user 10 is absent. Hereinafter, the communication terminal 100M may be read as a robot. The communication terminal 100M may be interpreted as a smartphone or a smart AI device. The robot action may be read as a communication terminal action.

The action determination unit 236 may determine the first action content in a case in which the camera included in the sensor is directed in an advancing direction of the user. As a result, the communication terminal 100M can start leading the user only in a case in which the user desires to execute route guidance. The action determination unit 236 may determine the fourth action content in a case in which the camera included in the sensor is directed to the work place of the user. As a result, the communication terminal 100M can start to support the user only in a case in which the user is performing the work or in a case in which the user desires the support for the work.

The action determination unit 236 may perform, as the communication terminal action, the first action content in the above "(11)", that is, reproduce an image of a route along which the user can walk on the screen of the communication terminal 100M.

The action determination unit 236 may perform, as the communication terminal action, the first action content in the above "(12)", that is, reproduce vocal sound related to a route along which the user can walk.

The action determination unit 236 may perform, as the communication terminal action, the first action content in the above "(13)", that is, reproduce an image of a route different from the set guidance route as the first action content of correcting the action of the user on the screen of the communication terminal 100M.

The action determination unit 236 may perform, as the communication terminal action, the first action content in the above "(14)", that is, reproduce vocal sound related to a route different from the set guidance route.

The action determination unit 236 may perform, as the communication terminal action, the first action content in the above "(15)", that is, reproduce an image of a route for return to the set guidance route on the screen of the communication terminal 100M in a case in which the user led along the set guidance route deviates from the guidance route.

The action determination unit 236 may perform, as the communication terminal action, the first action content in the above "(16)", that is, reproduce vocal sound related to a route for return to the set guidance route in a case in which the user led along the set guidance route deviates from the guidance route.

The action determination unit 236 may execute the second action content or the fourth action content different from the first action content or the fourth action content. Specifically, the action determination unit 236 may perform, as the communication terminal action, the second action content or the fourth action content in the above "(17)", that is, reproduce vocal sound praising the user for the action.

The action determination unit 236 may perform, as the communication terminal action, the second action content or the fourth action content in the above "(18)", that is, reproduce vocal sound expressing gratitude for the action of the user.

The action determination unit 236 may perform, as the communication terminal action, the second action content or the fourth action content in the above "(19)", that is, reproduce an image praising the user for the action.

The action determination unit 236 may perform, as the communication terminal action, the second action content or the fourth action content in the above "(20)", that is, reproduce an image expressing gratitude for the action of the user.

The action determination unit 236 may execute the third action content or the fourth action content different from the first action content. Specifically, the action determination unit 236 may perform, as the communication terminal action, the third action content or the fourth action content in the above "(21)", that is, transmit the specific information to a person other than the user.

The action determination unit 236 may perform, as the movement system action, the third action content or the fourth action content in the above "(22)", that is, execute reproduction of sound that attracts the interest of the user.

The action determination unit 236 may perform, as the movement system action, the third action content or the fourth action content in the above "(23)", that is, execute reproduction of an image that attracts the interest of the user.

The action determination unit 236 may perform, as the communication terminal action, the fourth action content in the above "(24)" that is, execute reproduction of vocal sound or an image that supports work performed by the user.

In a case in which, for example, an image of a route along which the user can walk is reproduced as the first action content indicated in the above "(11)" to "(16)", the related information collection unit 270 may store data such as an image of a route along which the user can walk in the collected data 223.

In a case in which, for example, vocal sound praising the user for the action is reproduced as the second action content or the fourth action content indicated in the above "(17)" to "(20)", and "(24)", the related information collection unit 270 may store data such as an image of a route along which the user can walk in the collected data 223.

In a case in which, for example, specific information is transmitted to a person other than the user as the third action content or the fourth action content indicated in the above "(21)" to "(24)", the related information collection unit 270 may store data of the specific information or the like in the collected data 223.

In a case in which vocal sound or an image that supports the work is reproduced as the fourth action content indicated in "(24)" for the user performing the work, the related information collection unit 270 may store data of a tool, equipment, a material, and the like used by the user in the collected data 223.

The agent system 500 may be mounted on the communication terminal 100M (see Fig. 24). In this case, the action determination unit 236 of the agent system 500 may determine any of a plurality of actions of the agent for interacting with the user, including no action as the action of the electronic apparatus that is the communication terminal 100M (see Fig. 24).

### (1. Case in Which User Tends to Walk For a Long Time)

In a case in which there is the tendency, the agent itself asks the generative AI a question "What kind of guidance should be given to the user who takes such an action?". In a case in which the answer of the generative AI to this question is, for example, "Propose the same route as the route along which the user has previously passed", "Propose a route along which the user has not passed", "Propose a route with a relatively small number of vehicles such as bicycles", or "Propose a route with undulations", the agent may store the action of the user and the answer of the generative AI in association with each other.

### (2. Case in Which User Tends to Run for a Long Time)

In a case in which there is the tendency, the agent itself asks the generative AI a question "What kind of guidance should be given to the user who takes such an action?". In a case in which the answer of the generative AI to this question is, for example, "Provide guidance for encouraging hydration periodically", "Propose a route with fewer bicycles and clean air", or "Propose a route with fewer traffic lights", the agent may store the action of the user and the answer of the generative AI in association with each other.

### (3. Case in Which User Tends to Walk on Roadway)

In a case in which there is the tendency, the agent itself asks the generative AI a question "What kind of situation can occur to the user who takes such an action?". In a case in which the answer of the generative AI to this question is, for example, "There is a possibility of coming into contact with vehicles", "There is a possibility of coming into contact with bicycles", or "There is a possibility of entering an expressway interchange", the agent may store the user's action of walking on the roadway and the answer of the generative AI in association with each other. The agent may store action content of correcting the action in association with the answer.

The action content of correcting the action may include at least one of reproduction of vocal sound for correcting the dangerous action of the user or reproduction of an image for correcting the dangerous action of the user.

The vocal sound for correcting the dangerous action of the user may include vocal sound for guiding the user to a specific place. The specific place may include a place other than the place where the user is currently located, for example, a vicinity of the communication terminal 100M and a sidewalk. Specifically, the vocal sound may include vocal sound such as "Please stop walking on the roadway", "That is not a sidewalk", "The roadway is dangerous", and "Please move to the sidewalk immediately".

As described above, in the autonomous processing, a table in which the answer of the generative AI corresponding to the state or action of the user, the content of the state or action, and the action content of correcting the state or action are associated with each other may be recorded in a storage medium such as a memory.

In the autonomous processing, after the table is recorded, the action of the user may be autonomously or periodically detected, and the action schedule of the agent for attracting the attention of the user may be set on the basis of the detected action of the user and the content of the stored table. Specifically, the action determination unit 236 of the agent system 500 may set the first action content on the basis of the detected action of the user and the content of the stored table.

Specifically, the action determination unit 236 of the agent system 500 may spontaneously or periodically detect an action of the user on the basis of the sensor of the communication terminal 100M, and determine the first action content in the case of determining to lead the user as an action of the electronic apparatus on the basis of the detected action of the user and the specific information stored in advance.

### (First Action Content)

Hereinafter, an example of the first action content will be described.

The action determination unit 236 of the agent system 500 may execute reproduction of at least one of vocal sound or an image for assisting in walking of the user as the first action content for performing route guidance so that the walking user moves along a specific route. Specifically, as illustrated in Fig. 25, the action determination unit 236 may reproduce an image of a route along which the user can walk on the screen of the communication terminal 100M, and may reproduce vocal sound related to the route. The vocal sound may include, for example, "Turn right 10 m ahead".

The action determination unit 236 of the agent system 500 may execute reproduction of at least one of vocal sound or an image for showing a route different from the set guidance route. Specifically, as illustrated in Fig. 19, the action determination unit 236 may reproduce an image of a route different from the set guidance route on the screen of the communication terminal 100M, and may reproduce vocal sound related to the route. The vocal sound may include, for example, "The road is closed ahead. Would you like me to show another route?".

In a case in which the user being led along the set guidance route deviates from the guidance route, the action determination unit 236 of the agent system 500 may execute reproduction of at least one of vocal sound or an image for guiding the user along a path for returning the user to the guidance route. Specifically, as illustrated in Fig. 27, the action determination unit 236 may reproduce an image of the route for return to the set guidance route on the screen of the communication terminal 100M, and may reproduce vocal sound related to the route. The vocal sound may include "You have deviated from the route. I will show you the path for return to the original route." and the like. The action determination unit 236 of the agent system 500 may execute reproduction of at least one of the vocal sound or the image described above, for example, "I will guide you to the route along which you have previously passed", "Please rehydrate frequently", or "Please return to the sidewalk".

### (Second Action Content)

The action determination unit 236 of the agent system 500 may determine whether or not the action of the user has been corrected by detecting the action of the user while the movement unit 601 was leading the user or after the movement unit 601 reproduced at least one of vocal sound or an image that assists in the walking of the user, and may determine second action content different from the first action content in a case in which the action of the user has been corrected.

The case in which the action of the user has been corrected may be interpreted as a case in which the user has stopped a specific action and a specific behavior or a case in which a specific situation has been resolved as a result of execution of the operation of the agent according to the first action content. Specifically, the case in which the action of the user has been corrected may include a case in which the user has started walking along the proposed different route, a case in which the user has started walking along a path for return to the original route when the path was shown due to deviation from the original route, and the like.

The second action content may include reproduction of at least one of vocal sound praising the user for action, vocal sound expressing gratitude for the action of the user, an image praising the user for the action, or an image expressing gratitude for the action of the user.

Specifically, the vocal sound praising the user for the action may include, for example, "Well done! Excellent" as illustrated in Fig. 28. The vocal sound expressing gratitude for the action of the user may include vocal sound such as "Thank you for coming back" or "Thank you for selecting another route" as illustrated in Fig. 29. The image praising the user for the action may include an image of a character taking a good pose. The image expressing gratitude for the action of the user may include an image of a character who expresses gratitude.

### (Third Action Content)

The action determination unit 236 may determine whether or not the action of the user has been corrected by detecting the action of the user while the movement unit 601 was leading the user or after the movement unit 601 reproduced at least one of vocal sound or an image that assists in the walking of the user, and determine third action content different from the first action content in a case in which the action of the user has not been corrected.

The case in which the action of the user has not been corrected may be interpreted as a case in which the user has continued the dangerous action and behavior even though the operation of the agent according to the first action content was executed, a case in which the dangerous situation has not been resolved, or a case in which the user has started or continued an action different from the proposal of the agent.

Specifically, the case in which the action of the user has not been corrected may include a case in which the user has walked along a route different from the proposed different route without walking along the proposed different route, a case in which the user has neglected a path for return to the original route and continued walking when the path was shown due to deviation from the route, and the like.

The third action content may include at least one of transmission of specific information to a person other than the user, reproduction of sound that attracts an interest of the user, or reproduction of an image that attracts an interest of the user.

The transmission of the specific information to a person other than the user may include distribution of an email containing a warning message to a relative of the user, a spouse of the user, a guardian of the user, or the like. The transmission of the specific information to a person other than the user may include distribution of an image (a still image or a moving image) including the user and the surrounding scenery to a relative of the user, a spouse of the user, a guardian of the user, or the like. The transmission of the specific information to a person other than the user may include distribution of vocal sound having a warning message.

The reproduction of the sound that attracts the interest of the user may include specific music that the user likes, and may also include vocal sound such as "Please come back here" or "Let's take a safe road".

The reproduction of the image that attracts the interest of the user may include reproduction of an image of an animal kept by the user, an image of parents of the user, an image of a character of the animal, and the like.

### (Fourth Action Content)

In a case in which the user wearing the communication terminal 100M is performing work at a position lower than the eyes, the action determination unit 236 of the agent system 500 may determine the fourth action content of supporting the work for the user.

In this case, the device operation determined as the action of the communication terminal 100M by the action determination unit 236 of the agent system 500 includes recognizing content and a situation of the work being performed by the user by the communication terminal 100M reproducing at least one of vocal sound or an image and setting the fourth action content of supporting the work.

For example, as illustrated in Fig. 64, the action determination unit 236 of the agent system 500 determines, as an action of the communication terminal 100M, to output vocal sound such as "Attach the lid to the housing.". The action determination unit 236 may determine, as the action of the communication terminal 100M, to output vocal sound that notifies the user of more specifically what attachment method to use for attachment. For example, as illustrated in Fig. 65, the action determination unit 236 determines to output vocal sound "Attach the lid to the housing to close the opening by using bolts and nuts." as the action of the communication terminal 100M. In a case in which a tool, an instrument, or an implement to be used is set according to the attachment method, the type of the tool, the instrument, or the implement to be used may also be included in the output content using vocal sound.

In a case in which the user uses a tool, an instrument, or an implement, the action determination unit 236 of the agent system 500 may determine, as the action of the communication terminal 100M, to output advice about a method of using the tool, the instrument, or the implement. In this case, the camera included in the communication terminal 100M may recognize the type of the tool, the instrument, or the implement used by the user, capture an image of work content and a work situation, and set advice about a use method on the basis of the captured image. The user's actual use of a tool, an instrument, or an implement may be monitored, and advice about more appropriate use may be given.

The work performed on the work target device by the user may be completed with single work content, or may be sequentially performed with a plurality of pieces of work content. In a case in which the work performed by the user on the work target device includes a plurality of pieces of work content, the action determination unit 236 of the agent system 500 may determine, as the action of the communication terminal 100M, to output vocal sound that conveys a work procedure to the user. In this case, the progress status of the work is detected by the camera of the communication terminal 100M, and after the end of one work is detected, vocal sound output for supporting the next work may be emitted.

In a case in which the work that the user is performing is delayed, the action determination unit 236 of the agent system 500 includes supporting the work of the user by replacing the action content of supporting the work of the user with content different from the output content of the vocal sound so far. For example, as illustrated in Fig. 66, the action determination unit 236 may determine to output vocal sound "The upper side of the black box is wide open. Cover the open portion with a white plate and attach the white plate to the black plate with bolts and nuts." as the action of the communication terminal 100M.

In a case in which the work performed by the user is wrong, the action determination unit 236 of the agent system 500 includes supporting the work of the user by replacing the action content of stopping the work of the user with the content different from the output content of the vocal sound so far. For example, as illustrated in Fig. 68, the action determination unit 236 may determine to output vocal sound "The red box is not a box to attach a lid to. Attach a white lid to the black box." as the action of the communication terminal 100M.

In a case in which the user is performing disassembly work, inspection work, cleaning work, repair work, and the like of the work target device, the action determination unit 236 of the agent system 500 determines the fourth action content of supporting the work for the user wearing the communication terminal 100M. For example, in a case in which the work is disassembly work for the work target device, the action determination unit 236 determines to output vocal sound such as "Detach the lid from the housing." as illustrated in Fig. 68 as an action of the communication terminal 100M.

In a case in which the user is performing work on the work target device, the output from the communication terminal 100M as the device operation determined by the action determination unit 236 of the agent system 500 as the action of the communication terminal 100M may include analysis and evaluation on the work content. For example, in a case in which the time required by the user for specific work becomes short, for example, the action determination unit 236 may determine, as the action of the communication terminal 100M, to output vocal sound "Wasted work is reduced and accurate assembly can be performed in a short time." as illustrated in Fig. 69. As described above, highly evaluating the work of the user is to bring the emotion of the user closer to "pleased", and as a result, supports the work of the user. As illustrated in Fig. 70, the action determination unit 236 may determine, as the action of the communication terminal 100M, to output vocal sound such as "Work is being continued for a long time. Please take a break.". As described above, calling some kind of attention or the like to the user enhances work efficiency or avoids danger, and as a result, supports the work of the user.

In a case in which the work performed by the user is a creation activity, the action determination unit 236 of the agent system 500 determines action content of supporting the work of the creation activity as an action of the communication terminal 100M. For example, in a case in which the creation activity is production of a painting, monitoring the production status with a camera of the communication terminal 100M and giving advice about a specific technique or the like are included. For example, as illustrated in Fig. 71, the action determination unit 236 of the agent system 500 determines to output vocal sound "When drawing fine lines, please be careful with the brush tip." as the action of the communication terminal 100M. As another example, the action determination unit 236 of the agent system 500 may determine to output vocal sound "There are many objects to draw, so please draw with attention to the order." as illustrated in Fig. 72. As still another example, as illustrated in Fig. 73, the action determination unit 236 of the agent system 500 may determine to output vocal sound "Please color while paying attention to the balance of hue.".

In a case in which the creation activity performed by the user is performance of a musical instrument, monitoring the performance with the camera of the communication terminal 100M and giving advice about a specific performance method or the like are included. For example, as illustrated in Fig. 74, the action determination unit 236 of the agent system 500 determines to output vocal sound "Please use your fingers smoothly." as the action of the communication terminal 100M. As still another example, as illustrated in Fig. 75, the action determination unit 236 of the agent system 500 may determine to output vocal sound "Please keep the tempo correctly.".

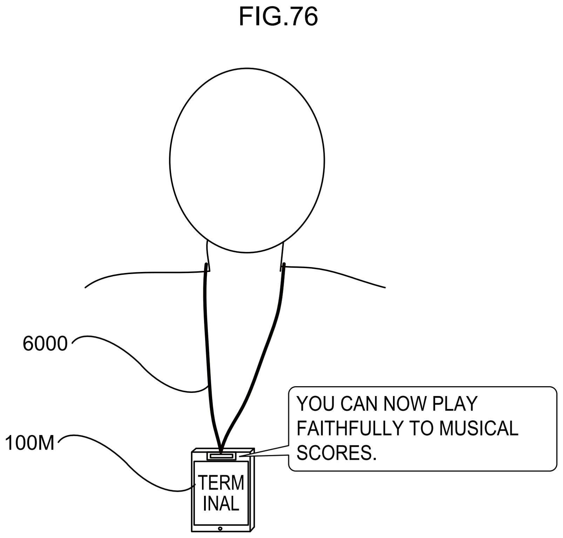

In a case in which the creation activity performed by the user is performance of a musical instrument, the output from the communication terminal 100M as the device operation determined by the action determination unit 236 of the agent system 500 as the action of the communication terminal 100M may include analysis and evaluation of the musical instrument performance. For example, as illustrated in Fig. 76, the action determination unit 236 determines to output vocal sound "You can now play faithfully to musical scores." as the action of the communication terminal 100M.

According to the agent system 500 of the disclosure, an action of leading the user can be executed through autonomous processing. As a result, the agent can guide the user walking in a town that the user visits for the first time to an appropriate route. It is possible to guide a user who is interested in walking to a route along which the user has not passed before. A route along which the user cannot pass is detected in advance, and the user can be guided to another route. A user who gets lost and deviates from a specific route can be guided to an original route of the user.

The action determination unit 236 determines, by using at least one of the state of the user 10, the emotion of the user 10, the emotions of the robot 100 and the communication terminal, or the states of the robot 100 and the communication terminal, and the action determination model 221 at a predetermined timing, any of a plurality of types of robot actions and communication terminal actions including no action as an action of the agent. Here, a case in which a sentence generation model having an interaction function is used as the action determination model 221 will be described as an example.

Specifically, the action determination unit 236 inputs text representing at least one of the state of the user 10, the emotion of the user 10, the emotion of the agent, or the state of the agent and text for inquiry about the agent action to the sentence generation model, and determines the action of the agent on the basis of the output of the sentence generation model.

For example, the plurality of types of agent actions include the following (1) to (24). The users in (1) to (24) include users working at positions lower than the eyes.
(1) The agent does nothing.
(2) The agent has a dream.
(3) The agent speaks to the user.
(4) The agent creates a picture diary.
(5) The agent proposes an activity.
(6) The agent proposes a person with whom the user should meet.
(7) The agent introduces news in which the user is interested.
(8) The agent edits pictures and moving images.
(9) The agent studies with the user.
(10) The agent evokes memories.
(11) The agent may reproduce an image of a route along which the user can walk on the screen of the communication terminal as the first action content of leading the user.
(12) The agent may reproduce vocal sound related to a route along which the user can walk as the first action content of correcting the action of the user.
(13) The agent may reproduce an image of a route different from the set guidance route on the screen of the communication terminal 100M as the first action content of correcting the action of the user.
(14) The agent may reproduce vocal sound related to a route different from the set guidance route as the first action content of correcting the action of the user.
(15) The agent may reproduce, as the first action content of correcting the action of the user, an image of a route for return to the set guidance route on the screen of the communication terminal 100M in a case in which the user led along the set guidance route deviates from the guidance route.
(16) The agent may reproduce, as the first action content of correcting the action of the user, vocal sound related to a route for return to the set guidance route in a case in which the user led along the set guidance route deviates from the guidance route.
(17) The agent may reproduce vocal sound praising the user for the action as the second action content or the fourth action content different from the first action content.
(18) The agent may reproduce vocal sound expressing gratitude for the action of the user as the second action content or the fourth action content different from the first action content.
(19) The agent may reproduce an image praising the user for the action as the second action content or the fourth action content different from the first action content.
(20) The agent may reproduce an image expressing gratitude for the action of the user as the second action content or the fourth action content different from the first action content.
(21) The agent may transmit specific information to a person other than the user as the third action content or the fourth action content different from the first action content.
(22) The agent may reproduce sound that attracts the interest of the user as the third action content or the fourth action content different from the first action content.
(23) The agent may reproduce an image that attracts the interest of the user as the third action content or the fourth action content different from the first action content.
(24) The agent may reproduce at least one of vocal sound or an image that supports the work performed by the user as the fourth action content different from the first action content.

The action determination unit 236 may spontaneously or periodically detect the action of the user on the basis of the sensor, and in a case in which it is determined to lead the user as the action of the agent on the basis of the detected action of the user and the specific information stored in advance, execute the following first action content.

The action determination unit 236 of the agent system 500 may perform, as the agent action, the first action content in the above "(11)", that is, reproduce an image of a route along which the user can walk on the screen of the communication terminal 100M.

The action determination unit 236 of the agent system 500 may perform, as the agent action, the first action content in the above "(12)", that is, reproduce vocal sound related to a route along which the user can walk.

The action determination unit 236 of the agent system 500 may perform, as the agent action, the first action content in the above "(13)", that is, reproduce an image of a route different from the set guidance route as the first action content of correcting the action of the user on the screen of the communication terminal 100M.

The action determination unit 236 of the agent system 500 may perform, as the agent action, the first action content in the above "(14)", that is, reproduce vocal sound related to a route different from the set guidance route.

The action determination unit 236 of the agent system 500 may perform, as the agent action, the first action content in the above "(15)", that is, reproduce an image of a route for return to the set guidance route on the screen of the communication terminal 100M in a case in which the user led along the set guidance route deviates from the guidance route.

The action determination unit 236 of the agent system 500 may perform, as the agent action, the first action content in the above "(16)", that is, reproduce vocal sound related to a route for return to the set guidance route in a case in which the user led along the set guidance route deviates from the guidance route.

The action determination unit 236 of the agent system 500 may execute the second action content or the fourth action content different from the first action content or the first action content. Specifically, the action determination unit 236 may perform, as the agent action, the second action content or the fourth action content in the above "(17)", that is, reproduce vocal sound praising the user for the action.

The action determination unit 236 of the agent system 500 may perform, as the agent action, the second action content or the fourth action content in the above "(18)", that is, reproduce vocal sound expressing gratitude for the action of the user.

The action determination unit 236 of the agent system 500 may perform, as the agent action, the second action content or the fourth action content in the above "(19)", that is, reproduce an image praising the user for the action.

The action determination unit 236 of the agent system 500 may perform, as the agent action, the second action content or the fourth action content in the above "(20)", that is, reproduce an image expressing gratitude for the action of the user.

The action determination unit 236 of the agent system 500 may execute the third action content different from the first action content or the fourth action content. Specifically, the action determination unit 236 may perform, as the agent action, the third action content or the fourth action content in the above "(21)", that is, transmit the specific information to a person other than the user.

The action determination unit 236 of the agent system 500 may perform, as the agent action, the third action content or the fourth action content in the above "(22)", that is, execute reproduction of sound that attracts the interest of the user.

The action determination unit 236 of the agent system 500 may perform, as the agent action, the third action content or the fourth action content in the above "(23)", that is, execute reproduction of an image that attracts the interest of the user.

The action determination unit 236 of the agent system 500 may perform, as the communication terminal action, the fourth action content in the above "(24)", that is, execute reproduction of vocal sound or an image that supports the work performed by the user.

In a case in which, for example, an image of a route along which the user can walk is reproduced as the first action content indicated in the above "(11)" to "(16)", the related information collection unit 270 may store data such as an image of a route along which the user can walk in the collected data 223.

In a case in which, for example, vocal sound praising the user for the action is reproduced as the second action content or the fourth action content indicated in the above "(17)" to "(20)", and "(24)", the related information collection unit 270 may store data such as an image of a route along which the user can walk in the collected data 223.

In a case in which, for example, specific information is transmitted to a person other than the user as the third action content or the fourth action content indicated in the above "(21)" to "(24)", the related information collection unit 270 of the agent system 500 may store data of the specific information or the like in the collected data 223.

In a case in which vocal sound or an image that supports the work is reproduced as the fourth action content indicated in "(24)" for the user performing the work, the related information collection unit 270 of the agent system 500 may store data of a tool, an instrument, an implement, and the like used by the user in the collected data 223.

A part of the agent system 500 (for example, the sensor module unit 210, the storage unit 220, and the control unit 228B) may be provided outside a communication terminal such as a smartphone possessed by the user (for example, a server), and the communication terminal may function as each unit of the agent system 500 by communicating with the outside.

### (Supplementary Note 1)

A control system including:
a state recognition unit that recognizes a user state including an action of a user and a state of an electronic apparatus;
an emotion determination unit that determines an emotion of the user or an emotion of the electronic apparatus; and
an action determination unit that determines, by using at least one of the user state, the state of the electronic apparatus, the emotion of the user, or the emotion of the electronic apparatus, and an action determination model at a predetermined timing, any of a plurality of types of device operations including non-operation as an action of the electronic apparatus, in which
the electronic apparatus detachably provided on a neck strap includes one or a plurality of sensors that collect information, and
the device operations include setting action content of supporting work performed by the user at a position lower than eyes of the user by the electronic apparatus reproducing at least one of vocal sound or an image generated by the action determination model on the basis of the information collected by the electronic apparatus.

### (Supplementary Note 2)

The control system according to Supplementary Note 1, in which the action determination unit determines the action content of giving advice about a work procedure for work performed by the user on a work target device.

### (Supplementary Note 3)

The control system according to Supplementary Note 2, in which the action determination unit determines the action content of giving advice about assembly work or disassembly work for the work target device as the work.

### (Supplementary Note 4)

The control system according to Supplementary Note 1, in which the action determination unit determines the action content of giving advice about a work procedure for work performed by the user on a creation activity.

### (Supplementary Note 5)

The control system according to Supplementary Note 4, in which the action determination unit determines the action content of giving advice about a creation activity of an art work as the creation activity.

### (Supplementary Note 6)

The control system according to Supplementary Note 1, in which the sensor detects a status of the work performed by the user.

### (Supplementary Note 7)

The control system according to Supplementary Note 1, in which the sensor detects a tool or an implement used by the user.

### (Supplementary Note 8)

The control system according to Supplementary Note 1, in which the action content includes reproduction of at least one of vocal sound praising the user for the action, vocal sound expressing gratitude for the action of the user, an image praising the user for the action, and an image expressing gratitude for the action of the user.

### (Supplementary Note 9)

The control system according to Supplementary Note 1, in which the action determination unit determines whether or not the action of the user has been corrected by detecting the action of the user after supporting the work for the user, and determines action content different from previous action content in a case in which the action of the user has not been corrected.

### [Thirteenth Embodiment]

Fig. 24 is an external view of the communication terminal according to the embodiment of the disclosure. As illustrated in Fig. 24, the communication terminal 100M may be interpreted as an electronic apparatus detachably provided on a neck strap 6000. The communication terminal 100M may be interpreted as a smartphone that will be described later, or may be interpreted as a smart AI device. The communication terminal 100M may have the functions of the robots 100 to 102 described above. The neck strap 6000 may include a hook-shaped member that is locked to the communication terminal 100M.

### (First Action Content)

The first action content may include an action of the communication terminal 100M that leads the user when the communication terminal 100M reproduces at least one of vocal sound or an image. Hereinafter, an example of the first action content will be described.

The action determination unit 236 may execute reproduction of at least one of vocal sound or an image for assisting in walking of the user as the first action content for performing route guidance so that the walking user moves along a specific route. Specifically, in a case in which it is detected that the communication terminal 100M is attached to the neck strap 6000, the action determination unit 236 may spontaneously search for a place where the user tends to go regularly and guide the user along a route to the place that has been searched for. In a case in which it is detected that the camera included in the sensor of the communication terminal 100M is directed in an advancing direction of the user, the action determination unit 236 may spontaneously search for a place where the user tends to go regularly and guide the user along a route to the place that has been searched for. The action determination unit 236 may reproduce vocal sound related to guidance of a route along which the user can walk. The vocal sound may include "I searched for a place where Mr. A frequently goes at this time. I will start guiding.", "Turn right 10 m ahead" illustrated in Fig. 24, and the like. The action determination unit 236 may reproduce an image related to guidance of a route along which the user can walk on the screen of the communication terminal 100M.

The action determination unit 236 may execute reproduction of at least one of vocal sound and an image for guiding a route different from the set guidance route. Specifically, the action determination unit 236 may spontaneously re-search for a place where the user tends to go regularly, including the traffic condition of the set guidance route, the climate near the route, the current time, and the like, and reproduce vocal sound or the like related to a route to the place that has been re-searched for, that is, a route different from the set guidance route. More specifically, the vocal sound may include "I searched for another route because of the heavy traffic today. Would you like me to show another route?", "The road is closed ahead. Would you like me to show another route?" as illustrated in Fig. 26, and the like.

In a case in which the user led along the set guidance route deviates from the guidance route, the action determination unit 236 may spontaneously search for a path for returning the user to the guidance route and execute reproduction of at least one of vocal sound or an image for guiding the user to the path that has been searched for. Specifically, the action determination unit 236 may spontaneously re-search for a place where the user can return, including the traffic condition of a path deviating from the guidance route, the climate near the path, the current time, events that have occurred in the past near the route, and the like, and reproduce vocal sound related to the route to the re-searched place. That is, the vocal sound related to the path for return to the set guidance route may be reproduced. More specifically, the vocal sound may include "Since there are few people and it is dangerous, I searched for a path for return to the original route.", "It started to rain. It seems to be an area where a mudslide occurred before. Do you want to return to the original road?", "You have deviated from the route. I will show you the path for return to the original route." as illustrated in Fig. 27, and the like. The action determination unit 236 of the robot 100 and the communication terminal may execute reproduction of at least one of vocal sound such as the above vocal sound, for example, "I will guide you to the route along which you have previously passed", "Please rehydrate frequently", and "Please return to the sidewalk" or an image.

### (Second Action Content)

The action determination unit 236 may determine whether or not the action of the user has been corrected by detecting the action of the user while the communication terminal 100M was leading the user or after the communication terminal 100M reproduced at least one of vocal sound or an image that assists in the walking of the user, and may determine second action content different from the first action content in a case in which the action of the user has been corrected.

The case in which the action of the user has been corrected may be interpreted as a case in which the user has stopped a specific action and a specific behavior or a case in which a specific situation has been resolved as a result of execution of the operation of the communication terminal 100M according to the first action content. Specifically, the case in which the action of the user has been corrected may include a case in which the user has started walking along the proposed different route, a case in which the user has started walking along a path for return to the original route when the path was shown due to deviation from the original route, and the like.

The second action content may include reproduction of at least one of vocal sound praising the user for action, vocal sound expressing gratitude for the action of the user, an image praising the user for the action, or an image expressing gratitude for the action of the user.

Specifically, the vocal sound praising the user for the action may include, for example, "Well done! Excellent" as illustrated in Fig. 28. The vocal sound expressing gratitude for the action of the user may include vocal sound such as "Thank you for coming back" or "Thank you for selecting another route" as illustrated in Fig. 29. The image praising the user for the action may include an image of a character taking a good pose. The image expressing gratitude for the action of the user may include an image of a character who expresses gratitude.

### (Third Action Content)

The action determination unit 236 may determine whether or not the action of the user has been corrected by detecting the action of the user while the communication terminal 100M was leading the user or after the communication terminal 100M reproduced at least one of vocal sound or an image that assists in the walking of the user, and determine third action content different from the first action content in a case in which the action of the user has not been corrected.

The case in which the action of the user has not been corrected may be interpreted as a case in which the user has continued the dangerous action and behavior even though the operation of the communication terminal 100M according to the first action content was executed, a case in which the dangerous situation has not been resolved, or a case in which the user has started or continued an action different from the proposal of the communication terminal 100M.

Specifically, the case in which the action of the user has not been corrected may include a case in which the user has walked along a route different from the proposed different route without walking along the proposed different route, a case in which the user has neglected a path for return to the original route and has continued walking when the path was shown due to deviation from the route, and the like.

The third action content may include at least one of transmission of specific information to a person other than the user, reproduction of sound that attracts an interest of the user, or reproduction of an image that attracts an interest of the user.

The transmission of the specific information to a person other than the user may include distribution of an email containing a warning message to a relative of the user, a spouse of the user, a guardian of the user, or the like. The transmission of the specific information to a person other than the user may include distribution of an image (a still image or a moving image) including the user and the surrounding scenery to a relative of the user, a spouse of the user, a guardian of the user, or the like. The transmission of the specific information to a person other than the user may include distribution of vocal sound having a warning message.

The reproduction of the sound that attracts the interest of the user may include specific music that the user likes, and may also include vocal sound such as "Please come back here" or "Let's take a safe road".

The reproduction of the image that attracts the interest of the user may include reproduction of an image of an animal kept by the user, an image of parents of the user, an image of a character of the animal, and the like.

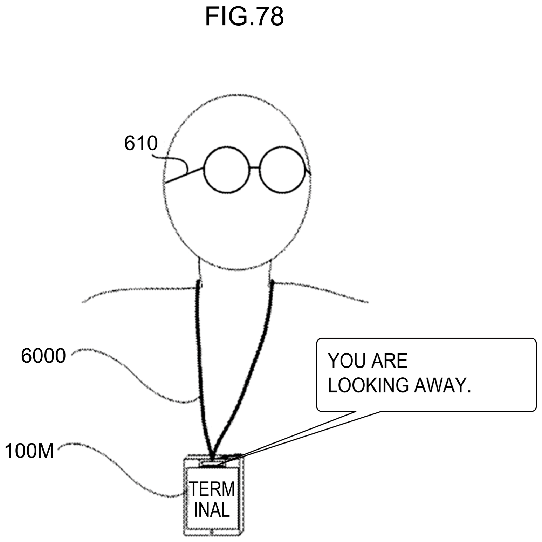

As illustrated in Fig. 78, the user may wear smart glasses 610 as a wearable terminal in a state in which the communication terminal 100M is worn on the neck strap 6000, and the communication terminal 100M and the smart glasses 610 may be linked. The smart glasses 610 may be interpreted as an electronic apparatus different from the communication terminal 100M. A configuration of the smart glasses 610 may be the same as the configuration of the smart glasses 720. The smart glasses 610 and the communication terminal 100M are connected via a wireless communication line such as Bluetooth (registered trademark). Through this connection, the communication terminal 100M and the smart glasses 610 are linked to each other. Specifically, the communication terminal 100M may receive information collected by the smart glasses 610, and may reproduce at least one of vocal sound or an image for supporting the user by the linked smart glasses 610 according to a situation to notify the user. That is, in the first action content, the second action content, and the third action content, at least one of the image or the vocal sound reproduced by the communication terminal 100M may be reproduced by the smart glasses 610 linked to the communication terminal 100M. By linking the communication terminal 100M and the smart glasses 610 as described above, for example, it is possible to provide better support to the user by using image information at a high position acquired from the smart glasses 610. It is easy to ascertain that the user is supported by reproducing at least one of the image and the vocal sound for supporting the user instead of the communication terminal 100M with the smart glasses 610 closer to the user's sensory organ (for example, vision and hearing).

### (Fourth Action Content)

In a case in which the user wears the communication terminal 100M and the smart glasses 610 linked to the communication terminal 100M, the action determination unit 236 may determine the fourth action content of supporting an operation of the user.

In this case, the device operations determined as the action of the communication terminal 100M by the action determination unit 236 include setting action content of supporting the user by the communication terminal 100M reproducing at least one of vocal sound or an image generated by the action determination model on the basis of the information collected by the communication terminal 100M and the information collected by the smart glasses 610. The communication terminal 100M reproducing at least one of the vocal sound or the image includes the communication terminal 100M causing the linked communication terminal 100M to reproduce at least one of the vocal sound or the image.

The action determination unit 236 determines action content of supporting the user on the basis of the information acquired by the camera of the communication terminal 100M and the information acquired by the camera (2D camera 203) of the smart glasses 610. The information acquired by each camera is image information. The image information may be a still image or a moving image.

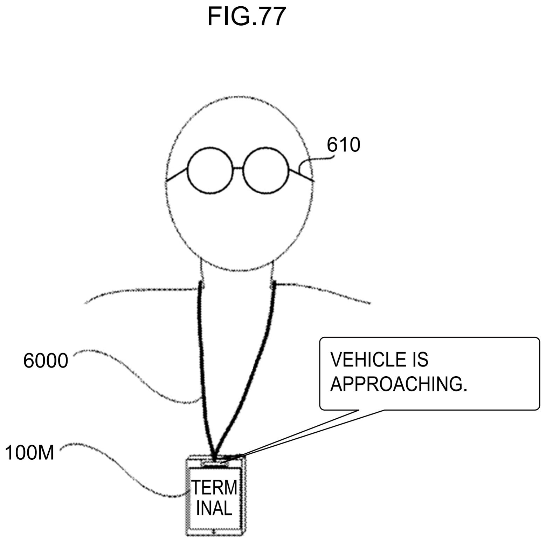

For example, in a case in which a moving object (for example, a vehicle) approaching the user is found in the image information acquired by the camera of the communication terminal 100M and the camera of the smart glasses 610 located at a position higher than the communication terminal 100M, the action determination unit 236 determines to output vocal sound "A vehicle is approaching." as the action of the communication terminal 100M as illustrated in Fig. 77. The action determination unit 236 determines, as the action of the communication terminal 100M, to output vocal sound "A vehicle is approaching. Please be careful." according to a distance between the user and the vehicle estimated from the image information. The action determination unit 236 determines, as the action of the communication terminal 100M, to output vocal sound "The vehicle is approaching at o km/h. Please move to the left." according to a speed of the vehicle estimated from the image information.

In a case in which the orientation of the camera of the communication terminal 100M is different from the orientation of the camera of the smart glasses 610, the action determination unit 236 determines action content of warning the user about looking away. For example, in a case in which the orientation of the camera of the communication terminal 100M and the orientation of the camera of the smart glasses 610 are different while the user is moving, the action determination unit 236 determines that there is a possibility that the user is looking away, and determines to output vocal sound "You are looking away." as the action of the communication terminal 100M as illustrated in Fig. 78. In a case in which the looking-away state continues for a predetermined time, the action determination unit 236 determines to output vocal sound "It is dangerous to look away." as the action of the communication terminal 100M. For example, in a case in which a moving object (for example, a vehicle) approaching the user is found in the image information acquired by the camera of the communication terminal 100M, the action determination unit 236 determines to output vocal sound "The vehicle is approaching. Please look in front." as the action of the communication terminal 100M.

According to the robot 100 and the communication terminal of the disclosure, an action of leading the user can be executed through autonomous processing. As a result, the robot 100 and the communication terminal can guide a user walking in a town that the user visits for the first time to an appropriate route. It is possible to guide a user who is interested in walking to a route along which the user has not passed before. A route along which the user cannot pass is detected in advance, and the user can be guided to another route. A user who gets lost and deviates from a specific route can be guided to an original route of the user. The linking between the communication terminal and the smart glasses 610 can accurately support the user.

The action determination unit 236 determines, by using at least one of the state of the user 10, the emotion of the user 10, the emotions of the robot 100 and the communication terminal, or the states of the robot 100 and the communication terminal, and the action determination model 221 at a predetermined timing, any of a plurality of types of robot actions and communication terminal actions including no action as actions of the robot 100 and the communication terminal. Here, a case in which a sentence generation model having an interaction function is used as the action determination model 221 will be described as an example.

Specifically, the action determination unit 236 inputs text representing at least one of the state of the user 10, the emotion of the user 10, the emotions of the robot 100 and the communication terminal, or the states of the robot 100 and the communication terminal, and text for inquiry about the robot action and the communication terminal action to the sentence generation model, and determines the actions of the robot 100 and the communication terminal on the basis of the output of the sentence generation model.

For example, the plurality of types of actions of the robot and the communication terminal include the following (1) to (24).
(1) The robot 100 and the communication terminal 100M do nothing.
(2) The robot 100 and the communication terminal 100M have a dream.
(3) The robot 100 and the communication terminal 100M talk to the user.
(4) The robot 100 and the communication terminal 100M create a picture diary.
(5) The robot 100 and the communication terminal 100M propose an activity.
(6) The robot 100 and the communication terminal 100M propose a person with whom the user should meet.
(7) The robot 100 and the communication terminal 100M introduce news in which the user is interested. (8) The robot 100 and the communication terminal 100M edit pictures and moving images.
(9) The robot 100 and the communication terminal 100M study with the user.
(10) The robot 100 and the communication terminal 100M evoke memories.
(11) The robot 100 and the communication terminal 100M may reproduce an image of a route along which the user can walk on the screen of the communication terminal 100M as the first action content of leading the user.
(12) The robot 100 and the communication terminal 100M may reproduce vocal sound related to a route along which the user can walk as the first action content of correcting the action of the user.
(13) The robot 100 and the communication terminal 100M may reproduce an image of a route different from the set guidance route on the screen of the communication terminal 100M as the first action content of correcting the action of the user.
(14) The robot 100 and the communication terminal 100M may reproduce vocal sound related to a route different from the set guidance route as the first action content of correcting the action of the user.
(15) The robot 100 and the communication terminal 100M may reproduce, as the first action content of correcting the action of the user, an image of a route for return to the set guidance route on the screen of the communication terminal 100M in a case in which the user led along the set guidance route deviates from the guidance route.
(16) The robot 100 and the communication terminal 100M may reproduce, as the first action content of correcting the action of the user, vocal sound related to a route for return to the set guidance route in a case in which the user led along the set guidance route deviates from the guidance route.
(17) The robot 100 and the communication terminal 100M may reproduce vocal sound praising the user for the action as the second action content different from the first action content.
(18) The robot 100 and the communication terminal 100M may reproduce vocal sound expressing gratitude for the action of the user as the second action content different from the first action content.
(19) The robot 100 and the communication terminal 100M may reproduce an image praising the user for the action as the second action content different from the first action content.
(20) The robot 100 and the communication terminal 100M may reproduce an image expressing gratitude for the action of the user as the second action content different from the first action content.
(21) The robot 100 and the communication terminal 100M may transmit specific information to a person other than the user as the third action content different from the first action content.
(22) The robot 100 and the communication terminal 100M may reproduce sound that attracts the interest of the user as the third action content different from the first action content.
(23) The robot 100 and the communication terminal 100M may reproduce an image that attracts the interest of the user as the third action content different from the first action content.
(24) The robot 100 and the communication terminal 100M may reproduce vocal sound that gives a warning to the user as the fourth action content. The actions in (1) to (24) may be reproduced in the smart glasses 610 linked to the communication terminal 100M.

The action determination unit 236 inputs, to the sentence generation model, text representing the state of the user 10 and the state of the robot 100 or the communication terminal 100M recognized by the state recognition unit 230, and the current emotion value of the user 10 and the current emotion value of the robot 100 or the current emotion value of the communication terminal 100M determined by the emotion determination unit 232, and text for inquiry about any of a plurality of types of robot actions including no action, each time a certain period of time elapses, and determines actions of the robot 100 and the communication terminal 100M on the basis of an output of the sentence generation model. Here, in a case in which the user 10 is absent around the robot 100 or the communication terminal 100M, the text to be input to the sentence generation model need not include the state of the user 10 and the current emotion value of the user 10, or may include the fact that the user 10 is absent. Hereinafter, the communication terminal 100M may be read as a robot. The communication terminal 100M may be interpreted as a smartphone or a smart AI device. The robot action may be read as a communication terminal action.

The action determination unit 236 may determine the first action content in a case in which the camera included in the sensor is directed in an advancing direction of the user. As a result, the communication terminal 100M can start leading the user only in a case in which the user desires to execute route guidance.

The action determination unit 236 may perform, as the communication terminal action, the first action content in the above "(11)", that is, reproduce an image of a route along which the user can walk on the screen of the communication terminal 100M.

The action determination unit 236 may perform, as the communication terminal action, the first action content in the above "(12)", that is, reproduce vocal sound related to a route along which the user can walk.

The action determination unit 236 may perform, as the communication terminal action, the first action content in the above "(13)", that is, reproduce an image of a route different from the set guidance route as the first action content of correcting the action of the user on the screen of the communication terminal 100M.

The action determination unit 236 may perform, as the communication terminal action, the first action content in the above "(14)", that is, reproduce vocal sound related to a route different from the set guidance route.

The action determination unit 236 may perform, as the communication terminal action, the first action content in the above "(15)", that is, reproduce an image of a route for return to the set guidance route on the screen of the communication terminal 100M in a case in which the user led along the set guidance route deviates from the guidance route.

The action determination unit 236 may perform, as the communication terminal action, the first action content in the above "(16)", that is, reproduce vocal sound related to a route for return to the set guidance route in a case in which the user led along the set guidance route deviates from the guidance route.

The action determination unit 236 may execute the second action content different from the first action content. Specifically, the action determination unit 236 may perform, as the communication terminal action, the second action content in the above "(17)", that is, reproduce vocal sound praising the user for the action.

The action determination unit 236 may perform, as the communication terminal action, the second action content in the above "(18)", that is, reproduce vocal sound expressing gratitude for the action of the user.

The action determination unit 236 may perform, as the communication terminal action, the second action content in the above "(19)", that is, reproduce an image praising the user for the action.

The action determination unit 236 may perform, as the communication terminal action, the second action content in the above "(20)", that is, reproduce an image expressing gratitude for the action of the user.

The action determination unit 236 may execute the third action content different from the first action content. Specifically, the action determination unit 236 may perform, as the communication terminal action, the third action content in the above "(21)", that is, transmit the specific information to a person other than the user.

The action determination unit 236 may perform, as the communication terminal action, the third action content in the above "(22)", that is, execute reproduction of sound that attracts the interest of the user.

The action determination unit 236 may perform, as the communication terminal action, the third action content in the above "(23)", that is, execute reproduction of an image that attracts the interest of the user.

The action determination unit 236 may perform, as the communication terminal action, the fourth action content in the above "(24)" that is, execute reproduction of vocal sound for giving a warning to the user.

In a case in which, for example, an image of a route along which the user can walk is reproduced as the first action content indicated in the above "(11)" to "(16)", the related information collection unit 270 may store data such as an image of a route along which the user can walk in the collected data 223.

In a case in which, for example, vocal sound praising the user for the action is reproduced as the second action content indicated in the above "(17)" to "(20)", the related information collection unit 270 may store data such as an image of a route along which the user can walk in the collected data 223.

In a case in which, for example, specific information is transmitted to a person other than the user as the third action content indicated in the above "(21)" to "(23)", the related information collection unit 270 of the agent system 500 may store data of the specific information or the like in the collected data 223.

In a case in which a warning is given to the user as the fourth action content indicated in the above "(24)", the related information collection unit 270 may store data regarding a situation in which the warning is given, and the like in the collected data 223. The situation in which the warning is given includes, for example, a looking-away time and a distance to a vehicle.

### (First Action Content)

Hereinafter, an example of the first action content will be described.

The first action content may include an action of the agent that leads the user by controlling the wheels 601a of the movement unit 601. Controlling the wheels 601a may include changing at least one of a rotation direction, a rotation speed, or a steering direction of the wheels 601a. Hereinafter, an example of the first action content will be described.

The action determination unit 236 of the agent system 500 may execute reproduction of at least one of vocal sound or an image for assisting in walking of the user as the first action content for performing route guidance so that the walking user moves along a specific route. Specifically, as illustrated in Fig. 25, the action determination unit 236 may reproduce an image of a route along which the user can walk on the screen of the communication terminal 100M, and may reproduce vocal sound related to the route. The vocal sound may include, for example, "Turn right 10 m ahead".

The action determination unit 236 may execute reproduction of at least one of vocal sound or an image for guiding the user along a route different from the set guidance route. Specifically, as illustrated in Fig. 26, the action determination unit 236 may reproduce an image of a route different from the set guidance route on the screen of the communication terminal 100M, and may reproduce vocal sound related to the route. The vocal sound may include, for example, "The road is closed ahead. Would you like me to show another route?".

In a case in which the user being led along the set guidance route deviates from the guidance route, the action determination unit 236 of the agent system 500 may execute reproduction of at least one of vocal sound or an image for guiding the user along a path for returning the user to the guidance route. Specifically, as illustrated in Fig. 27, the action determination unit 236 may reproduce an image of the route returning the user to the set guidance route on the screen of the communication terminal 100M, and may reproduce vocal sound related to the route. The vocal sound may include "You have deviated from the route. I will show you the path for return to the original route." and the like. The action determination unit 236 of the agent system 500 may execute reproduction of at least one of the vocal sound or the image described above, for example, "I will guide you to the route along which you have previously passed", "Please rehydrate frequently", or "Please return to the sidewalk".

### (Second Action Content)

The action determination unit 236 of the agent system 500 may determine whether or not the action of the user has been corrected by detecting the action of the user while the movement unit 601 was leading the user or after the movement unit 601 reproduced at least one of vocal sound or an image that assists in walking of the user, and may determine second action content different from the first action content in a case in which the action of the user has been corrected.

The case in which the action of the user has been corrected may be interpreted as a case in which the user has stopped a specific action and a specific behavior or a case in which a specific situation has been resolved as a result of execution of the operation of the movement system 1000M according to the first action content. Specifically, the case in which the action of the user has been corrected may include a case in which the user has started walking along the proposed different route, a case in which the user has started walking along a path for return to the original route when the path was shown due to deviation from the original route, and the like.

The second action content may include reproduction of at least one of vocal sound praising the user for action, vocal sound expressing gratitude for the action of the user, an image praising the user for the action, or an image expressing gratitude for the action of the user.

Specifically, the vocal sound praising the user for the action may include, for example, "Well done! Excellent" as illustrated in Fig. 28. The vocal sound expressing gratitude for the action of the user may include vocal sound such as "Thank you for coming back" or "Thank you for selecting another route" as illustrated in Fig. 29. The image praising the user for the action may include an image of a character taking a good pose. The image expressing gratitude for the action of the user may include an image of a character who expresses gratitude.

### (Third Action Content)

The action determination unit 236 may determine whether or not the action of the user has been corrected by detecting the action of the user while the movement unit 601 was leading the user or after the movement unit 601 reproduced at least one of vocal sound or an image that assists in the walking of the user, and determine third action content different from the first action content in a case in which the action of the user has not been corrected.

The case in which the action of the user has not been corrected may be interpreted as a case in which the user has continued the dangerous action and behavior even though the operation of the agent according to the first action content was executed, a case in which the dangerous situation has not been resolved, or a case in which the user has started or continued an action different from the proposal of the agent.

Specifically, the case in which the action of the user has not been corrected may include a case in which the user has walked along a route different from the proposed different route without walking along the proposed different route, a case in which the user has neglected a path for return to the original route and has continued walking when the path was shown due to deviation from the original route, and the like.

The third action content may include at least one of transmission of specific information to a person other than the user, reproduction of sound that attracts an interest of the user, or reproduction of an image that attracts an interest of the user.

The transmission of the specific information to a person other than the user may include distribution of an email containing a warning message to a relative of the user, a spouse of the user, a guardian of the user, or the like. The transmission of the specific information to a person other than the user may include distribution of an image (a still image or a moving image) including the user and the surrounding scenery to a relative of the user, a spouse of the user, a guardian of the user, or the like. The transmission of the specific information to a person other than the user may include distribution of vocal sound having a warning message.

The reproduction of the sound that attracts the interest of the user may include specific music that the user likes, and may also include vocal sound such as "Please come back here" or "Let's take a safe road".

The reproduction of the image that attracts the interest of the user may include reproduction of an image of an animal kept by the user, an image of parents of the user, an image of a character of the animal, and the like.

As illustrated in Fig. 77, the user may wear smart glasses 610 as a wearable terminal in a state in which the communication terminal 100M is worn on the neck strap 6000, and the communication terminal 100M and the smart glasses 610 may be linked. The smart glasses 610 may be interpreted as an electronic apparatus different from the communication terminal 100M. A configuration of the smart glasses 610 may be the same as the configuration of the smart glasses 720. The smart glasses 610 and the communication terminal 100M are connected via a wireless communication line such as Bluetooth (registered trademark). Through this connection, the communication terminal 100M and the smart glasses 610 are linked to each other. Specifically, the communication terminal 100M may receive information collected by the smart glasses 610, and may reproduce at least one of vocal sound or an image for supporting the user by the linked smart glasses 610 according to a situation to notify the user. That is, in the first action content, the second action content, and the third action content, at least one of the image or the vocal sound reproduced by the communication terminal 100M may be reproduced by the smart glasses 610 linked to the communication terminal 100M. By linking the communication terminal 100M and the smart glasses 610 as described above, for example, it is possible to provide better support to the user by using image information at a high position acquired from the smart glasses 610. It is easy to ascertain that the user is supported by reproducing at least one of the image and the vocal sound for supporting the user instead of the communication terminal 100M with the smart glasses 610 closer to the user's sensory organ (for example, vision and hearing).

### (Fourth Action Content)

In a case in which the user wears the communication terminal 100M and the smart glasses 610 linked to the communication terminal 100M, the action determination unit 236 of the agent system 500 may determine the fourth action content of supporting an operation of the user.

In this case, the device operations determined as the action of the communication terminal 100M by the action determination unit 236 of the agent system 500 include setting action content of supporting the user by the communication terminal 100M reproducing at least one of vocal sound or an image generated by the action determination model on the basis of the information collected by the communication terminal 100M and the information collected by the smart glasses 610. The communication terminal 100M reproducing at least one of the vocal sound or the image includes the communication terminal 100M causing the linked communication terminal 100M to reproduce at least one of the vocal sound or the image.

The action determination unit 236 of the agent system 500 determines action content of supporting the user on the basis of the information acquired by the camera of the communication terminal 100M and the information acquired by the camera (2D camera 203) of the smart glasses 610. The information acquired by each camera is image information. The image information may be a still image or a moving image.

For example, in a case in which a moving object (for example, a vehicle) approaching the user is found in the image information acquired by the camera of the communication terminal 100M and the camera of the smart glasses 610 located at a position higher than the communication terminal 100M, the action determination unit 236 of the agent system 500 determines to output vocal sound "A vehicle is approaching." as the action of the communication terminal 100M as illustrated in Fig. 77. The action determination unit 236 of the agent system 500 determines, as the action of the communication terminal 100M, to output vocal sound "A vehicle is approaching. Please be careful." according to a distance between the user and the vehicle. The action determination unit 236 determines, as the action of the communication terminal 100M, to output vocal sound "The vehicle is approaching at o km/h. Please move to the left." according to a speed of the vehicle estimated from the image information.

In a case in which the orientation of the camera of the communication terminal 100M is different from the orientation of the camera of the smart glasses 610, the action determination unit 236 of the agent system 500 determines action content of warning the user about looking away. For example, in a case in which the orientation of the camera of the communication terminal 100M and the orientation of the camera of the smart glasses 610 are different while the user is moving, the action determination unit 236 determines that there is a possibility that the user is looking away, and determines to output vocal sound "You are looking away." as the action of the communication terminal 100M as illustrated in Fig. 78. In a case in which the looking-away state continues for a predetermined time, the action determination unit 236 determines to output vocal sound "It is dangerous to look away." as the action of the communication terminal 100M. For example, in a case in which a moving object (for example, a vehicle) approaching the user is found in the image information acquired by the camera of the communication terminal 100M, the action determination unit 236 determines to output vocal sound "The vehicle is approaching. Please look in front." as the action of the communication terminal 100M.

According to the agent system 500 of the disclosure, an action of leading the user can be executed through autonomous processing. As a result, the agent can guide the user walking in a town that the user visits for the first time to an appropriate route. It is possible to guide a user who is interested in walking to a route along which the user has not passed before. A route along which the user cannot pass is detected in advance, and the user can be guided to another route. A user who gets lost and deviates from a specific route can be guided to an original route of the user.

The action determination unit 236 determines, as the action of the agent, any of a plurality of types of agent actions including no action, by using at least one of the state of the user 10, the emotion of the user 10, the emotion of the agent, or the state of the agent, and the action determination model 221 at a predetermined timing. Here, a case in which a sentence generation model having an interaction function is used as the action determination model 221 will be described as an example.

Specifically, the action determination unit 236 inputs text representing at least one of the state of the user 10, the emotion of the user 10, the emotion of the agent, or the state of the agent, and text for inquiry about agent actions to the sentence generation model, and determines the actions of the agent on the basis of an output of the sentence generation model.

For example, the plurality of types of agent actions include the following (1) to (24).
(1) The agent does nothing.
(2) The agent has a dream.
(3) The agent talks to the user.
(4) The agent creates a picture diary.
(5) The agent proposes an activity.
(6) The agent proposes a person with whom the user should meet.
(7) The agent introduces news in which the user is interested.
(8) The agent edits pictures and moving images.
(9) The agent studies with the user.
(10) The agent evokes memories.
(11) The agent may reproduce an image of a route along which the user can walk on the screen of the communication terminal as the first action content of leading the user.
(12) The agent may reproduce vocal sound related to a route along which the user can walk as the first action content of correcting the action of the user.
(13) The agent may reproduce an image of a route different from the set guidance route on the screen of the communication terminal 100M as the first action content of correcting the action of the user.
(14) The agent may reproduce vocal sound related to a route different from the set guidance route as the first action content of correcting the action of the user.
(15) The agent may reproduce, as the first action content of correcting the action of the user, an image of a route for return to the set guidance route on the screen of the communication terminal 100M in a case in which the user led along the set guidance route deviates from the guidance route.
(16) The agent may reproduce, as the first action content of correcting the action of the user, vocal sound related to a route for return to the set guidance route in a case in which the user led along the set guidance route deviates from the guidance route.
(17) The agent may reproduce vocal sound praising the user for the action as the second action content different from the first action content.
(18) The agent may reproduce vocal sound expressing gratitude for the action of the user as the second action content different from the first action content.
(19) The agent may reproduce an image praising the user for the action as the second action content different from the first action content.
(20) The agent may reproduce an image expressing gratitude for the action of the user as the second action content different from the first action content.
(21) The agent may transmit specific information to a person other than the user as the third action content different from the first action content.
(22) The agent may reproduce sound that attracts the interest of the user as the third action content different from the first action content.
(23) The agent may reproduce an image that attracts the interest of the user as the third action content different from the first action content.
(24) The robot 100 and the communication terminal 100M may reproduce vocal sound that gives a warning to the user as the fourth action content.

The actions of (1) to (24) may be reproduced in the smart glasses 610 linked to the communication terminal 100M.

The action determination unit 236 may spontaneously or periodically detect the action of the user on the basis of the sensor, and in a case in which it is determined to lead the user as the action of the agent on the basis of the detected action of the user and the specific information stored in advance, execute the following first action content.

The action determination unit 236 of the agent system 500 may perform, as the agent action, the first action content in the above "(11)", that is, reproduce an image of a route along which the user can walk on the screen of the communication terminal 100M.

The action determination unit 236 of the agent system 500 may perform, as the agent action, the first action content in the above "(12)", that is, reproduce vocal sound related to a route along which the user can walk.

The action determination unit 236 of the agent system 500 may perform, as the agent action, the first action content in the above "(13)", that is, reproduce an image of a route different from the set guidance route as the first action content of correcting the action of the user on the screen of the communication terminal 100M.

The action determination unit 236 of the agent system 500 may perform, as the agent action, the first action content in the above "(14)", that is, reproduce vocal sound related to a route different from the set guidance route.

The action determination unit 236 of the agent system 500 may perform, as the agent action, the first action content in the above "(15)", that is, reproduce an image of a route for return to the set guidance route on the screen of the communication terminal 100M in a case in which the user led along the set guidance route deviates from the guidance route.

The action determination unit 236 of the agent system 500 may perform, as the agent action, the first action content in the above "(16)", that is, reproduce vocal sound related to a route for return to the set guidance route in a case in which the user led along the set guidance route deviates from the guidance route.

The action determination unit 236 of the agent system 500 may execute the second action content different from the first action content. Specifically, the action determination unit 236 may perform, as the agent action, the second action content in the above "(17)", that is, reproduce vocal sound praising the user for the action.

The action determination unit 236 of the agent system 500 may perform, as the agent action, the second action content in the above "(18)", that is, reproduce vocal sound expressing gratitude for the action of the user.

The action determination unit 236 of the agent system 500 may perform, as the agent action, the second action content in the above "(19)", that is, reproduce an image praising the user for the action.

The action determination unit 236 of the agent system 500 may perform, as the agent action, the second action content in the above "(20)", that is, reproduce an image expressing gratitude for the action of the user.

The action determination unit 236 of the agent system 500 may execute the third action content different from the first action content. Specifically, the action determination unit 236 may perform, as the agent action, the third action content in the above "(21)", that is, transmit the specific information to a person other than the user.

The action determination unit 236 of the agent system 500 may perform, as the agent action, the third action content in the above "(22)", that is, execute reproduction of sound that attracts the interest of the user.

The action determination unit 236 of the agent system 500 may perform, as the agent action, the third action content in the above "(23)", that is, execute reproduction of an image that attracts the interest of the user.

The action determination unit 236 of the agent system 500 may perform, as the communication terminal action, the fourth action content in the above "(24)", that is, execute reproduction of vocal sound for giving a warning to the user.

In a case in which, for example, an image of a route along which the user can walk is reproduced as the first action content indicated in the above "(11)" to "(16)", the related information collection unit 270 of the agent system 500 may store data such as an image of a route along which the user can walk in the collected data 223.

In a case in which, for example, vocal sound praising the user for the action is reproduced as the second action content indicated in the above "(17)" to "(20)", the related information collection unit 270 of the agent system 500 may store data such as an image of a route along which the user can walk in the collected data 223.

In a case in which, for example, specific information is transmitted to a person other than the user as the third action content indicated in the above "(21)" to "(23)", the related information collection unit 270 of the agent system 500 may store data of the specific information or the like in the collected data 223.

In a case in which a warning is given to the user as the fourth action content indicated in the above "(24)", the related information collection unit 270 may store data regarding a situation in which the warning is given, and the like in the collected data 223. The situation in which the warning is given includes, for example, a looking-away time and a distance to a vehicle.

### (Supplementary Note 1)

A control system including:
a state recognition unit that recognizes a user state including an action of a user and a state of an electronic apparatus;
an emotion determination unit that determines an emotion of the user or an emotion of the electronic apparatus; and
an action determination unit that determines, by using at least one of the user state, the state of the electronic apparatus, the emotion of the user, or the emotion of the electronic apparatus, and an action determination model at a predetermined timing, any of a plurality of types of device operations including non-operation as an action of the electronic apparatus, in which
the electronic apparatus detachably provided on a neck strap includes one or a plurality of sensors that collect information,
the electronic apparatus is worn by the user and linked to another electronic apparatus including one or a plurality of sensors that collect information, and
the device operations include setting action content of supporting the user by the electronic apparatus reproducing at least one of vocal sound or an image generated by the action determination model on the basis of the information collected by the electronic apparatus and the information collected by the another electronic apparatus.

### (Supplementary Note 2)

The control system according to Supplementary Note 1, in which the action determination unit determines the action content of supporting the user on the basis of information acquired by a camera included in the sensors of the electronic apparatus and information acquired by a camera included in the sensors of the another electronic apparatus.

### (Supplementary Note 3)

The control system according to Supplementary Note 1 or 2, in which the another electronic apparatus is an eyeglass type terminal.

### (Supplementary Note 4)

The control system according to Supplementary Note 3, in which the action determination unit determines action content of warning the user about looking away in a case in which an orientation of a camera included in the sensors of the electronic apparatus is different from an orientation of a camera included in the sensors of the eyeglass type terminal.

The disclosure of Japanese Patent Application No. 2023-116312 filed on July 14, 2023, the disclosure of Japanese Patent Application No. 2023-116313 filed on July 14, 2023, the disclosure of Japanese Patent Application No. 2023-159212 filed on September 22, 2023, the disclosure of Japanese Patent Application No. 2023-166305 filed on September 27, 2023, the disclosure of Japanese Patent Application No. 2023-166490 filed on September 27, 2023, the disclosure of Japanese Patent Application No. 2023-184976 filed on October 27, 2023, the disclosure of Japanese Patent Application No. 2023-168886 filed on September 28, 2023, the disclosure of Japanese Patent Application No. 2023-203919 filed on December 1, 2023, the disclosure of Japanese Patent Application No. 2023-197352 filed on November 21, 2023, the disclosure of Japanese Patent Application No. 2023-132367 filed on August 15, 2023, the disclosure of Japanese Patent Application No. 2023-134572 filed on August 22, 2023, the disclosure of Japanese Patent Application No. 2023-133994 filed on August 21, 2023, the disclosure of Japanese Patent Application No. 2023-131173 filed on August 10, 2023, the disclosure of Japanese Patent Application No. 2023-131829 filed on August 14, 2023, the disclosure of Japanese Patent Application No. 2023-141433 filed on August 31, 2023, the disclosure of Japanese Patent Application No. 2023-140990 filed on August 31, 2023, and the disclosure of Japanese Patent Application No. 2023-147187 filed on September 11, 2023 are incorporated herein by reference in their entirety.

### [Description of Reference Numerals]

- 5: System
- 10, 11, 12: User
- 20: Communication network
- 100, 101, 102: Robot
- 100N: Stuffed toy
- 200: Sensor unit
- 201: Microphone
- 202: Depth sensor
- 203: Camera
- 204: Distance sensor
- 210: Sensor module unit
- 211: Vocal sound emotion recognition unit
- 212: Speech understanding unit
- 213: Expression recognition unit
- 214: Face recognition unit
- 220: Storage unit
- 221: Action determination model
- 222: History data
- 230: State recognition unit
- 232: Emotion determination unit
- 234: Action recognition unit
- 236: Action determination unit
- 238: Storage control unit
- 250: Action control unit
- 252: Control target
- 270: Related information collection unit
- 280: Communication processing unit
- 300: Server
- 500, 700: Agent system
- 600: Communication terminal
- 601: Movement unit
- 601a: Wheel
- 602: Mounting member
- 602a: First member
- 602b: Second member
- 602c: Third member
- 602d: Fourth member
- 602d1: Opening
- 1200: Computer
- 1210: Host controller
- 1212: CPU
- 1214: RAM
- 1216: Graphic controller
- 1218: Display device
- 1220: Input/output controller
- 1222: Communication interface
- 1224: Storage device
- 1226: DVD drive
- 1227: DVD-ROM
- 1230: ROM
- 1240: Input/output chip

## Claims

1. A control system comprising:
a state recognition unit that recognizes a user state including an action of a user and a state of an electronic apparatus;
an emotion determination unit that determines an emotion of the user or an emotion of the electronic apparatus; and
an action determination unit that determines, by using at least one of the user state, the state of the electronic apparatus, the emotion of the user, or the emotion of the electronic apparatus, and an action determination model at a predetermined timing, any of a plurality of types of device operations including non-operation as an action of the electronic apparatus, wherein
the electronic apparatus is detachably provided on a movement unit having a wheel for autonomous traveling,
the electronic apparatus includes one or a plurality of sensors that collect information for controlling rotation of the wheel, and
the device operations include setting first action content of leading the user by controlling the wheel.

2. The control system according to claim 1, wherein the action determination unit spontaneously or periodically detects an action of the user on the basis of the sensors, and in a case in which it is determined to lead the user as the action of the electronic apparatus on the basis of the detected action of the user and specific information stored in advance, the action determination unit determines the first action content.

3. The control system according to claim 2, wherein the first action content includes reproduction of at least one of vocal sound or an image for assisting in walking of the user.

4. The control system according to claim 3, wherein the reproduction includes reproduction of at least one of vocal sound or an image for guiding the user along a route different from a set guidance route.

5. The control system according to claim 4, wherein the reproduction includes reproduction of at least one of vocal sound or an image for guiding the user along a path for returning the user to the set guidance route in a case in which the user led along the guidance route deviates from the guidance route.

6. The control system according to claim 3, wherein the action determination unit determines whether or not the action of the user has been corrected by detecting the action of the user while the movement unit was leading the user or after the movement unit reproduced at least one of the vocal sound or the image, and determines second action content different from the first action content in a case in which the action of the user has been corrected.

7. The control system according to claim 6, wherein the second action content includes reproduction of at least one of vocal sound praising the user for the action, vocal sound expressing gratitude for the action of the user, an image praising the user for the action, or an image expressing gratitude for the action of the user.

8. The control system according to claim 3, wherein the action determination unit determines whether or not the action of the user has been corrected by detecting the action of the user while the movement unit was leading the user or after the movement unit reproduced at least one of the vocal sound or the image, and determines third action content different from the first action content in a case in which the action of the user has not been corrected.

9. The control system according to claim 8, wherein the third action content includes at least one of transmission of specific information to a person other than the user, reproduction of sound that attracts an interest of the user, or reproduction of an image that attracts the interest of the user.

10. The control system according to claim 1, wherein
the electronic apparatus is a communication terminal, and
the action determination unit determines any of a plurality of types of actions of an agent for interacting with the user, including no action, as an action of the communication terminal.

11. The control system according to claim 10, wherein
the action determination model is a sentence generation model having an interaction function, and
the action determination unit inputs text representing at least one of the user state, a state of the agent, the emotion of the user, or an emotion of the agent and text for inquiry about an action of the agent to the sentence generation model, and determines the action of the communication terminal on the basis of an output of the sentence generation model.

12. A control system comprising:
a state recognition unit that recognizes a user state including an action of a user and a state of an electronic apparatus;
an emotion determination unit that determines an emotion of the user or an emotion of the electronic apparatus; and
an action determination unit that determines, by using at least one of the user state, the state of the electronic apparatus, the emotion of the user, or the emotion of the electronic apparatus, and an action determination model at a predetermined timing, any of a plurality of types of device operations including non-operation as an action of the electronic apparatus, wherein
the electronic apparatus detachably provided on a neck strap includes one or a plurality of sensors that collect information, and
the device operations include setting first action content of leading the user by the electronic apparatus reproducing at least one of vocal sound or an image.

13. A control system comprising:
a state recognition unit that recognizes a user state including an action of a user and a state of an electronic apparatus;
an emotion determination unit that determines an emotion of the user or an emotion of the electronic apparatus; and
an action determination unit that determines, by using at least one of the user state, the state of the electronic apparatus, the emotion of the user, or the emotion of the electronic apparatus, and an action determination model at a predetermined timing, any of a plurality of types of device operations including non-operation as an action of the electronic apparatus, wherein
the electronic apparatus detachably provided on a neck strap includes one or a plurality of sensors that collect information, and
the device operations include setting first action content of leading the user by the electronic apparatus reproducing at least one of vocal sound or an image.

14. A control system comprising:
a state recognition unit that recognizes a user state including an action of a user and a state of an electronic apparatus;
an emotion determination unit that determines an emotion of the user, an emotion of another user existing around the user, or an emotion of the electronic apparatus;
an action determination unit that, in a case in which an emotion value indicating an intensity of the emotion of the another user changes by a predetermined value or more, determines to start capturing a moving image of the another user with a camera as an action of the electronic apparatus; and
an action control unit that controls the electronic apparatus to execute the action of the electronic apparatus determined by the action determination unit.

15. A control system comprising:
a state recognition unit that recognizes a user state including an action of a user and a state of an electronic apparatus;
an emotion determination unit that determines an emotion of the user or an emotion of the electronic apparatus; and
an action determination unit that determines, by using at least one of the user state, the state of the electronic apparatus, the emotion of the user, or the emotion of the electronic apparatus, and an action determination model at a predetermined timing, any of a plurality of types of device operations including non-operation as an action of the electronic apparatus, wherein
surrounding environment information is acquired from one or a plurality of sensors that collect information, the sensors being included in the electronic apparatus detachably provided on a body of the user, and
the action determination unit checks a health state of the user by the electronic apparatus outputting at least one of vocal sound or text according to the acquired environment information, and
the electronic apparatus makes an appropriate proposal to the user.

16. A control system comprising:
a state recognition unit that recognizes a user state including an action of a user and another user;
an emotion determination unit that determines an emotion of the user and another user; and
an action determination unit that determines any of a plurality of types of device operations as an operation of an electronic apparatus by using at least one of the user state or the emotion of the user and an action determination model at a predetermined timing, wherein
the state recognition unit recognizes a surrounding state from one or a plurality of sensors of the electronic apparatus detachably provided on a body of the user, and
the action determination unit determines that the electronic apparatus makes speech toward the another user in a case in which the another user exists near the user.

17. A control system comprising:
a state recognition unit that recognizes a user state including an action of a user and a state of an electronic apparatus;
an emotion determination unit that determines an emotion of the user or an emotion of the electronic apparatus; and
an action determination unit that determines, by using at least one of the user state, the state of the electronic apparatus, the emotion of the user, or the emotion of the electronic apparatus, and an action determination model at a predetermined timing, any of a plurality of types of device operations including non-operation as an action of the electronic apparatus, wherein
the emotion determination unit estimates an emotion of another person around the user from a video of the another person acquired from one or a plurality of sensors that collect information, the sensors being included in the electronic apparatus detachably provided on a body of the user, and
the action determination unit makes an appropriate proposal to the user in a case in which the emotion of the another person is in trouble.

18. A control system comprising:
a state recognition unit that recognizes a user state including an action of a user and a state of an electronic apparatus;
an emotion determination unit that determines an emotion of the user or an emotion of the electronic apparatus; and
an action determination unit that determines, by using at least one of the user state, the state of the electronic apparatus, the emotion of the user, or the emotion of the electronic apparatus, and an action determination model at a predetermined timing, any of a plurality of types of device operations including non-operation as an action of the electronic apparatus, wherein
the electronic apparatus detachably provided on a neck strap includes one or a plurality of sensors that collect information, the sensors including an imaging unit that captures an image of surroundings, and
the device operations include issuing a warning to a person satisfying a predetermined condition among persons approaching the imaging unit.

19. A control system comprising:
a state recognition unit that recognizes a user state including an action of a user and a state of an electronic apparatus;
an emotion determination unit that determines an emotion of the user or an emotion of the electronic apparatus; and
an action determination unit that determines, by using at least one of the user state, the state of the electronic apparatus, the emotion of the user, or the emotion of the electronic apparatus, and an action determination model at a predetermined timing, any of a plurality of types of device operations including non-operation as an action of the electronic apparatus, wherein
the electronic apparatus detachably provided on a neck strap includes one or a plurality of sensors that collect information, and
the device operations include setting action content of supporting work performed by the user at a position lower than eyes of the user by the electronic apparatus reproducing at least one of vocal sound or an image generated by the action determination model on the basis of the information collected by the electronic apparatus.

20. A control system comprising:
a state recognition unit that recognizes a user state including an action of a user and a state of an electronic apparatus;
an emotion determination unit that determines an emotion of the user or an emotion of the electronic apparatus; and
an action determination unit that determines, by using at least one of the user state, the state of the electronic apparatus, the emotion of the user, or the emotion of the electronic apparatus, and an action determination model at a predetermined timing, any of a plurality of types of device operations including non-operation as an action of the electronic apparatus, wherein
the electronic apparatus is detachably provided on a movement unit having a wheel for autonomous traveling,
the electronic apparatus includes one or a plurality of sensors that collect information regarding a surrounding situation of the user,
the electronic apparatus controls rotation of the wheel on the basis of the information regarding the surrounding situation of the user, and
the action determination unit determines, in a case in which it is detected that there is a dangerous obstacle around the user, first action content of leading the user to a safe place so that the user does not approach the dangerous obstacle as the action of the electronic apparatus.

21. A control system comprising:
a state recognition unit that recognizes a user state including an action of a user and a state of an electronic apparatus;
an emotion determination unit that determines an emotion of the user or an emotion of the electronic apparatus; and
an action determination unit that determines, by using at least one of the user state, the state of the electronic apparatus, the emotion of the user, or the emotion of the electronic apparatus, and an action determination model at a predetermined timing, any of a plurality of types of device operations including non-operation as an action of the electronic apparatus, wherein
the electronic apparatus is provided in a movement unit capable of autonomous traveling,
the electronic apparatus includes one or a plurality of sensors that collect information regarding a surrounding situation of the user, and
the action determination unit determines, in a case in which it is detected that the user is in imminent danger on the basis of the information regarding the surrounding situation of the user, to take an action of being a substitute for the user as the action of the electronic apparatus.

22. A control system comprising:
a state recognition unit that recognizes a user state including an action of a user and a state of an electronic apparatus;
an emotion determination unit that determines an emotion of the user or an emotion of the electronic apparatus; and
an action determination unit that determines, by using at least one of the user state, the state of the electronic apparatus, the emotion of the user, or the emotion of the electronic apparatus, and an action determination model at a predetermined timing, any of a plurality of types of device operations including non-operation as an action of the electronic apparatus, wherein
the electronic apparatus is detachably provided on a movement unit having a wheel for autonomous traveling,
the electronic apparatus includes one or a plurality of sensors that collect information regarding a surrounding situation of the user, and
the device operations include setting first action content of assisting the user in walking an animal by controlling the wheel on the basis of the information regarding the surrounding situation of the user.

23. A control system comprising:
a state recognition unit that recognizes a user state including an action of a user and a state of an electronic apparatus;
an emotion determination unit that determines an emotion of the user or an emotion of the electronic apparatus; and
an action determination unit that determines, by using at least one of the user state, the state of the electronic apparatus, the emotion of the user, or the emotion of the electronic apparatus, and an action determination model at a predetermined timing, any of a plurality of types of device operations including non-operation as an action of the electronic apparatus, wherein
the electronic apparatus detachably provided on a neck strap includes one or a plurality of sensors that collect information regarding a surrounding situation, and
the device operations include setting action content of assisting in walking of the user by notifying the user of the surrounding situation by the electronic apparatus reproducing at least one of vocal sound or an image.

24. A control system comprising:
a state recognition unit that recognizes a user state including an action of a user and a state of an electronic apparatus;
an emotion determination unit that determines an emotion of the user or an emotion of the electronic apparatus; and
an action determination unit that determines, by using at least one of the user state, the state of the electronic apparatus, the emotion of the user, or the emotion of the electronic apparatus, and an action determination model at a predetermined timing, any of a plurality of types of device operations including non-operation as an action of the electronic apparatus, wherein
the electronic apparatus detachably provided on a neck strap includes one or a plurality of sensors that collect biological information of the user, and
the device operations include notifying the user of attention calling information based on the collected biological information by the electronic apparatus reproducing at least one of vocal sound or an image.

25. A control system comprising:
a state recognition unit that recognizes a user state including an action of a user and a state of an electronic apparatus;
an emotion determination unit that determines an emotion of the user or an emotion of the electronic apparatus; and
an action determination unit that determines, by using at least one of the user state, the state of the electronic apparatus, the emotion of the user, or the emotion of the electronic apparatus, and an action determination model at a predetermined timing, any of a plurality of types of device operations including non-operation as an action of the electronic apparatus, wherein
the electronic apparatus detachably provided on a neck strap includes one or a plurality of sensors that collect surrounding environment information, and
the device operations include notifying the user of attention calling information based on the collected surrounding environment information by the electronic apparatus reproducing at least one of vocal sound or an image.

26. A control system comprising:
a state recognition unit that recognizes a user state including an action of a user and a state of an electronic apparatus;
an emotion determination unit that determines an emotion of the user or an emotion of the electronic apparatus; and
an action determination unit that determines, by using at least one of the user state, the state of the electronic apparatus, the emotion of the user, or the emotion of the electronic apparatus, and an action determination model at a predetermined timing, any of a plurality of types of device operations including non-operation as an action of the electronic apparatus, wherein
the electronic apparatus detachably provided on a neck strap includes one or a plurality of sensors that collect information, and
the device operations include setting action content of supporting work performed by the user at a position lower than eyes of the user by the electronic apparatus reproducing at least one of vocal sound or an image generated by the action determination model on the basis of the information collected by the electronic apparatus.

27. A control system comprising:
a state recognition unit that recognizes a user state including an action of a user and a state of an electronic apparatus;
an emotion determination unit that determines an emotion of the user or an emotion of the electronic apparatus; and
an action determination unit that determines, by using at least one of the user state, the state of the electronic apparatus, the emotion of the user, or the emotion of the electronic apparatus, and an action determination model at a predetermined timing, any of a plurality of types of device operations including non-operation as an action of the electronic apparatus, wherein
the electronic apparatus detachably provided on a neck strap includes one or a plurality of sensors that collect information,
the electronic apparatus is linked to another electronic apparatus that is worn by the user and includes one or a plurality of sensors that collect information, and
the device operations include setting action content of supporting the user by the electronic apparatus reproducing at least one of vocal sound or an image generated by the action determination model on the basis of the information collected by the electronic apparatus and the information collected by the another electronic apparatus.
